# EUROPEAN PATENT SPECIFICATION

(11) **EP 3 946 437 B1**
(45) Date of publication and mention of the grant of the patent: **19.11.2025**
(21) Application number: 20720677.2
(22) Date of filing: 27.03.2020
(51) Int. Cl.: A61K 39/00, A61P 35/00, A61P 37/04, C12N 5/0784, C12N 5/0783

(54) **EX VIVO METHODS FOR PRODUCING A T CELL THERAPEUTIC AND RELATED COMPOSITIONS AND METHODS**
EX-VIVO-VERFAHREN ZUR HERSTELLUNG EINES T-ZELL-THERAPEUTIKUMS UND ZUGEHÖRIGE ZUSAMMENSETZUNGEN UND VERFAHREN
PROCÉDÉS EX VIVO DE PRODUCTION D'UN AGENT THÉRAPEUTIQUE DE LYMPHOCYTES T ET COMPOSITIONS ET PROCÉDÉS ASSOCIÉS

(30) Priority: 29.03.2019 US 201962826974 P; 27.11.2019 US 201962941610 P; 18.02.2020 US 202062978298 P
(43) Date of publication of application: 09.02.2022
(73) Proprietor: H. Lee Moffitt Cancer Center and Research Institute, Inc., Tampa, FL 33612-9497 (US)
(72) Inventor: LANGER, Timothy J., New York, New York 10010 (US); CECCARELLI, Jacob, New York, New York 10010 (US)
(74) Representative: J A Kemp LLP
(86) International application number: PCT/US2020/025541
(87) International publication number: WO 2020/205662

(56) References cited:
- WO-A1-2016/053339
- WO-A1-2018/183485
- US-A1- 2017 224 800
- RAMI YOSSEF ET AL: "Enhanced detection of neoantigen-reactive T cells targeting unique and shared oncogenes for personalized cancer immunotherapy", JCI INSIGHT, vol. 3, no. 19, 4 October 2018 (2018-10-04), XP055542887, DOI: 10.1172/jci.insight.122467
- Y. LI ET AL: "MART-1-Specific Melanoma Tumor-Infiltrating Lymphocytes Maintaining CD28 Expression Have Improved Survival and Expansion Capability Following Antigenic Restimulation In Vitro", THE JOURNAL OF IMMUNOLOGY, vol. 184, no. 1, 30 November 2009 (2009-11-30), pages 452 - 465, XP055210596, ISSN: 0022-1767, DOI: 10.4049/jimmunol.0901101

## Description

### Field

The present disclosure provides methods for ex vivo expansion of a T cells, including tumor-reactive T cells, and compositions containing such T cells for use in methods for treating cancer.

### Background

Cancer cells accumulate lots of different DNA mutations as part of the tumorigenic process. These mutations can cause amino acid changes in protein coding regions. For a mutation to be recognized by the immune system the protein needs to be processed intracellularly and present the mutant peptide presented on the surface with the Major Histocompatibility Complex (MHC). Neoantigens are the mutant peptides presented by the MHC complex that can be recognized by a T-cell via TCR binding. Neoantigens are ideal targets for immunotherapies. These antigens were not present in the body before the cancer developed and are truly cancer specific, not expressed on normal cells and are not subjected to off target immune toxicity. Clinical studies have demonstrated that T cells isolated from surgically resected tumor possess TCRs that recognize neoantigens, and expanding these neoantigen reactive TIL populations and re-infusing them into the patient can in some cases result in a dramatic clinical benefit. However, a major obstacle to applications of such cells in cell therapy is the difficulty in obtaining such cells. Improved methods are needed for obtaining and manufacturing cell compositions containing tumor-reactive T cells for therapeutic use. Provided herein are embodiments that meet such needs. Yossef et al. JCI Insight, 3 (2018) describes a method of enhanced detection of neoantigen-reactive T cells targeting unique and shared oncogenes for personalized cancer immunotherapy.

### Summary of the invention

The present invention, as defined in the claims, provides a method for manufacturing tumor-reactive T cells, the method comprising:
(1) culturing T cells by a process that comprises:
   (a) incubating a population of cells comprising T cells from a biological sample obtained from a subject that has a tumor with a first T cell stimulatory agent(s) under conditions to stimulate expansion of T cells of the population to produce a population of stimulated T cells, wherein the first T cell stimulatory agent(s) comprises the presence of one or more recombinant cytokines;
   (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells;
   (c) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and
   (d) incubating the population of T cells enriched in tumor reactive T cells with a second T cell stimulatory agent(s) under conditions to stimulate expansion of T cells in the population, wherein the second T cell stimulatory agent(s) comprises the presence of one or more recombinant cytokines; and
   wherein one or more steps of the culturing is carried out in the presence of at least one T cell adjuvant that is: (i) an apoptosis inhibitor that inhibits caspase activation or activity, (ii) a costimulatory agonist which is a tumor necrosis factor receptor superfamily (TNFRSF) agonist, or (iii) a checkpoint inhibitor which inhibits the activity of an immune checkpoint selected from the group consisting of PD-1/PD-L1, CTLA-4, OX40, LAG-3, TIM-3 and B7-H3; and
(2) harvesting cells produced by the method to produce a composition of expanded T cells enriched in tumor reactive T cells.

The invention also provides a composition comprising expanded T cells enriched in tumor reactive T cells for use in a method of treating a subject having a cancer, wherein the method comprises manufacturing the composition using the method of any one of the claims and administering the composition to a subject having a tumor a therapeutic dose of the composition.

Further aspects of the invention and preferred embodiments are defined in the dependent claims. Any aspects, embodiments and examples of the present disclosure which do not fall under the scope of the appended claims do not form part of the invention and are merely provided for illustrative purposes.

### Summary of the technical teachings

The technical information set out below may in some respects go beyond the disclosure of the invention, which is defined by the appended claims. The additional technical information is provided to place the actual invention in a broader technical context and to illustrate possible related technical developments. Such additional technical information which does not fall within the scope of the appended claims, is not part of the invention. Accordingly, any aspects, embodiments and examples of the present disclosure which do not fall under the scope of the appended claims do not form part of the invention and are merely provided for illustrative purposes.

Provided herein is a method for manufacturing tumor-reactive T cells, the method comprising culturing T cells by a process that comprises: (a) incubating a population of cells comprising T cells from a biological sample obtained from a subject with a T cell stimulatory agent(s) under conditions to stimulate expansion of T cells of the population to produce a population of stimulated T cells; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (c) enriching from the co-culture the population of tumor reactive T cells reactive to the one or more peptides; and (d) incubating the population of T cells enriched in tumor reactive T cells with a T cell stimulatory agent(s) under conditions to stimulate expansion of T cells in the population, wherein one or more steps of the culturing is carried out in the presence of at least one T cell adjuvant selected from a costimulatory agonist or an apoptosis inhibitor. In some embodiments, the method further comprises (e) harvesting cells produced by the method.

Provided herein is a method for manufacturing tumor-reactive T cells, the method comprising (1) culturing T cells by a process that comprises: (a) incubating a population of cells comprising T cells from a biological sample obtained from a subject that has a tumor with a first T cell stimulatory agent(s) under conditions to stimulate expansion of T cells of the population to produce a population of stimulated T cells; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (d) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and (e) incubating the population of T cells enriched in tumor reactive T cells with a second T cell stimulatory agent(s) under conditions to stimulate expansion of T cells in the population; and wherein one or more steps of the culturing is carried out in the presence of at least one T cell adjuvant selected from a costimulatory agonist or an apoptosis that is an apoptosisinhibitor that inhibits caspase activation or activity; and (2) harvesting cells produced by the method to produce a composition of expanded T cells enriched in tumor reactive T cells.

In some of any of the provided embodiments, the first or second T cell stimulatory agent(s) comprises the presence of one or more recombinant cytokines, wherein the incubating in the presence of the T cell stimulatory agent(s) is carried out prior to, during and/or subsequent to the incubating in the presence of the one or more T cell adjuvants.

In some of any of the provided embodiments, the incubation with the at least one T cell adjuvant is carried out during at least a portion of the co-culturing. In some of any of the embodiments, at least a portion of the incubating the population of T cells with the at least one T cell adjuvant is carried out concurrently with the incubating the population of T cells with the T cell stimulatory agent(s).

Provided herein is a method for manufacturing tumor-reactice T cells, the method comprising: (1) culturing T cells by a process comprising: (a) incubating a population of cells comprising T cells from a biological sample from a subject that has a tumor with a first T cell stimulatory agent(s) comprising one or more recombinant cytokinesunder conditions to stimulate expansion of T cells in the population to produce a population of stimulated T cells, wherein the incubation with the one or more recombinant cytokines is in the presence of at least one T cell adjuvantthat is an apoptosis inhibitor that inhibits caspase activation or activity; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (c) enriching tumor-reactive T cells from the co-culture, said tumor-reactive T cells comprising an endogenous TCR that is reactive to a tumor-associated antigen, thereby producing a population of T cells enriched for tumor-reactive T cells; (d) incubating the population of T cells enriched for tumor-reactive T cells with a second T cell stimulatory agent(s) under conditions to stimulate expansion of cells of cells of the population of cells; and (2) harvesting cells produced by the method.

In some of any of the provided embodiments, the at least one T cell adjuvant further comprises at least one costimulatory agonist. In some of any of the provided embodiments, the incubating with the at least one costimulatory agonist and the apoptosis inhibitor is carried out simultaneously, intermittenttly or sequentially.

In some of any of the provided embodiments, the at least one T cell adjuvant further comprises at least one checkpoint inhibitor. In some of any of the provided embodiments, the incubating with the at least one checkpoint inhibitor and the apoptosis inhibitor is carried out simultaneously, intermittenttly or sequentially.

Provided herein is a method for manufacturing tumor-reactive T cells, the method comprising: (1) culturing T cells by a process that comprises: (a) incubating a population of cells comprising T cells from a biological sample obtained from a subject that has a tumor with a first T cell stimulatory agent(s) under conditions to stimulate expansion of T cells of the population to produce a population of stimulated T cells; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (d) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and (d) incubating the population of T cells enriched in tumor reactive T cells with a second T cell stimulatory agent(s) under conditions to stimulate expansion of T cells in the population; and wherein one or more steps of the culturing is carried out in the presence of at least one T cell adjuvant selected from that is a costimulatory agonist or an apoptosis; and (2) harvesting cells produced by the method to produce a composition of expanded T cells enriched in tumor reactive T cells.

In some of any of the provided embodiments, the first or second T cell stimulatory agent(s) comprises the presence of one or more recombinant cytokines, wherein the incubating in the presence of the T cell stimulatory agent(s) is carried out prior to, during and/or subsequent to the incubating in the presence of the one or more T cell adjuvants.

Provided herein is a method for manufacturing tumor reactive T cells, the method comprising (1) culturing T cells by a process comprising incubating an ex vivo population of T cells comprising tumor-reactive T cells, said tumor-reactive T cells comprising an endogenous TCR that is reactive to a tumor-associated antigen, in the presence of at least one T cell adjuvant selected from a costimulatory agonist or an apoptosis inhibitor, wherein the population of T cells is enriched for CD4+ and CD8+ T cells and wherein at least a portion of the incubation is carried out prior to, concurrently with, or subsequent to incubating T cells with a T cell stimulatory agent(s) under conditions to activate T cells, wherein the culturing stimulates expansion of cells of the population of T cells; and (2) harvesting cells produced by the method.

Provided herein is a method for manufacturing tumor reactive T cells, the method comprising: (1) culturing T cells by a process comprising: (a) incubating a population of cells comprising T cells from a biological sample from a subject that has a tumor with a first T cell stimulatory agent(s) comprising one or more recombinant cytokinesunder conditions to stimulate expansion of T cells in the population to produce a population of stimulated T cells, wherein the incubation with the one or more recombinant cytokines is in the presence of at least one T cell adjuvant that is a costimulatory agonist; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (c) enriching tumor-reactive T cells from the co-culture, said tumor-reactive T cells comprising an endogenous TCR that is reactive to a tumor-associated antigen, thereby producing a population of T cells enriched for tumor-reactive T cells; (d) incubating the population of T cells enriched for tumor-reactive T cells with a second T cell stimulatory agent(s) under conditions to stimulate expansion of cells of cells of the population of cells; and (2) harvesting the expanded cells produced by the method.

In some of any of the provided embodiments, at least one T cell adjuvant further comprises at least one apoptosis inhibitor. In some of any of the provided embodiments, at least one apoptosis inhibitor and the checkpoint agonist is carried out simultaneously, intermittently or sequentially. In some of any of the provided embodiments, at least one T cell adjuvant further comprises at least one checkpoint inhibitor. In some of any of the embodiments, the at least one checkpoint inhibitor and the costimulatory agonist is carried out simultaneously, intermittently or sequentially.

Provided herein is a method for manufacturing tumor reactive T cells, the method comprising: (1) culturing T cells by a process comprising: (a) incubating an ex vivo population of T cells comprising tumor-reactive T cells, said tumor-reactive T cells comprising an endogenous TCR that is reactive to a tumor-associated antigen, with at least one T cell adjuvant selected from a costimulatory agonist or an apoptosis inhibitor, wherein the population of T cells is enriched for CD4+ and CD8+ T cells; and (b) incubating T cells with a T cell stimulatory agent(s) under conditions to activate T cells, wherein at least a portion of the incubating is carried out prior to, concurrently with, or subsequent to the incubating in (a), wherein the culturing stimulates expansion of cells of the population of T cells; and (2) harvesting cells produced by the method.

Provided herein is a method for manufacturing tumor-reactive T cells, the method comprising: (1) culturing T cells by a process that comprises: (a) incubating a population of cells comprising T cells from a biological sample obtained from a subject that has a tumor with a first T cell stimulatory agent(s) under conditions to stimulate expansion of T cells of the population to produce a population of stimulated T cells: (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (d) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and (d) incubating the population of T cells enriched in tumor reactive T cells with a second T cell stimulatory agent(s) under conditions to stimulate expansion of T cells in the population; and wherein one or more steps of the culturing is carried out in the presence of at least one T cell adjuvant-that is a checkpoint inhibitor; and (2) harvesting the expanded cells produced by the method.

In any of the embodiments, the culturing the T cells further comprises incubating the cells in the presence of one or more recombinant cytokines, wherein the incubating in the presence of one or more cytokines is carried out prior to, during and/or subsequent to the incubating with the T cell stimulatory agents(s) and/or incubating in the presence of the one or more T cell adjuvants.

In some of any of the provided embodiments, the first or second T cell stimulatory agent(s) comprises the presence of one or more recombinant cytokines, wherein the incubating in the presence of the T cell stimulatory agent(s) is carried out prior to, during and/or subsequent to the incubating in the presence of the one or more T cell adjuvants. In some of any of the provided embodiments, the incubation with the at least one T cell adjuvant is carried out during at least a portion of the co-culturing. In some of any of the provided embodiments, at least a portion of the incubating the population of T cells with the at least one T cell adjuvant is carried out concurrently with the incubating the population of T cells with the T cell stimulatory agent(s).

In some of any of the embodiments, the T cell stimulating agent(s) comprises the presence of one or more recombinant cytokines, wherein the incubating in the presence of the T cell stimulatory agent(s) is carried out prior to, during and/or subsequent to the incubating in the presence of the one or more T cell adjuvants.

In some of any of the embodiments, the T cell stimulating agent(s) comprises the an agent that initiates TCR/CD3 intracellular signaling and an agent that initiates signaling via a costimulatory receptor, optionally wherein the costimulatory receptor is CD28, wherein the incubating in the presence of the T cell stimulating agent(s) is carried out prior to, during and/or subsequent to the incubating in the presence of the one or more T cell adjuvants.

In some of any of the provided embodiments, the method further comprises washing the cells during one or more of the steps for culturing, such as during incubation with T cell stimulatory agent(s) and/or the one or more T cell adjuvants, e.g. costimulatory agonist, apoptosis inhibitor (e.g. caspase inhibitor), or checkpoint inhibitor. In some embodiments, the provided methods include adding during the one or more steps a transient addition (e.g. only one time, such as at the initiation of the incubation or culture) of the T cell adjuvant, e.g. costimulatory agonist, apoptosis inhibitor (e.g. caspase inhibitor), or checkpoint inhibitor. In some embodiments, the provided methods include continuous addition of the T cell adjuvant during the one or more steps. For example, the one or more T cell adjuvants, e.g. costimulatory agonist, apoptosis inhibitor (e.g. caspase inhibitor), or checkpoint inhibitor is added continuously during the one or more steps of incuating the cells, such as during incubation with a T cell stimulatory agent(s). For example, after washing the cells during or after the one or more steps the culture media is replenished with the T cell adjuvant, such as at the same concentration. In some cases, the one or more T cell stimulatory agent(s) also is replenished with the wash. The washes can be carried out daily, every other day, every third day, every fourth day, every fifth day, every sixth day or once a week during the step, such as during the incubation with the one or more T cell stimulatory agent(s).

In some of any of the embodiments, the method further comprises washing the cells after one or more of the steps of incubating the cells with the T cell stimulatory agent(s), the at least one T cell adjuvant and/or the one or more recombinant cytokines.

In some of any of the embodiments, the culturing is carried out until a threshold amount of cells is obtained and/or is carried out for up to 20 days.

In some of any of the embodiments, the population of T cells comprising tumor-reactive T cells comprise T cells surface positive for one or more T cell activation marker selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3. In some of any of the embodiments, the population of T cells comprising tumor-reactive T cells is enriched for T cells surface positive for one or more T cell activation marker selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3. In some of any of the provided embodiments, the population of T cells comprising tumor-reactive T cells is enriched for the tumor-reactive T cells.

In some of any of the provided embodiments, the T cells comprise primary T cells from a subject, optionally a human subject.

In some of any of the provided embodiments, the population of T cells comprising tumor-reactive T cells are present in an ex vivo co-culture comprising autologous T cells from a biological sample from a subject and antigen-presenting cells (APCs), said co-culturing carried out under conditions in which the APCs have been induced to present one or more peptides from a tumor-associated antigen from the subject. In some of any of the provided embodiments, the population of T cells comprising tumor-reactive T cells is generated by a process comprising the steps of: (a) identifying somatic mutations associated with one or more tumor-associated antigen by exome sequencing of healthy and tumor tissue from a subject; (b) identifying at least one epitope of the one or more tumor-associated antigens, optionally wherein the at least one epitope is a neoepitope; (c) isolating a population of autologous T cells from a biological sample from the subject; and (d) co-culturing the population of T cells with antigen presenting cells (APCs) that have been exposed to or contacted with one or more peptides comprising the at least one epitope of the one or more tumor-associated antigens under conditions to present one or more of the peptides on the surface of a major histocompatibility complex (MHC), thereby generating the population of T cells comprising tumor-reactive T cells reactive to the one or more peptides.

In some embodiments, exposing or contacting APCs with the one or more peptides comprises generating a mutation library of peptides comprising the one or more epitopes, optionally wherein the peptides are 8 to 32 amino acids in length, 8 to 24 amino acids in length, 8 to 18 amino acids in length, 8 to 10 amino acids in length, 10 to 32 amino acids in length, 10 to 24 amino acids in length, 10 to 18 amino acids in length, 18 to 32 amino acids in length, 18 to 24 amino acids in length or 24 to 32 amino acids in length, optionally at or about 9mers; and pulsing the APCs with the mutation library of peptides under conditions to present one or more of the peptides on the surface of a major histocompatibility complex (MHC), optionally wherein the MHC is MHC class I. In some embodiments, exposing or contacting APCs with the one or more peptides comprises: generating DNA, optionally a minigene construct, encoding the one or more tumor-associated antigens or a portion thereof comprising the somatic mutation; in vitro transcribing the DNA into RNA; and introducing the in vitro transcribed RNA into the APCs under conditions to present one or more of the peptides on the surface of a major histocompatibility complex (MHC), optionally wherein the MHC is MHC class II.

In some of any of the embodiments, the co-culturing is carried out for 1 to 7 days. In some embodiments, the incubation with the at least one T cell adjuvant is carried out during at least a portion of the co-culturing. In some embodiments, the method includes further selecting from the co-culture T cells reactive to the one or more peptides, thereby enriching for the population of T cells comprising tumor-reactive T cells. In some embodiments, the enriching is carried out prior to the incubation with the at least one T cell adjuvant. In some embodiments, the incubation with the T cell stimulatory agent(s) is carried out before the co-culturing, wherein the population of autologous T cells from the biological sample from the subject is incubated with the T cell stimulatory agent(s). In some embodiments, the incubation with the T cell stimulatory agent(s) is carried out after the enriching for the population of T cell cells comprising tumor-reactive T cells. In some embodiments, the APCs are autologous to the subject or allogeneic to the subject.

In some of any of the provded embodiments, the co-culture ratio of antigen presenting cells to T Cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1. In some of any of the provided embodiments, the co-culture ratio of antigen presenting cells to T cells is or is about 1:1. In some of any of the provided embodiments, the co-culturing is for 2 hours to 24 hours. In some of any of the provided embodiments, the co-culturing is for at or about 6 hours.

In some of any of the provided embodiments, the subject is healthy or is not known to exhibit a disease or condition.

In some of any of the provided embodiments, the culturing comprises: isolating a population of cells comprising T cells from a biological sample from a subject; incubating the population of cells comprising T cells with a T cell stimulatory agent(s) under conditions to activate T cells of the population to produce a population of T cells comprising activated cells; co-culturing the population of T cells comprising activated cells in the presence of APCs under conditions in which the APCs have been induced to present one or more peptides from a tumor-associated antigen from the subject, thereby generating a population of T cells comprising tumor reactive T cells, wherein the co-culturing is carried out in the presence of the at least one T cell adjuvant thereby incubating the population of T cells comprising tumor-reactive T cells with the at least one adjuvant; selecting from the co-culture T cells reactive to the one or more peptides, thereby enriching for the population of T cells comprising tumor-reactive T cells; and further incubating the cells in the presence of one or more recombinant cytokines, optionally wherein the incubating in the presence of one or more cytokines is carried out prior to, during and/or subsequent to the incubating with the T cell stimulatory agents(s), co-culturing in the presence of APCs and/or incubating in the presence of one or more T cell adjuvants , wherein the culturing stimulates expansion of cells of the population of T cells.

In some of any of the provided embodiments, the culturing comprises:isolating a population of cells comprising T cells from a biological sample from a subject; incubating the population of cells comprising T cells with a T cell stimulatory agent(s) under conditions to activate T cells of the population to produce a population of T cells comprising activated cells; co-culturing the population of T cells comprising activated cells in the presence of APCs under conditions in which the APCs have been induced to present one or more peptides from a tumor-associated antigen from the subject, thereby generating a population of T cells comprising tumor reactive T cells; selecting from the co-culture T cells reactive to the one or more peptides, thereby enriching for the population of T cells comprising tumor-reactive T cells; incubating the enriched population of T cells comprising tumor-reactive cells in the presence of the at least one T cell adjuvant; and further incubating the cells in the presence of one or more recombinant cytokines, optionally wherein the incubating in the presence of one or more cytokines is carried out prior to, during and/or subsequent to the incubating with the T cell stimulatory agents(s), co-culturing in the presence of APCs and/or incubating in the presence of one or more T cell adjuvants, wherein the culturing stimulates expansion of cells of the population of T cells.

In some of any of the provided embodiments, the culturing comprises:isolating a population of cells comprising T cells from a biological sample from a subject; co-culturing the population of T cells in the presence of APCs under conditions in which the APCs have been induced to present one or more peptides from a tumor-associated antigen from the subject, thereby generating a population of T cells comprising tumor reactive T cells; incubating the population comprising tumor-reactive T cells with a T cell stimulatory agent(s) under conditions to activate T cells of the population to produce a population of T cells comprising activated cells; selecting from the co-culture T cells reactive to the one or more peptides, thereby enriching for the population of T cells comprising tumor-reactive T cells, wherein the selecting is carried out prior to or after the incubating with the T cells stimulatory agent(s); incubating the population of T cells enriched for tumor-reactive T cellscells in the presence of the at least one T cell adjuvant; and further incubating the cells in the presence of one or more recombinant cytokines, optionally wherein the incubating in the presence of one or more cytokines is carried out prior to, during and/or subsequent to the incubating with the T cell stimulatory agents(s), co-culturing in the presence of APCs and/or incubating in the presence of one or more T cell adjuvants, wherein the culturing stimulates expansion of cells of the population of T cells.

In some of any of the provided embodiments, the co-culturing is carried out for 1-7 days.

In some of any of the provided embodiments, the population of T cells comprising tumor-reactive T cells are isolated or selected directly from a biological sample from a subject. In some of any of the provided embodiments, the subject exhibits a disease or condition, optionally wherein the disease or condition is a cancer. In some of any of the provided embodiments, the subject has been previously administered a costimulatory agonist prior to the isolating or selecting of the population of T cells.

In some of any of the provided embodiments, the population of T cells comprising tumor-reactive T cells are allogeneic to a subject to be treated or autologous to a subject to be treated, optionally wherein the subject to be treated has a tumor or cancer. In some of any of the provided embodiments, the biological sample is a peripheral blood sample, a lymph node sample, or a tumor sample.

In some of any of the provided embodiments, the biological sample is a peripheral blood sample and the peripheral blood sample is collected by a blood draw or by apheresis, optionally wherein the apheresis is leukapheresis. In some of any of the provided embodiments, the biological sample comprises a volume of between 50 mL and 400 mL. In some of any of the provided embodiments, the enriching for the tumor reactive T cells or T cells surface positive for the one or more activation markers is by flow cytometry, optionally carried out by automated high-throughput flow cytometry In some embodiments, the enriching can be carried out using an FX500 cell sorter. In some of any of the provided embodiments, 1 run, 2 runs, 3 runs or 4 runs by flow cytometry is carried out to enrich the tumor-reactive T cells from the sample.

In some of any of the provided embodiments, the biological sample is a lymph node sample or a tumor sample, wherein the sample is collected by a needle biopsy, optionally a core needle biopsy or a fine-needle aspiration. In some of any of the emobdiments, the biological sample includes tumor fragments. In some of any of the provided embodiments, the population of T cells comprise tumor infiltrating lymphocytes.

Provided herein is a method for manufacturing tumor reactive T cells, the method comprising: (1) culturing T cells by a process comprising: (a) enriching tumor-reactive T cells from a biological sample comprising T cells, said tumor-reactive T cells comprising an endogenous TCR that is reactive to a tumor-associated antigen, thereby producing a population of T cells enriched for tumor-reactive T cells; and (b) incubating the population of T cells enriched for tumor-reactive T cells in the presence of at least one T cell adjuvant selected from a costimulatory agonist or an apoptosis inhibitor, wherein at least a portion of the incubation is carried out prior to, concurrently with, or subsequent to incubating the population of T cells with a T cell stimulatory agent(s) under conditions to stimulate expansion of cells of the population of T cells, wherein the culturing stimulates expansion of cells of the population of T cells; and (2) harvesting cells produced by the method.

In some embodiments, provided herein is a method for manufacturing tumor reactive T cells, the method comprising: (1) culturing T cells by a process comprising: (a) enriching tumor-reactive T cells from a biological sample comprising T cells, said tumor-reactive T cells comprising an endogenous TCR that is reactive to a tumor-associated antigen, thereby producing a population of T cells enriched for tumor-reactive T cells; (b) incubating the population of T cells enriched for tumor-reactive T cells with at least one T cell adjuvant selected from a costimulatory agonist or an apoptosis inhibitor; and (c) incubating the population of T cells enriched for tumor-reactive T cells with a T cell stimulatory agent(s) under conditions to stimulate expansion of cells of cells of the population of cells, wherein at least a portion of the incubating is carried out prior to, concurrently with, or subsequent to the incubating in (b), wherein the culturing stimulates expansion of cells of the population of T cells; and (2) harvesting cells produced by the method.

In some of any of the provided embodiments, the culturing the T cells further comprises incubating the cells in the presence of one or more recombinant cytokines, wherein the incubating in the presence of one or more cytokines is carried out prior to, during and/or subsequent to the incubating with the T cell stimulatory agents(s) and/or incubating in the presence of the one or more T cell adjuvants.

In some of any of the embodiments, the T cell stimulatory agent(s) comprises the presence of one or more recombinant cytokines, wherein the incubating in the presence of the T cell stimulatory agent(s) is carried out prior to, during and/or subsequent to the incubating in the presence of the one or more T cell adjuvants.

In some of any of the embodiments, the T cell stimulating agent(s) comprises an agent that initiates TCR/CD3 intracellular signaling and an agent that initiates signaling via a costimulatory receptor, optionally wherein the costimulatory receptor is CD28, wherein the incubating in the presence of the T cell stimulating agent(s) is carried out prior to, during and/or subsequent to the incubating in the presence of the one or more T cell adjuvants.

In some of any of the provided embodiments, the method further comprises washing the cells after one or more of the steps of incubating the cells with the T cell stimulatory agent(s), the at least one T cell adjuvant and/or the one or more recombinant cytokines. In some of any of the provided embodiments, the culturing is carried out until a threshold amount of cells is obtained and/or is carried out for up to 20 days.

In some of any of the provided embodiments, at least a portion of the incubating the population of T cells with the at least one T cell adjuvant is carried out concurrently with the incubating the population of T cells with the T cell stimulatory agent(s).

In some of any of the provided embodiments, the incubating the population of T cells with the at least one T cell adjuvant is carried out after the incubating the population of T cells with the T cell stimulatory agent(s).

In some of any of the provided embodiments, the culturing comprises: enriching tumor-reactive T cells from a biological sample comprising T cells, said tumor-reactive T cells comprising an endogenous TCR that is reactive to a tumor-associated antigen, thereby producing a population of T cells enriched for tumor-reactive T cells; incubating the population of T cells comprising reactive T cells with a T cell stimulatory agent(s) under conditions to activate T cells of the population to produce a population of T cells comprising activated cells; incubating the population of T cells comprising activated T cells in the presence of the at least one T cell adjuvant; and further incubating the cells in the presence of one or more recombinant cytokines, optionally wherein the incubating in the presence of one or more cytokines is carried out prior to, during and/or subsequent to the incubating with the T cell stimulatory agents(s) and/or incubating in the presence of one or more T cell adjuvants, wherein the culturing stimulates expansion of cells of the population of T cells. In some of any of the provided embodiments, the incubating in the presence of the at least one T cell adjuvant is carried out between 1 and 5 days prior to the harvesting of the cells.

In some of any of the provided embodiments, the incubating the population of T cells with the at least one T cell adjuvant is carried out prior to the incubating of the population of T cells with the T cell stimulatory agent(s). In some of any of the provided embodiments, subsequent to the incubating the population of T cells with the at least one T cell adjuvant and prior to incubating the population of T cells with the T cell stimulatory agent(s), enriching for the tumor-reactive T cells and/or the T cells expressing one or more T cell activation marker, wherein the population of enriched T cells is incubated with the T cell stimulatory agent(s).

In some of any of the provided embodiments, the T cells are primary T cells from a subject, optionally a human subject.

In some of any of the provided embodiments, the biological sample is an ex vivo co-culture comprising autologous T cells from a subject and antigen-presenting cells (APCs), said co-culturing carried out under conditions in which the APCs have been induced to present one or more peptides from a tumor-associated antigen from the subject. In some of any of the provided embodiments, the APCs are autologous to the subject or allogeneic to the subject. In some of any of the provided embodiments, the subject is healthy or is not known to exhibit a disease or condition.

In some of any of the provided embodiments, the biological sample is from a subject and the selecting comprises directly isolating the T cells from the biological sample. In some of any of the provided embodiments, the subject exhibits a disease or condition, optionally wherein the disease or condition is a cancer. In some of any of the provided embodiments, the subject has been previously administered a costimulatory agonist prior to the isolating or selecting of the population of T cells. In some of any of the provided embodiments, the population of T cells are allogeneic to a subject to be treated or autologous to a subject to be treated, optionally wherein the subject to be treated has a tumor or cancer. In some of any of the provided embodiments, the biological sample is a peripheral blood sample, a lymph node sample, or a tumor sample.

In some of any of the provided embodiments, the biological sample is a peripheral blood sample and the peripheral blood sample is collected by a blood draw or by apheresis, optionally wherein the apheresis is leukapheresis. In some of any of the provided embodiments, the biological sample comprises a volume of between 50 mL and 400 mL. In some of any of the provided embodiments, the enriching for the tumor reactive T cells or T cells surface positive for the one or more activation markers is by flow cytometry, optionally carried out by automated high-throughput flow cytometry, optionally by the FX500 cell sorter. In some of any of the provided embodiments, 1 run, 2 runs, 3 runs or 4 runs by flow cytometry is carried out to enrich the tumor-reactive T cells from the sample.

In some of any of the provided embodiments, the biological sample is a lymph node sample or a tumor sample, wherein the sample is collected by a needle biopsy, optionally a core needle biopsy or a fine-needle aspiration. In some embodiments, the biological sample includes tumor fragments. In some of any of the provided embodiments, the population of T cells comprise tumor infiltrating lymphocytes.

Provided herein is a method for manufacturing tumor reactive T cells, the method comprising: (1) culturing T cells by a process comprising: (a) incubating a population of T cells from a biological sample from a subject with one or more recombinant cytokines for stimulating expansion of T cells in the population, wherein the incubation with the one or more recombinant cytokines is in the presence of at least one T cell adjuvant selected from a costimulatory agonist or an apoptosis inhibitor; (b) enriching tumor-reactive T cells from the population of T cells, said tumor-reactive T cells comprising an endogenous TCR that is reactive to a tumor-associated antigen, thereby producing a population of T cells enriched for tumor-reactive T cells; (c) incubating the population of T cells enriched for tumor-reactive T cells with a T cell stimulatory agent(s) under conditions to stimulate expansion of cells of cells of the population of cells; and (2) harvesting cells produced by the method.

Provided herein is a method for manufacturing tumor reactive T cells, the method comprising: (1) culturing T cells by a process comprising: (a) incubating a population of T cells with at least one T cell adjuvant selected from a costimulatory agonist or an apoptosis inhibitor; (b) enriching tumor-reactive T cells from the population of T cells, said tumor-reactive T cells comprising an endogenous TCR that is reactive to a tumor-associated antigen, thereby producing a population of T cells enriched for tumor-reactive T cells; (c) incubating the population of T cells enriched for tumor-reactive T cells with a T cell stimulatory agent(s) under conditions to stimulate expansion of cells of cells of the population of cells; and (d) further incubating the cells in the presence of one or more recombinant cytokines, optionally wherein the incubating in the presence of one or more cytokines is carried out prior to, during and/or subsequent to the incubating with the T cell stimulatory agents(s) and/or incubating in the presence of one or more T cell adjuvants, wherein the culturing stimulates expansion of cells of the population of T cells; and (2) harvesting cells produced by the method.

Provided herein is a method for manufacturing tumor reactive T cells, the method comprising: (1) culturing T cells by a process comprising: (a) incubating a population of cells comprising T cells from a biological sample from a subject that has a tumor with a T cell stimulatory agent(s) comprising one or more recombinant cytokinesunder conditions to stimulate expansion of T cells in the population to produce a population of stimulated T cells, wherein the incubation with the one or more recombinant cytokines is in the presence of at least one T cell adjuvant that is a checkpoint inhibitor; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (c) enriching tumor-reactive T cells from the co-culture, said tumor-reactive T cells comprising an endogenous TCR that is reactive to a tumor-associated antigen, thereby producing a population of T cells enriched for tumor-reactive T cells; (d) incubating the population of T cells enriched for tumor-reactive T cells with a second T cell stimulatory agent(s) under conditions to stimulate expansion of cells of cells of the population of cells; and (2) harvesting the expanded cells produced by the method.

In some of any of the provided embodiments, at least one T cell adjuvant further comprises at least one apoptosis inhibitor. In some of any of the provided emdbodiments, at least one apoptosis inhibitor and the checkpoint inhibitor is carried out simultaneously, intermittently or sequentially. In some of any of the provided embodiments, at least one T cell adjuvant further comprises at least one costimulatory agonist. In some of any of the provided embodiments, at least one costimulatory agonist and the checkpoint inhibitor is carried out simultaneously, intermittently or sequentially. In some of any of the provided embodiments, the apoptosis inhibitor inhibits caspase activation or activity.

In some of any of the provided emdbodiments, the apoptosis inhibitor inhibits one or more of caspase 2, a caspase 8, a caspase 9, a caspase 10, a caspase 3, a caspase 6 or a caspase 7. In some of any of the provided embodiments, the apoptosis inhibitor is selected from the group consisting of Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8), Wedelolactone, NS3694, NSCI and Z- fluoromethyl ketone Z-VAD-FMK or a flouromethyl ketone variant thereof.. In some of any of the provided emdbodiments, the apoptosis inhibitor is a pan-caspase inhibitor that inhibits activation or activity of two or more caspases. In some of any of the provided embodiments, the apoptosis inhibitor is Z-VAD-FMK. In some of any of the provided embodiments, the apoptosis inhibitor is selected from the group consisting of Z-FA-FMK, Z-VAD(OH)-FMK, Z-DEVD-FMK, Z-VAD(OM2)-FMK, and Z-VDVAD-FMK.

In some of any of the provided embodiments, the apoptosis inhibitor (e.g. caspase inhibitor) is added at a concentration of between at about 0.5 µM and at about 50 µM, between at about 0.5 µM and at about 40 µM, between at about 0.5 µM and at about 30 µM, between at about 0.5 µM and at about 20 µM, between at about 0.5 µM and at about 10 µM, between at about 0.5 µM and at about 5 µM, between at about 0.5 µM and at about 1 µM, between at about 1 µM and at about 50 µM, between at about 1 µM and at about 40 µM, between at about 1 µM and at about 30 µM, between at about 1 µM and at about 20 µM, between at about 1 µM and at about 10 µM, between at about 1 µM and at about 5 µM, each inclusive. In some embodiemtns, the apoptosis inhibitor (e.g. caspase inhibitor) is added at a concentration of at or about 2 µM. In some embodiemtns, the apoptosis inhibitor (e.g. caspase inhibitor) is added at a concentration of at or about 10 µM. In some embodiemtns, the apoptosis inhibitor (e.g. caspase inhibitor) is added at a concentration of at or about 25 µM. In some of any of the provided embodiments, the apoptosis inhibitor (e.g. caspase inhibitor) is added at a concentration of between at about 0.5 µg/mL and at or about 25 µg/mL, between at or about 0.5 µg/mL and at or about 10 µg/mL, between at or about 0.5 µg/mL and at or about 5 µg/mL, between at or about 0.5 µg/mL and at or about 1 µg/mL, between at or about 1 µg/mL and at or about 25 µg/mL, between at or about 1 µg/mL and at or about 10 µg/mL, between at or about 1 µg/m L and at or about 5 µg/mL, between at or about 5 µg/mL and at or about 25 µg/mL, between at or about 5 µg/mL and at or about 10 µg/mL, or between at or about 10 µg/mL and at or about 25 µg/mL, each inclusive.

Provided herein is a method for manufacturing tumor reactive T cells, the method comprising: (1) culturing T cells by a process comprising: (a) incubating a population of T cells from a biological sample from a subject with one or more recombinant cytokines for stimulating expansion of T cells in the population, wherein the incubation with the one or more recombinant cytokines is in the presence of at least one T cell adjuvant selected from a costimulatory agonist or an apoptosis inhibitor; (b) co-culturing the population of T cells produced in (a) in the presence of antigen presenting cells (APCs) under conditions in which the APCs have been induced to present one or more peptides from a tumor-associated antigen from the subject, thereby generating a population containing T cells comprising tumor reactive T cells; (c) enriching tumor-reactive T cells produced in (b), said tumor-reactive T cells comprising an endogenous TCR that is reactive to a tumor-associated antigen, thereby producing a population of T cells enriched for tumor-reactive T cells; (d) incubating the population of T cells enriched for tumor-reactive T cells with a T cell stimulatory agent(s) under conditions to stimulate expansion of cells of cells of the population of cells; and (2) harvesting cells produced by the method.

In some of any of the provided embodiments, the method further comprises washing the cells after one or more of the steps of incubating the cells with the T cell stimulatory agent(s), the at least one T cell adjuvant and/or the one or more recombinant cytokines. In some of any of the provided embodiments, the culturing is carried out until a threshold amount of cells is obtained and/or is carried out for up to 30 days. In some of any of the provided embodiments, the culturing is carried out until a threshold amount of cells is obtained and/or is carried out for up to 20 days. In some of any of the provided embodiments, the population of T cells comprises primary T cells from a subject, optionally a human subject. In some of any of the provided embodiments, the population of T cells are isolated from an ex vivo co-culture comprising autologous T cells from a biological sample from a subject and antigen-presenting cells (APCs), said co-culturing carried out under conditions in which the APCs have been induced to present one or more neotantigen peptides from a tumor from the subject. In some of any of the provided embodiments, the APCs are autologous to the subject or allogeneic to the subject. In some of any of the provided embodiments, the subject is healthy or is not known to exhibit a disease or condition. In some of any of the provided embodiments, the population of T cells are isolated or selected directly from a biological sample from a subject. In some of any of the provided embodiments, the subject exhibits a disease or condition, optionally wherein the disease or condition is a cancer. In some of any of the provided embodiments, the population of T cells are allogeneic to a subject to be treated or autologous to a subject to be treated, optionally wherein the subject to be treated has a tumor or cancer.

In some of any of the provided embodiments, the biological sample is a peripheral blood sample, a lymph node sample, or a tumor sample.

In some of any of the provided embodiments, the biological sample is a peripheral blood sample and the peripheral blood sample is collected by a blood draw or by apheresis, optionally wherein the apheresis is leukapheresis. In some of any of the provided embodiments, the biological sample comprises a volume of between 50 mL and 400 mL. In some of any of the provided embodiments, the enriching for the tumor reactive T cells or T cells surface positive for the one or more activation markers is by flow cytometry, optionally carried out by automated high-throughput flow cytometry, optionally by the FX500 cell sorter. In some of any of the provided embodiments, 1 run, 2 runs, 3 runs or 4 runs by flow cytometry is carried out to enrich the tumor-reactive T cells from the sample.

In some of any of the provided embodiments, the biological sample is a lymph node sample or a tumor sample, wherein the sample is collected by a needle biopsy, optionally a core needle biopsy or a fine-needle aspiration. In some embodiments, the biological sample includes tumor fragments. In some of any of the provided embodiments, the population of T cells comprise tumor infiltrating lymphocytes.

In some of any of the provided embodiments, the enriching for tumor-reactive T cells comprises selecting for T cells surface positive for one or more T cell activation marker selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3.

In some of any of the provided embodiments, the enriching for the tumor reactive T cells comprises selection of T cells surface positive for the one or more T cell activation markers. In some of any of the provided embodiments, the one or more T cell activation marker is selected from the group consisting of CD107, CD107a, CD39, CD103, CD137 (4-1BB), CD59, CD69, CD90, CD38, CD30, CD154, CD252, CD134, CD258, CD256, PD-1, TIM-3 and LAG-3. In some of any of the provided embodiments, the one or more T cell activation marker is selected from the group consisting of CD38, CD39, CD6, CD90, CD134 and CD137. In some of any of the provided embodiments, the one or more T cell activation marker is CD134 and/or CD137. In some of any of the provided embodiments, the one or more T cell activation marker is selected from the group consisting of CD107, CD107a, CD39, CD103, CD59, CD90, CD38, CD30, CD154, CD252, CD134, CD258 and CD256. In some of any of the provided embodiments, the one or more T cell activation marker is selected from the group consisting of CD107a, CD39, CD103, CD59, CD90 and CD38.

In some of any of the provided embodiments, the one or more T cell activation marker comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38. In some of any of the provided embodiments, the one or more T cell activation marker further comprises CD137. In some of any of the provided embodiments, the one or more T cell activation marker comprises at least two markers selected from CD107a and CD137, CD38 and CD137, CD103 and CD137, CD59 and CD137, CD90 and CD137 and CD38 and CD137. In some of any of the provided embodiments, the one or more T cell activation marker further comprises at least one marker selected from the group consisting of PD-1, TIM-3 and LAG-3.

In some of any of the provided embodiments, the T cells comprise CD4+ T cells, CD8+ T cells, or CD4+ and CD8+ T cells. In some of any of the provided embodiments, the population of T cells is enriched for CD4+ T cells and CD8+ T cells. In some of any of the provided embodiments, enriching for CD4+ and CD8+ T cells comprises selecting for T cells surface positive for the cell surface marker CD3 or comprises sequential or simultaneous selection for T cells surface positive for the cell surface marker CD4 and T cells surface positive for the cell surface marker CD8. In some of any of the provided embodiments, the population enriched for CD4+ T cell and CD8+ T cells comprises CD3+ T cells as a percentage of total cells in the population that is greater than or greater than about 60%, greater than or greater than about 70%, greater than or greater than about 80%, greater than or greater than about 90% or greater than or greater than about 95%; or the population enriched for CD4+ T cell and CD8+ T cells comprises CD4+ T cells and CD8+ T cells as a percentage of total cells in the population that is greater than or greater than about 60%, greater than or greater than about 70%, greater than or greater than about 80%, greater than or greater than about 90% or greater than or greater than about 95%. In some of any of the provided embodiments, the ratio of CD8+ T cells to CD4+ T cells is between at or about 1:100 and at or about 100:1, between at or about 1:50 and at or about 50:1, between at or about 1:25 and at or about 25: 1, between at or about 1:10 and at or about 10:1, between at or about 1:5 and at or about 5:1, or between at or about 1:2.5 and at or about 2.5:1.

In some of any of the provided embodiments, the tumor-associated antigen is a neoantigen; and/or the tumor-reactive T cells comprise T cells comprising an endogenous TCR that is reactive to a neoantigen, In some of any of the provided embodiments, the population enriched for tumor-reactive T cells comprises tumor-reactive T cells as a percentage of total cells or total T cells in the population that is greater than or greater than about 60%, greater than or greater than about 70%, greater than or greater than about 80%, greater than or greater than about 90% or greater than or greater than about 95%; and/or the population enriched for tumor-reactive T cells comprises T cells with an activation phenotype as a percentage of total cells or total T cells in the population that is greater than or greater than about 60%, greater than or greater than about 70%, greater than or greater than about 80%, greater than or greater than about 90% or greater than or greater than about 95%, wherein the activation phenotype comprises T cells surface positive for one or more T cell activation marker selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3.

In some of any of the provided embodiments, the one or more T cell activation marker is selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3. In some of any of the provided embodiments, the one or more T cell activation marker is selected from the group consisting of CD107, CD107a, CD39, CD103, CD59, CD90, CD38, CD30, CD154, CD252, CD134, CD258 and CD256. In some of any of the provided embodiments, the method comprising selecting, from a biological sample, T cells that are surface positive for one or more T cell activation marker selected from the group consisting of CD107, CD107a, CD39, CD103, CD59, CD90, CD38, CD30, CD154, CD252, CD134, CD258 and CD256.

In some of any of the provided embodiments, further comprising incubating selected cells in the presence of at least one T cell adjuvant and/or a T cell stimulatory agent(s) under conditions for expansion or proliferation of the cells.

In some of any of the provided embodiments, the T cells are primary T cells from a subject, optionally a human subject. In some of any of the provided embodiments, the biological sample is an ex vivo co-culture comprising autologous T cells from a subject and antigen-presenting cells (APCs), said co-culturing carried out under conditions in which the APCs have been induced to present one or more peptides from a tumor-associated antigen from the subject. In some of any of the provided embodiments, the APCs are autologous to the subject or allogeneic to the subject. In some of any of the provided embodiments, the subject is healthy or is not known to exhibit a disease or condition.

In some of any of the provided embodiments, the antigen presenting cells are nucleated cells such as dendritic cells, mononuclear phagocytes, B lymphocytes, endothelial cells or thymic epithelium. In some of any of the provided embodiments, the antigen presenting cells are dendritic cells. In some of any of the provided embodiments, the antigen presenting cells are autologous to the subject or allogeneic to the subject. In some of the provided embodiments, the one or more peptides comprises at least one neoepitope from tumor-associated antigens from the subject.

In some of any of the provided embodiments, the biological sample is from a subject and the selecting comprises directly isolating the T cells from the biological sample. In some of any of the provided embodiments, the subject exhibits a disease or condition, optionally wherein the disease or condition is a cancer. In some of any of the provided embodiments, the subject has been previously administered a costimulatory agonist prior to the isolating or selecting of the population of T cells. In some of any of the provided embodiments, the T cells are allogeneic to a subject to be treated or autologous to a subject to be treated, optionally wherein the subject to be treated has a tumor or cancer.

In some of any of the provided embodiments, the biological sample is a peripheral blood sample, a lymph node sample, or a tumor sample. In some of any of the provided embodiments, the biological sample is a peripheral blood sample and the peripheral blood sample is collected by a blood draw or by apheresis, optionally wherein the apheresis is leukapheresis. In some of any of the provided embodiments, the biological sample is a lymph node sample or a tumor sample, wherein the sample is collected by a needle biopsy, optionally a core needle biopsy or a fine-needle aspiration.

In some of any of the provided embodiments, the one or more T cell activation marker is selected from the group consisting of CD107a, CD39, CD103, CD59, CD90 and CD38. In some of any of the provided embodiments, the one or more T cell activation marker comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38.

In some of any of the provided embodiments, the one or more T cell activation marker further comprises CD137. In some of any of the provided embodiments, the one or more reactive T cell activation marker comprises at least two markers selected from CD107a and CD137, CD38 and CD137, CD103 and CD137, CD59 and CD137, CD90 and CD137 and CD38 and CD137. In some of any of the provided embodiments, the one or more reactive T cell marker further comprises at least one marker selected from the group consisting of PD-1, TIM-3 and LAG-3.

In some of any of the provided embodiments, the selecting comprises immunoaffinity-based selection. In some of any of the provided embodiments, the immununoaffinity-based selection is carried out by contacting cells with an antibody that specifically binds to the marker and recovering cells bound to the antibody. In some of any of the provided embodiments, the antibody is immobilized on a particle, optionally wherein the particle is a bead or a nanoparticle, optionally wherein the particle is a magnetic particle. In some of any of the provided embodiments, the selecting comprises magnetic-based selection. In some of any of the provided embodiments, the antibody is labeled with a detectable agent, optionally wherein the detectable agent is a fluorophore. In some of any of the provided embodiments, the selecting comprises flow cytometry.

In some of any of the provided embodiments, the at least one T cell adjuvant is soluble and/or is not bound or attached to a solid support, optionally wherein the solid support is a bead.
In some of any of the provided embodiments, the at least one T cell adjuvant comprises at least one costimulatory agonist and at least one apoptotic blocker.

In some of any of the provided embodiments, the incubating with the at least one costimulatory agonist and the at least one apoptotic blocker is carried out simultaneously, intermittenttly or sequentially.

In some of any of the provided embodiments, the at least one T cell adjuvant comprises at least one costimulatory agonist. In some of any of the provided embodiments, the costimulatory agonist does not specifically bind CD28 or is not an anti-CD28 antibody. In some of any of the provided embodiments, the costimulatory agonist is a tumor necrosis factor receptor superfamily (TNFRSF) agonist. In some of any of the provided embodiments, the costimulatory agonist is an antibody or antigen-binding fragment that specifically binds a TNFRSF member or is a fusion protein comprising an extracellular domain or binding portion thereof of a ligand of a TNFRSF member. In some of any of the provided embodiments, the TNFRSF member is selected from OX40, 4-1BB, GITR and CD27.

In some of any of the provided embodiments, the costimulatory agonist specifically binds OX40. In some of any of the provided embodiments, the costimulatory agonist is an antibody or antigen-binding fragment selected from Tavolixizumab, Pogalizumab, 11D4, 18D8, Hu119-122, Hu106-222,PF-04518600, GSK3174998, MEDI6469, BMS 986178 or 9B12, or is an antigen-binding fragment thereof . In some of any of the provided embodiments, the costimulatory agonist is an OX40L fusion protein that is MEDI6383. In some of any of the provided embodiments, the costimulatory agonist is Tavolixizumab.

In some of any of the provided embodiments, the costimulatory agonist specifically binds 4-1BB. In some of any of the provided embodiments, the costimulatory agonist is urelumab or Utomilumab, or is an antigen-binding fragment of any of the foregoing.

In some of any of the provided embodiments, the costimulatory agonist specifically bind CD27.

In some of any of the provided embodiments, the costimulatory agonist is Varlilumab, or is an antigen-binding fragment of any of the foregoing. In some of any of the provided embodiments, the costimulatory agonist specifically bind GITR. In some of any of the provided embodiments, the costimulatory agonist is MK-1248, or is an antigen-binding fragment of any of the foregoing.

In some of any of the provided embodiments, the at least one T cell adjuvant comprises at least one apoptosis inhibitor. In some of any of the provided embodiments, the apoptosis inhibitor reduces apoptosis induced by CD95 (Fas), optionally wherein the apoptosis inhibitor specifically binds CD95 (Fas) or CD95 ligand (Fas ligand). In some of any of the provided embodiments, the apoptosis inhibitor is an antibody or antigen-binding fragment, optionally wherein the apoptosis inhibitor is an anti-Fas antibody or an anti-Fas ligand antibody. In some of any of the provided embodiments, the apoptosis inhibitor is a fusion protein comprising the extracellular domain of CD95 (Fas) or a specific binding fragment thereof that binds to CD95 ligand (Fas ligand) fused to an Fc immunoglobulin domain, optionally wherein the apoptosis inhibitor is APG101 or CAN008.

In some of any of the provided embodiments, the apoptosis inhibitor inhibits caspase activation or activity, optionally wherein the caspase is a caspase 2, a caspase 8, a caspase 9, a caspase 10, a caspase 3, a caspase 6 or a caspase 7, optionally wherein the caspase is a caspase 3. In some of any of the provided embodiments, the apoptosis inhibitor is selected from the group consisting of NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8). In some of any of the provided embodiments, the apoptosis inhibitor is emericasan.

In some of any of the provided embodiments, the apoptosis inhibitor is added at a concentration of between at about at or about at or about 0.5 µg/mL and at or about 25 µg/mL, between at or about 0.5 µg/mL and at or about 10 µg/mL, between at or about 0.5 µg/mL and at or about 5 µg/mL, between at or about 0.5 µg/mL and at or about 1 µg/mL, between at or about 1 µg/mL and at or about 25 µg/mL, between at or about 1 µg/mL and at or about 10 µg/mL, between at or about 1 µg/mL and at or about 5 µg/mL, between at or about 5 µg/mL and at or about 25 µg/mL, between at or about 5 µg/mL and at or about 10 µg/mL, and between at or about 10 µg/mL and at or about 25 µg/mL, each inclusive.

In some of any of the proided embodiments, the checkpoint inhibitor inhibits the activity of an immune checkpoint selected from the group consisting of PD-1/PD-L1, CTLA-4, OX40, LAG-3, TIM-3 and B7-H3. In some of any of the provided embodiments, the immune checkpoint is selected from PD-1/PD-L1. In some of any of the provided embodiments, the checkpoint inhibitor is an anti-PD-1 antibody, optionally wherein the antibody is selected from Pembrolizumab, cemiplimab, nivolumab, or is an antigen-binding fragment of any of the foregoing. In some of any of the provided embodiments, the checkpoint inhibitor is Pembrolizumab. In some of any of the provided embodiments, the checkpoint inhibitor is an anti-PDL1 antibody, optionally wherein the antibody is selected from avelumab, durvalumab and atezolizumab, or is an antigen-binding fragment of any of the foregoing.

In some of any of the provided embodiments, the immune checkpoint is OX40. In some of any of the provided embodiments, the checkpoint inhibitor is an anti-OX40L antibody, optionally wherein the antibody is Oxelumab or is an antigen-binding fragment thereof.

In some of any of the provided embdiments, the immune checkpoint is CTLA-4. In some of any of the provided embodiments, the checkpoint inhibitor is an anti-CTLA-4 antibody, optionally wherein the antibody is Ipilimumab or is an antigen-binding fragment thereof.

In some of any of the provided embodiments, the checkpoint inhibitor is added at a concentration of between at about at or about at or about 0.5 µg/mL and at or about 25 µg/mL, between at or about 0.5 µg/mL and at or about 10 µg/mL, between at or about 0.5 µg/mL and at or about 5 µg/mL, between at or about 0.5 µg/mL and at or about 1 µg/mL, between at or about 1 µg/mL and at or about 25 µg/mL, between at or about 1 µg/mL and at or about 10 µg/mL, between at or about 1 µg/mL and at or about 5 µg/mL, between at or about 5 µg/mL and at or about 25 µg/mL, between at or about 5 µg/mL and at or about 10 µg/mL, and between at or about 10 µg/mL and at or about 25 µg/mL, each inclusive.

In some of any of the provided embodiments, the T cell adjuvant is added continuously during the one or more steps of the culturing, wherein the T cell adjuvant is replenished or replaced one or more times during the culturing. In some of any of the provided embodiments, the T cell adjuvant is added continuously during the incubation with the one or more recombinant cytokines, wherein the T cell adjuvant is replenished or replaced one or more times during the incubation. In some of any of the provided embodiments, the T cell adjuvant is added transiently during the one or more steps of the culturing, wherein the T cell adjuvant is added only one time during the one or more steps of culturing.

In some of any of the provided embodiments, the T cell stimulatory agent(s) is selected from an agent that initiates TCR/CD3 intracellular signaling and an agent that initiates signaling via a costimulatory receptor, optionally wherein the costimulatory receptor is CD28. In some of any of the provided embodiments, the agent that initiates TCR/CD3 intracellular signaling is an anti-CD3 antibody, optionally OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble.

In some embodiments, the recombinant cytokine selected from the group consisting of IL-10, IL-1a, IL-5, IL-7, IL-12, IL-23p40, IL-16, IL-17A, IL-15, IL-22, IL-2, IL-4, IL-6, IL-8, IL-10, IL12p70, IL13, and IL-1b, optionally wherein the recombinant cytokine is selected from one or more of IL-2, IL-15, IL-7 and IL-21.

In some of any of the provided embodiments, the second T cell stimulatory agent(s) comprise a recombinant cytokine selected from the group consisting of IL-10, IL-1a, IL-5, IL-7, IL-12, IL-23p40, IL-16, IL-17A, IL-15, IL-22, IL-2, IL-4, IL-6, IL-8, IL-10, IL12p70, IL13, and IL-1b.

In some of any of the provided embodiments, the T cell stimulatory agent(s) is or includes recombinant IL-2.

In some of any of the provded embodiments, the one or more recombinant cytokine comprises recombinant IL-2.

Provided herein is a method for manufacturing tumor-reactive T cells, the method comprising (1) culturing T cells by a process that comprises: (a) incubating a population of cells comprising T cells from a biological sample obtained from a subject that has a tumor with a first T cell stimulatory agent(s) comprising one or more recombinant cytokine selected from one or more of IL-7, IL-15 and IL-21 under conditions to stimulate expansion of T cells of the population to produce a population of stimulated T cells; (b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells; (d) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and (d) incubating the population of T cells enriched in tumor reactive T cells with a second T cell stimulatory agent(s) under conditions to stimulate expansion of T cells in the population; and (2) harvesting the expanded cells produced by the method.

In some of any of the provided embodiments, the first T cell stimulatory agent(s) comprises one or more recombinant cytokines comprises IL-7 and IL-15. In some of any of the provided embodiments, the first T cell stimulatory agent(s) further comprises recombinant IL-2. In some of any of the provided embodiments, the first T cell stimulatory agent(s) does not comprise recombinant IL-2.

In some of any of the provided embodiments, the second T cell stimulatory agent(s) comprise one or more recombinant cytokine selected from the group consisting of IL-10, IL-1a, IL-5, IL-7, IL-12, IL-23p40, IL-16, IL-17A, IL-15, IL-22, IL-2, IL-4, IL-6, IL-8, IL-10, IL12p70, IL13, and IL-1b. In some of any of the provided embodiments, the recombinant cytokine is selected from one or more of IL-2, IL-15, IL-7 and IL-21. In some of any of the provided embodiments, the one or more recombinant cytokine comprises recombinant IL-2.

In some of any of the provided embodiments, the concentration of each of the one or more recombinant cytokine individually is 100 IU/mL to 6000 IU/mL. In some of any of the provided embodiments, the concentration of each of the one or more recombinant cytokine individually is from 300 IU/mL to 1000 IU/mL, optionally wherein the concentration of each of the one or more recombinant cyokine is at or about 300 IU/mL or is at or about 1000 IU/mL.

In some of any of the provided embodiments, the second T cell stimulatory agent(s) further comprises an anti-CD3 antibody, optionally OKT3, optionally wherein the concentration of the anti-CD3 antibody is at or about 50 ng/mL.

In some of any of the provided embodiments, the co-culturing is carried out by a process comprising: (a) identifying somatic mutations associated with one or more tumor-associated antigen by exome sequencing of healthy and tumor tissue from a subject; (b) identifying at least one neoepitope of the one or more tumor-associated antigens; (c) isolating a population of autologous T cells from a biological sample from the subject; and (d) co-culturing the population of T cells with antigen presenting cells (APCs) that have been exposed to or contacted with the one or more peptides, said one or more peptides comprising the at least one neoepitope of the one or more tumor-associated antigens under conditions to present one or more of the peptides on the surface of a major histocompatibility complex (MHC), thereby generating the population of T cells comprising tumor-reactive T cells reactive to the one or more peptides.

In some of any of the provided embodiments, the MHC molecule is a class I molecule. In some of any of the provided embodiments, the MHC molecule is a Class II molecule. In some of any of the provided embodiments, the MHC molecule is MHC class I and II.

In some of any of the provided embodiments, the T cells are CD4+ cells. In some of any of the provided embodiments, the T cells are CD8+ cells. In some of any of the provided embodiments, the T cells are CD4+ cells and CD8+ cells.

In some of any of the provided embodiments, the one or more peptide comprises an individual peptide or a pool of peptides. In some of any of the provided embodiments, the one or more peptides are loaded on antigen presenting cells by transfection of in vitro transcribed synthesized minigene constructs encoding for the one or more peptides, optionally wherein the one or more peptides are flanked on each side by 12 amino acids from endogenous proteins, in tandem, wherein the transcribed minigene constructs generate individual peptides. In some of any of the provided embodiments, the one or more peptides are loaded on antigen presenting cells by peptide pulse, optionally by electroporation. In some of any of the provided embodiments, the one or more peptide is each individually 5-30 amino acids, optionally 12-25 amino acids, optionally at or about 25 amino acids in length.

In some of any the provided embodiments, the one or more peptides are a pool of peptides and the concentration of peptides in the pool of peptides for the peptide pulse is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL; or the one or more peptides is an individual peptide and the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL. In some of any of the provided embodiments, the concentration of individual peptides of the one or more peptide, on average, is from at or about 0.00001 µg/mL to at or about 0.01 µg/mL. In some of any of the provided embodiments, the concentration of individual peptide of the one or more peptide, on average, is from at or about 0.0001 µg/mL and at or about 0.001 µg/mL.

In some of any of the provided embodiments, harvesting is carried out within 20 days after initiation of the culturing and/or the enriching of T cells comprising tumor-reactive cells. In some of any of the provided embodiments, the culturing is carried out in the presence of a recombinant cytokine selected from the group consisting of IL-2, IL-15, IL-7 and IL-21.

In some of any of the provided embodiments, the cells are harvested 7 to 20 days, 7 to 14 days, 7 to 10 days, 10 to 20 days, 10 to 14 days or 14 to 20 days after the initiation of the culturing.

In some of any of the provided embodiments, the culturing is carried out until a threshold amount of cells is achieved that is between at or about 0.5 x 10⁸ and at or about 50 x 10⁹ total cells or total viable cells, between at or about 0.5 x 10⁸ and at or about 30 x 10⁹ total cells or total viable cells, between 0.5 x 10⁸ and at or about 12 x 10⁹ total cells or total viable cells, between at or about 0.5 x 10⁸ and at or about 60 x 10⁸ total cells or total viable cells, between at or about 0.5 x 10⁸ and at or about 15 x 10⁸ total cells or total viable cells, between at or about 0.5 x 10⁸ and at or about 8 x 10⁸ total cells or total viable cells, between at or about 0.5 x 10⁸ and at or about 3.5x 10⁸ total cells or total viable cells, between at or about 0.5 x 10⁸ and at or about 1 x 10⁸ total cells or total viable cells, between 1 x 10⁸ and at or about 50 x 10⁹ total cells or total viable cells, between at or about 1 x 10⁸ and at or about 30 x 10⁹ total cells or total viable cells, between 1 x 10⁸ and at or about 12 x 10⁹ total cells or total viable cells, between at or about 1 x 10⁸ and at or about 60 x 10⁸ total cells or total viable cells, between at or about 1 x 10⁸ and at or about 15 x 10⁸ total cells or total viable cells, between at or about 1 x 10⁸ and at or about 8 x 10⁸ total cells or total viable cells, between at or about 1 x 10⁸ and at or about 3.5x 10⁸ total cells or total viable cells, between at or about 3.5 x 10⁸ and at or about 50 x 10⁹ total cells or total viable cells, between at or about 3.5 x 10⁸ and at or about 30 x 10⁹ total cells or total viable cells, between at or about 3.5 x 10⁸ and at or about 12 x 10⁹ total cells or total viable cells, between at or about 3.5 x 10⁸ and at or about 60 x 10⁸ total cells or total viable cells, between at or about 3.5 x 10⁸ and at or about 15 x 10⁸ total cells or total viable cells, between at or about 3.5 x 10⁸ and at or about 8 x 10⁸ total cells or total viable cells, between at or about 8 x 10⁸ and at or about 50 x 10⁹ total cells or total viable cells, between at or about 8 x 10⁸ and at or about 30 x 10⁹ total cells or total viable cells, between at or about 8 x 10⁸ and at or about 12 x 10⁹ total cells or total viable cells, between at or about 8 x 10⁸ and at or about 60 x 10⁸ total cells or total viable cells, between at or about 8 x 10⁸ and at or about 15 x 10⁸ total cells or total viable cells, between at or about 15 x 10⁸ and at or about 50 x 10⁹ total cells or total viable cells, between at or about 15 x 10⁸ and at or about 30 x 10⁹ total cells or total viable cells, between at or about 15 x 10⁸ and at or about 12 x 10⁹ total cells or total viable cells, between at or about 15 x 10⁸ and at or about 60 x 10⁸ total cells or total viable cells, between at or about 60 x 10⁸ and at or about 50 x 10⁹ total cells or total viable cells, between at or about 60 x 10⁸ and at or about 30 x 10⁹ total cells or total viable cells, between at or about 60 x 10⁸ and at or about 12 x 10⁹ total cells or total viable cells, between at or about 12 x 10⁹ and at or about 50 x 10⁹ total cells or total viable cells, between at or about 12 x 10⁹ and at or about 30 x 10⁹ total cells or total viable cells, or between at or about 30 x 10⁹ and at or about 60 x 10⁹ total cells or total viable cells, each inclusive.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more.

In some of any of the provided embodiments, the biological sample is a peripheral blood sample, optionally an apheresis sample, and wherein: the number of cells at the initiation of the culturing is between at or about 1 x 10⁹ and 7 x 10⁹ total viable cells; or is at or about 1 x 10⁹ total viable cells, at or about 2 x 10⁹ total viable cells, 3 x 10⁹ total viable cells, 4 x 10⁹ total viable cells, 5 x 10⁹ total viable cells, 6 x 10⁹ total viable cells, or 7 x 10⁹ total viable cells, or any value between any of the foregoing; and/or the percentage of tumor reactive T cells at the initiation of the culturing is between at or about 0.02% and at or about 40%, at or about 0.02% and at or about 24%, at or about 0.02% and at or about 18%, at or about 0.02% and at or about 0.9% or at or about 0.02% and at or about 6.0%; and/or the number of T cells surface positive for the T cell activation marker at the initiation of the culturing is between at or about 0.1 x 10⁶ and at or about 60 x 10⁶ T cells, 0.1 x 10⁶ and at or about 8 x 10⁶ T cells, 0.1 x 10⁶ and at or about 20 x 10⁶ T cells, 0.3 x 10⁶ and at or about 35 x 10⁶ T cells or 0.3 x 10⁶ and at or about 60 x 10⁶ T cells; or is at or about 0.1 x 10⁶ T cells, 0.3 x 10⁶ T cells, 0.6 x 10⁶ T cells, 1 x 10⁶ T cells, 5 x 10⁶ T cells, 10 x 10⁶ T cells, 35 x 10⁶ T cells or 60 x 10⁶ T cells, or any value between any of the foregoing.

In some of any of the provided embodiments, the biological sample is a lymph sourced sample or a tumor sourced sample, and wherein: the number of cells at the initiation of the culturing is between at or about 10 x 10⁶ and 100 x 10⁶ total viable cells, 20 x 10⁶ and 100 x 10⁶ total viable cells, or 12 x 10⁶ and 43 x 10⁶ total viable cells; or is at or about 10 x 10⁶ total viable cells, at or about 12 x 10⁶ total viable cells, 20 x 10⁶ total viable cells, 40 x 10⁶ total viable cells, 60 x 10⁶ total viable cells, or 100 x 10⁶ total viable cells, , or any value between any of the foregoing; and/or the percentage of tumor reactive T cells at the initiation of the culturing is between at or about 1% and at or about 90%, at or about 1% and at or about 75%, at or about 1% and at or about 50%, at or about 1% and at or about 25% or at or about 1% and at or about 14%; and/or the number of T cells surface positive for the T cell activation marker at the initiation of the culturing is between at or about 0.7 x 10⁶ and at or about 15 x 10⁶ T cells, 1 x 10⁶ and at or about 15 x 10⁶ T cells, or at or about 0.7 x 10⁶ and at or about 5.4 x 10⁶ T cells; or is at or about 0.7 x 10⁶ T cells, 1 x 10⁶ T cells, 5.4 x 10⁶ T cells, or 15 x 10⁶ T cells, or any value between any of the foregoing.

In some of any of the provided embodiments, the population of cells comprising T cells comprise tumor infiltrating lymphocytes, lymph lymphocytes or peripheral blood mononuclear cells.

In some of any of the provided embodiments, the biological sample is a tumor and the population of cells comprising T cells comprise tumor infiltrating lymphocytes. In some of any of the provided embodiments, the biological sample is a resected tumor and the population of cells comprising T cells are one or more tumor fragments from the resected tumor. In some of any of the provided embodiments, the one or more tumor fragments are seeded for incubation with the first T cell stimulatory agent(s) at about 1 tumor fragment per 2 cm². In some of any of the provided embodiments, the tumor is a melanoma. In some of any of the provided embodiments, the biological sample is a resected tumor and the population of cells comprising T cells are processed as a single cell suspension by homogenization and/or enzymatic digestion of one or more tumor fragments from the resected tumor. In some of any of the provided embodiments, the biological sample is a resected tumor and the population of cells comprising T cells are a single cell suspension processed by homogenization and enzymatic digestion of one or more tumor fragments from the resected tumor. In some of any of the provided embodiments, the enzymatic digestion is by incubation with a collagenase, optionally collagenase IV or collagenase I/II.

In some of any of the provided embodiments, the population of cells comprising T cells are seeded for incubation with the first T cell stimulatory agent(s) at about 5 x 10⁵ to at or about 2 x 10⁶ total cells per 2 cm². In some of any of the provided embodiments, the tumor is a colorectal cancer (CRC).

In some of any of the provided embodiments, the incubation with the at least one T cell adjuvant is for a minimum of 24 hours and up to the time of harvest of the cells, or is between at or about 24 hours and at or about 96 hours or is between at or about 24 hour and at or about 48 hours, or is at or about 24 hours, at or about 36 hours or at or about 48 hours.

In some of any of the provided embodiments, the incubation with the T cell stimulatory agent(s) is between at or about 24 hours and at or about 96 hours or is between at or about 24 hour and at or about 48 hours, or is at or about 24 hours, at or about 36 hours or at or about 48 hours.

In some of any of the provided embodiments, the incubating with the first T cell stimulatory agent(s) is for 7 to 21 days, optionally 7 to 14 days. In some of any of the provided embodiments, the incubating with the first T cell stimulatory agent(s) is in a closed system. In some of any of the provided embodiments, the incubating with the first T cell stimulatory agent(s) is in a gas permeable culture vessel. In some of any of the provided embodiments, the incubating with the first T cell stimulatory agent(s) is performed using a bioreactor. In some of any of the provided embodiments, the incubating with the second T cell stimulatory agent(s) is for 7 to 21 days, optionally 7 to 14 days. In some of any of the provided embodiments, the incubating with the second T cell stimulatory agent(s) is in a closed system. In some of any of the provided embodiments, the incubating with the second T cell stimulatory agent(s) is in a gas permeable culture vessel. In some of any of the provided embodiments, the incubating with the second T cell stimulatory agent(s) is performed using a bioreactor.

In some of any of the provided embodiments, one or more of the steps of the method is carried out in a closed system, optionally wherein one or more of the steps for selection and/or enrichment or incubation is carried out in a closed system.

In some of any of the provided embodiments, the method further comprises formulating the harvested cells for administration to a subject. In some of any of the provided embodiments, the formulating comprises cryopreservation, wherein the cells are thawed prior to administration to the subject.

Provided herein is a composition produced by any of the provided methods.

Provided herein is a composition comprising tumor-reactive T cells, wherein at least at or about 40%, at least at or about 50%, at least at or about 60%, at least at or about 70%, at least at or about 80%, or at least at or about 90% of the total cells or total T cells in the composition are tumor reactive T cells or are surface positive for one or more T cell activation marker.

Provided herein is a composition comprising expanded T cells enriched in tumor reactive T cells produced by any of the provided methods.

Provided herein is a composition comprising a pharmaceutically acceptable excipient produced by any of the provided methods.

In some of any of the embodiments of the provided compositions, the one or more T cell activation marker is selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3. In some embodiments, the one or more T cell activation marker is selected from the group consisting of CD107, CD107a, CD39, CD103, CD59, CD90, CD38, CD30, CD154, CD252, CD134, CD258 and CD256. In some embodiments, the one or more T cell activation marker is selected from the group consisting of CD107a, CD39, CD103, CD59, CD90 and CD38. In some embodiments, the one or more T cell activation marker comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38. In some embodiments, one or more T cell activation marker further comprises CD137. In some embodiments, the one or more reactive T cell activation marker comprises at least two markers selected from CD107a and CD137, CD38 and CD137, CD103 and CD137, CD59 and CD137, CD90 and CD137 and CD38 and CD137. In some embodiments. the one or more reactive T cell marker further comprises at least one marker selected from the group consisting of PD-1, TIM-3 and LAG-3.

In some of any of the embodiments of the provided compositions, the T cells are CD3+ T cells or comprise CD4+ T cells and/or CD8+ T cells. In some embodiments, the T cells comprise CD4+ T cells and CD8+ T cells, wherein the ratio of CD8+ T cells to CD4+ T cells is between at or about 1: 100 and at or about 100: 1, between at or about 1:50 and at or about 50:1, between at or about 1:25 and at or about 25: 1, between at or about 1:10 and at or about 10: 1, between at or about 1:5 and at or about 5:1, or between at or about 1:2.5 and at or about 2.5:1.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more.

In some of any of the provided embodiments, the composition of expanded cells are able to produce IFNgamma at a concentration of greater than at or about 30 pg/mL, optionally greater than at or about 60 pg/mL, following antigen-specific stimulation. In some of any of the provided embodiments, the method comprises formulating the harvested cells with a cryoprotectant. ,

In some of any of the provided embodiments, the T cells are CD3+ T cells or comprise CD4+ T cells and/or CD8+ T cells. In some of any of the provided embodiments, the T cells comprise CD4+ T cells and CD8+ T cells, wherein the ratio of CD8+ T cells to CD4+ T cells is between at or about 1:100 and at or about 100:1, between at or about 1:50 and at or about 50:1, between at or about 1:25 and at or about 25:1, between at or about 1:10 and at or about 10:1, between at or about 1:5 and at or about 5:1, or between at or about 1:2.5 and at or about 2.5:1.

In some of any of the provided embodiments, the number of tumor reactive T cells or total T cells surface positive for the T cell activation marker, or of viable cells thereof, in the composition is between at or about 0.5 x 10⁸ and at or about 50 x 10⁹, between at or about 0.5 x 10⁸ and at or about 30 x 10⁹, between 0.5 x 10⁸ and at or about 12 x 10⁹, between at or about 0.5 x 10⁸ and at or about 60 x 10⁸, between at or about 0.5 x 10⁸ and at or about 15 x 10⁸, between at or about 0.5 x 10⁸ and at or about 8 x 10⁸, between at or about 0.5 x 10⁸ and at or about 3.5x 10⁸, between at or about 0.5 x 10⁸ and at or about 1 x 10⁸, between 1 x 10⁸ and at or about 50 x 10⁹, between at or about 1 x 10⁸ and at or about 30 x 10⁹, between 1 x 10⁸ and at or about 12 x 10⁹, between at or about 1 x 10⁸ and at or about 60 x 10⁸, between at or about 1 x 10⁸ and at or about 15 x 10⁸, between at or about 1 x 10⁸ and at or about 8 x 10⁸, between at or about 1 x 10⁸ and at or about 3.5x 10⁸, between at or about 3.5 x 10⁸ and at or about 50 x 10⁹, between at or about 3.5 x 10⁸ and at or about 30 x 10⁹, between at or about 3.5 x 10⁸ and at or about 12 x 10⁹, between at or about 3.5 x 10⁸ and at or about 60 x 10⁸, between at or about 3.5 x 10⁸ and at or about 15 x 10⁸, between at or about 3.5 x 10⁸ and at or about 8 x 10⁸, between at or about 8 x 10⁸ and at or about 50 x 10⁹, between at or about 8 x 10⁸ and at or about 30 x 10⁹, between at or about 8 x 10⁸ and at or about 12 x 10⁹, between at or about 8 x 10⁸ and at or about 60 x 10⁸, between at or about 8 x 10⁸ and at or about 15 x 10⁸, between at or about 15 x 10⁸ and at or about 50 x 10⁹, between at or about 15 x 10⁸ and at or about 30 x 10⁹, between at or about 15 x 10⁸ and at or about 12 x 10⁹, between at or about 15 x 10⁸ and at or about 60 x 10⁸, between at or about 60 x 10⁸ and at or about 50 x 10⁹, between at or about 60 x 10⁸ and at or about 30 x 10⁹, between at or about 60 x 10⁸ and at or about 12 x 10⁹, between at or about 12 x 10⁹ and at or about 50 x 10⁹, between at or about 12 x 10⁹ and at or about 30 x 10⁹, or between at or about 30 x 10⁹ and at or about 60 x 10⁹, each inclusive.

In some of any of the embodiments of the provided compositions, the number of tumor reactive T cells or total T cells surface positive for the T cell activation marker, or of viable cells thereof, in the composition is between at or about 0.5 x 10⁸ and at or about 50 x 10⁹, between at or about 0.5 x 10⁸ and at or about 30 x 10⁹, between 0.5 x 10⁸ and at or about 12 x 10⁹, between at or about 0.5 x 10⁸ and at or about 60 x 10⁸, between at or about 0.5 x 10⁸ and at or about 15 x 10⁸, between at or about 0.5 x 10⁸ and at or about 8 x 10⁸, between at or about 0.5 x 10⁸ and at or about 3.5x 10⁸, between at or about 0.5 x 10⁸ and at or about 1 x 10⁸, between 1 x 10⁸ and at or about 50 x 10⁹, between at or about 1 x 10⁸ and at or about 30 x 10⁹, between 1 x 10⁸ and at or about 12 x 10⁹, between at or about 1 x 10⁸ and at or about 60 x 10⁸, between at or about 1 x 10⁸ and at or about 15 x 10⁸, between at or about 1 x 10⁸ and at or about 8 x 10⁸, between at or about 1 x 10⁸ and at or about 3.5x 10⁸, between at or about 3.5 x 10⁸ and at or about 50 x 10⁹, between at or about 3.5 x 10⁸ and at or about 30 x 10⁹, between at or about 3.5 x 10⁸ and at or about 12 x 10⁹, between at or about 3.5 x 10⁸ and at or about 60 x 10⁸, between at or about 3.5 x 10⁸ and at or about 15 x 10⁸, between at or about 3.5 x 10⁸ and at or about 8 x 10⁸, between at or about 8 x 10⁸ and at or about 50 x 10⁹, between at or about 8 x 10⁸ and at or about 30 x 10⁹, between at or about 8 x 10⁸ and at or about 12 x 10⁹, between at or about 8 x 10⁸ and at or about 60 x 10⁸, between at or about 8 x 10⁸ and at or about 15 x 10⁸, between at or about 15 x 10⁸ and at or about 50 x 10⁹, between at or about 15 x 10⁸ and at or about 30 x 10⁹, between at or about 15 x 10⁸ and at or about 12 x 10⁹, between at or about 15 x 10⁸ and at or about 60 x 10⁸, between at or about 60 x 10⁸ and at or about 50 x 10⁹, between at or about 60 x 10⁸ and at or about 30 x 10⁹, between at or about 60 x 10⁸ and at or about 12 x 10⁹, between at or about 12 x 10⁹ and at or about 50 x 10⁹, between at or about 12 x 10⁹ and at or about 30 x 10⁹, or between at or about 30 x 10⁹ and at or about 60 x 10⁹, each inclusive.

In some of any of the provided embodiments, the therapeutically effective dose is between 1 x 10⁹ and 10 x 10⁹ T cells.

In some of any of the embodiments of the provided compositions, the composition further contains a pharmaceutically acceptable excipient. In some embodiments the composition includes a cyroprotectant.

In some of any of the embodiments of the provided compositions, the composition is is sterile.

Provided herein is a method of treatment, comprising administering to a subject having a tumor a therapeutic dose of the composition produced by any of the provided methods.

Provided herein is a method of treatment, comprising administering any of the provided compositions to a subject having a cancer. In some embodiments, the cells of the administered composition are autologous to the subject. In some embodiments, the cancer is an epithelial cancer.

In some embodiments, the cancer is melanoma, lung squamous, lung adenocarcinoma, bladder cancer, lung small cell cancer, esophageal cancer, colorectal cancer, cervical cancer, head and neck cancer, stomach cancer or uterine cancer. In some embodiments, the cancer is non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

In some of any of the provided embodiments, a composition of comprising expanded cells produced by the method are used to treat the cancer in the subject. In some of any of the provided embodiments, the tumor is a tumor of an epithelial cancer. In some of any of the provided methods, the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer. In some of any of the provided methods, the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

### Brief Description of the Drawings

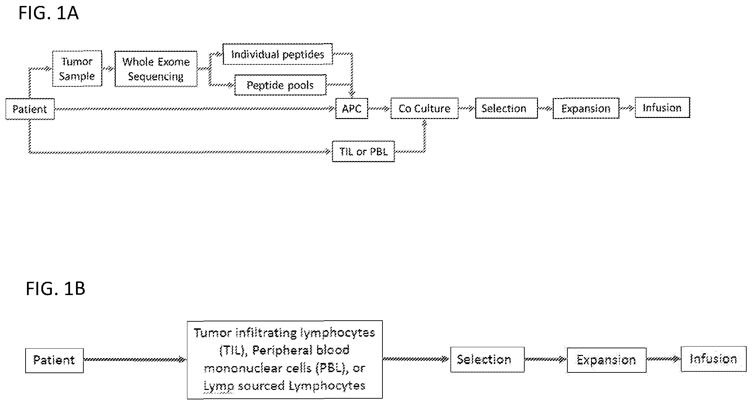

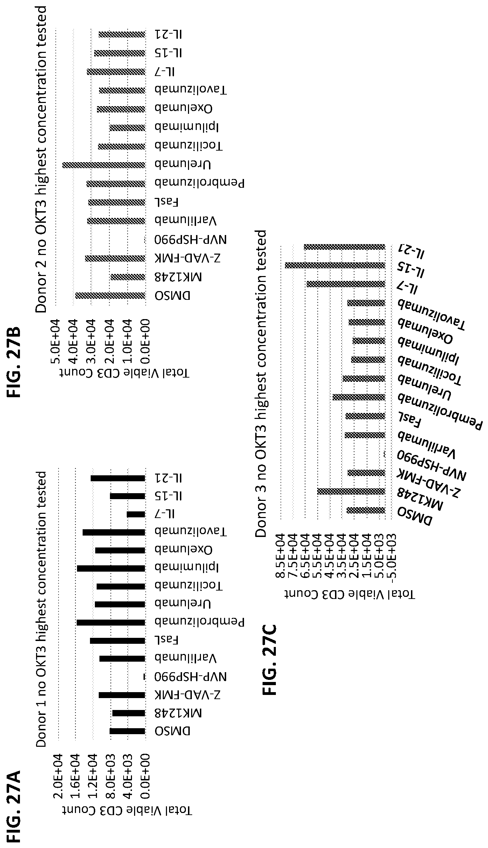

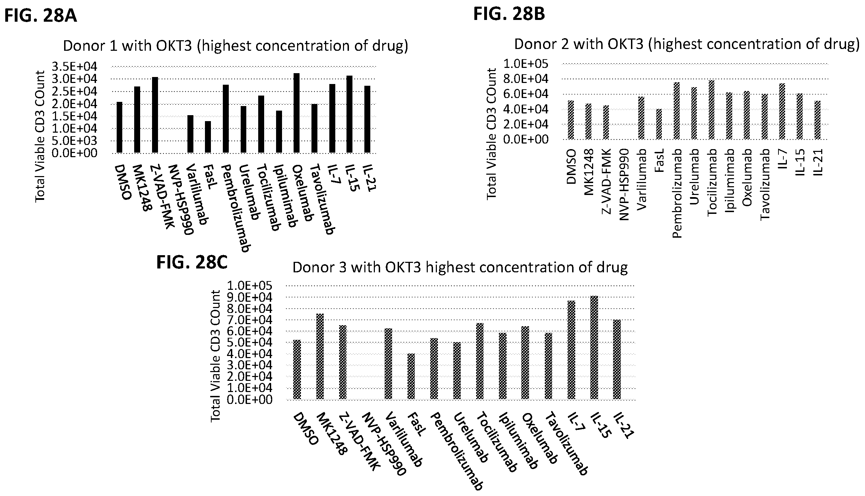

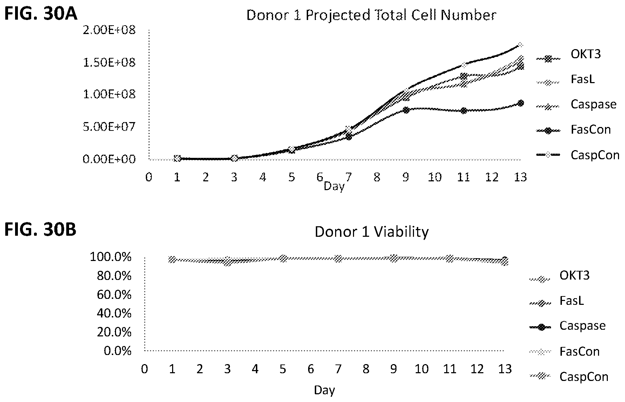

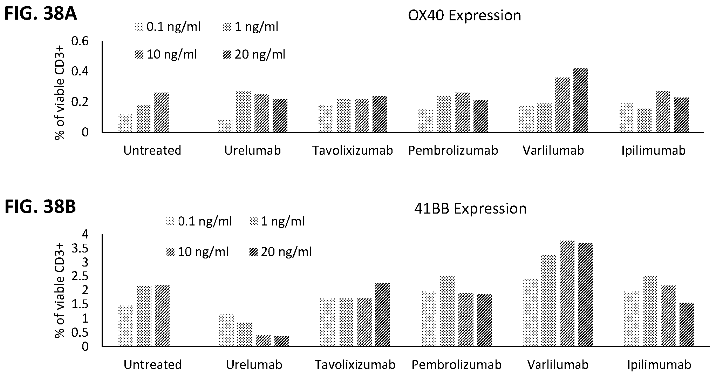

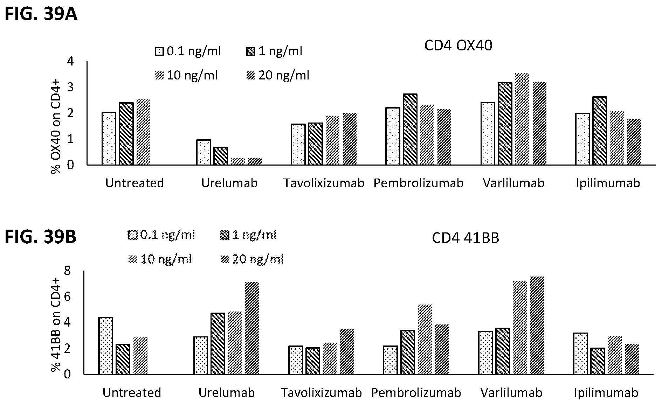

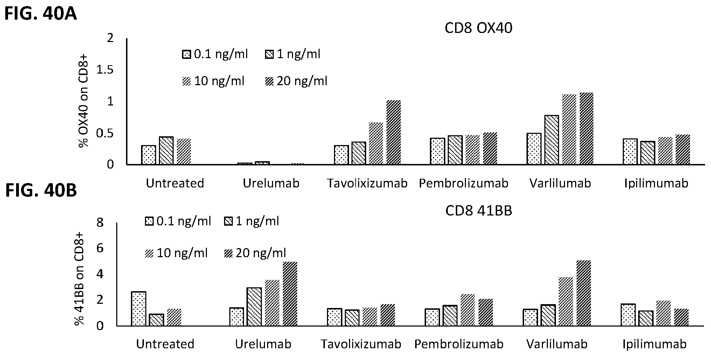

**FIG.** 1A depicts a schematic of an exemplary process for manufacturing a T cell therapeutic composition in accord with the provided methods. In the exemplary process a tumor sample is obtained from a patient for identification and generation of peptides for use in co-culturing methods with autologous T cells obtained from the same subject. In some cases, a population of T cells from the patient, e.g. containing tumor infiltrating lymphocytes (TIL) or peripheral blood lymphocytes (PBL), is stimulated under conditions to expand the cells prior to co-culture with antigen presenting cells that have been contacted or exposed to peptide neoepitopes for presentation on a major histocompability complex. Following co-culture under conditions in which the antigen presenting cells present peptides in the context of a major histocompatibility complex, tumor-reactive T cells or T cells surface positive for one or more T cell activation marker (e.g. CD70a) associated with tumor reactive T cells can be selected and cultured under conditions for expansion in accord with the provided methods, such as incubation with a T cell stimulatory agent(s) (e.g. anti-CD3/anti-CD28). The steps can include incubation with a T cell adjuvant that is a costimulatory agonist (e.g. OX40 or 4-1BB agonist) or an apoptosis inhibitor (e.g. Fas/Fas ligand inhibitor or caspase inhibitor) in accord with the provided methods.. The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion.

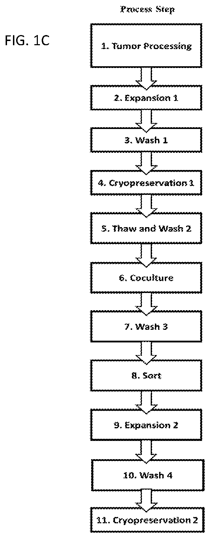

**FIG. 1B** depicts a schematic of an exemplary process for manufacturing a T cell therapeutic composition in accord with the provided methods. In the exemplary process, a biological sample containing T cells is used as a cellular source for the methods. The biological sample can include tumor infiltrating lymphocytes, peripheral blood mononuclear cells (e.g. apheresis), or lymph sourced lymphocytes. Tumor-reactive T cells or T cells surface positive for one or more T cell activation marker (e.g. CD70a) associated with tumor reactive T cells can be selected directly from the sample and cultured under conditions for expansion in accord with the provided methods, including incubation with a T cell adjuvant that is a costimulatory agonist (e.g. OX40 or 4-1BB agonist) or an apoptosis inhibitor (e.g. Fas/Fas ligand inhibitor or caspase inhibitor), and incubation with a T cell stimulatory agent(s) (e.g. anti-CD3/anti-CD28). The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion. **FIG. 1C** depicts a full process flow chart for the generation of a population of patient specific tumor-derived infiltrating T cells.

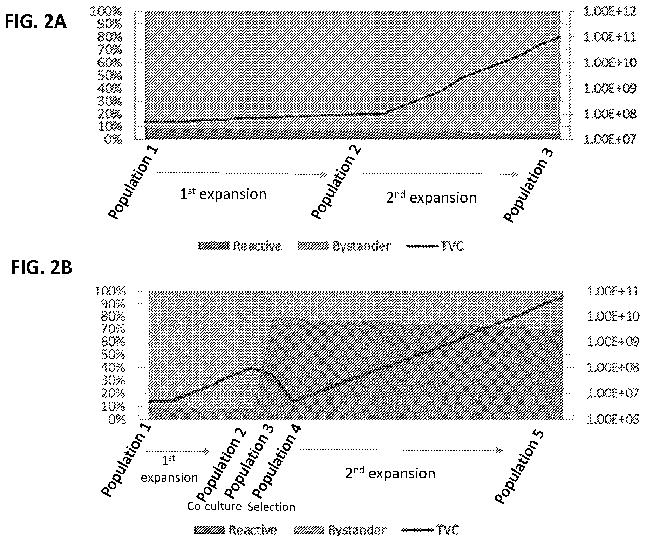

**FIG. 2A** depicts exemplary kinetics and T cell neoantigen reactivity in a typical TIL expansion process involving a bulk expansion of T cells with a first initial expansion and a second rapid expansion wherein reactivity remains low throughout the process, including within the final product. **FIG. 2B** further depicts the exemplary kinetics of a TIL expansion process involving a first initial expansion, followed by an enrichment of tumor-reactive T cells by co-culture with neoantigen peptide-presenting antigen presenting cells, selection of tumor-reactive cells for T cell activation (upregulation) markers, and a second expansion of enriched reactive cells.

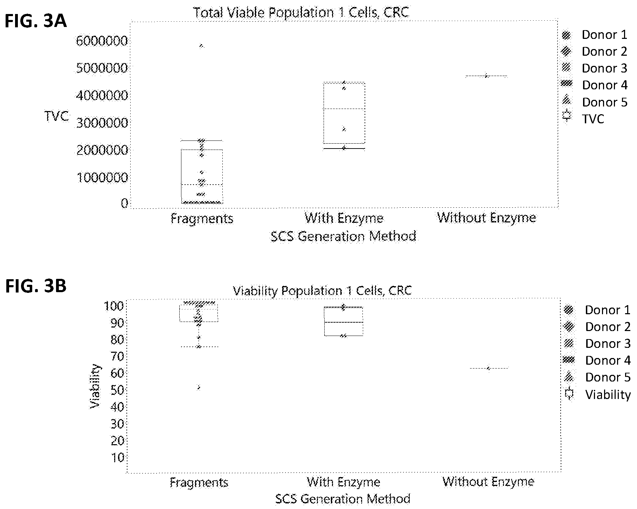

**FIG. 3A** depicts the generation of total viable Population 1 cells from patient derived CRC tumor tissue using fragment culture, homogenization with enzyme, and homogenization without enzyme. Digestion with and without enzyme both yielded more total cells than culture from fragments. Percent viability of these cells is shown in **FIG. 3B****.** Viabilities of cultures generated from fragments and digested with enzyme were higher than those derived using homogenization without enzyme.

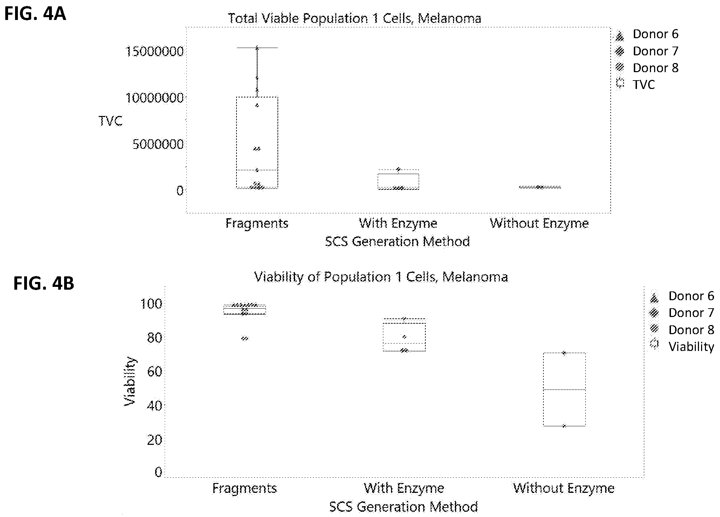

**FIG. 4A** depicts the generation of Population 1 cells from patient derived melanoma tumor tissue using fragment culture or homogenization with or without enzyme. Fragment culture yielded more total cells than cultures initiated from single cell suspensions. Percent viability of these cells is shown in **FIG. 4B****.** The population generated from fragments showed higher viability than cells from single cell suspensions.

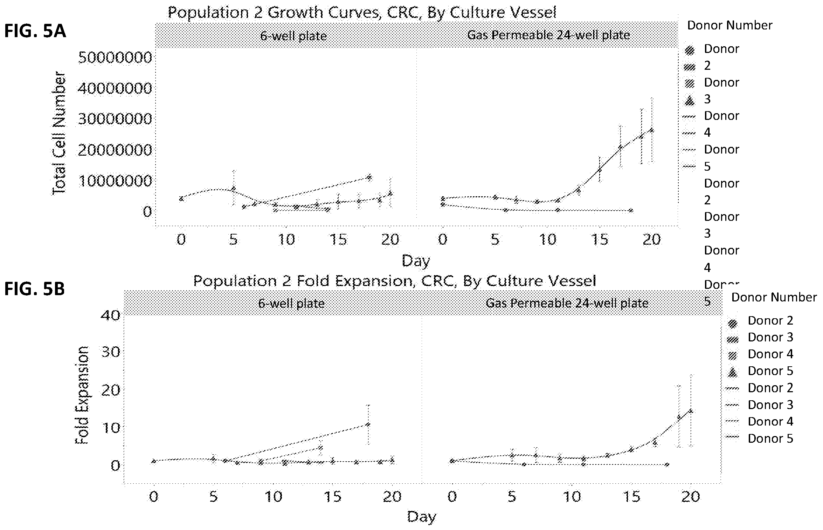

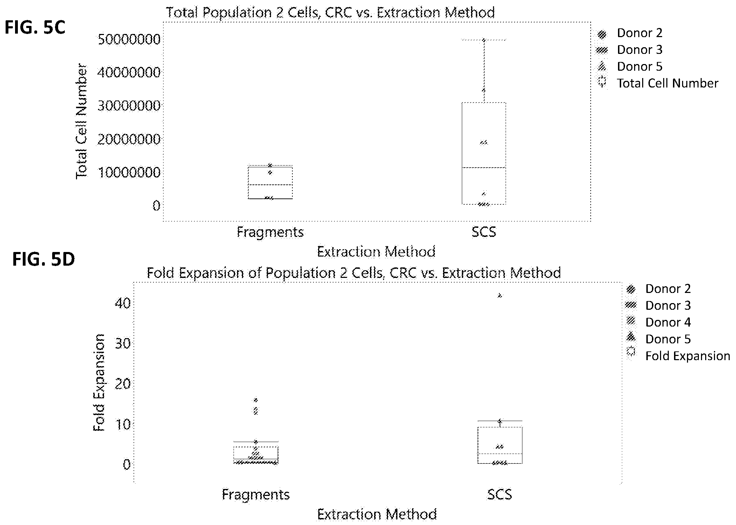

**FIG. 5** depicts growth curves **(****FIG. 5A****)** as well as fold expansion **(****FIG. 5B****)** of Population 2 cells derived from primary CRC tumors in either a conventional 6-well culture plate or a 24-well gas permeable culture plate. **FIG. 5** also depicts total cell number **(****FIG. 5C****)** as well as fold expansion **(****FIG. 5D****)** of Population 2 cells derived from primary CRC tumors contrasted by cellular extraction method, either fragment or single cell suspension culture.

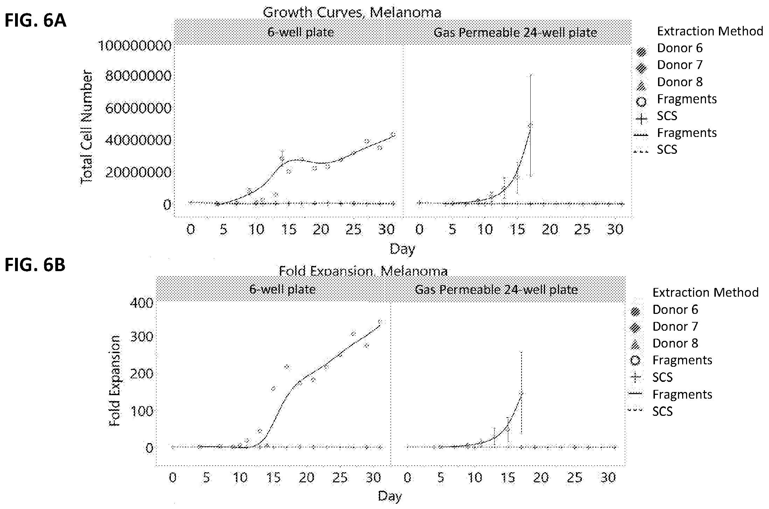

**FIG. 6** depicts growth curves **(****FIG. 6A****)** as well as fold expansion **(****FIG. 6B****)** of Population 2 cells derived from primary melanoma tumors in either a 6-well culture plate or a 24-well gas permeable culture plate.

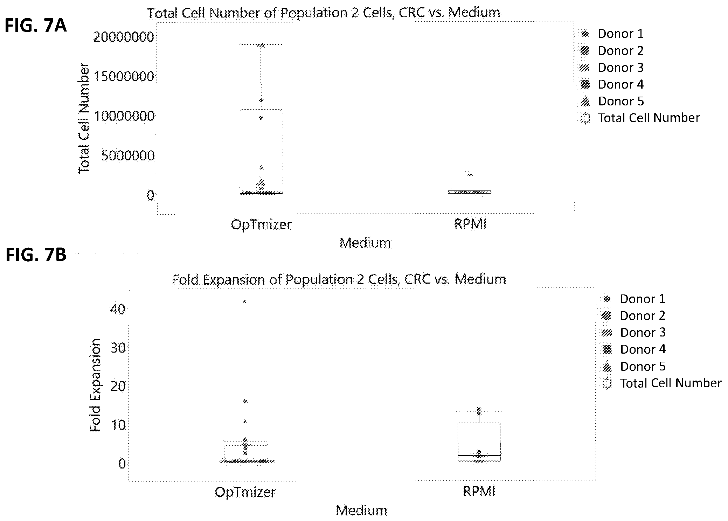

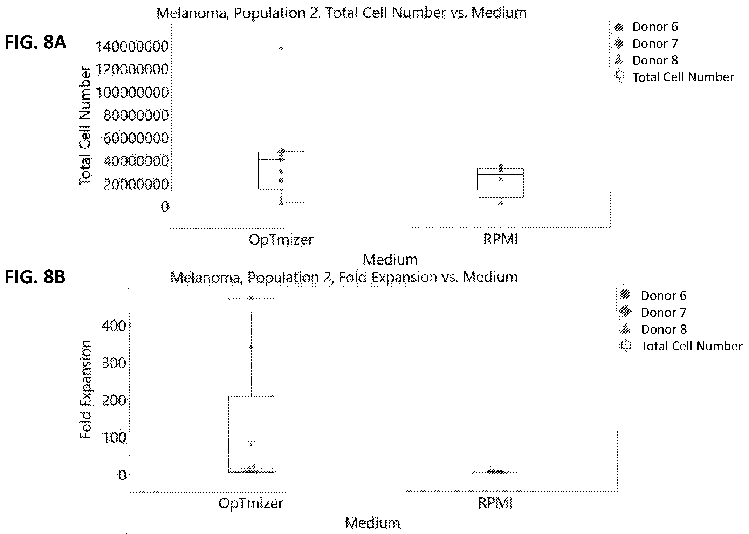

**FIG. 7** depicts total cell number **(****FIG. 7A****)** as well as fold expansion **(****FIG. 7B****)** of Population 2 cells derived from primary CRC tumors using serum free OpTmizer or RPMI media supplemented with 5% human serum. Similarly, **FIG. 8** depicts total cell number **(****FIG. 8A****)** as well as fold expansion **(****FIG. 8B****)** of Population 2 cells derived from primary melanoma tumors using serum free OpTmizer or RPMI media supplemented with 5% human serum.

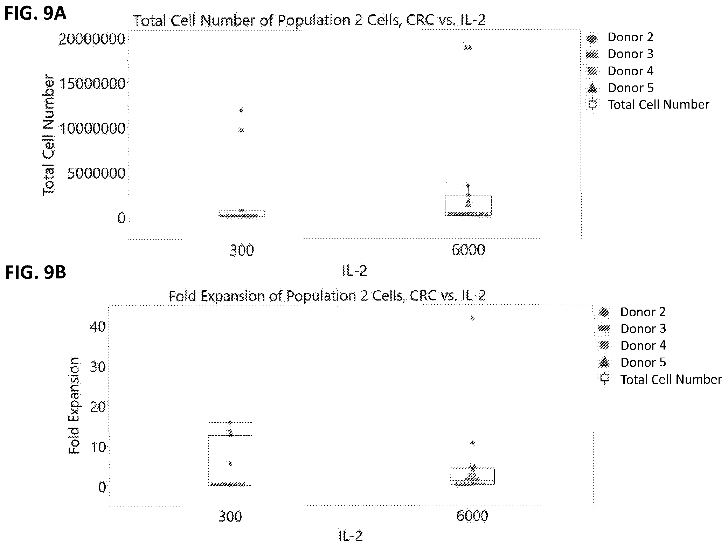

**FIG. 9** depicts total cell number **(****FIG. 9A****)** as well as fold expansion **(****FIG. 9B****)** of Population 2 cells derived from CRC tumors and cultured in media supplemented with either a low concentration (300 IU/mL) or a high concentration (600 IU/mL) of recombinant human IL-2. These data are similarly depicted for melanoma tumor derived cells in

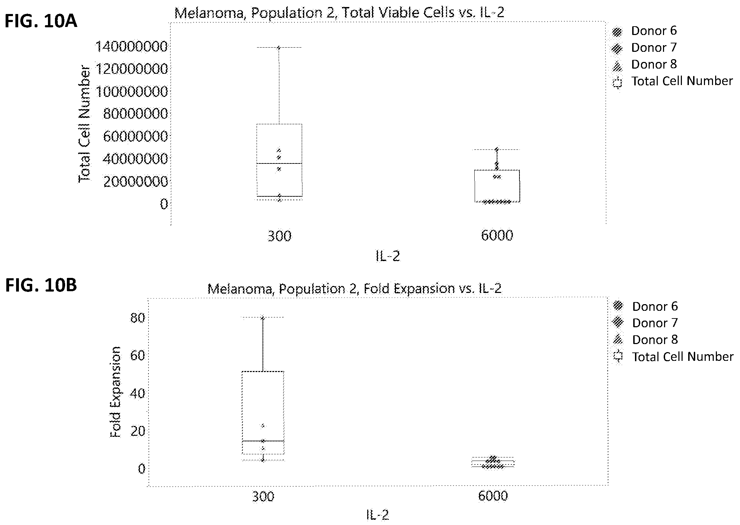

**FIG. 10A-B****.** A high concentration of IL-2 was not observed to be necessary for cellular expansion.

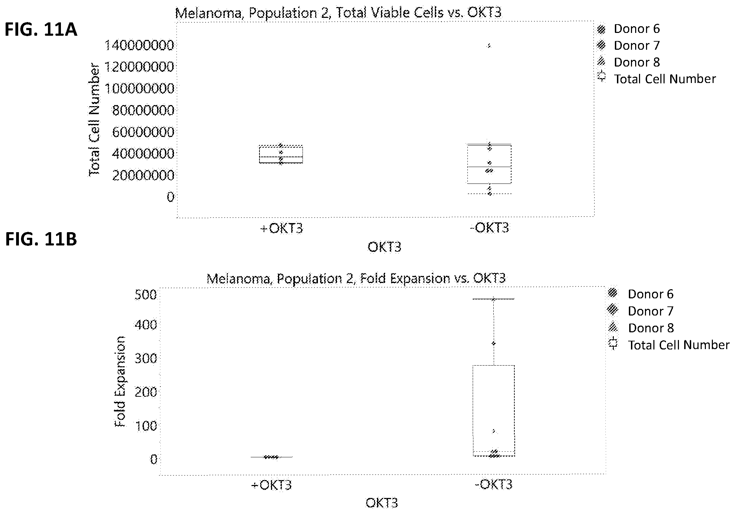

**FIG. 11A** depicts Population 2 total cell number and **FIG. 11B** depicts fold expansion from melanoma derived cell cultures that were unstimulated or stimulated with OKT3, an anti-CD3 monoclonal antibody, were observed to be largely similar.

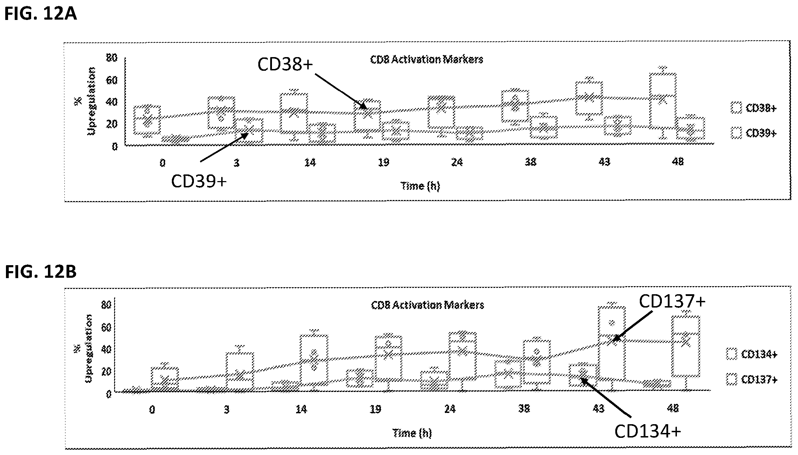

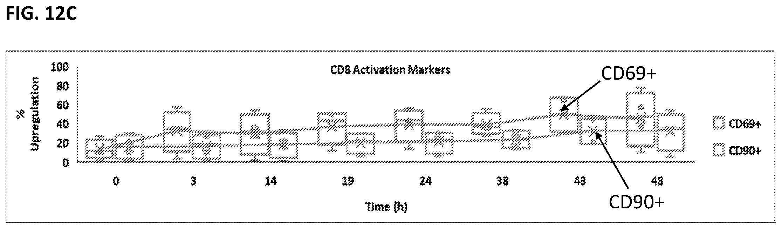

**FIG. 12A-C** depict percent upregulation of activation markers on CD8+ T cells, CD38 and CD39 **(****FIG. 12A****),** CD134 and CD137 **(****FIG. 12B****),** and CD69 and CD90 **(****FIG. 12C****),** between 0 and 48 hours after activation with OKT3.

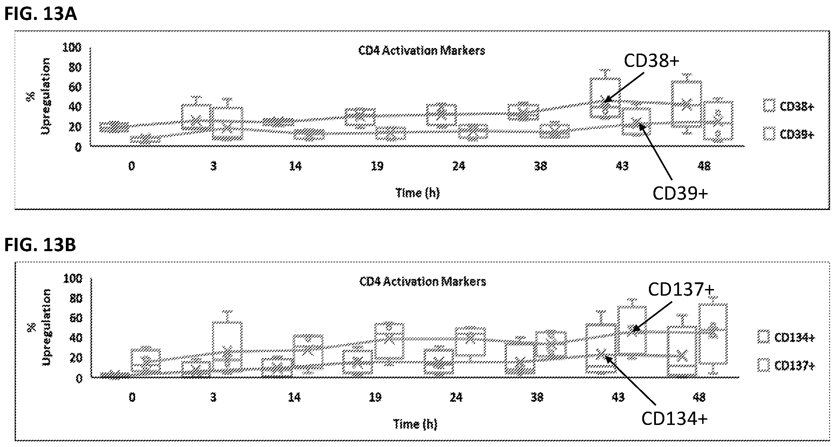

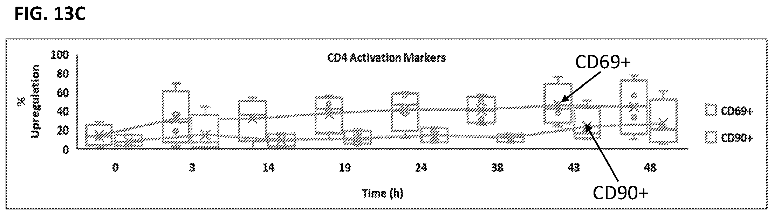

**FIG. 13A-C** depict percent upregulation of activation markers on CD4+ T cells, CD38 and CD39 **(****FIG. 13A****),** CD134 and CD137 **(****FIG. 13B****),** and CD69 and CD90 **(****FIG. 13C****),** between 0 and 48 hours after activation with OKT3.

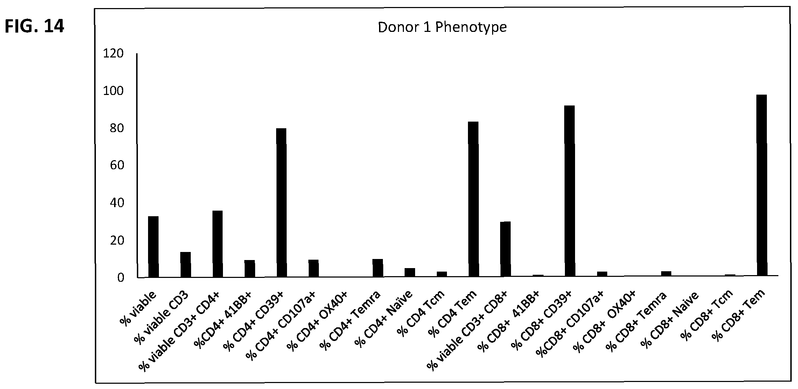

**FIG. 14** depicts expression of selected exemplary markers in a single cell suspension culture generated from a CRC tumor on Day 0.

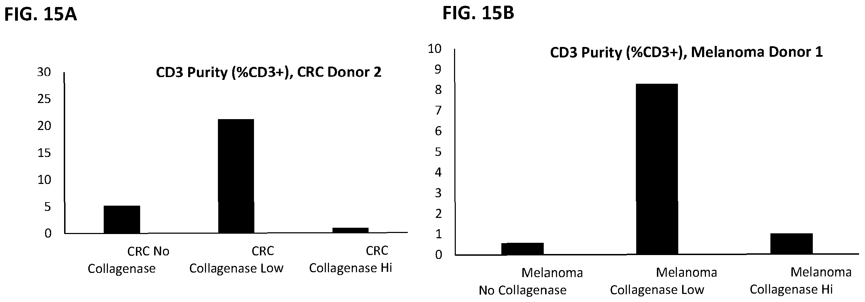

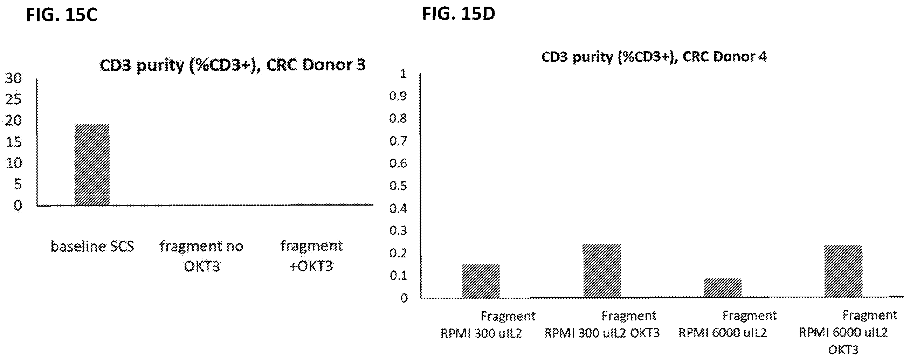

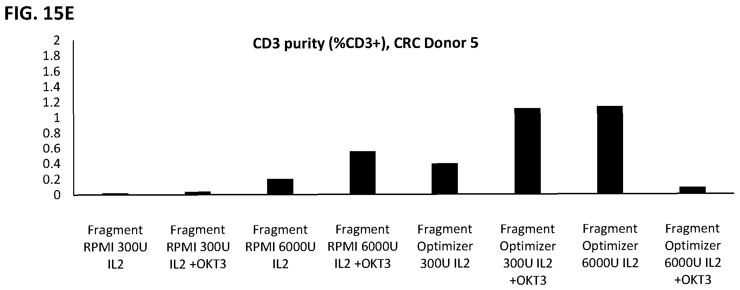

**FIG. 15A-E** depict CD3+ cell purity as a percent of Population 1 cells. **FIG. 15A** depicts the purity of cells from Day 0 SCS from a CRC tumor after homogenization without enzyme, with 1 mg/ml (low) enzyme, and 5 mg/ml (high) enzyme. These data are similarly shown for a melanoma derived culture in **FIG. 15B****.** **FIG. 15C** depicts the purity of CD3+ Population 1 cells from Day 0 (baseline SCS) and Day 6 from fragments cultured in the presence or absence of OKT3 stimulation. **FIG. 15D** shows the relative purity of CD3+ cells from a CRC donor on Day 11 using fragments cultured in media supplemented with either 6000 IU/mL (high) or 300 IU/mL (low) recombinant IL-2. **FIG. 15E** depicts Population 1 cells (Day 9) from fragments cultured in either serum free OpTmizer media or RPMI with either OKT3 stimulation and/or IL-2 at high or low concentrations. These observations support that SCSs from tumor biopsies of CRC patients may be more capable of providing a greater number of T cells for expansion than cells obtained from culture of tumor fragments.

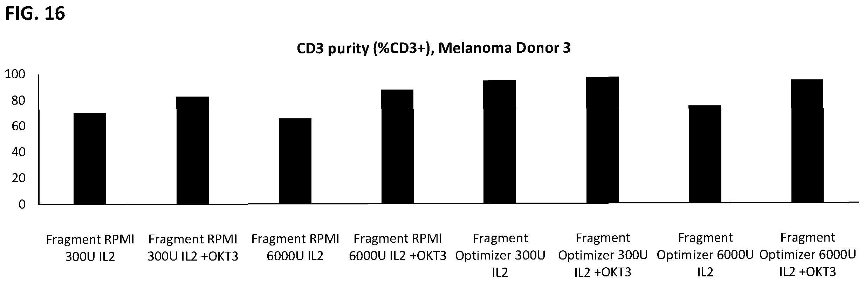

**FIG. 16** depicts the purity of CD3+ Population 1 cells derived from a melanoma patient as fragment cultures from Day 9 at high and low IL-2 concentrations and with serum containing RPMI medium or serum free OpTmizer.

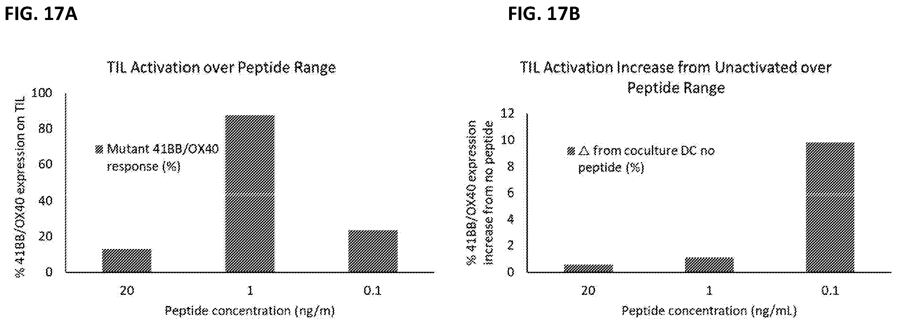

**FIG. 17A** depicts the generation of Population 3 cells following co-culture with dendritic cells loaded with peptide at concentrations from 0.1 ng/mL to 20 ng/mL. **FIG. 17B** depicts the fold increase in the same experiment from T cells which were co-cultured with unloaded dendritic cells **(****FIG. 17B****).**

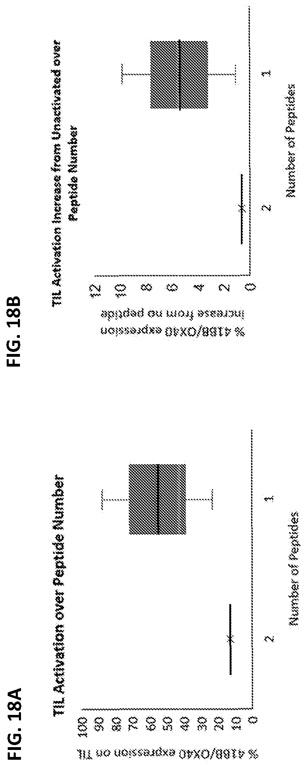

**FIG. 18A** compares stimulation with one peptide or two peptides reported as % 41BB/OX40 expression. **FIG. 18B** depicts stimulation with one peptide or two peptides reported as fold increase from T cells which were unactivated.

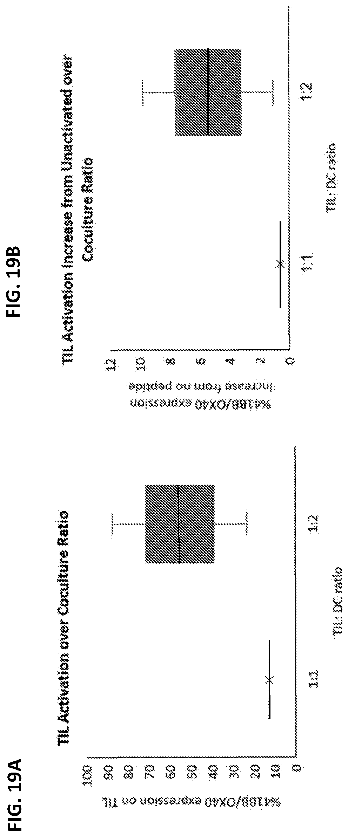

**FIG. 19A** compares two T cell to dendritic cell ratios, 1:1 and 1:2, reported as % 41BB/OX40 expression. **FIG. 19B** compares two T cell to dendritic cell ratios, 1:1 and 1:2, reported as fold increase from T cells which were unactivated.

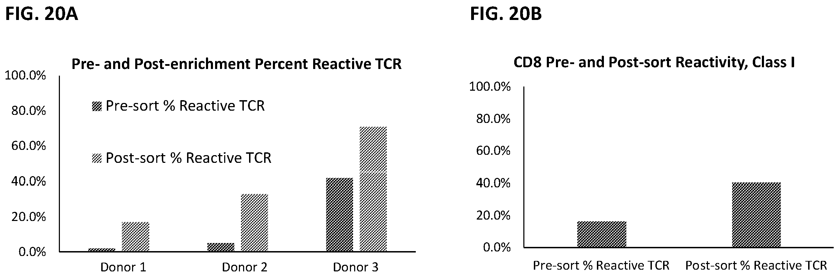

**FIG. 20A** depicts percent neoantigen reactive TCR before and after co-culture with autologous neoantigen peptides and sorting of T cells sourced from the peripheral blood of three healthy donors. **FIG. 20B** depicts average class I reactivity pre- and post-co-culture and sorting of CD8+ cells.

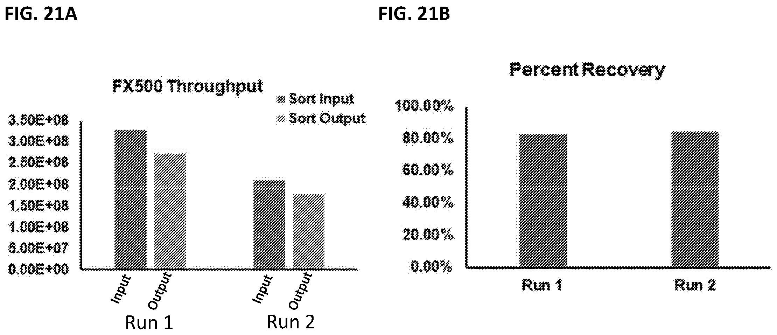

**FIG. 21A and FIG. 21B** depict recovery from cell sorting using the Sony FX500 as both total cell input and output for two independent runs **(****FIG. 21A****)** and percent recovery **(****FIG. 21B****).**

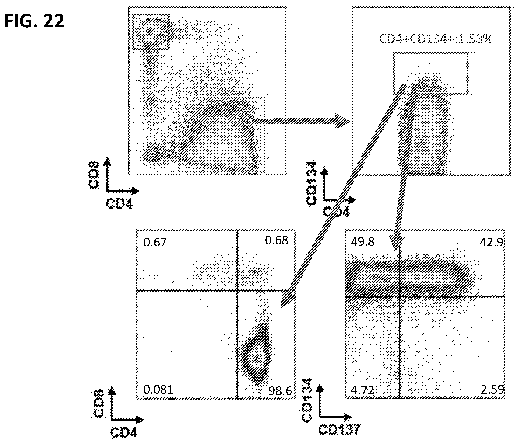

**FIG. 22** depicts purity and gating of a CD4+ population from cell sorting using Sony FX500. The results demonstrate a high recovery of cells after selection and sorting of cells positive for upregulation markers.

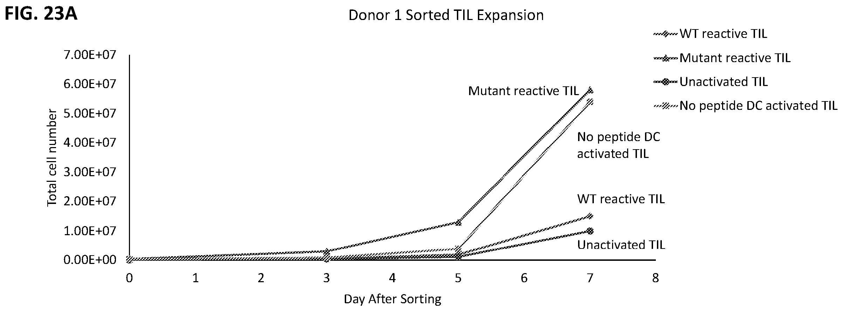

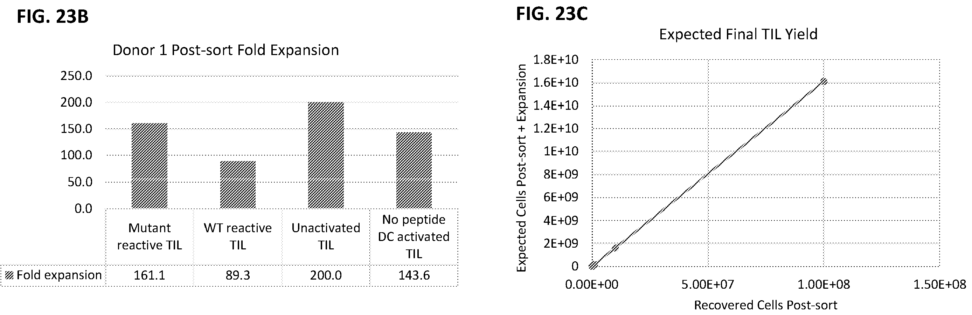

**FIG. 23A****-****FIG. 23C** depict expansion of tumor infiltrating T lymphocytes after sorting. **FIG. 23A** depicts total cell number and **FIG. 23B** depicts fold expansion, of Population 5 cells derived from Population 4 cells following co-culture with or without dendritic cells loaded with wild-type peptide, tumor associated peptide, or no peptide. Projected cell numbers after expansion of Population 4 cells into Population 5 cells at various cell recovery numbers post-sort are shown in **FIG. 23C****.**

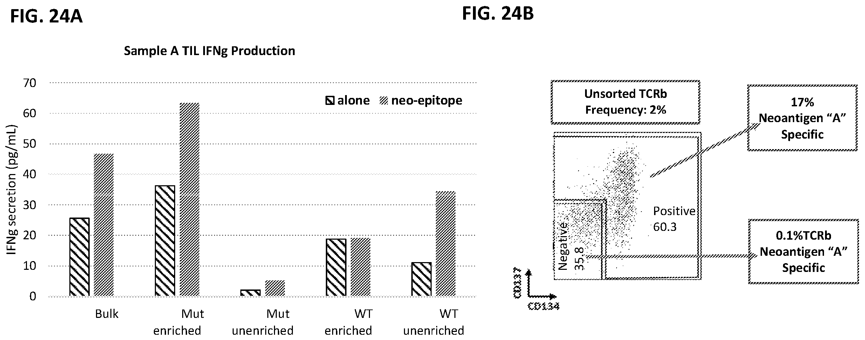

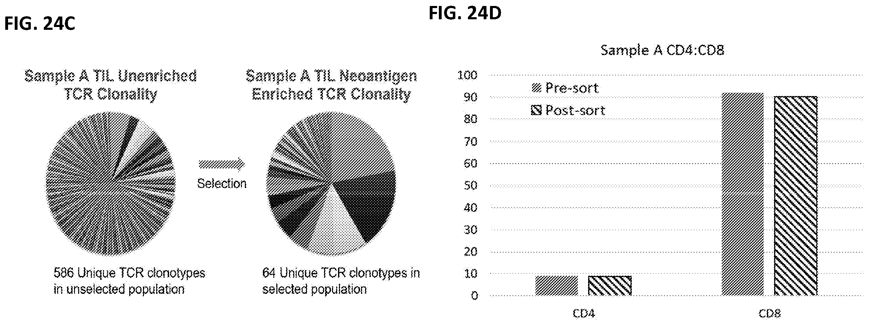

**FIG. 24A** depicts measured IFN-gamma secretion within a bulk co-culture, positive sorted (selected) population by expression of CD137 and/or CD134 from bulk co-culture cells (enriched), or negative sorted (unselected) population form bulk co-culture cells, following stimulation with mutant (mut) peptide or normal, wild-type (WT) peptide from an ovarian cancer patient. **FIG. 24B** depicts enrichment of neoantigen specific population of the tumor-reactive specific cells in the positive sort and negative sort compared to the bulk unsorted T cells. **FIG. 24C** depicts the number of TCR clonotypes present in the unselected and selected populations and demonstrates that the diversity of incoming TCRs is high in the unsorted T cell population and that there is enrichment of unique TCR clones in the selected population. **FIG. 24D** depicts the pre- and post-sort cell populations from Sample A which were observed to contain CD4+ and CD8+ cells, indicating that class I and class II reactive cells are present in the enriched population.

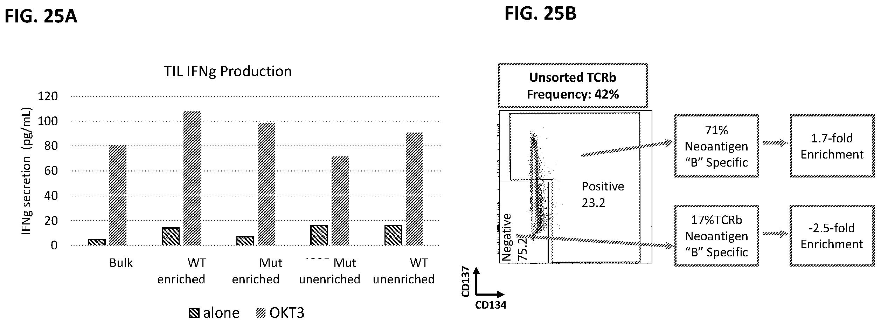

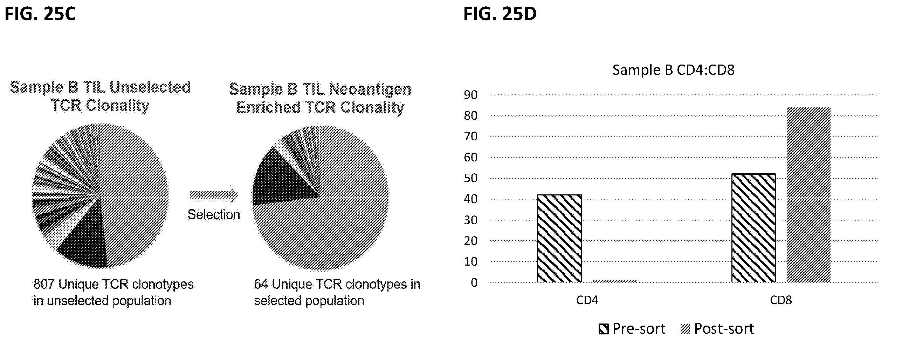

**FIG. 25A** depicts measured IFN-gamma secretion within a bulk co-culture, positive sorted (selected) population by expression of CD137 and/or CD134 from bulk co-culture cells (enriched), or negative sorted (unselected) population from bulk co-culture cells, following stimulation with anti-CD3 (OKT3) from colorectal cancer patient. **FIG. 25B** depicts enrichment of neoantigen specific population of the tumor-reactive specific cells in the positive sort and negative sort compared to the bulk unsorted T cells. **FIG. 25C** depicts the TCR clonality profile present in the unselected and selected populations. **FIG. 25D** depicts the pre- and post-sort cell populations which were observed to contain CD4+ and CD8+ cells, indicating that class I and class II reactive cells are present in the enriched population.

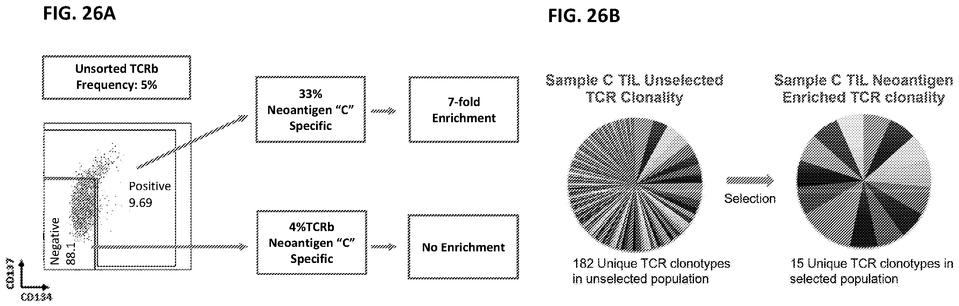

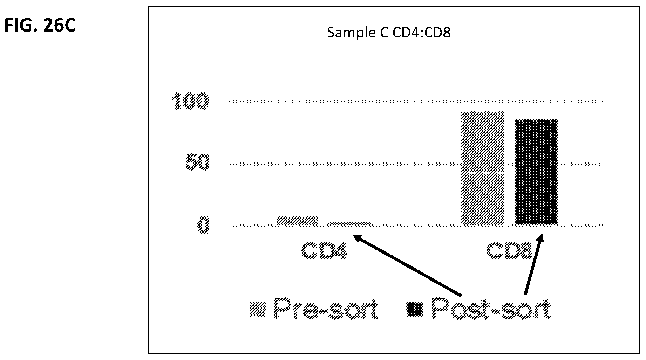

**FIG. 26A** depicts enrichment of neoantigen specific population of tumor-reactive specific cells in a bulk co-culture, positive sorted (selected) population by expression of CD137 and/or CD134 from bulk co-culture cells (enriched), or negative sorted (unselected) population fromm bulk co-culture cells. **FIG. 26B** depicts the TCR clonality profile present in the unselected and selected populations. **FIG. 26C** depicts Pre- (bulk) and post-sort cell populations, which were observed to contain both CD4+ class I reactive and CD8+ class II reactive cells.
Total viable CD3+ cell count for cells grown in the presense of numerous T cell adjuvants are shown with **(FIG. 27A-C)** and without **(FIG. 28A-C)** supplemental OKT3 stimulation. The results shown are for the following adjuvants: Tavolixizumab, Oxelumab, Ipilimumab, Tocilizumab, Urelumab, Pembrolizumab, Varlilumab, anti-GITR MK-1248, anto-human FasL at 10µg/mL; 25 µM for Z-VAD-FMK pan-caspase inhibitor; 250 nM for HSP inhibitor NVP-HSP990; and 1000 IU/mL for cytokine (IL-7, IL-15 or IL-21).

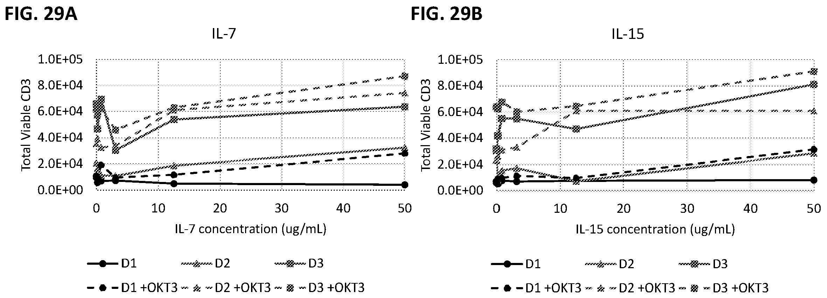

**FIG. 29** shows dose response curves for IL-7 **(****FIG. 29A****)** and IL-15 **(****FIG. 29B****).**
Total cell number and and cell viability for cells derived from each of three healthy donors and grown in experimental conditions as described are shown in **FIG.30A****-B-****FIG.32A-B**. It was observed that cells grown in the presense of continuous caspase inhibition showed superior growth despite inherent donor variability.
Cellular viability for a single activation with anti-CD3/anti-CD28 (transient activation) treatment groups for two donors are shown in **FIG. 33A-B,** and total cell number for the same treatments are shown in **FIG. 34A-B.** Cellular viability for the continuous activation with anti-CD3/anti-CD28 treatment groups for two donors are shown in **FIG 35A-B,** and total cell number for the same treatments are shown in **FIG. 36A-B.**

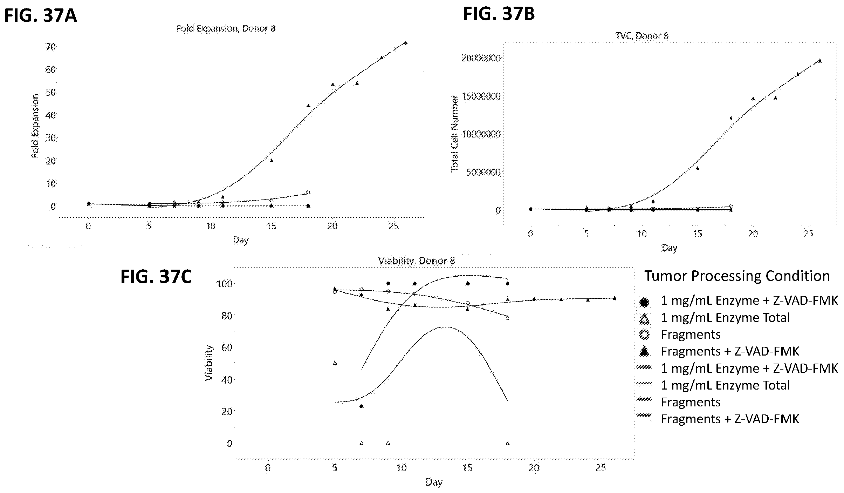

**FIG. 37A-C** shows the fold expansion **(****FIG. 37A****),** total viable cells **(****FIG. 37B****)** and percent viability **(****FIG. 37C****),** of both SCS and tumor fragment derived cultures grown in the presence or absence of pan-caspase inhibitor Z-VAD-FMK.
T cell phenotype is shown for CD3+ **(****FIG. 38A-D),** CD4+ **(****FIG. 39A-D)** and CD8+ **(****FIG. 40A-D)** cells grown in the presense of Ipilimumab (anti-CTLA4), Pembrolizumab (anti-PD1), Tavolixizumab (anti-TNFRSF4), Urelumab (anti-CD137), and Varlilumab (anti-CD27) at varying concentrations.

### Detailed Description

Provided herein is a method for manufacturing T cells that express a cell surface receptor that recognizes peptides on the surface of a target cells, such as a tumor. The T cells can be tumor-reactive T cells that recognize tumor-associated antigens, such as neoantigens. The methods include culturing of T cells ex vivo in which the T cells have been isolated or obtained from a biological sample as a cellular source for T cells. In some cases, the cellular source includes peripheral blood lymphocytes, lymph node sourced lymphocytes or tumor infiltrating lymphocytes. The methods of culturing the cells include methods to proliferate and expand cells, particularly involving steps to enrich for proliferation and expansion of tumor-reactive T cells such as by selection of such cells or based on T cell activation markers associated with such cells. The provided methods also use certain T cell adjuvants in the ex vivo production of a T cell therapy. In some embodiments, the T cell adjuvant is a costimulatory agonist(s). In some embodiments, the T cell adjuvant is an agent that inhibits apoptosis or an apoptotic pathway in a cell (hereinafter "apoptosis inhibitor"). In some embodiments, the T cell adjuvant is an immune checkpoint modulator. In some embodiments, the T cell adjuvant is an agent that inhibits heat shock proteins or heat shock protein activity in the cell. The T cell adjuvant can be added to the manufacturing of T cells to increase the functionality of the T cells ex vivo and for use in in vivo methods of treatment. In particular embodiments, such methods can enrich for expansion of reactive T cells compared to non-reactive and promote their survival and growth in culture ex vivo. It is contemplated that the provided methods can increase expansion to a therapeutic dose to a much greater extent than existing methods and/or increase functionality of the T cell therapy for therapeutic effect. The provided methods can be used to support the growth and survival of donor cells outside of the body, such as in connection with methods of producing a T cell therapy for redelivery back to the patient donor or another patient.

The provided methods relate to producing a T cell therapy reactive to tumor-associated antigens, such as neoantigens. Cancer cells accumulate many different DNA mutations as part of the tumorigenic process. These mutations can cause amino acid changes in protein coding regions. For a mutation to be recognized by the immune system the protein needs to be processed intracelluarlly and presented the mutant peptide presented on the surface with the Major Histocompatibility Complex (MHC). Peptide neoantigens (also referred to herein as neoepitopes or peptide neoepitopes) are the mutant peptides presented by the MHC complex that can be recognized by a T cell via via TCR binding. In order for the immune system to recognize the mutation, it must be expressed on the surface of the cancer cell via the MHC complex and the T cell must have a TCR that recognizes the mutating peptide. These neoantigens may be presented by MHC class I and MHC class II, and are recognized by CD8+ and CD4+ T cells respectively.

In particular embodiments of the provided methods, the population of T cells is or includes reactive T cells that express cell surface receptors, such as a T cell receptor (TCR), able to recognize peptide antigens on the surface of a target cells. Specifically, for an antigen to be recognized by the immune system the protein needs to be processed intracellularly to peptide fragments that are then presented on the surface with the Major Histocompatibility Complex (MHC). A TCR has two protein chains, which are designed to bind with specific peptides presented by a major histocompatibility complex (MHC) protein on the surface of certain cells. Since TCRs recognize peptides in the context of MHC molecules expressed on the surface of a target cell, TCRs have the potential to recognize antigens not only presented directly on the surface of target cells, e.g. cancer cells, but also presented by antigen-presenting cells, such as in tumor, inflammatory and infected microenvironments, and in secondary lymphoid organs. Reactive T cells expressing such cell surface receptors may be used to target and kill any target cell, including, but not limited to, infected cells, damaged cells, or dysfunctional cells. Examples of such target cells may include cancer cells, virally infected cells, bacterially infected cells, dysfunctionally activated inflammatory cells (e.g., inflammatory endothelial cells), and cells involved in dysfunctional immune reactions (e.g., cells involved in autoimmune diseases).

In some embodiments, a "T cell receptor" or "TCR" is a molecule that contains a variable α and β chains (also known as TCRα and TCRβ, respectively) or a variable γ and δ chains (also known as TCRγ and TCRδ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a T cells (or T lymphocytes) where it is generally responsible for recognizing antigens bound to major histocompatibility complex (MHC) molecules.

In some aspects, the reactive T cells are tumor-reactive T cells that recognize a cancer antigen. Cancer cells accumulate many different DNA mutations as part of the tumorigenic process. These mutations can cause amino acid changes in protein coding regions. Neoantigens are the mutant peptides encoded by tumor-specific mutated genes and presented by the MHC complex that can be recognized by a T cell via TCR binding. In order for the immune system to recognize the mutation, a neoantigen is expressed on the surface of the cancer cell via the MHC complex for recognition by a T cell that has a TCR that recognizes the mutating peptide. These neoantigens may be presented by MHC class I and MHC class II, and are recognized by CD8+ and CD4+ T cells respectively. The majority of neoantigens arise from passenger mutations, meaning they do not infer any growth advantage to the cancer cell. A smaller number of mutations actively promote tumor grown, these are known as driver mutations. Passenger mutations are likely to give rise to neoantigens that are unique to each patient and may be pressing in a subset of all cancer cells. Driver mutation give rise to neoantigens that are likely to be present in all the tumor cells of an individual and potentially shared. In some embodiments of the provided method, the population of T cells contain tumor-reactive T cells that can recognize neoantigens containing passenger and/or driver mutations.

In particular aspects, the provided methods can be used for the ex vivo production of a T cell therapy, including for the ex vivo expansion of autologous tumor-reactive T cells. In some aspects, neoantigens are ideal targets for immunotherapies because they represent disease-specific targets. For example, such antigens generally are not present in the body before the cancer developed and are truly cancer specific, not expressed on normal cells and are not subjected to off target immune toxicity. Thus, the unique repertoire of neoantigens specific to the patient can elicit a strong immune response specific to the cancer cells, avoiding normal cells. This is an advantage over other cell therapy targets that may not be disease-specific targets, since even low levels of target antigen on normal cells can lead to severe fatal autoimmune toxicity in the contexts of engineered therapies that target common antigens. For example an anti MAGE-A3-TCR program in melanoma patients was halted due to study related deaths attributed to cross reactivity with a similar target MAGE-A12, which is expressed at a low level in the brain. A significant challenge in cancer immunotherapy has been the identification of cancer targets.

Recent clinical studies have demonstrated that T cells isolated from surgically resected tumors possess TCRs that recognize neoantigens, and expanding these neoantigen reactive TIL populations and re-infusing them into the patient can in some cases result in a dramatic clinical benefit. This personalized therapy has generated remarkable clinical responses in certain patients with common epithelial tumors.

Existing methods for obtaining and generating tumor-reactive T cells are not entirely satisfactory. For example, direct isolation of tumor-reactive T cells from a subject without expansion is not feasible because therapeutically effective amounts of such cells cannot be obtained. As an alternative, these cells can be identified through ex vivo co-culture methods of autologous bulk T cells in the presence of autologous antigen-presenting cells. In such methods, autologous antigen-presenting cells are contacted with, or made to present, a source or potential tumor peptides to identify TCRs that are reactive to neoantigen mutations. Although existing methods may result in producing reactive T cells, the procedures often are long, require single cell co-culturing using droplet techniques, and/or involve methods outside of a GMP controlled environment leading to safety risks associated with endotoxins, mycoplasma, and sterility. Attempts also have been made to identify TCRs specific to a desired neoantigen for recombinant engineering of the TCR into T cells for use in adoptive cell therapy methods. Such approaches, however, produce only a single TCR against a specific neoantigen and thereby lack diversity to recognize a broader repertoire of multiple tumor-specific mutations. Other methods involve bulk expansion of T cells from a tumor source, which has the risk of expanding T cells that are not reactive to a tumor antigen and/or that may include a number of bystander cells that could exhibit inhibitory activity. For example, tumor regulatory T cells (Tregs) are a subpopulation of CD4⁺ T cells, which specialize in suppressing immune responses and could limit reactivity of a T cell product. These further approaches that have sought to expand tumor-reactive T cells ex vivo are not selective such that non-reactive T cells in the culture may preferentially expand over reactive T cells resulting in a final product that lacks satisfactory reactivity and/or in which the number of tumor-reactive T cells remains insufficient. Methods to produce tumor-reactive T cells for therapy are needed.

The provided embodiments relate to improved methods for identifying and expanding T cells ex vivo, including tumor-reactive T cells, for use in T cell therapy. In some embodiments, the provided methods improve or increase the growth and survival of T cells, such as tumor-reactive T cells, outside of the body. In particular embodiments, the methods enrich for expansion of reactive T cells compared to non-reactive and promote their survival and growth in culture ex vivo. In some embodiments, the resulting methods can be carried out in a closed system. The methods in some embodiments are carried out in an automated or partially automated fashion.

In provided embodiments, the methods include *ex vivo* incubation of cells enriched for a population of T cells with a costimulatory agonist under conditions to stimulate or activate a costimulatory receptor expressed by one or more of the T cells in the sample. In particular embodiments, the costimulatory agonist is a 4-1BB agonist. In other particular embodiments, the costimulatory agonist is an OX40 agonist. One or more recombinant cytokines from recombinant IL-2, IL-7, IL-15 and/or IL-21, also can be included during the incubation to initially expand T cells in a population of cells from a subject. In some embodiments, subsequent to or concurrently with incubation with the costimulatory agonist, the population of T cells also is contacted with a T cell stimulatory agent(s), such as recombinant IL-2 alone or in combination with one or more other recombinant cytokines (e.g. IL-7, IL-15 and/or IL-21) under conditions to induce or mediate proliferation of T cells in the population. In some embodiments, subsequent to or concurrently with incubation with the costimulatory agent, the population of T cells also is contacted with a T cell stimulatory agent(s), such as an anti-CD3 antibody (e.g. OKT3) or an anti-CD3/anti-CD28 stimulatory agent, e.g. as anti-CD3/anti-CD28 beads, under conditions to induce or mediate proliferation of T cells in the population. In some such aspects, the costimulatory agonist, such as a 4-1BB agonist or an OX40 agonist, provides an initial stimulation to enhance or boost the proliferative capacity and/or functional activity of T cells in the population.

In provided embodiments, the methods include *ex vivo* incubation of cells enriched for a population of T cells with an apoptosis inhibitor under conditions to reduce or prevent apoptosis of T cells in the sample. In particular embodiments, the apoptosis inhibitor is an inhibitor of the Fas/Fas ligand axis or is an inhibitor of caspase, both of which are involved in inducing apoptosis particularly of activated T cells. In particular embodiments, the apoptosis inhibitor is an inhibitor of one or more caspase (also called caspase inhibitor). As shown herein, caspase inhibitors are found herein to strikingly improve expansion potential of tumor-reactive T cells, particular from a patient tumor or when cells are activated under conditions that may be present in a tumor microenvironment. One or more recombinant cytokines from recombinant IL-2, IL-7, IL-15 and/or IL-21, also can be included during the incubation to initially expand T cells in a population of cells from a subject. In some embodiments, subsequent to or concurrently with incubation with the apoptosis inhibitor, the population of T cells also is contacted with a T cell stimulatory agent(s), such as recombinant IL-2 alone or in combination with one or more other recombinant cytokines (e.g. IL-7, IL-15 and/or IL-21) under conditions to induce or mediate proliferation of T cells in the population. In some embodiments, subsequent to or concurrently with incubation with the apoptosis inhibitor, the population of T cells also is contacted with a T cell stimulatory agent(s), such as an anti-CD3 antibody (e.g. OKT3) or an anti-CD3/anti-CD28 stimulatory agent, e.g. as anti-CD3/anti-CD28 beads, under conditions to induce or mediate proliferation of T cells in the population. In some such aspects, the apoptosis inhibitor protects the T cells from apoptosis thereby rejuvenating their potential of T cells in the population to proliferate and expand.

In particular embodiments, the T cell adjuvant, such as a costimulatory agonist, an apoptosis inhibitor, an immune checkpoint modulator, and/or heat shock protein inhibitor. is a soluble protein, such as a protein that is not bound or attached to a solid surface (e.g. a bead or other solid support). The T cell adjuvants can include small molecules, peptides or proteins. Among such T cell adjuvants are soluble ligands, antibody or antigen-binding fragments or other binding agents. In some embodiments, a costimulatory agonist can include a molecule that specifically binds to a costimulatory molecule, such as 4-1BB or OX40, to induce or stimulate a costimulatory signal in the cells. In some embodiments, an apoptosis inhibitor can include a molecule that specifically binds to a receptor that mediates or is involved in inducing apoptosis in a cell. In some embodiments, an immune check point modulator can include a molecule that specficially binds to a "check point" protein , such as PD1. In some embodiments, a heat shock protein inhibitor can include a molecule that specficially binds to a heat shock protein, such as Hsp90. In some embodiments, these molecules can be easily removed during the manufacturing process, such as by washing the cells in connection with cell manufacturing or prior to final formulation of the cells for administration.

In some embodiments of the provided methods a source of potential tumor peptides is used to identify TCRs that are reactive to neoantigens in a process that includes expansion of the T cells reactive to the tumor neoantigenic peptides. Provided methods include ex vivo co-culture methods in which a population of T cells that have been expanded from a biological sample (e.g. tumor fragments or peripheral blood or other source of T cells) is incubated in the presence of antigen-presenting cells that have been contacted with, or made to present, the neoantigenic peptides. In particular aspects, the T cells and antigen-presenting cells are autologous to the tumor-bearing subject from which the peptides were identified. The provided methods further include steps to separate, enrich for and/or select for tumor-reactive T cells from the co-culture prior to or in connection with their further ex vivo expansion.

**FIG. 1A** depicts a schematic of an exemplary process for manufacturing a T cell therapeutic composition in accord with the provided methods. In the exemplary process a tumor sample is obtained from a patient for identification and generation of peptides for use in co-culturing methods with antigen presenting cells (APCs) presenting the peptides and autologous antigen T cells obtained from the same subject. In some cases, a population of T cells from the patient, e.g. containing tumor infiltrating lymphocytes (TIL) or peripheral blood lymphocytes (PBL), is stimulated under conditions to expand the cells, prior to co-culture with antigen presenting cells that have been contacted or exposed to peptide neoepitopes for presentation on a major histocompability complex. Following co-culture under conditions in which the antigen presenting cells present peptides in the context of a major histocompatibility complex, tumor-reactive T cells or T cells surface positive for one or more T cell activation marker (also called an upregulation marker or reactive T cell marker, e.g. CD70a) associated with tumor reactive T cells can be selected and cultured under conditions for expansion in accord with the provided methods, such as incubation with a T cell stimulatory agent(s) (e.g. recombinant IL-2, anti-CD3 and/or anti-CD28). The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion. In provided examples, one or more of the steps are carried out in the presence of a T cell adjuvant, such as a costimulatory agonist, an apoptosis inhibitor, an immune checkpoint modulator, and/or heat shock protein inhibitor. **FIG. 1B** depicts an exemplary process in which a cryopreservation step can be carried out after one or more of the steps.

The provided methods offer advantages compared to existing methods for producing an expanding TILs because the provided methods involve steps to enrich for tumor reactive cells, such as by the co-culturing step with peptide-presenting APCs followed by selection of reactive T cell clones that have upregulated one or more T cell activation marker. By virtue of this process, the initial small population of tumor reactive T cells expanded from the biological sample (e.g. tumor) are enriched for cells that are or likely to be tumor reactive cells before a subsequent second expansion step, thereby promoting preservation and expansion of cells of interest and limiting expansion of bystander T cells that are not reactive to a tumor antigen and/or that may include cells that exhibit inhibitory activity **(****FIG. 2A****).** This is in contrast to existing methods that involve passive expansion of bulk T cells in which all T cells from a tumor are subjected to a first initial expansion, e.g. with high IL-2 concentrations, followed by a second rapid expansion of T cells present after the initial expansion. In such other methods, while total viable cells (TVC) can be greatly expanded by these alternative processes, there is no step of actively ensuring that tumor reactive T cells are predominantly propagated **(****FIG. 2B****).** Further, the provided methods are carried out to maximize the numbers of tumor reactive cells that may be collected, for example by co-culturing all of the cells propagated after the first expansion with peptide-presenting APCs, and then by selecting from among all of the bulk cells after the co-culturing for cells positive for the one or more activation markers before the subsequent second expansion. In aspects of the provided methods, all steps of the method are carried out in a closed system.

The provided methods include one or more features that provide for or relate to an improved, more efficient and/or more robust process for producing a tumor-reactive T cell therapeutic composition ex vivo. In particular, the disclosure relates to methods that provide advantages over available methods for producing a TIL therapeutic cell composition. Such advantages include, for example, reduced cost, streamlining, improved enrichment of tumor-reactive T cells in the therapeutic composition, and increased efficacy of the therapeutic composition, including among different subjects and tumor conditions.

Among the findings herein is the observation that lower concentrations of recombinant IL-2 can be employed during one or both expansion steps with success. Many existing methods use high concentrations of IL-2 of 6000 IU/mL for T cell expansion of TIL. However, high IL-2 concentrations can increase the cost of the process and may be limiting. In some cases, high IL-2 concentrations may lead to negative impacts on T cell differentiation by driving effector T cell differentiation over early memory T cells that may be more desirable in a therapeutic T cell composition. The provided methods can be carried out with concentrations that are several-fold lower than 6000 IU/mL, such as concentrations less than at or about 1000 IU/mL, for example from at or at about 300 IU/mL to at or about 1000 IU/mL. In particular embodiments, the concentration of IL-2 is at or about 300 IU/mL.

In embodiments of the provided methods, the population of T cells is obtained from a biological sample known to contain T cells. In some embodiments, the population of T cells is enriched from a biological sample from a subject, in particular a human subject. The biological sample can be any sample containing a bulk population of T cells. In some embodiments, the biological sample is or includes peripheral blood mononuclear cells. In some embodiments, the biological sample is a peripheral blood or serum sample. In some embodiments, the biological sample is a lymph node sample. In some embodiments, the biological sample is a tumor sample. In some aspects, the bulk T cells can include tumor-infiltrating T cells (TILs). In some embodiments, the subject is a human subject. In some embodiments the subject is a subject having a cancer, viral infection, bacterial infection, or is a subject with an inflammatory condition. In particular embodiments, the subject has a cancer.

In aspects of the provided methods, the starting source of cells in the method can be tumor fragments (e.g. 1-8 mm diameter fragments) or can be a single cell suspension preparation from enzymatic digestion of tumor fragments. It is found herein that, while certain sources may be superior for some tumor types, both fragments and single cell suspensions can support T cell expansion and enrichment of tumor-reactive T cells. In some cases, the tumor cell source can be chosen depending on the tumor type or cancer, such as to optimize or increase expansion and enrichment of tumor-reactive T cells from the tumor. In one example, the cancer is a melanoma and the starting population of lymphocytes are tumor fragments, such as from a resected tumor. In another example, the cancer is a colorectal cancer and the starting population of lymphocytes is a single cell suspension obtained by enzymatic digestion, e.g. collagenase, of tumor fragments.

In some embodiments, the methods include a step of co-culturing initially expanded T cells with autologous antigen presenting cells that have been loaded with peptide. Findings herein demonstrate that relatively low concentrations of peptide or a peptide pool (containing a plurality of peptides, e.g. 2, 3, 4, 5, 6, 7, 8, 9, 10, 20, 30, 40, 50, 60, 70, 80, 90 or 100 more, or any value between any of the foregoing), such where each individual peptide is less than 20 ng/mL, and even as low as 0.1 ng/mL, can lead to an increase in activation of T cell during the culture. In some embodiments, this can lead to an improved enrichment of tumor-reactive T cells in the co-culture prior to selection of cells positive for one or more T cell activation marker (i.e. upregulation marker or reactive T cell marker). In some embodiments, the co-culturing step in the provided methods include a ratio of tumor-derived cells containing T cells to autologous APCs (e.g. dendritic cells) of at or about 1:5 to at or about 5:1, such as 1:3 to at or about 3:1, for example as at or about 1:1, and involves loading the APCs with an individual peptide or a pool of peptides. In some embodiments, the APCs are loaded with a concentration of peptide or peptide pool in which the individual peptide, or individual peptides of the pool of peptides on average, is less than at or about 20 ng/mL, such as from at or about 0.1 ng/mL to at or about 1 ng/mL, for example at or about 0.1 ng/mL.

In some embodiments, the provided methods include enriching or selecting for a population of T cells from the biological sample. In some aspects, T cells or specific subpopulations of T cells, such as cells positive or expressing high levels of one or more surface markers, e.g., CD3+, CD4+ or CD8+ T cells, are isolated by positive or negative selection techniques. In some aspects, the enriched T cells are enriched or selected for CD4+ T cells. In some aspects, the enriched T cells are enriched or selected for CD8+ T cells. In some aspects, the enriched T cells are enriched or selected for CD4+ and CD8+ T cells. For example, CD4+ and CD8+ T cells can be positively selecting for bulk T cells that express CD3. Alternatively, CD4+ and CD8+ T cells can be selected separately, either simultaneously or sequentially in either order, by positive selection of a T cell subpopulation that express CD4 and positive selection of a T cell subpopulation that express CD8. Selection for CD4+ and CD8+ T cells ensures enrichment of T cells expressing MHC class II and MHC class I to provide for a T cell therapy that is a pan-tumor scanning target able to recognize a diverse repertoire of antigens, such as cancer antigens.

In some embodiments, the provided methods include enriching T cells, such as CD3+ T cells or a CD4 and/or CD8 subset thereof, further based on one or more marker that is whose expression is upregulated on (e.g. compared to resting or non-activated T cells) or specific to reactive or activated T cells (hereinafter "reactive T cell marker" or T cell activation marker). Reactive T cells will express certain reactive markers when their endogenous TCR recognizes an antigen on a target cell or tissue, such as when a TCR recognizes a neoantigen on the tumor. Exemplary reactive T cell markers include one or more, such as two, three, four or more of, CD107, CD107a, CD39, CD103, CD137 (4-1BB), CD59, CD90, CD38, CD30, CD154, CD252, CD134, CD258, CD256, PD-1, TIM-3 or LAG-3. The enrichment or selection for cells positive for one or more such reactive T cell marker can be carried out prior to or during one or more steps of the expansion method. In particular embodiments, the provided methods include enrichment or selection for cells positive for one or more upregulation marker on reactive or activated T cells after activation of a population of T cells by the co-culture incubation with peptide-presenting APCs (e.g. dendritic cells, DCs). In some embodiments, the step of selecting cells positive for one or more upregulation marker on reactive or activated T cells from the co-culture can result in 2-fold or greater enrichment of antigen-specific tumor-reactive T cells and/or a substantial decrease in TCR clonality evidencing enrichment of TCR clonotypes consistent with enrichment of tumor-reactive T cells. Furthermore, such enriched T cells can exhibit an improved ability to produce IFN-gamma following antigen-specific stimulation compared to non-selected T cells or bulk T cells from the co-culture.

In some embodiments, the methods produce or expand T cells for use in adoptive cell therapy methods for treating a disease or condition in which cells or tissue associated with the disease or condition is known or suspected of expressing an antigen target recognized by the T cells. In some embodiments, the T cell therapy is autologous to the subject. In some embodiments, the T cell therapy is allogeneic to the subject.

If a definition set forth herein is contrary to or otherwise inconsistent with a definition set forth in the patents, applications, published applications and other publications that are referenced, the definition set forth herein prevails over the definition that is in the reference.

The section headings used herein are for organizational purposes only and are not to be construed as limiting the subject matter described.

### I. Ex Vivo Expansion Methods

Provided herein is a method for the ex vivo expansion and production of a T cell therapeutic composition, particularly for use in connection with treating cancer. In some embodiments, the method of manufacturing involves the growth and manipulation of patient cells outside of the body. In particular embodiments, the methods relate to methods for expanding T cells containing an endogenous TCR specific to a tumor-associated antigen (hereinafter "tumor reactive T cells"). For purposes of this disclosure, reference to tumor reactive T cells includes T cells that exhibit reactivity to a tumor antigen or that are likely or suspected of being tumor reactive T cells due to upregulation or surface positive expression of a T cell activation marker. In some aspects, the frequency of these cells can be low and in order to expand these cells to a therapeutic dose ex vivo methods for enrichment and expansion are necessary.

Provided methods for expansion of tumor-reactive T cells involve a series of expansion steps to stimulate or induce proliferation of T cells in a population of T cells. In some cases, the methods including incubation with recombinant IL-2 alone or in combination with one or more other recombinant cytokines (e.g. IL-7, IL-21 and/or IL-15) and, in some cases, one or more other stimulatory T cell agents to provide a primary and secondary (costimulatory) signal to the cells. Additionally, in some cases, one or more T cell modulatory agents can be used including apoptosis and heat shock protein inhibitors and immune check point modulators. Standard methods for culturing T cells to provide a primary and secondary (costimulatory) signal to the cells, involve incubation with T cell stimulating agents provided by anti-CD3 (e.g. OKT3) and anti-CD28 reagents. In some embodiments, the T cell stimulatory agents include an anti-CD3 antibody (e.g. OKT3) and an anti-CD28 antibody. Typically such stimulations also include one or more additional recombinant cytokine (e.g. IL-2, IL-7, IL-21 and/or IL-15) and nutrient containing media so that the cells can survive outside of the body.

In the provided methods, the methods include culturing a population of T cells containing tumor reactive T cells ex vivo in which at least a portion of the culturing includes incubation with additional T adjuvants, including pharmaceutical agonists and in some cases inhibitors of apoptosis or heat shock protein mediated pathways. The addition of one or more T cell adjuvants to the manufacturing of T cells can increase the functionality of the T cells ex vivo and in-vivo. In connection with the provided methods, the methods further include enrichment of T cells containing an endogenous TCR specific to a tumor-associated antigen ("tumor reactive T cells") to maximize expansion of desired therapeutic cells. In some embodiments, the tumor-associated antigen is or includes a neoantigen.

Thus, among the provided methods are methods of culturing T cells for manufacture of tumor reactive T cells that involve both (1) the use of additional T cell modulatory agents, such as prior to or concurrently with standard T cell stimulatory agent(s) such as anti-CD3/anti-CD28 and/or recombinant cytokines (e.g. IL-2, IL-7, IL-21 and/or IL-15), and (2) further involve enrichment or selection of tumor reactive T cells or T cells that are surface positive for one or more T cell activation markers associated with tumor reactive T cells. It is contemplated that the provided methods can increase expansion to a therapeutic dose to a much greater extent than existing methods and/or increase functionality of the T cell therapy for therapeutic effect.

The provided methods involve collecting a biological sample from a subject that is known or likely to contain tumor reactive T cells. In some embodiments, the biological sample can be collected directly from a subject that has a tumor, in which, in some cases, such isolated or obtained T cells may have been co-cultured or exposed to a tumor in vivo. In embodiments of provided methods, a population containing T cells (hereinafter also called first population of T cells) is a population of T cells obtained, selected or isolated from the biological sample from a subject, such as a human subject. In some embodiments, the population containing T cells can be from any source sample that is known or suspected of containing T cells that are or that may include or potentially could include tumor reactive T cells. The sample can include a tumor sample containing tumor infiltrating lymphocytes (TILs), a blood sample (e.g. apheresis or leukapheresis sample) containing peripheral blood mononuclear cells (PBMCs) or a lymph node sample. In some embodiments, the sample is a tumor sample or a tumor fragment containing tumor infiltrating lymphocytes or TILs. The population containing T cells can be directly obtained from a subject (e.g. healthy or cancer subject), such as by selection of T cells or a subset thereof from the biological sample from the subject. In particular embodiments, the biological sample is from a subject that has a tumor and that contains tumor reactive T cells or that has the potential to or that may contain tumor reactive T cells that can be enriched by the provided methods.

Provided methods for expansion of tumor-reactive T cells involve a first expansion involving culturing the selected or isolated population containing T cells (i.e. the first population of T cells) with a recombinant cytokine from one or more of (e.g. IL-2, IL-7, IL-21 and/or IL-15), typically generally including recombinant IL-2. In some cases, the T cell stimulatory agent(s) tfurther provide a primary and secondary (costimulatory) signal to the cells, such as provided by anti-CD3 (e.g. OKT3) and anti-CD28 reagents, such as an anti-CD3 antibody (e.g. OKT3) and an anti-CD28 antibody.. In some cases, the first expansion also is carried out in the presence of one or more T cell modulatory agent, e.g. TNFSFR agonists and /or immune checkpoint modulators and/or apoptosis inhibitors and/or heat shock protein inhibitors as described. The initial or first expansion results in a second population of T cells that is enriched for T cells as a result of expansion or proliferation of T cells present in the first population.

In other embodiments of the provided methods, the biological sample can be collected and used for ex vivo co-culture in which T cells directly obtained from a subject (e.g. healthy or cancer subject) have been incubated with artificial presenting cells (APCs) under conditions in which the APCs have been induced to present one or more peptides from a tumor-associated antigen from the subject. The collected samples contain lymphocytes that have endogenous TCRs that are reactive to mutations present on the tumor. These cells can be identified via a variety of methods such as, but not limited to, co-culture in the presence of antigen presenting cells or co-culture with tumor. In the provided methods, tumor reactive T cells can be further identified or enriched from the stimulated T cells expanded in the first step by one or more further steps that include ex vivo co-culture of the stimulated or expanded T cells (i.e. second population of T cells) with antigen presenting cells and one or a plurality of peptides that include neoepitopes of a tumor antigen (APCs/peptide neoepitopes). Provided methods include ex vivo co-culture in which the second population of T cells are incubated with artificial presenting cells (APCs) that have been exposed to or contacted with one or more peptides, e.g. synthetic peptides, under conditions in which the APCs have been induced to present one or more peptides from a tumor-associated antigen. In some embodiments, the population T cells are autologous T cells from a subject with a tumor and the source of synthetic peptides are tumor antigenic peptides from a tumor antigen of the same subject. In some embodiments, cells from the ex vivo co-culture include population of T cells (in some cases a third population of T cells) that represent a source of cells that are enriched for tumor reactive T cells. In some cases, the co-culture of T cells with APCs and peptides can also be carried out in the presence of one or more T cell modulatory agent (e.g. TNFSFR agonist and/or apoptosis inhibitor).

In some cases, the tumor reactive T cells can be further enriched by separation or selection of cells that express one or more activation markers associated with tumor-reactive T cells. Among the provided aspects, a biological sample containing tumor-reactive T cells, including a sample obtained directly from a subject or a co-culture sample generated ex vivo, can be further enriched for tumor-reactive T cells or T cells that express one or more activation markers associated with tumor-reactive T cells. The T cell activation markers include cell surface markers whose expression is upregulated or specific to T cells that have been exposed to antigen and activated. Exemplary of such markers are described below. In provided aspects, a population of T cells containing tumor-reactive T cells is isolated, selected or enriched from a biological sample prior to or in connection with expanding such cells in accord with the provided methods. In some embodiments, the tumor-reactive T cells, or T cells that express certain activation markers associated with tumor-reactive T cells, are those present endogenously in a cancer subject known to have a tumor. In other embodiments, the tumor-reactive T cells, or T cells that express certain activation markers associated with tumor-reactive T cells, are obtained following an ex vivo co-culture of autologous T cells from a subject with a tumor with a source of tumor antigenic peptides under conditions in which tumor-reactive T cell can be generated and identified.

In particular embodiments, a second expansion is performed on T cells enriched or isolated from the co-culture, such as after separation or selection of tumor reactive T cells or T cells that are surface positive for one or more T cell activation markers associated with tumor reactive T cells. The second expansion involves incubation to further stimulate T cells with a T cell stimulatory agent(s), such as anti-CD3 antibody (e.g. OKT3), anti-CD28 antibody, and recombinant cytokine(s) (e.g. IL-2, IL-7, IL-21 and/or IL-15), and optionally one or more T cell modulatory agent (e.g. costimulatory agonists and /or immune checkpoint modulators and/or apoptosis inhibitors and/or heat shock protein inhibitors as described). The T cells, such as tumor reactive T cells or T cells that are surface positive for one or more T cell activation markers associated with tumor reactive T cells, are allowed to expand for a certain number of days as desired and/or until a therapeutic dose or harvest dose is met. The composition of expanded T cells can then be harvested and formulated for administration to a subject for treatment of a cancer in the subject.

Provided herein are methods for manufacturing tumor-reactive T cells in which cells are cultured ex vivo by a process that includes incubating a population of T cells containing, or that is likely or may contain, tumor-reactive T cells, said tumor-reactive T cells comprising an endogenous TCR that is reactive to a tumor-associated antigen, in the presence of at least one T cell adjuvant selected from a costimulatory agonist or an apoptosis inhibitor, wherein at least a portion of the incubation is carried out prior to, concurrently with, or subsequent to incubating the population of T cells with a T cell stimulatory agent(s), e.g. anti-CD3 antibody (e.g. OKT3), anti-CD28 antibody and/or a recombinant cytokine(s) (e.g.from IL-2, IL-7, IL-21 and/or IL-15), under conditions to stimulate expansion of cells of the population of T cells. The T cell adjuvant, e.g. costimulatory agonists and /or immune checkpoint modulators and/or apoptosis inhibitors and/or heat shock protein inhibitors as described, can be included during either one or both of the first expansion or the second expansion in accord with the provided methods. In some embodiments, the methods of culturing includes (a) incubating an ex vivo population of T cells comprising tumor-reactive T cells, said tumor-reactive T cells comprising an endogenous TCR that is reactive to a tumor-associated antigen, with at least one T cell adjuvant selected from a costimulatory agonist or an apoptosis inhibitor; and (b) incubating the population of T cells with a T cell stimulatory agent(s) under conditions to stimulate expansion of cells of the population of cells, wherein at least a portion of the incubating to stimulate or expand the T cells, e.g. in either one or both of the first or second expansion, is carried out prior to, concurrently with, or subsequent to the incubating with the at least one T cell adjuvant. In some embodiments, the population of T cells that is incubated in the presence of the at least one T cell adjuvant can be the first population of T cells following obtaining, selecting or isolating the population of T cells from a biological sample from a subject. In embodiments, the population of T cells is enriched for CD4+ and CD8+ T cells. In some cases, tumor-reactive T cells can be enriched from a biological sample comprising T cells prior to the incubating with the at least one T cell adjuvant and/or prior to the incubating with the at least one T cell stimulatory agent(s). In some embodiments, the population of T cells that is incubated in the presence of the at least one T cell adjuvant can be the population of T cells that has been enriched for or selected for tumor reactive T cells or T cells that express an activation marker following co-culture with APCs/peptide neoepitopes (e.g. in some cases the fourth population of T cells). In some embodiments, the method comprises culturing the cells under conditions for expansion until a threshold amount of cells is obtained and/or until up to 20 days after initiation of incubation with the at least one T cell adjuvant, wherein at least a portion of the culturing is carried out during the incubation with the one or more T cell adjuvant and/or the incubation with the T cell stimulatory agent(s).

Provided herein are methods for manufacturing tumor-reactive T cells in which cells are cultured ex vivo by a process that includes a step of enriching tumor-reactive T cells from a sample comprising T cells, said tumor-reactive T cells comprising an endogenous TCR that is reactive to a tumor-associated antigen, thereby producing a population of T cells enriched for tumor-reactive T cells; and incubating the population of T cells enriched for tumor-reactive T cells in the presence of at least one T cell adjuvant selected from a costimulatory agonist or an apoptosis inhibitor, wherein at least a portion of the incubation is carried out prior to, concurrently with, or subsequent to incubating the population of T cells with a T cell stimulatory agent(s), e.g. anti-CD3 antibody (e.g. OKT3), anti-CD28 antibody and/or recombinant cytokine(s) (e.g. IL-2, IL-7, IL-21 and/or IL-15), under conditions to stimulate expansion of cells of the population of T cells. In some embodiments, the tumor-reactive T cells are selected or isolated after co-culturing a population of T cells containing stimulated T cells (in some cases the second population of T cells) in the presence of antigen presenting cells (APCs) that have been contacted or exposed to one or more of the plurality of peptides under conditions in which the APCs present one or more MHC-associated non-native peptide. In some embodiments, the methods of culturing include (a) enriching tumor-reactive T cells from a biological sample comprising T cells, such as from a co-culture of T cells and APCs/neoepitope peptides, said tumor-reactive T cells comprising an endogenous TCR that is reactive to a tumor-associated antigen, thereby producing a population of T cells enriched for tumor-reactive T cells; (b) incubating the population of T cells enriched for tumor-reactive T cells with at least one T cell adjuvant selected from a costimulatory agonist or an apoptosis inhibitor; and (c) incubating the population of T cells enriched for tumor-reactive T cells with a T cell stimulatory agent(s) under conditions to stimulate expansion of cells of cells of the population of cells, wherein at least a portion of the incubating with the T cell stimulatory agent is carried out prior to, concurrently with, or subsequent to the incubating with the at least one T cell adjuvant. In embodiments, the population of T cells is enriched for CD4+ and CD8+ T cells. In some embodiments, the method comprises culturing the cells under conditions for expansion until a threshold amount of cells is obtained and/or until up to 20 days after initiation of incubation with the at least one T cell adjuvant, wherein at least a portion of the culturing is carried out during the incubation with the one or more T cell adjuvant and/or the incubation with the T cell stimulatory agent(s).

Provided herein is a method for manufacturing tumor reactive T cells in which cells are cultured ex vivo by a process that includes incubating a population of T cells with a T cell stimulatory agent(s), e.g. arecombinant cytokine(s) from amongIL-2, IL-7, IL-21 and/or IL-15, such as generally at least including recombinant IL-2, and at least one T cell adjuvant selected from a costimulatory agonist or an apoptosis inhibitor, in which the incubation is carried out under conditions to stimulate or expand T cells in the population to produce a second population of T cells; enriching tumor-reactive T cells from the population of T cells, said tumor-reactive T cells comprising an endogenous TCR that is reactive to a tumor-associated antigen, thereby producing a population of T cells enriched for tumor-reactive T cells; and incubating the population of T cells enriched for tumor-reactive T cells with a T cell stimulatory agent(s), e.g. anti-CD3 antibody (e.g. OKT3), anti-CD28 antibody and recombinant cytokine(s) (e.g. IL-2, IL-7, IL-21 and/or IL-15), under conditions to further stimulate expansion of cells of the population of cells enriched in tumor reactive T cells. In some embodiments, the tumor-reactive T cells are selected or isolated after co-culturing a population of T cells containing stimulated T cells (in some cases the second population of T cells) in the presence of antigen presenting cells (APCs) that have been contacted or exposed to one or more of the plurality of peptides under conditions in which the APCs present one or more MHC-associated non-native peptide. In embodiments, the population of T cells is enriched for CD4+ and CD8+ T cells. In some embodiments, the method comprises culturing the cells under conditions for expansion until a threshold amount of cells is obtained and/or until up to 20 days after initiation of incubation with the at least one T cell adjuvant, wherein at least a portion of the culturing is carried out during the incubation with the one or more T cell adjuvant and/or the incubation with the T cell stimulatory agent(s).

In particular embodiments, the provided methods include, but are not limited to the steps of (1) identifying, obtaining or generating a plurality of peptides that contain neoepitopes specific to a subject's tumor (2) obtaining a population of T cells obtained from a donor subject, such as from a resected tumor or by directly selecting T cells from a biological sample, e.g. a tumor, blood, bone marrow, lymph node, thymus or other tissue or fluids (first population of T cells); (3) performing a first expansion by stimulating or activating the first population of T cells with a T cell stimulatory agent(s), such as one or more recombinant cytokines from IL-2, IL-7, IL-21 and/or IL-15 (e.g. at least including recombinant IL-2), and optionally one or more further T cell modulatory agent, such as a TNFRSF agonist and/or apoptosis inhibitor to produce a second population of T cells containing expanded or stimulated T cells, (3) co-culturing the second population containing stimulated T cells in the presence of antigen presenting cells (APCs) that have been contacted or exposed to one or more of the plurality of peptides under conditions in which the APCs present one or more MHC-associated non-native peptide to produce a third population of T cells; and (5) enriching, from the third population of T cells, T cells containing an endogenous TCR that are reactive to peptides present on antigen presenting cells (APCs) to produce a fourth population of T cells. In some aspects, T cells containing an endogenous TCR are enriched by separating the antigen presenting cells from the population of T cells. Alternatively or additionally, such cells are enriched by selecting T cells that are surface positive for one or more activation markers associated with tumor-reactive T cells. In particular embodiments, a second expansion is performed on T cells enriched or isolated from the co-culture, such as after separation or selection of tumor reactive T cells or T cells that are surface positive for one or more T cell activation markers associated with tumor reactive T cells. The second expansion involves incubation to further stimulate T cells with a T cell stimulatory agent(s), such as anti-CD3 antibody (e.g. OKT3), anti-CD28 antibody and recombinant cytokine(s) (e.g. IL-2, IL-7, IL-21 and/or IL-15), and optionally one or more T cell modulatory agent (e.g. TNFSFR agonists and /or immune checkpoint modulators and/or apoptosis inhibitors and/or heat shock protein inhibitors ).

In embodiments of the provided methods, one or more of the steps can be carried out in serum-free media. In one embodiment, the serum free medium is OpTmizer CTS (LifeTech), Immunocult XF (Stemcell technologies), CellGro (CellGenix), TexMacs (Miltenyi), Stemline (Sigma), Xvivo15 (Lonza), PrimeXV (Irvine Scientific), or Stem XVivo (RandD systems). The serum-free medium can be supplemented with a serum substitute such as ICSR (immune cell serum replacement) from LifeTech. The level of serum substitute (e.g., ICSR) can be, e.g., up to 5%, e.g., about 1%, 2%, 3%, 4%, or 5%. In some embodiments, the serum-free media contains 0.5 mM to 5 mM of a dipeptide form of L-glutamine, such L-alanyl-L-glutamine (Glutamax^{™}). In some embodiments, the concentration of the dipeptide form of L-glutamine, such as L-alanyl-L-glutamine, is from or from about 0.5 mM to 5 mM, 0.5 mM to 4 mM, 0.5 mM to 3 mM, 0.5 mM to 2 mM, 0.5 mM to 1 mM, 1 mM to 5 mM, 1 mM to 4 mM, 1 mM to 3 mM, 1 mM to 2 mM, 2 mM to 5 mM, 2 mM to 4 mM, 2 mM to 3 mM, 3 mM to 5 mM, 3 mM to 4 mM or 4 mM to 5 mM, each inclusive. In some embodiments, the concentration of the dipeptide form of L-glutamine, such as L-alanyl-L-glutamine, is or is about 2 mM.

In embodiments of any of the provided methods, the incubation with each of the at least one T cell adjuvant, such as one or more costimulatory agonist, immune checkpoint modulator, heat shock protein inhibitor, or apoptosis inhibitor, is independently continued during the entire course of the culturing or during a portion thereof. In some embodiments, the incubation with each of the at least one T cell adjuvant is for no more than 14 days, no more than 12 days, no more than 10 days, no more than 7 days, no more than 5 days, no more than 3 days or no more than 2 days. In some embodiments, the incubation with each of the at least one T cell adjuvant is independently for 12 hours to 96 hours, such as 24 hours to 48 hours, and generally is at or about 48 hours.

In embodiments, the incubation with each of the T cell stimulatory agent(s), such as a recombinant cytokine (e.g. IL-2) and/or an anti-CD3/anti-CD28 antibody, can be continued for a time period sufficient to activate or stimulate the cells. In some embodiments, the incubation with the T cell stimulatory agent(s) is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days. In some embodiments, the incubation with each of the T cell stimulatory agent(s), such as a recombinant cytokine (e.g. IL-2) and/or anti-CD3/anti-CD28 antibody, is for 12 hours to 96 hours, such as 24 hours to 48 hours, and generally at or about 48 hours.

In some embodiments, the cells are washed one or more times during the culturing to remove agents present during the culturing and/or to replenish the culture medium with one or more additional agents. In some embodiments, the cells are washed during the cultureing to reduce or remove the at least one T cell adjuvant and/or at least one T cell stimulatory agent(s) prior to completion of the culturing.

In some embodiments, the methods of culturing T cells provided herein, including incubation with a T cell adjuvant and/or a T cell stimulatory agent(s), include temperature suitable for the growth of human T lymphocytes, for example, at least about 25 degrees Celsius, generally at least about 30 degrees, and generally at or about 37 degrees Celsius. In some embodiments, the methods of culturing, including incubation with a T cell adjuvant and/or a T cell stimulatory agent(s) is carried out in serum-free media.

In particular embodiments, the provided methods include enriching from a biological sample (directly sourced from a sample in vivo or from an ex vivo co-culture with antigen presenting cells (APCs) T cells that have an endogenous TCR that recognize tumor-associated antigens, e.g. neoantigens, such as by selecting for T cells that are surface positive for one or more T cell activation marker (e.g. CD107, CD107a, CD039, CD137, CD59, CD90, CD38, or CD103).

In some embodiments, any one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, the methods of culturing for expanding cells in accord with any of the provided methods is carried out until a threshold amount of cells, such as tumor-reactive cells or cells positive for one or more T cell activation marker, is obtained and/or until up to 20 days after initiation of the incubation with the at least one T cell adjuvant. In some embodiments, the culturing is carried out for 7 to 20 days, 7 to 14 days, 7 to 10 days, 10 to 20 days, 10 to 14 days or 14 to 20 days.

In some embodiments, the culturing is carried out until a threshold amount of cells is obtained in which the threshold amount is between at or about 0.5 x 10⁸ and at or about 50 x 10⁹ total cells or total viable cells, between at or about 0.5 x 10⁸ and at or about 30 x 10⁹ total cells or total viable cells, between 0.5 x 10⁸ and at or about 12 x 10⁹ total cells or total viable cells, between at or about 0.5 x 10⁸ and at or about 60 x 10⁸ total cells or total viable cells, between at or about 0.5 x 10⁸ and at or about 15 x 10⁸ total cells or total viable cells, between at or about 0.5 x 10⁸ and at or about 8 x 10⁸ total cells or total viable cells, between at or about 0.5 x 10⁸ and at or about 3.5x 10⁸ total cells or total viable cells, between at or about 0.5 x 10⁸ and at or about 1 x 10⁸ total cells or total viable cells, between 1 x 10⁸ and at or about 50 x 10⁹ total cells or total viable cells, between at or about 1 x 10⁸ and at or about 30 x 10⁹ total cells or total viable cells, between 1 x 10⁸ and at or about 12 x 10⁹ total cells or total viable cells, between at or about 1 x 10⁸ and at or about 60 x 10⁸ total cells or total viable cells, between at or about 1 x 10⁸ and at or about 15 x 10⁸ total cells or total viable cells, between at or about 1 x 10⁸ and at or about 8 x 10⁸ total cells or total viable cells, between at or about 1 x 10⁸ and at or about 3.5x 10⁸ total cells or total viable cells, between at or about 3.5 x 10⁸ and at or about 50 x 10⁹ total cells or total viable cells, between at or about 3.5 x 10⁸ and at or about 30 x 10⁹ total cells or total viable cells, between at or about 3.5 x 10⁸ and at or about 12 x 10⁹ total cells or total viable cells, between at or about 3.5 x 10⁸ and at or about 60 x 10⁸ total cells or total viable cells, between at or about 3.5 x 10⁸ and at or about 15 x 10⁸ total cells or total viable cells, between at or about 3.5 x 10⁸ and at or about 8 x 10⁸ total cells or total viable cells, between at or about 8 x 10⁸ and at or about 50 x 10⁹ total cells or total viable cells, between at or about 8 x 10⁸ and at or about 30 x 10⁹ total cells or total viable cells, between at or about 8 x 10⁸ and at or about 12 x 10⁹ total cells or total viable cells, between at or about 8 x 10⁸ and at or about 60 x 10⁸ total cells or total viable cells, between at or about 8 x 10⁸ and at or about 15 x 10⁸ total cells or total viable cells, between at or about 15 x 10⁸ and at or about 50 x 10⁹ total cells or total viable cells, between at or about 15 x 10⁸ and at or about 30 x 10⁹ total cells or total viable cells, between at or about 15 x 10⁸ and at or about 12 x 10⁹ total cells or total viable cells, between at or about 15 x 10⁸ and at or about 60 x 10⁸ total cells or total viable cells, between at or about 60 x 10⁸ and at or about 50 x 10⁹ total cells or total viable cells, between at or about 60 x 10⁸ and at or about 30 x 10⁹ total cells or total viable cells, between at or about 60 x 10⁸ and at or about 12 x 10⁹ total cells or total viable cells, between at or about 12 x 10⁹ and at or about 50 x 10⁹ total cells or total viable cells, between at or about 12 x 10⁹ and at or about 30 x 10⁹ total cells or total viable cells, or between at or about 30 x 10⁹ and at or about 60 x 10⁹ total cells or total viable cells, each inclusive.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more.

Non-limiting descriptions of aspects of the provided methods are further described in the following subsections.

### A. Neoepitope Identification and Peptide Generation

The provided methods include a step of generating or identifying in silico a plurality of peptides (also referred to as "P" or "n-mers") that contain at least one cancer-specific cancer neoepitope, and a further step of filtering in silico the peptides to so obtain a subset of neoepitope sequences. In some embodiments, at least one synthetic peptide is prepared using sequence information from the subset of neoepitope sequences, and the synthetic peptide is then employed in methods to enrich for tumor-reactive T cells in accord with the provided methods.

In some embodiments, methods for ex-vivo generation of tumor-reactive T cells include identifying or isolating a tumor-associated antigen or peptide sequence thereof from a cancer cell from a subject. The cancer cell may be obtained from any bodily sample derived from a patient which contains or is expected to contain tumor or cancer cells. The bodily sample may be any tissue sample such as blood, a tissue sample obtained from the primary tumor or from tumor metastases, a lymph node sample or any other sample containing tumor or cancer cells. In some aspects, nucleic acid from such cancer cells is obtained and sequenced. In embodiments, the protein-coding region of genes in a genome is sequenced, such as by whole exome sequencing. To identify tumor-specific sequences, sequencing data can be compared to a reference sequencing data, such as data obtaining by sequencing a normal cell or noncancerous cell from the same subject. In some embodiments, next-generation sequencing (NGS) methods are used.

In some embodiments, the tumor is a hematological tumor. Non- limiting examples of hematological tumors include leukemias, including acute leukemias (such as l lq23- positive acute leukemia, acute lymphocytic leukemia, acute myelocytic leukemia, acute myelogenous leukemia and myeloblastic, promyelocytic, myelomonocytic, monocytic and erythroleukemia), chronic leukemias such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some embodiments, the tumor is a solid tumor. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some embodiments, the cancer is a gastrointestinal cancer involving a cancer of the gastrointestinal tract (GI tract), including cancers or the upper or lower digestive tract, or an accessory organ of digestion, such as esophagus, stomach, biliary system, pancreas, small intestine, large intestine, rectum or anus. In some embodiments, the cancer is an espphageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular embodiments, the cancer is a colorectal cancer.

In some embodiments, the tumor is from a breast cancer, such as a ductal carcinoma or a lobular carcinoma. In some embodiments, the tumor is from a prostate cancer. In some embodiments, tumor is from a skin cancer, such as a basal cell carcinoma, a squamous cell carcinoma, a Kaposi's sarcoma, or a melanoma. In some embodiments, the tumor is from a lung cancer, such as an adenocarcinoma, a bronchiolaveolar carcinoma, a large cell carcinoma, or a small cell carcinoma. In some embodiments, the tumor is from a brain cancer, such as a glioblastoma or a meningioma. In some embodiments, the tumor is from a gastrointestinal cancer, such as any described above. In some embodiments, the tumor is from a colon cancer. In some embodiments, the tumor is from a liver cancer, such as a hepatocellular carcinoma. In some embodiments, the tumor is from a pancreatic cancer. In some embodiments, the tumor is from a kidney cancer, such as a renal cell carcinoma. In some embodiments, the tumor is from a testicular cancer.

In some embodiments, the cancer is not a melanoma. Melanoma is a cancer that generally has a high mutational rate. High tumor mutation burden has been thought to be a particularly desired prognostic marker for success related to treatment with an immunotherapy targeting tumor neoantigens (Simpson et al., Journal of Clinical Oncology 2017, 35:15 suppl, 9567-9567*;* McGranahan et al. Science 2016, 351:1463-1469*)* In some embodiments, the provided methods can be used in cancers that have a lower tumor mutation burden, since the methods are carried out to actively (as opposed to passively) enrich for tumor reactive T cells.

In some embodiments, the subject is a subject with a tumor mutational burden (TMB) of less than 8 mutations.TMB includes the number of non-synomymous mutations per tumor. In some embodiments, TMB can be calculated by counting the number of synonymous and non-synonymous mutations across a 0.8- to 1.2-megabase (Mb) region, and reporting the result as mutations/Mb. In some embodiments, TMB can be determined by next generation sequencing (NGS) on tumor tissue samples. In some cases, whole exome sequencing can be used or computational germline status filtering can be used (Chalmers et al. Genome Med 2017 9:34). In some embodiments, the subject has a TMB of less than at or about 60 mutations/Mb, such as less than at or about 55 mutations/Mb, less than at or about 50 mutations/Mb, less than at or about 45 mutations/Mb, less than at or about 40 mutations/Mb, less then at or about 30 mutations/Mb, less than at or about 25 mutations per Mb, or less than at or about 20 mutations/Mb, or any value between any of the foregoing. In some embodiments, the subject has a TMB of less than at or about 41 mutations/Mb, less than at or about 40 mutations/Mb, less than at or about 39 mutations/Mb, less than at or about 38 mutations/Mb, less than at or about 37 mutations/Mb or less.

In some embodiments, the peptide (P) is a tumor-associated antigen derived from premalignant conditions, such as variants of carcinoma in situ, or vulvar intraepithelial neoplasia, cervical intraepithelial neoplasia, or vaginal intraepithelial neoplasia.

In some aspects, nucleic acid from such cells of the tumor or cancer is obtained and sequenced. In embodiments, the protein-coding region of genes in a genome is obtained, such as by omics analysis, such as by analysis of whole genomic sequencing data, exome sequencing data, and/or transcriptome data. To identify tumor-specific sequences, sequencing data can be compared to a reference sequencing data, such as data obtaining from a normal cell or noncancerous cell from the same subject. In some embodiments, next-generation sequencing (NGS) methods are used.

In some embodiments, the methods include a step of using matched normal omics data of a tumor. In such methods, the in silico analysis involves an omics analysis to identify mutations in the tumor relative to normal tissue of the same patient, such as non-diseased tissue of the same patient. It is generally contemplated that matched normal omics data are whole genomic sequencing data, exome sequencing data, and/or transcriptome data, and that the matched normal omics data are matched against normal before treatment of the patient. In a particular embodiment, whole exome sequencing is performed on healthy and diseased tissue to identify somatic mutations associated with the tumor.

In some embodiments, omics data are obtained from one or more patient biopsy samples following standard tissue processing protocol and sequencing protocols. In particular embodiments, the data are patient matched tumor data (e.g., tumor versus same patient normal). In some cases, non- matched or matched versus other reference (e.g., prior same patient normal or prior same patient tumor, or homo statisticus) are also deemed suitable for use herein. The omics data may be fresh omics data or omics data that were obtained from a prior procedure (or even different patient). For example, neoepitopes may be identified from a patient tumor in a first step by whole genome and/or exome analysis of a tumor biopsy (or lymph biopsy or biopsy of a metastatic site) and matched normal tissue (i.e., non-diseased tissue from the same patient such as peripheral blood). In some embodiments, genomic analysis can be processed via location-guided synchronous comparison of the so obtained omics information.

The genomic analysis can be performed by any number of analytic methods. In particular embodiments, the methods include WGS (whole genome sequencing) and exome sequencing of both tumor and matched normal sample using next generation sequencing such as massively parallel sequencing methods, ion torrent sequencing, pyrosequencing. Computational analysis of the sequence data may be performed in numerous manners. In some embodiments, the data format is in SAM, BAM, GAR, or VCF format. As an example, analysis can be performed in silico by location-guided synchronous alignment of tumor and normal samples as, for example, disclosed in US 2012/0059670A1 and US 2012/0066001 Al using BAM files and BAM servers. Alternative file formats for sequence analysis (e.g., SAM, GAR, FASTA, etc.) are also contemplated.

In some of any embodiments, peptides (P) comprising neoantigens arising from a missense mutation encompass the amino acid change encoded by 1 or more nucleotide polymorphisms. Peptides (P) comprising neoantigens that arise from frameshift mutations, splice site variants, insertions, inversions and deletions should encompass the novel peptide sequences and junctions of novel peptide sequences. Peptides (P) comprising neoantigens with novel post-translational modifications should encompass the amino acids bearing the post-translational modification(s), such as a phosphate or glycan.

Once these mutations are identified, neoepitopes are then identified. Neoepitopes are mutant peptides that are recognized by a patient's T cells. These neoepitopes must be presented by a tumor or antigen presenting cell by the MHC complex and then be recognized by a TCR on the T cell. In some embodiments, the provided methods include a step of calculation of one or more neoepitopes to define neoepitopes that are specific to the tumor and patient. Consequently, it should be recognized that patient and cancer specific neoepitopes can be identified from omics information in an exclusively in silico environment that ultimately predicts potential epitopes that are unique to the patient and tumor type. In particular aspects, the so identified cancer neoepitopes are unique to the patient and the particular cancer in the patient (e.g., having a frequency of less than 0.1% of all neoepitopes, and more typically less than 0.01% in a population of cancer patients diagnosed with the same cancer), but that the so identified cancer neoepitopes have a high likelihood of being presented in a tumor.

In some of any embodiments, the length of the peptide (P) depends on the specific application and is typically between about 5 to about 50 amino acids. In preferred embodiments, the peptide (P) is between about 7 to 35 amino acids, e.g., 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids. In some aspects, the methods can be carried out with an individual peptide that includes a change(s) (e.g. mutations) in the amino acid sequences. In some aspects, the methods can be carried out with a pool of peptides, where peptides of the pool contain a change(s) (e.g. mutations) in the amino acid sequences. The pool of peptides can include tens to hundreds of individual peptides. In some cases, the pool of peptides includes 2, 3, 4, 5, 6, 7, 8, 9, 10, 11, 12, 13, 14, 15, 16, 17, 18, 19, 20, 30, 40, 50, 60, 70, 80, 90, 100 or more individual peptides, or any value between any of the foregoing. The pool of peptides can represent one neo-antigen or can represent several neoantigens. In some cases, a pool of peptides can include multiple overlapping peptides of the same neo-antigen. Thus, for a tumor-associated antigen, the antigen may be divided into 7 to 35 amino acid, e.g., 25 amino acid, peptides (P) wherein each peptide (P) contains a unique composition of amino acids; or, the peptides (P) can be overlapping peptide pools wherein an antigen is divided into a set number of 7 to 35 amino acid, e.g., 25 amino acid, peptides (P) that have overlapping sequences. For example, an overlapping peptide pool comprising a 100 amino acid antigen may be divided into eight 25 amino acid peptides (P) that are each offset by 12 amino acids (i.e., each subsequent 25 amino acid peptide comprising a 100 amino acid peptide sequence starts at the 13^{th} amino acid position from the prior peptide). Those skilled in the art understand that many permutations exist for generating a peptide pool from an antigen.

The neoepitope sequences as contemplated herein can be defined as sequence stretches with relatively short length (e.g., 5-30 mers, more typically 7- 11 mers, or 12-25 mers) wherein such stretches include the change(s) (e.g. mutations) in the amino acid sequences. Most typically, the change(s) is/are located centrally or near the center (e.g., less than 4, or less than 5, or less than 6 amino acids from center position). In particular aspects, neoepitope sequences contemplated herein will especially include those in which a single amino acid is exchanged relative to the matched normal sequence, and in which the position of the changed amino acid is centrally located, or near the center of the neoepitope sequence (e.g., in a 9-mer, the changed amino acid is at position 2, 3, 4, or 5, and more typically at position 3, 4, or 5, and most typically at position 4 or 5). It should be appreciated that a single amino acid change may be presented in numerous neoepitope sequences that include the changed amino acid, depending on the position of the changed amino acid.

In particular embodiments, neoepitopes will be calculated to have a length of between 2-50 amino acids, more typically between 5-30 amino acids, and most typically between 9- 15 amino acids. For example, where the epitope is to be presented by the MHC-I complex, a typical epitope length will be about 8- 11 amino acids, while the typical epitope length for presentation via MHC-II complex will have a length of about 13- 17 amino acids. As will be readily appreciated, since the position of the changed amino acid in the neoepitope may be other than central, the actual peptide sequence and with that actual topology of the neoepitope may vary considerably. Moreover, where the neoepitope is presented to an immune competent (or other) cell as a synthetic peptide, it should be appreciated that the synthetic peptide may be significantly longer than the peptide portion that is ultimately bound by the MHC-I or MHC-II system to so allow for proteolytic processing in the cell. For example, contemplated synthetic peptides may therefore have between 8 and 15 amino acids upstream and downstream of the changed amino acid.

Various algorithms have been developed and can be used to map T cell epitopes (both MHC Class I and Class II-restricted) within protein molecules of various origins. In some embodiments, many programs utilize availability of the large-scale peptide-MHC binding affinity matrix from experimental measurements, to train machine learning (ML)-based classifiers to distinguish MHC-binders from non-binders (see e.g., Zhao et al. (2018) PLoS Comput Biol 14(11): e1006457)*.* Exemplary predictor methods for MHC class I (e.g. 9-mer) include *smm,* smmpmbec, ann (NetMHC3.4), NetMHC4, PickPocket, consensus, NetMHCpan2.8, NetMHCpan3, NetMHCpan4, NetMHCcons, mhcflurry, mheflurry_pan, or MixMHCpred. Exemplary predictor methods for MHC class II (e.g. 15-mer ) include NetMHCIIpan, NetMHCII2.3, nn_align, smm_align, consensus, comblib, tepitope, or mhcflurry. Any of such methods can be used.

In embodiments where the synthetic peptide is used for direct MHC-I binding, the overall length will be between 8 and 10 amino acids. In embodiments, where the synthetic peptide is used for direct MHC-II binding, the overall length will be between 12 and 25 amino acids, such as between 14 and 20 amino acids. In some cases, where the synthetic peptide is processed in the cell (typically via proteasome processing) prior to MHC presentation, the overall length will typically be between 10 and 40 amino acids, with the changed amino at or near a central position in the synthetic peptide. In some embodiments, a peptide for MHC-I binding is a 9-mer. In some embodiments, a peptide for MHC-II binding is a 23-mer. In some embodiments, a peptide for MHC-II binding is a 25-mer.

As an example, a peptide (P) can include 0-25 amino acids on either side flanking the amino acid change or novel junction that arises due to a mutation. In one embodiment, the peptide (P) is a neoantigen sequence that comprises the 12 amino acids on either side flanking the amino acid change that arises from a single nucleotide polymorphism, for example, a 25 amino acid peptide, wherein the 13^{th} amino acid is the amino acid residue resulting from the single nucleotide polymorphism. In some embodiments, the peptide (P) is a neoantigen sequence that comprises the 12 amino acids on either side flanking an amino acid with a novel post-translational modification, for example, a 25 amino acid peptide, wherein the 13^{th} amino acid is the amino acid residue resulting from the novel post- translational modification site. In other embodiments, the peptide (P) is a neoantigen sequence that comprises 0-12 amino acids on either side flanking a novel junction created by an insertion, deletion or inversion. In some cases, the peptide (P) comprising neoantigens resulting from novel sequences can encompass the entire novel sequence, including 0-25 amino acids on either side of novel junctions that may also arise.

In some embodiments, further downstream analysis may be performed on the so identified sequence differences to identify those that lead to a new peptide sequence based on the cancer and patient specific mutation. Neoepitopes may therefore be identified by considering the type (e.g., deletion, insertion, transversion, transition, translocation) and impact of the mutation (e.g., non-sense, missense, frame shift, etc.), and may as such serve as a content filter through which silent and other non-relevant (e.g., non-expressed) mutations are eliminated.

In some embodiments, identified neoepitopes can be further filtered in silico against an identified patient HLA- type. Such HLA-matching is thought to ensure strong binding of the neoepitopes to the MHC-I complex of nucleated cells and the MHC-II complex of specific antigen presenting cells. Targeting both antigen presentation systems is particularly thought to produce a therapeutically effective and durable immune response involving both, the cellular and the humoral branch of the immune system. It should also be appreciated that thusly identified HLA-matched neoepitopes can be biochemically validated in vitro.

HLA determination for both MHC-I and MHC-II can be done using various methods. In some embodiments, the HLA-type can be predicted from omics data in silico using a reference sequence containing most or all of the known and/or common HLA-types. For example, a patient' s HLA-type is ascertained (using wet chemistry or in silico determination), and a structural solution for the HLA-type is calculated or obtained from a database, which is then used as a docking model in silico to determine binding affinity of the neoepitope to the HLA structural solution. Suitable systems for determination of binding affinities include the NetMHC platform (see e.g., Nucleic Acids Res. 2008 Jul 1; 36(Web Server issue): W509-W512.), HLAMatchmaker (http://www.epitopes.net/downloads.html), and IEDB Analysis Resource (http://tools.immuneepitope.org/ mhcii/). Neoepitopes with high affinity (e.g., less than 100 nM, less than 75 nM, less than 50 nM for MHC-I; less than 500 nM, less than 300 nM, less than 100 nM for MHC-II) against the previously determined HLA-type are then selected. In calculating the highest affinity, modifications to the neoepitopes may be implemented by adding N- and/or C-terminal modifications to the epitope to further increase binding of a synthetic neoepitope to the HLA-type of the patient. Thus, neoepitopes may be native as identified or further modified to better match a particular HLA-type. In some embodiments, neoepitopes can be scored/ranked based on allele frequency multiplied by the transcripts per million number to get a likelihood score. This score can then be further augmented using HLA information and calculated or actual binding affinity to the patient' s HLA type.

Among provided embodiments are embodiments in which the neoepitopes are compared against a database that contains known human sequences to so avoid use of a human-identical sequence.

After the in silico identification of suitable neoepitope sequences, corresponding synthetic peptides are then prepared in vitro (e.g. , using solid phase synthesis). In particular embodiments, a library of synthetic peptides is prepared representing a plurality of different neoepitopes from the subject. The library can include 100, 1000, 10000 or more different peptides. To obtain a synthetic antibody against the identified neoepitope(s), it is contemplated that the in silico identified is prepared in vitro to yield a synthetic peptide.

Various methods can be used to prepare synthetic peptides. For example, peptides with cancer neoepitope sequences can be prepared on a solid phase (e.g., using Merrified synthesis), via liquid phase synthesis, or from smaller peptide fragments. Peptide epitopes can be obtained by chemical synthesis using a commercially available automated peptide synthesizer. In some embodiments, the peptides can be synthesized, for example, by using the Fmoc-polyamide mode of solid-phase peptide synthesis which is disclosed by Lu et al (1981).J. Org. Chem. 46,3433 and the references therein. In some aspects, peptides can be produced by expression of a recombinant nucleic acid in a suitable host and with a suitable expression system. In some aspects, recombinant methods can be used where multiple neoepitopes are on a single peptide chain, such as with spacers between neoepitopes or cleavage sites).

The peptides can be purified by any one, or a combination of techniques such as recrystallization, size exclusion chromatography, ion-exchange chromatography, hydrophobic interaction chromatography, and reverse-phase high performance liquid chromatography using e.g. acetonitrile/water gradient separation. In some embodiments, peptides can be precipitated and further purified, for example by high performance liquid chromatography (HPLC). Analysis of peptides can be carried out using thin layer chromatography, electrophoresis, in particular capillary electrophoresis, solid phase extraction (CSPE), reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

### B. Cell Samples Containing Tumor-Reactive T cells

The provided methods include obtaining and enriching or selecting a population of T cells from a biological sample for use as a first or input population of T cells. In some cases, the first population of T cells is one that is known or likely to contain T cells reactive to a tumor antigen or that are capable of being reactive to a tumor antigen, such as following an ex vivo co-culture with an autologous source of tumor antigen. For example, typically the first population of T cells is from a biological sample from a tumor or from a subject known or likely to have a tumor. In particular embodiments, the first population of T cells is further stimulated with one or more T cell stimulatory agent(s) (e.g. one or more recombinant cytokines, such as IL-2) and, in some cases one or more T cell adjuvant, to produce a second or stimulated population of T cells containing expanding T cells.

In some cases, conditions for stimulating the T cells by culture with one or more T cell stimulatory agent(s), and in some cases one or more T cell adjuvant, results in expansion or outgrowth of T cells present in the first or input population of T cells. In some embodiments, the conditions for stimulating the T cells with one or more T cell stimulatory agent(s), and in some cases one or more T cell adjuvant, can include culturing the T cells under condition that results in bulk expansion of the T cells. In other particular embodiments, conditions for stimulating the T cells may include culturing the T cells under conditions that are carried out to result in preferential or favored enrichment or outgrowth of desired T cells while minimizing or reducing certain T cell subsets that may not be desired.

In the provided methods, the stimulated composition of T cells is then employed in subsequent downstream steps for enrichment and expansion of tumor reactive T cells, including steps that include co-culture of the stimulated T cells with antigen presenting cells (APCs) in the presence of T cell neopitope (mutated) peptide antigens to produce, yield or to pull out T cells that are tumor reactive T cells. In particular embodiments, the provided methods also can include a step for selecting or enriching T cells reactive to a tumor antigen (tumor reactive T cells), after co-culturing T cells with APCs/peptide neoepitopes. The tumor reactive T cell populations can be cultured under conditions for expansion, such as to produce a therapeutic T cell composition.

In some embodiments, a biological sample is a sample from a subject having a tumor that is known to or is likely to contain tumor-reactive T cells for which such T cells have been exposed to or activated by a tumor neoantigen in vivo. In some embodiments, selecting the T cells from the biological sample further includes enriching or selecting for tumor-reactive T cells or T cells that express one or more activation markers associated with tumor-reactive T cells. The T cell activation markers include cell surface markers whose expression is upregulated or specific to T cells that have been exposed to antigen and activated. Exemplary markers are described in Section I.D below.

In aspects of any of the provided methods, the input or first population of T cells is incubated in the presence of a T cell stimulatory agent(s). In particular embodiments, the incubation is carried out under conditions in which the T cell stimulatory agent(s) activates or stimulates the cells or promotes expansion of T cells present in the input or first population of T cells.

In some embodiments, the T cell stimulatory agent(s) include a recombinant T cell stimulating cytokine, such as IL-2, IL-7, IL-15 and/or IL-21. In some embodiments, the T cell stimulating cytokine includes IL-2, alone or in combination with another cytokine from among IL-7, IL-15 and/or IL-21. In some embodiments, the T cell stimulating cytokines are IL-7 and IL-15.

In some embodiments, the T cell stimulatory (agent(s) can include an agent or agents that engage CD3 and a costimulatory molecule, such as CD28. The T cell stimulatory agent(s) can include an anti-CD3 antibody, such as OKT3, and an anti-CD28 agent (presented by APC's or as a soluble antibody. In embodiments, prior to and/or during at least a portion of the co-culturing of T cells with APCs, the T cells selected from the biological sample (i.e. input or first population) are incubated in the presence of a T cell stimulatory agent(s), such as an anti-CD3 (e.g. OKT3)/anti-CD28 antibody. Thus, either before the co-culture in the presence of APCs or after the selection of reactive cells, the T cells are incubated with one or more T-cell stimulating agents of lymphocytes, such as but not limited to anti-CD3 antibody (e.g. OKT3) and anti-CD28 (presented by APC's or as soluble antibodies), to produce a second population of T cells that include activated or stimulated T cells. In particular embodiments, one or more recombinant cytokines also are present as additional T cell stimulatory agents during the incubation.

In some embodiments, the incubation with the T cell stimulatory agent(s) is carried out directly on an input or first population of T cells selected from a biological sample from a subject, wherein the population of T cells selected from the biological sample (e.g. autologous T cells from the subject) is incubated with the T cell stimulatory agent(s). In other embodiments, the input population of T cells includes T cells that are likely to be or are suspected to be tumor reactive T cells, in which such cells are first selected from a population of T cells selected from a biological sample from a subject by selecting for cells positive for a surface marker that is upregulated on activated T cells (e.g. 4-1BB or OX40). In such embodiments, the incubation with the T cell stimulatory agent(s) is carried out after the enriching for the population of T cell cells comprising tumor-reactive T cells. In the provided embodiments, the incubation with the T cell stimulatory agent(s) is carried out before the co-culturing of such T cells (stimulated T cells) with the APCs/peptide neoepitopes.

### 1. Selecting a Population of T cells

The provided methods include selecting or obtaining a population of T cells from a biological sample, which can be used as the source or input of T cells for stimulation with one or more T cell stimulatory agents(s) (e.g. recombinant IL-1 and/or anti-CD3/anti-CD28) and, in provided embodiments, also a T cell modulatory agent (e.g. T cell agonist or apoptosis inhibitor). In some embodiments, the T cells are from a biological sample from a subject that is known or likely to contain tumor reactive T cells. The collected biological sample contains or is suspected to contain lymphocytes that have endogenous TCRs that are reactive to mutations present on a tumor.

In aspects of any of the provided embodiments, a suitable biological sample from a subject, such as from a patient of interest, i.e., a patient suspected of having or known to have cancer, is obtained. In some embodiments, the sample is one that is known or suspected of containing T cells, such as T cells that may be or may likely express an endogenous T cell receptor (TCR) specific to, binds or recognizes a tumor-associated antigen. The sample may be derived from any initial source that would contain or is suspected of containing such T cells. In some aspects, biological sample sources of interest include, but are not limited to, many different physiological sources, e.g. tissue derived samples, e.g. homogenates, and blood or derivatives thereof.

Any of a variety of samples can be used as a source of potentially reactive T cells. Although the tumor and downstream lymph nodes may have the highest frequency of reactive T cells (Powell et al., Clin. Cancer. Res., 2014), other sample sources also can be used. In some cases the sample is a tumor sample, a tertiary lymphoid site, a draining lymph node, peripheral blood or bone marrow. In some embodiments, the sample is a tumor sample. In some embodiments, the sample is a lymph sample. In some embodiments, the sample is a peripheral blood sample.

The samples include tissue, fluid, and other samples taken directly from the subject, as well as samples resulting from one or more processing steps, such as separation, e.g. selection or enrichment, centrifugation, washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In many embodiments, the sample may be derived from fluids in which the T cells of interest are at least suspected of being present. In many embodiments, a suitable initial source for the sample is blood. In some embodiments, the biological sample is a blood-derived sample. The blood-derived sample may be derived from whole blood or a fraction thereof, e.g. serum, plasma, etc., where in many embodiments the sample is derived from blood cells harvested from whole blood. In some aspects, the sample source contains mononuclear cells. For example, a biological sample is or contains peripheral blood mononuclear cells (PBMCs) or is derived from PBMCs.

In some embodiments in which the sample is a PBMC derived sample, the sample is generally a fluid PBMC derived sample. Any convenient methodology for producing a fluid PBMC sample may be employed. In many embodiments, the fluid PBMC derived sample is prepared by separating PBMCs from whole blood, i.e., collecting PBMCs, e.g., by centrifugation (such as by Ficoll-Hypaque density gradient centrifugation, where representative protocols for such separation procedures are disclosed in WO 98/15646 and U.S. Pat. No. 5,985,565).

In some embodiments, the sample is a tumor sample and thereby provides a source of tumor-infiltrating lymphocytes (TILs). In some aspects, TILs are T cells that have left the bloodstream of a subject and migrated into or infiltrated a tumor. In particular aspects, TILs are reactive to a tumor antigen.

A patient tumor sample may be obtained by any of a variety of methods in which the method obtains a sample that contains a mixture of tumor and TIL cells. In some embodiments, the tumor sample is obtained by surgical resection. In some embodiments, the tumor sample is obtained by needle biopsy. In general, the tumor sample may be from any solid tumor, including primary tumors, invasive tumors or metastatic tumors. The tumor sample may also be a liquid tumor, such as a tumor obtained from a hematological malignancy. The solid tumor may be of any cancer type, including, but not limited to, breast, pancreatic, prostate, colorectal, lung, brain, renal, stomach (gastrointestinal), and skin (including but not limited to squamous cell carcinoma, basal cell carcinoma, and melanoma). In particular embodiments, the tumor is any as described in Section IV. In some embodiments, the tumor sample is from the same tumor source as was used to identify a neoantigen for preparing peptide neoepitopes.

In provided embodiments, the obtained tumor sample is fragmented into small pieces of between at or about 1 mm³ and at or about 8 mm³ in size, such as between at or about 1 mm³ and at or about 6 mm³, between at or about 1 mm³ and at or about 4 mm³, between at or about 1 mm³ and at or about 2 mm³. In some embodiments, the tumor fragment is from about 2-3 mm³. In some embodiments, the tumor fragment is from about 1-2 mm³. In some embodiments, the tumor fragment is obtained by physical fragmentation, such as by dissection. In some embodiments, the tumor fragment is obtained by sharp dissection.

In some of any of the provided embodiments, the obtained tumor sample is fragmented into small pieces of between at or about 1 mm and at or about 8 mm in diameter, such as between at or about 1 mm and at or about 6 mm in diameter, between at or about 1 mm and at or about 4 mm in diameter, between at or about 1 mm and at or about 2 mm in diameter. In some embodiments, the tumor fragment is from about 2-3 mm in diameter. In some embodiments, the tumor fragment is from about 1-2 mm in diameter. In some embodiments, the tumor fragment is obtained by physical fragmentation, such as by dissection. In some embodiments, the tumor fragment is obtained by sharp dissection.

In some embodiments, the tumor sample is cryopreserved prior to fragmentation. In some embodiments, the tumor fragments are cryopreserved.

In some embodiments, obtained tumor fragments are placed into culture media under conditions and with appropriate nutrients to sustain T cell expansion, such as any of the conditions described in Subsection I.B.2 below for stimulation of T cells. In some embodiments 1 to 500 tumor fragments (e.g. each 1-8 mm in size) are placed in an appropriate culture vessel under conditions for expansion. In some embodiments, 10, 20, 30, 40, 50 or more fragments are cultured under conditions for expansion. The culture vessel can be a microwell, flask, tube, bag or other closed system device. In some embodiments the culture vessel is a closed container that provides a gas-permeable surface area, such as a a gas permeable flask. An exemplary culture vessel that provides a gas-permeable surface area include G-Rex plates or flasks. In some embodiments, 1 tumor fragment (about 1-8 mm in diameter) is placed for each about 2 cm² area of a culture vessel. The particular culture vessel can be chosen based on the number of tumor fragments available and/or the desired yield of cells. The choice of culture vessel (e.g. G-Rex) can be chosen by linearly scaling the number of fragments seeded to the surface area of the culture vessel. In some embodiments, the surface areas of the culture vessel is about 2 cm² (e.g. G-Rex 24 well plate) and about 1 tumor fragment (about 1-8 mm in diameter) is placed in the culture vessel. In some embodiments, the surface area of a culture vessel is about 10 cm² (e.g. G-Rex 10 or G-Rex 10M) and about 5 tumor fragments (each about 1-8 mm in diameter) are placed in the culture vessel. In some embodiments, the surface area of a culture vessel is about 100 cm² (e.g. G-Rex 100 M/100M-CS) and about 50 tumor fragments (each about 1-8 mm in diameter) are placed in the culture vessel. In some embodiments, the surface area of a culture vessel is about 500 cm² (e.g. G-Rex 500 M/500M-CS) and about 250 tumor fragments (each about 1-8 mm in diameter) are placed in the culture vessel. In aspects of the provided methods, increasing the size of the culture vessel, and hence the number of tumor fragments per vessel, may decrease variability as compared to methods involving smaller culture vessels and/or fewer fragments per vessel, such by pooling larger numbers of fragments to minimize inter-tumor variability among fragments.

In some embodiments, the tumor fragments are placed in culture media for stimulation of the cells using any of the conditions described in Subsection I.B.2 below. In some embodiments, the culture media is a serum-free media containing recombinant cytokine from IL-2, IL-7, IL-15, and/or IL-21, such as recombinant IL-12 or recombinant IL-7 and IL-15. The concentration of recombinant cytokine can include any as described. In particular embodiments, the culture media is a serum-free media containing recombinant IL-2, such as from at or about 300 IU/mL to at or about 1000 IU/mL, for example at or about 300 IU/mL. In some embodiments, the culture media is a serum-free media containing an anti-CD3 antibody and/or CD28 targeting agent (e.g. anti-CD28 antibody) and one or more recombinant cytokines (e.g. IL-2). In some embodiments, the culture media contains one or more additional T cell stimulatory agonist or apoptosis inhibitor as described in Section II.

In some embodiments, the provided methods involve obtaining cells from the tumor fragments, such as by enzymatic digestion of tumor fragments to obtain TILs. Enzymatic digestion can be carried out using a collagenase, such as a type IV collagenase or a type I/II collagenase. The enzyme, such as a collagenase, can be present in media for the enzymatic digestion at a concentration of from at or about 1 mg/mL to at or about 5 mg/mL, such as at or about 1 mg/mL, at or about 2 mg/mL, at or about 3 mg/mL, at or about 4 mg/mL or at or about 5 mg/mL, or any value between any of the foregoing. In some embodiments, the enzymatic digestion is with a media that includes type IV collagenase, such as from at or about 1 mg/mL to at or about 5 mg/mL. In some embodiments, the enzymatic digestion is with a media that includes type I/II collagenase, such as from at or about 1 mg/mL to at or about 5 mg/mL. In other embodiments, enzymes from the Miltenyi human tumor dissociation kit can be used (e.g. Cat. O. 130-095-929; Miltenyi Biotec). The enzymatic media containing the enzyme can be a serum-free media, such as any as described. In particular embodiments, enzymatic media includes collagenase, e.g., Roswell Park Memorial Institute (RPMI) 1640 buffer, 2 mM glutamate (e.g. GlutaMAX), 10 mg/mL gentamicin, 30 units/mL of DNase and 1.0 mg/mL of collagenase). In some embodiments, enzymatic media includes a serum free media (e.g. OpTmizer) containing 2 mM glutamate (e.g. GlutaMAX), 10 µg/mL gentamicin, an immune cell serum replacement (e.g. CTS Immune Cell Serum Replacement) and 1.0 mg/mL to 5.0 mg/mL of collagenase). In some embodiments, the collagenase is a type IV collagenase. In some embodiments, the collagenase is a type I/II collagenase.

The tumor fragment is then mechanically dissected to dissociate the TILs, e.g., using a tissue dissociator. Tumor digests may be produced by placing the tumor in enzymatic media and mechanically dissociating the tumor for approximately 1 minute, followed by incubation for 30 minutes at 37 °C in 5% C0₂, followed by repeated cycles of mechanical dissociation and incubation under the foregoing conditions until only small tissue pieces are present. At the end of this process, if the cell suspension contains a large number of red blood cells or dead cells, a density gradient separation using FICOLL can be performed to remove these cells. Alternative methods known in the art may be used, such as those described in U.S. Patent Application Publication No. 2012/0244133 A1. Any of the foregoing methods may be used in any of the embodiments described herein for methods of obtaining TILs for use in the provided methods.

In some embodiments, digested cells from the tumor fragments are placed into culture media under conditions and with appropriate nutrients to sustain T cell expansion, such as any of the conditions described in Subsection I.B.2 below for stimulation of T cells. The cells are seeded at a particular density suitable for the particular culture vessel. The culture vessel can be a microwell, flask, tube, bag or other closed system device. In some embodiments the culture vessel is a closed container that provides a gas-permeable surface area, such as a a gas permeable flask. An exemplary culture vessel that provides a gas-permeable surface area include G-Rex plates or flasks. In some embodiments approximately 5 x 10⁵ to 2 x 10⁶ cells of an enzymatically digested single cell suspension are seeded for each about 2 cm² area of a culture vessel. The particular culture vessel can be chosen based on the number of cells available and/or the desired yield of cells. The choice of culture vessel (e.g. G-Rex) can be chosen by linearly scaling the number of cells seeded to the surface area of the culture vessel. In some embodiments, the surface areas of the culture vessel is about 2 cm² (e.g. G-Rex 24 well plate) and about 5 x 10⁵ to 2 x 10⁶ cells of an enzymatically digested single cell suspension is placed in the culture vessel. In some embodiments, the surface area of a culture vessel is about 10 cm² (e.g. G-Rex 10 or G-Rex 10M) and about 2.5 x 10⁶ to 1 x 10⁷ cells of an enzymatically digested single cell suspension are placed in the culture vessel. In some embodiments, the surface area of a culture vessel is about 100 cm² (e.g. G-Rex 100 M/100M-CS) and about 2.5 x 10⁷ to 1 x 10⁸ cells of an enzymatically digested single cell suspension are placed in the culture vessel. In some embodiments, the surface area of a culture vessel is about 500 cm² (e.g. G-Rex 500 M/500M-CS) and about 1.25 x 10⁸ to 5 x 10⁸ cells of an enzymatically digested single cell suspension are placed in the culture vessel.

In some embodiments, the culture media is a serum-free media containing recombinant IL-2. In some embodiments, one or more additional T cell stimulating agent can also be included. In some embodiments, the culture media is a serum-free media containing an anti-CD3 antibody and/or a CD28 targeting agent (e.g. anti-CD28 antibody) and one or more recombinant cytokines (e.g. IL-2). In some embodiments, the culture media contains one or more additional T cell stimulatory agonist or apoptosis inhibitor as described in Section II.

The sample may be obtained from a variety of different subjects/patients/hosts. Generally such hosts are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the hosts will be humans.

In some aspects, the subject is a human. Accordingly, the cells in some embodiments are primary cells, e.g., primary human cells. In some embodiments, the sample is autologous to a subject to be treated, such as a subject who is a patient in need of a particular therapeutic intervention, such as the adoptive cell therapy for which cells are being isolated, processed, and/or expanded in accord with the provided methods. In some embodiments, the sample is allogenic to a subject to be treated.

In some embodiments, the T cells for use in connection with the provided methods can be enriched or sorted a variety of ways including, but not limited to, magnetic bead separation, fluorescent cell sorting, and disposable closed cartridge based cell sorters. In particular aspects, one or more reagents specific to T cells or a subset thereof, such as reagents specific to T cell activation markers for selecting reactive cells, can be used including, but not limited to, florescent antibodies, nanoparticles or beads on cell selection equipment, but not limited to, the CliniMACS, Sony FX500 or the Tyto cell sorting systems (Miltenyi).

In some aspects, T cells can be selected from a biological sample, such as based on T cell markers CD3, CD4 or CD8. In some embodiments, selecting for a T cell that is surface positive for one or more cell surface marker includes any method for separation based on such markers.

In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. In some embodiments, the immunoaffinity-based selections include contacting a sample containing cells, such as a sample containing a bulk population of T cells, e.g. primary human T cells, containing CD3+ T cells or CD4+ and CD8+ cells, with an antibody or binding partner that specifically binds to the cell surface marker or markers. In some embodiments, the antibody or binding partner is bound to a solid support or matrix, such as a sphere or bead, for example a nanoparticle, microbeads, nanobeads, including agarose, magnetic bead or paramagnetic beads, to allow for separation of cells for positive and/or negative selection. In some embodiments, the spheres or beads can be packed into a column to effect immunoaffinity chromatography, in which a sample containing cells, such as primary human T cells containing CD3+ T cells or CD4+ and CD8+ cells, is contacted with the matrix of the column and subsequently eluted or released therefrom. In other embodiments, the antibody or binding partner is detectably labeled.

In some aspects, the sample or composition of cells to be separated is incubated with small, magnetizable or magnetically responsive material, such as magnetically responsive particles or microparticles, such as nanoparticles or paramagnetic beads. The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select. Such beads are known and are commercially available from a variety of sources including, in some aspects, Dynabeads^{®} (Life Technologies, Carlsbad, CA), MACS^{®} beads (Miltenyi Biotec, San Diego, CA) or Streptamer^{®} bead reagents (IBA, Germany). In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained.

In certain embodiments, the sample is contacted with a binding agent, e.g., a detectably labeled binding agent, that specifically binds to a cell surface marker. In certain embodiments, the detectably labeled binding agent(s) are fluorescently labeled. In certain embodiments, T cells labeled with binding agents specific to a cell surface marker are identified by flow cytometry. In certain embodiments, the method further includes separating any resultant T cells labeled with the binding agent(s) from other components of the sample to produce a composition enriched for T cells surface positive for the one or more cell surface marker. Cell selection sorting equipment can be used that has a sufficiently high-throughput to handle large volumes and cell numbers. Non-limiting cell sorting equipment includes, for example, Sony FX500 or the Tyto cell sorting systems (Miltenyi).

The incubation generally is carried out under conditions whereby the antibodies or binding partners, or molecules, such as secondary antibodies or other reagents, which specifically bind to such antibodies or binding partners, which are attached to the magnetic particle or bead and/or are detectably labeled, specifically bind to cell surface molecules if present on cells within the sample. In some aspects, cells bound to the antibodies can be recovered or separated from non-bound cells in the sample.

In some aspects, a combination of positive and negative selection is performed during the same selection step, where the positive and negative fractions are retained and further processed or subject to further separation steps. Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells. For example, in some aspects, a selection of one of the CD4+ or CD8+ population enriches for said population, either the CD4+ or CD8+ population, but also can contain some residual or small percentage of other non-selected cells, which can, in some cases, include the other of the CD4 or CD8 population still being present in the enriched population.

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In particular embodiments, a T cell population is one that includes both CD4+ and CD8+ T cells. In some cases, a CD4+ and CD8+ T cell population is isolated, selected or enriched from the biological sample. Many cancers, including solid tumors, such as many common epithelial indications (e.g. GI), express class I and class II restricted mutations. In order for a T cell product to target such indications, e.g. common epithelial indications, it is contemplated that both CD8+ T cells to recognize class I MHC-restricted molecules and CD4+ T cells to recognize Class II MHC-restricted molecules are necessary.

In some embodiments, the methods include isolation, selection and/or enrichment of CD3+ cells. In some embodiments, the methods include isolation, selection and/or enrichment of CD4+ and CD8+ cells. In some aspects, a CD4⁺ or CD8⁺ selection step, such as positive selection for CD4 and positive selection for CD8, is used to separate CD4⁺ helper and CD8⁺ cytotoxic T cells. Such selections in some aspects are carried out simultaneously and in other aspects are carried out sequentially, in either order. In some embodiments, the methods include enriching for CD4+ and CD8+ T cells by selecting for T cells surface positive for CD3 or by sequential or simultaneous selection for T cells surface positive for CD4 and T cells surface positive for CD8. Such CD3+ T cells, or CD4⁻ and/or CD8⁺ populations, can be further sorted into sub-populations by positive or negative selection for markers expressed or expressed to a relatively higher degree on tumor-reactive T cells or on T cells having expression of T cell activation markers associated with tumor-reactive T cells, e.g. as described in Section I.D..

In some embodiments, isolation of the cells or populations further includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some embodiments, the selected population is enriched for CD3+ T cells and comprises CD3+ T cells as a percentage of total cells in the population that is greater than or greater than about 60%, greater than or greater than about 70%, greater than or greater than about 80%, greater than or greater than about 90% or greater than or greater than about 95%. In some embodiments, the selected population is enriched for CD4+ T cell and CD8+ T cells and comprises CD4+ T cells and CD8+ T cells as a percentage of total cells in the population that is greater than or greater than about 60%, greater than or greater than about 70%, greater than or greater than about 80%, greater than or greater than about 90% or greater than or greater than about 95%. In particular embodiments, the ratio of CD8+ T cells to CD4+ T cells is between at or about 1:100 and at or about 100:1, between at or about 1:50 and at or about 50:1, between at or about 1:25 and at or about 25: 1, between at or about 1:10 and at or about 10: 1, between at or about 1:5 and at or about 5:1, or between at or about 1:2.5 and at or about 2.5:1.

In some of any of the provided embodiments, the biological sample is a peripheral blood sample, optionally an apheresis sample, and wherein: the number of cells at the initiation of the culturing is between at or about 1 x 10⁹ and 7 x 10⁹ total viable cells; or is at or about 1 x 10⁹ total viable cells, at or about 2 x 10⁹ total viable cells, 3 x 10⁹ total viable cells, 4 x 10⁹ total viable cells, 5 x 10⁹ total viable cells, 6 x 10⁹ total viable cells, or 7 x 10⁹ total viable cells, or any value between any of the foregoing; and/or the percentage of tumor reactive T cells at the initiation of the culturing is between at or about 0.02% and at or about 40%, at or about 0.02% and at or about 24%, at or about 0.02% and at or about 18%, at or about 0.02% and at or about 0.9% or at or about 0.02% and at or about 6.0%; and/or the number of T cells surface positive for the T cell activation marker at the initiation of the culturing is between at or about 0.1 x 10⁶ and at or about 60 x 10⁶ T cells, 0.1 x 10⁶ and at or about 8 x 10⁶ T cells, 0.1 x 10⁶ and at or about 20 x 10⁶ T cells, 0.3 x 10⁶ and at or about 35 x 10⁶ T cells or 0.3 x 10⁶ and at or about 60 x 10⁶ T cells; or is at or about 0.1 x 10⁶ T cells, 0.3 x 10⁶ T cells, 0.6 x 10⁶ T cells, 1 x 10⁶ T cells, 5 x 10⁶ T cells, 10 x 10⁶ T cells, 35 x 10⁶ T cells or 60 x 10⁶ T cells, or any value between any of the foregoing.

In some of any of the provided embodiments, the biological sample is a lymph sourced sample or a tumor sourced sample, and wherein: the number of cells at the initiation of the culturing is between at or about 10 x 10⁶ and 100 x 10⁶ total viable cells, 20 x 10⁶ and 100 x 10⁶ total viable cells, or 12 x 10⁶ and 43 x 10⁶ total viable cells; or is at or about 10 x 10⁶ total viable cells, at or about 12 x 10⁶ total viable cells, 20 x 10⁶ total viable cells, 40 x 10⁶ total viable cells, 60 x 10⁶ total viable cells, or 100 x 10⁶ total viable cells, , or any value between any of the foregoing; and/or the percentage of tumor reactive T cells at the initiation of the culturing is between at or about 1% and at or about 90%, at or about 1% and at or about 75%, at or about 1% and at or about 50%, at or about 1% and at or about 25% or at or about 1% and at or about 14%; and/or the number of T cells surface positive for the T cell activation marker at the initiation of the culturing is between at or about 0.7 x 10⁶ and at or about 15 x 10⁶ T cells, 1 x 10⁶ and at or about 15 x 10⁶ T cells, or at or about 0.7 x 10⁶ and at or about 5.4 x 10⁶ T cells; or is at or about 0.7 x 10⁶ T cells, 1 x 10⁶ T cells, 5.4 x 10⁶ T cells, or 15 x 10⁶ T cells, or any value between any of the foregoing.

In some embodiments, the selected T cells can be further enriched for tumor-reactive T cells based on expression of a marker associated with activated T cells. Particular markers for use in selecting or enriching for such tumor-reactive T cells is described in Section I.D. below. In other cases, selection or enrichment of tumor-reactive T cells is carried out in one or more subsequent step of the process, such as after co-culture with one or more mutated peptide (peptide neoepitope).

### 2. Stimulation of T cells for Initial Expansion

In aspects of the provided methods, the T cells from the biological sample (input or first population of T cells, such as present in a resected tumor fragment) are incubated or cultured in the presence of one or more T cell stimulatory agent(s) under conditions for stimulating the T cells, such as to expand the T cells. In some cases, the culturing or incubation is further carried out in the presence of one or more T cell modulatory agent or adjuvant, such as a T cell agonist or apoptosis inhibitor. In some embodiments, the incubation or culturing with one or more T cell stimulatory agent(s) and/or T cell modulatory agent or adjuvants results in expansion or outgrowth of selected T cells, or a desired subset or subtype thereof or for viable cells thereof, for use in subsequent steps of the provided methods. Non-limiting examples of T cell stimulatory agent(s) and/or T cell modulatory agents or adjuvants and conditions for incubation or culture are described herein.

In some embodiments, the T cell stimulatory agent(s) include a recombinant T cell stimulating cytokine, such as IL-2, IL-7, IL-15 and/or IL-21. In some embodiments, the T cell stimulating cytokine includes IL-2, alone or in combination with another cytokine from among IL-7, IL-15 and/or IL-21. In some embodiments, the T cell stimulating cytokine includes IL-7 and IL-15.

In aspects of any of the provided methods, the population of T cells is incubated in the presence of a T cell stimulatory agent(s). In particular embodiments, the incubation is carried out under conditions in which the T cell stimulatory agent(s) activates or stimulates the cells or promotes expansion of cells.

In some of any of the provided embodiments, the T cell stimulatory agent(s) is selected from an agent that initiates TCR/CD3 intracellular signaling and an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble.

Thus, among the provided methods are methods of culturing T cells for manufacture of tumor reactive T cells in which T cells are cultured or incubated in the presence of a T cell stimulatory agent under conditions to expand T cells, such as present in the co-culture. In some embodiments, the T cell stimulatory agent is or includes an anti-CD3 antibody and anti-CD28 antibody.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include theUHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the T cell stimulatory agent(s) can include adding adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

In some embodiments, the T cell stimulatory agent(s) include one or more recombinant cytokine. In some embodiments, the cytokine is added or is exogenous to the culture media. In some embodiments, the recombinant cytokine can include one or more of IL-2, IL-7, IL-15 or IL-21. In some embodiments, the culturing and incubation is carried out in the presence of recombinant IL-2, IL-15 and IL-7. In some embodiments, the culturing is carried out in the presence of a IL-2. In some embodiments, the culturing is carried out in the presence of IL-15 and IL-7, which, in some aspects does not additionally include IL-2. In particular embodiments, the recombinant cytokine(s) is human.

The recombinant cytokine generally is a recombinant human protein. In particular embodiments, the recombinant cytokine is present in the cell culture medium during the incubation at a concentration of at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. IL-2 is a cytokine that supports T cell recovery and proliferation. IL-2 also supports the homeostasis of T cells, thereby supporting their phenotype, differentiation status, and immune memory. In some cases, induction of regulatory T cells in the tumor microenvironment may lead to low bioavailability of IL-2. Recombinant IL-2 has been regularly used in broad expansion of T cells in various contexts. Recombinant IL-2 is commercially available. In particular embodiments, recombinant IL-2 is GMP grade (e.g. MACS GMP Recombinant Human IL-2, Miltenyi Biotec).

Recombinant IL-2 can be included in cell culture media during various stages of the provided process. In some cases, recombinant IL-2 can be included in the initial T cell expansion (first expansion), such as to promote TIL outgrowth and allow their proliferation from solid tumor. IL-2 also can be included in antigen-presenting cell co-culture as described in Section I.C, such as to allow for peak activation of neo-antigen reactive T prior to their separation or selection. In some cases, recombinant IL-2 can also be included in cultures to expand tumor-reactive T cells during the second expansion phase, such as described in Section I.E.

In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. IL-15 is a cytokine that is involved in memory T cell homeostasis and activation. In some cases, IL-15 can promote effector functions of antigen-experienced T cells in the absence of antigen and prevent their differentiation into an exhausted phenotype. IL-15 also plays a role in T cell proliferation. Recombinant IL-15 is commercially available. In particular embodiments, recombinant IL-15 is GMP grade (e.g. MACS GMP Recombinant Human IL-15, Miltenyi Biotec).

Recombinant IL-15 can be included in cell culture media during various stages of the provided process. In some cases, recombinant IL-15 can be included in the initial T cell expansion (first expansion), such as to promote TIL expansion to promote their outgrowth and allow their proliferation and/or stabilize phenotype from solid tumor. Recombinant IL-15 also can be included in antigen-presenting cell co-culture as described in Section I.C, such as to allow for peak activation of neo-antigen reactive T prior to their separation or selection. In some cases, recombinant IL-15 can also be included in cultures to expand tumor-reactive T cells during the second expansion phase, such as described in Section I.E. In some cases, recombinant IL-15 can be combined with recombinant IL-7 to provide for activation, survival and/or expansion of tumor-reactive T cells in the provided methods. In some such embodiments, the combination of recombinant IL-7 and IL-15 is an alternative to the use of recombinant IL-2 in the culture, and the culture media does not additionally contain recombinant IL-2.

In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. IL-7 is a cytokine that is involved in promoting T cell maintenance and homeostasis. In some cases, IL-7 can boost memory T cell survival and proliferation, particularly the central memory compartment. Recombinant IL-7 is commercially available. In particular embodiments, recombinant IL-7 is GMP grade (e.g. MACS GMP Recombinant Human IL-7, Miltenyi Biotec).

Recombinant IL-7 can be included in cell culture media during various stages of the provided process. In some cases, recombinant IL-7 can be included in the initial T cell expansion (first expansion), such as to promote TIL expansion to promote their outgrowth and allow their proliferation and/or stabilize phenotype from solid tumor. IL-7 also can be included in antigen-presenting cell co-culture as described in Section I.C, such as to allow for peak activation of neo-antigen reactive T prior to their separation or selection. In some cases, recombinant IL-7 can also be included in cultures to expand tumor-reactive T cells during the second expansion phase, such as described in Section I.E. Inclusion of recombinant IL-7 in the process can maintain or support expansion of memory T cell subsets in the process. In some cases, recombinant IL-7 can be combined with recombinant IL-15 to provide for activation, survival and/or expansion of tumor-reactive T cells in the provided methods. In some such embodiments, the combination of recombinant IL-7 and IL-15 is an alternative to the use of recombinant IL-2 in the culture, and the culture media does not additionally contain recombinant IL-2.

In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. IL-21 is a cytokine that supports a broad range of T cell activation without increasing regulatory T cell signaling. In some cases, IL-21 can support memory cell stabilization, effector function, and proliferation of antigen-experienced T cells. IL-21 can induce upregulation of effector molecules in both CD4 and CD8 T cells. Recombinant IL-21 is commercially available. In particular embodiments, recombinant IL-21 is GMP grade (e.g. MACS GMP Recombinant Human IL-21, Miltenyi Biotec).

Recombinant IL-21 can be included in cell culture media during various stages of the provided process. In some cases, recombinant IL-21 can be included in the initial T cell expansion (first expansion), such as to promote TIL outgrowth from solid tumor, including by stabilizing memory T cell activation, function and/or proliferation. In some aspects, the presence of IL-21 allows for improved recovery of TIL. Recombinant IL-21 also can be included in antigen-presenting cell co-culture as described in Section I.C, such as due to the ability to stimulate expression of T cell activation markers, including expression of activation markers on neo-antigen reactive TIL. In some cases, recombinant IL-21 can also be included in cultures to expand tumor-reactive T cells during the second expansion phase as described in Section I.E, such as to support proliferation and stabilization of memory phenotype.

In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

In particular embodiments, T cell stimulatory agent(s) present during the incubation, such as for expansion of cells, contains recombinant IL-2. In some embodiments, one or more other stimulating agent can be included such as one or more other recombinant cytokine from IL-7, IL-15, IL-21 or an anti-CD3 antibody (e.g. OKT-3). In some cases in which an anti-CD3 antibody (e.g. OKT-3) the T cell stimulating agent(s) also can include a costimulating agent, such as provided by antigen-presenting feeder cells, such as PBMCs, or a soluble anti-CD28 antibody.

In particular embodiments, T cell stimulatory agent(s) present during the incubation, such as for expansion of cells contains recombinant IL-2, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-15 and recombinant IL-7, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-15 and recombinant IL-7, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In some embodiments, the incubation with the T cell stimulatory agent(s) is carried out under conditions for initial expansion of T cells from the biological sample. In some embodiments, the cells are cultured at about 37 °C with about 5% CO₂.

In some embodiments, the incubation with the T cell stimulatory agent(s) is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days.

The incubation, such as for initial expansion of T cells in the biological sample, can be carried out under GMP conditions. In some embodiments, the incubation is in a closed system, which in some aspects may be a closed automated system. In some embodiments, the culture media containing the T cell stimulatory agent(s) can be a serum-free media. In some embodiments, the incubation is carried out in a closed automated system and with serum-free media.

In some embodiments, the initial expansion of cells under the one or more stimulatory conditions is in a culture vessel suitable for cell expansion. In some embodiments, the culture vessel is a gas permeable culture vessel, such as a G-Rex system (e.g. G-Rex 10, G-Rex 10M, G-Rex 100 M/100M-CS or G-Rex 500 M/500M-CS). In some embodiments the culture vessel is a microplate, flask, bar or other culture vessel suitable for expansion of cells in a closed system. In some embodiments, expansion can be carried out in a bioreactor. In some embodiments, the initial expansion can be carried out using a cell expansion system by transfer of the cells to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 x 10⁶ cells/mL to 1.5 x 10⁶ cells/mL. In some embodiments, the density is at or about 0.5 x 10⁶ cells/mL, 0.75 x 10⁶ cells/mL, 1 x 10⁶ cells/mL, 1.25 x 10⁶ cells/mL or 1.5 x 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

The expansion methods can be carried out under GMP conditions, including in a closed automated system and using serum free medium. In some embodiments, any one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, immediately after the incubation, the stimulated cells can be collected for subsequent co-culture with APCs, such as in accord with methods described in Section I.C below.

In some embodiments, the stimulated cells are collected and are cryofrozen. The provision of an intermediate hold step by cryopreservation after the initial expansion phase can be used to coordinate timing with the neoepitope identification and peptide generation, such as described in Section I.A and/or the generation of APCs as described in Section I.C. In some embodiments, for cryopreservation, the stimulated cells are formulated as a composition with a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to -150°C, such as or about 80 °C ± 6.0 ° C.

In some embodiments, the cryopreserved cells are prepared for subsequent steps by thawing. In some cases, the cells can be ready for subsequent culturing with APCs and peptides immediately after thawing following one or more wash steps.

### C. Co-Culture of T cells with APCs

In some embodiments, the source of cells for expansion can be obtained from an ex vivo co-culture source of cells generated to be enriched for tumor reactive T cells. Methods for generating cells with antigen-specificity are known, see e.g. US published application No. US2017/0224800. Thus, embodiments, the provided methods include those in which the population of T cells containing tumor-reactive T cells, such as T cells that exhibit antigenic specificity for a tumor-associated antigen (e.g. neoantigen) or a peptide of a tumor-associated antigen, are identified or generated ex vivo. Such methods include, but are not limited to the steps of (1) enriching a population of T cells obtained from a donor subject, such as by directly selecting T cells from a biological sample, e.g. a tumor, blood, bone marrow, lymph node, thyus or other tissue or fluids; (2) stimulating the population with one or more T-cell stimulating agents, such as described above (e.g. anti-CD3 (e.g. OKT3) and anti-CD28 reagents, such as an anti-CD3 antibody (e.g. OKT3) and an anti-CD28 antibody, and one or more additional recombinant cytokine such as IL-2, IL-7, IL-21 and/or IL-15), to produce a population containing activated T cells; (3) co-culturing the population containing activated T cells in the presence of antigen presenting cells (APCs) that present one or more MHC-associated non-native peptide; and (4) enriching T cells containing an endogenous TCR that are reactive to peptides present on antigen presenting cells (APCs). In some aspects, T cells containing an endogenous TCR are enriched by separating the antigen presenting cells from the population of T cells. Alternatively or additionally, such cells are enriched by selecting T cells that are surface positive for one or more activation markers associated with tumor-reactive T cells.

In particular embodiments, once the neopitopes that encode for proteins are synthesized a plurality of the synthetic peptides are contacted with antigen presenting cells under conditions to present peptides in the context of an MHC molecule and incubated with T cells from a population of T cells for recognition of the peptides presented on the APCs. In some embodiments, the synthetic peptides are pulsed into autologous or allogeneic APCs that are then cultured with patient T cells. Antigen presenting cells are used to present these peptides. T cells that recognize these peptides on the surface of the APC can then be isolated, such as by methods described below. The incubated cells can be cultured under conditions that enrich for and expand tumor-reactive T cells, i.e. T cells containing endogenous TCR that are reactive to peptides present on the APCs, in the culture. In some embodiments, the methods include culturing the T cells under conditions for expansion until a threshold amount of T cells is obtained and/or until up to 20 days after initiation of incubation. In some embodiments, of the provided methods the method can include co-culturing the T cells with the APCs over the course of several hours to days and then separating antigen presenting cells from the population of T cells for the expansion of the T cells under conditions to enrich or expand tumor-reactive T cells. In some embodiments, of the provided methods the method can include co-culturing the T cells with the APCs over the course of 1-7 days, and then separating antigen presenting cells from the population of T cells for the expansion of the T cells under conditions to enrich or expand tumor-reactive T cells. In some embodiments, the separating can include isolating or selecting reactive T cells from culture based on one or more T cell activation markers on T cells.

The method may comprise inducing autologous antigen presenting cells (APCs) of the patient to present the mutated amino acid sequence. The method may comprise inducing autologous antigen presenting cells (APCs) of the patient to present the mutated amino acid sequence. The APCs may include any cells which present peptide fragments of proteins in association with major histocompatibility complex (MHC) molecules on their cell surface. The APCs may include, for example, any one or more of macrophages, DCs, Langerhans cells, B-lymphocytes, and T-cells. Preferably, the APCs are DCs. By using autologous APCs from the patient, the methods may identify T cells that have antigenic specificity for a mutated amino acid sequence encoded by a cancer-specific mutation that is presented in the context of an MHC molecule expressed by the patient. The MHC molecule can be any MHC molecule expressed by the patient including, but not limited to, MHC Class I, MHC Class II, HLA-A, HLA-B, HLA-C, HLA-DM, HLA-DO, HLA-DP, HLA-DQ, and HLA-DR molecules.

In particular embodiments, the APCs include cells that are able to present Class I and Class II restricted molecules. For example, B cells and DCs both have the ability to present MHC class I and MHC class II restricted molecules. In some embodiments, the APC cell sample includes B cells and DCs. In some embodiments, the APC cell sample is enriched for B cells, such as by selection or isolation from a primary cell sample. In some embodiments, the APC cell sample is enriched for DCs, such as by selection or isolation from a primary cell sample.

In some embodiments, the APCs express MHC class I and/or MHC class II molecules with a matched HLA from which the source of T cells has been obtained. In particular embodiments, both the APCs and T cells have been isolated from the same subject, i.e. are autologous to the cancer patient. In some embodiments, the method may comprise inducing autologous antigen presenting cells (APCs) of the patient to present the mutated amino acid sequence. By using autologous APCs from the patient, the methods may identify T cells that have antigenic specificity for a mutated amino acid sequence encoded by a cancer-specific mutation that is presented in the context of an MHC molecule expressed by the patient.

In some embodiments, the APCs are cells from a blood or apheresis sample from a subject, such as the patient. In some embodiments, the APCs include cells present in a peripheral blood mononuclear cell (PBMC) sample Typically, APCs function in a PBMC culture primarily involves monocytes and B cells. In some embodiments, a population of isolated PBMCs can be used as APCs in the provided methods. PBMCs can be obtained using standard methods such as Ficoll-Paque gradient separation. In some cases, the APCs are or include B cells that are isolated from the blood or apheresis sample or from a PBMC sample. In other cases, the APCs are or include monocytes isolated from the blood or apheresis sample or from a PBMC sample. In some aspects, the monocytes can be used as a source for preparing monocyte-derived DCs for use as APCs. In some embodiments, a source of monocyte-derived DCs (e.g. CD11c^{high}MHCII^{high}CD14^{low} cells) can be generated ex vivo from isolated monocytes, by culture with GM-CSF and IL-4 for 4 to 6 days to produce monocyte-derived dendritic cells. In particular embodiments, the monocytes are isolated from PBMCs such as by CD14 selection, and then are cultured with GM-CSF and IL-4 for 4 to 6 days.

In some embodiments, the APCs are primary cells (e.g. B cells or monocyte-derived DCs) that are replication competent, for example, the cells are not subjected to irradiation, heat treatment or other method that would result in their inactivation. In particular embodiments, the provided methods do not use irradiated APCs. In some embodiments, the APCs are freshly isolated primary cells obtained from the subject, or are derived from primary cells obtained from the subject. In some embodiments, the APCs have been cryopreserved and subsequently thawed prior to the co-culture with the stimulated T cells in accord with provided methods.

In some particular embodiments, B cells are used as a source of APCs and are generated from a patient apheresis, such as an apheresis autologous to the subject from which the tumor fragments and/or T cells were obtained. In other particular embodiments, monocyte-derived dendritic cells are used as a source of APCs and are generated from monocytes from a patient apheresis, such as an apheresis autologous to the subject from which the tumor fragment and/or T cells are obtained.

In some embodiments, the isolated or generated APCs are collected and are cryofrozen. The provision of an intermediate hold step by cryopreservation after the isolation or generation of APCs can be used to coordinate timing with the neoepitope identification and peptide generation such as described in Section I.A and/or initial expansion of T cells, such as described in Section I.B. In some embodiments, for cryopreservation, the isolated or generated APCs are formulated as a composition with a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to -150°C, such as or about 80 °C ± 6.0 ° C.

In some embodiments, the cryopreserved cells are prepared for subsequent steps by thawing. In some cases, the cells can be ready for subsequent culturing with T cells and peptides immediately after thawing following one or more wash steps.

In particular embodiments, the methods for enriching or selecting tumor reactive cells are initiated by contacting PBMCs with the mutated amino acid sequence, such as one or more, such as a plurality of, neoepitope peptides. The PBMCs/peptides can then be cultured with stimulated T cells. The PBMCs and T cells can be obtained from the same subject.

In particular embodiments, the methods for enriching or selecting tumor reactive cells are initiated by contacting B cells with the mutated amino acid sequence, such as one or more, such as a plurality of, neoepitope peptides. The B cell/peptides can then be cultured with stimulated T cells. The B cells and T cells can be obtained from the same subject.

In particular embodiments, the methods for enriching or selecting tumor reactive cells are initiated by contacting monocyte-derived DCs with the mutated amino acid sequence, such as one or more, such as a plurality of, neoepitope peptides. The monocyte-derived Dcs/peptides can then be cultured with stimulated T cells. The monocyte-derived DCs and T cells can be obtained or derived from the same subject.

In some embodiments, the APC is an artificial antigen presenting cell (aAPC). Typically, aAPCs include features of natural APCs, including expression of an MHC molecule, stimulatory and costimulatory molecule(s), Fc receptor, adhesion molecule(s) and/or the ability to produce or secrete cytokines (e.g. IL-2). Normally, an aAPC is a cell line that lacks expression of one or more of the above, and is generated by introduction (e.g. by transfection or transduction) of one or more of the missing elements from among an MHC molecule, a low affinity Fc receptor (CD32), a high affinity Fc receptor (CD64), one or more of a co-stimulatory signal (e.g. CD7, B7-1 (CD80), B7-2 (CD86), PD-L1, PD-L2, 4-1BBL, OX40L, ICOS-L, ICAM, CD30L, CD40, CD70, CD83, HLA-G, MICA, MICB, HVEM, lymphotoxin beta receptor, ILT3, ILT4, 3/TR6 or a ligand of B7-H3; or an antibody that specifically binds to CD27, CD28, 4-1BB, OX40, CD30, CD40, PD-1, ICOS, LFA-1, CD2, CD7, LIGHT, NKG2C, B7-H3, Toll ligand receptor or a ligand of CD83), a cell adhesion molecule (e.g. ICAM-1 or LFA-3) and/or a cytokine (e.g. IL-2, IL-4, IL-6, IL-7, IL-10, IL-12, IL-15, IL-21, interferon-alpha (IFNα), interferon-beta (IFNβ), interferon-gamma (IFNγ), tumor necrosis factor-alpha (TNFα), tumor necrosis factor-beta (TNFβ), granulocyte macrophage colony stimulating factor (GM-CSF), and granulocyte colony stimulating factor (GCSF)). In some cases, an aAPC does not normally express an MHC molecule, but can be engineered to express an MHC molecule or, in some cases, is or can be induced to express an MHC molecule, such as by stimulation with cytokines. In some cases, aAPCs also can be loaded with a stimulatory or co-stimulatory ligand, which can include, for example, an anti-CD3 antibody, an anti-CD28 antibody or an anti-CD2 antibody. Exemplary of a cell line that can be used as a backbone for generating an aAPC is a K562 cell line or fibroblast cell line. Various aAPCs are known in the art, see e.g., U.S. Patent No. 8,722,400, published application No. US2014/0212446; Butler and Hirano (2014) Immunol Rev., 257(1):10. 1111/imr.12129; Suhoshki et al. (2007) Mol. Ther., 15:981-988). In particular embodiments, the methods for enriching or selecting tumor reactive cells are initiated by contacting aAPCs with the mutated amino acid sequence, such as one or more, such as a plurality of, neoepitope peptides. The aAPC/peptides can then be cultured with stimulated T cells.

In a particular embodiment, whole exome sequencing is performed on healthy and diseased tissue to identify somatic mutations associated with the tumor. Once these mutations are identified, neoepitopes, the reactive neoantigens are then identified. Neoantigens are mutant peptides that are recognized by a patient's T cells. These neoantigens must be presented by a tumor or antigen presenting cell by the MHC complex and then be recognized by a TCR on the T cell. Once the neopitopes that encode for proteins are identified a mutation library can be generated. Long peptides can be synthesized and pulsed using electroporation into an antigen presenting cell. Long peptides can then be presented by the antigen presenting cells to be recognized by CD8 cells. Long peptides are suitable for expression by MHC class I restricted molecules for recognition by CD8 cells. Long peptides do not work for MHC class II restricted molecules for recognition by CD4 cells. MHC class II restricted molecules must be presented as a gene encoding DNA of the mutation and electroporated into the antigen presenting cell.

Inducing autologous APCs (e.g. B cells or monocyte-derived DCs) of the patient to present the mutated amino acid sequence may be carried out using various suitable methods. In an embodiment, inducing autologous APCs of the patient to present the mutated amino acid sequence (i.e. peptide neoepitope) comprises pulsing the autologous APCs with peptides comprising the mutated amino acid sequence or a pool of peptides, each peptide in the pool comprising a different mutated amino acid sequence. In some cases, the APCs are pulsed with the peptides using electroporation into an antigen presenting cell. The synthetic peptides can then be presented by the antigen presenting cells to be recognized by CD8 cells (MHC class I) or CD4 cells (MHC class II). In certain particular embodiments, synthetic peptides are generated to be suitable for expression by MHC class I restricted molecules for recognition by CD8 cells. In other particular embodiments, synthetic peptides are generated to be suitable for expression by MHC class II restricted molecules for recognition by CD4 cells.

In some embodiments, the APCs (e.g. PBMCs, B cells or monocyte-derived DCs) are contacted with a single peptide or a pool of peptides. The pool of peptide can represent many different mutated amino acid sequences, such as 5, 10, 20, 25, 30, 40, 50, 60, 70, 80, 90, 100, 150, 200, 300, 400, 500, 600, 700, 800, 900 or 100 peptides, or any value between any of the foregoing.

The peptides or pool of peptides are loaded onto antigen presenting cells (e.g. dendritic cells), such as by peptide pulsing, at a concentrations suitable for their presentation on the surface of a major histocompatibility complex (MHC).

In some embodiments, the peptide concentration representing an individual or single peptide can range between at or about 0.00000 1 µg/mL and at or about 10 µg/m L. In some embodiments, the peptide concentration representing an individual or single peptide can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration representing an individual or single peptide can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the peptides are a pool of peptides representing many different mutated amino acid sequences and the concentration on average of individual or single peptides in the pool can range between at or about 0.00000 1 µg/mL and at or about 10 µg/mL. In some embodiments, the peptides are a pool of peptides representing many different mutated amino acid sequences and the concentration on average of individual or single peptides in the pool can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration on average of individual or single peptides in the pool can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the concentration of individual peptides of the one or more non-native peptide is, on average, less than 0.02 µg/mL. In some embodiments, the concentration of individual peptides of the one or more non-native peptides is, on average, from at or about 0.00001 µg/mL to at or about 0.01 µg/mL, such as at or about 0.00001 µg/mL to at or about 0.005 µg/mL, at or about 0.00001 µg/mL to at or about 0.002 µg/mL, at or about 0.00001 µg/mL to at or about 0.001 µg/mL, at or about 0.00001 µg/mL to at or about 0.0005 µg/mL, at or about 0.00001 µg/mL to at or about 0.0002 µg/mL, at or about 0.00001 µg/mL to at or about 0.0001 µg/mL, at or about 0.00001 µg/mL to at or about 0.00005 µg/mL, at or about 0.00001 µg/mL to at or about 0.00002 µg/mL, at or about 0.00002 µg/mL to at or about 0.005 µg/mL, at or about 0.00002 µg/mL to at or about 0.002 µg/mL, at or about 0.00002 µg/mL to at or about 0.001 µg/mL, at or about 0.00002 µg/mL to at or about 0.0005 µg/mL, at or about 0.00002 µg/mL to at or about 0.0002 µg/mL, at or about 0.00002 µg/mL to at or about 0.0001 µg/mL, at or about 0.00002 µg/mL to at or about 0.00005 µg/mL, at or about 0.00005 µg/mL to at or about 0.005 µg/mL, at or about 0.00005 µg/mL to at or about 0.002 µg/mL, at or about 0.00005 µg/mL to at or about 0.001 µg/mL, at or about 0.00005 µg/mL to at or about 0.0005 µg/mL, at or about 0.00005 µg/mL to at or about 0.0002 µg/mL, at or about 0.00005 µg/mL to at or about 0.0001 µg/mL, at or about 0.0001 µg/mL to at or about 0.005 µg/mL, at or about 0.0001 µg/mL to at or about 0.002 µg/mL, at or about 0.0001 µg/mL to at or about 0.001 µg/mL, at or about 0.0001 µg/mL to at or about 0.0005 µg/mL, at or about 0.0001 µg/mL to at or about 0.0002 µg/mL, at or about 0.0005 µg/mL to at or about 0.005 µg/mL, at or about 0.0005 µg/mL to at or about 0.002 µg/mL, at or about 0.0005 µg/mL to at or about 0.001 µg/mL, at or about 0.001 µg/mL to at or about 0.005 µg/mL, at or about 0.001 µg/mL to at or about 0.002 µg/mL, or at or about 0.002 µg/mL to at or about 0.005 µg/mL.

In some embodiments, the peptide concentration, representing the single peptide or pool of peptides, can range between at or about 0.0001 µg/mL and at or about 40 µg/mL. The peptide concentration, representing the single peptide or pool of peptides, can range between at or about 0.001 µg/mL and at or about 40 µg/mL, at or about 0.001 µg/mL and at or about 25 µg/mL, 0.001 µg/mL and at or about 10 µg/mL, 0.001 µg/mL and at or about 5 µg/mL, 0.001 µg/mL and at or about 1 µg/mL , 0.001 µg/mL and at or about 0.5 µg/mL, 0.001 µg/mL and at or about 0.1 µg/mL, 0.001 µg/mL and at or about 0.01 µg/mL, at or about 0.01 µg/mL and at or about 40 µg/mL, such as at or about 0.01 µg/mL and at or about 25 µg/mL, at or about 0.01 µg/mL and at or about 10 µg/mL, at or about 0.01 µg/mL and at or about 5 µg/mL, at or about 0.01 µg/mL and at or about 1 µg/mL, at or about 0.01 µg/mL and at or about 0.5 µg/mL, at or about 0.01 µg/mL and at or about 0.1 µg/mL, at or about 0.01 µg/mL and at or about 0.05 µg/mL, 0.05 µg/mL and at or about 40 µg/mL, at or about 0.05 µg/mL and at or about 25 µg/mL, at or about 0.05 µg/mL and at or about 10 µg/mL, at or about 0.05 µg/mL and at or about 5 µg/mL, at or about 0.05 µg/mL and at or about 1 µg/mL, at or about 0.05 µg/mL and at or about 0.5 µg/mL, at or about 0.05 µg/mL and at or about 0.1 µg/mL, 0.1 µg/mL and at or about 40 µg/mL, such as at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL and at or about 40 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL and at or about 40 µg/mL, at or about 1 µg/mL and at or about 25 µg/mL, at or about 1 µg/mL and at or about 10 µg/mL, at or about 1 µg/mL and at or about 5 µg/mL, 5 µg/mL and at or about 40 µg/mL, at or about 5 µg/mL and at or about 25 µg/mL, at or about 5 µg/mL and at or about 10 µg/mL, 10 µg/mL and at or about 40 µg/mL, at or about 10 µg/mL and at or about 25 µg/mL, or at or about 25 µg/mL and at or about 40 µg/mL. In some embodiments, the peptide concentration, representing the single peptide or pool of peptides can be at or about 0.0001 µg/mL, at or about 0.001 µg/mL, at or about 0.01 µg/mL, at or about 0.1 µg/mL, at or about 1 µg/mL, at or about 10 µg/mL, at or about 20 µg/mL, at or about 30 µg/mL or at or about 40 µg/mL or any value between any of the foregoing. In some embodiments, the peptide concentrations is the concentration of a pool of peptides. In some embodiments, the peptide concentration is a concentration of a single or individual peptide.

In some embodiments, for peptide pulsing APCs (e.g. B cells or monocyte-derived DCs) are incubated with peptides for between at or about 2 hours and at or about 48 hours, such as between at or about 2 hours and at or about 36 hours, between at or about 2 hours and at or about 24 hours, between at or about 2 hours and at or about 24 hours, between at or about 2 hours and at or about 18 hours, between at or about 2 hours and at or about 12 hours, between at or about 2 hours and at or about 6 hours, between at or about 6 hours and at or about 48 hours, between at or about 6 hours and at or about 36 hours, between at or about 6 hours and at or about 24 hours, between at or about 6 hours and at or about 24 hours, between at or about 6 hours and at or about 18 hours, between at or about 6 hours and at or about 12 hours, between at or about 12 hours and at or about 48 hours, between at or about 12 hours and at or about 36 hours, between at or about 12 hours and at or about 24 hours, between at or about 12 hours and at or about 18 hours, between at or about 18 hours and at or about 48 hours, between at or about 18 hours and at or about 36 hours, between at or about 18 hours and at or about 24 hours, between at or about 24 hours and at or about 48 hours, between at or about 24 hours and at or about 36 hours, or between at or about 36 hours and at or about 48 hours. In some embodiments, the APCs (e.g. B cells or monocyte-derived DCs) are incubated with peptides for at or about 4 hours, at or about 6 hours, at or about 7 hours, at or about 8 hours, at or about 9 hours, at or about 10 hours, at or about 12 hours, at or about 14 hours, at or about 16 hours, at or about 18 hours, at or about 20 hours, at or about 22 hours, at or about 24 hours, or any value between any of the foregoing. In particular embodiments, the APCs (e.g. PBMCs, B cells or monocyte-derived DCs) are incubated with peptides overnight, such as for between at or about 8 to 12 hours. In some embodiments, the co-culture incubation is for at or about 6 hours.

In an embodiment, inducing autologous APCs of the patient to present the mutated amino acid sequence comprises introducing a nucleotide sequence encoding the mutated amino acid sequence into the APCs. The nucleotide sequence is introduced into the APCs so that the APCs express and display the mutated amino acid sequence, bound to an MHC molecule, on the cell membrane. The nucleotide sequence encoding the mutated amino acid may be RNA or DNA. Introducing a nucleotide sequence into APCs may be carried out in any of a variety of different ways. Non-limiting examples of techniques that are useful for introducing a nucleotide sequence into APCs include transformation, transduction, transfection, and electroporation. In an embodiment in which more than one gene is identified, the method may comprise preparing more than one nucleotide sequence, each encoding a mutated amino acid sequence encoded by a different gene, and introducing each nucleotide sequence into a different population of autologous APCs. In this regard, multiple populations of autologous APCs, each population expressing and displaying a different mutated amino acid sequence, may be obtained.

In some cases, peptides for binding MHC class II restricted molecules are presented as a gene encoding DNA of the mutation and electroporated into the antigen presenting cell. This DNA will then be in-vitro transcribed into RNA encoding peptides on the surface for recognition by CD4+ cells. In some cases, Tandem Mini Gene methods (National Cancer Institute) can be employed to do this for MHC class II restricted molecules, see e.g. U.S. Patent Application Number PCT/US2014/058796 and Parkhurst et al. (2016) Clin Cancer Res., 23:2491-505. In some cases where tandem minigenes are used, APCs (e.g. B cells or monocyte-derived DCs) are electroporated with a mixture of DNA (plurality of DNA) encoding a different mutated amino acid sequences, which will then be in-vitro transcribed into RNA encoding peptides for surface recognition by CD4+ T cells. In some embodiments, APCs (e.g. B cells or monocyte-derived DCs) are electroporated using the Lonza 4D Nucleofector continuous electroporation system.

Once these long peptides and DNA is synthesized and pulsed into autologous or allogeneic antigen presenting cells they are cultured with patient T cells. Antigen presenting cells are used to present these peptides. T cells that recognize these peptides on the surface of the APC can then be isolated, such as by methods described below. The methods include adding T cells (e.g. from patient having a tumor) with the culture of APCs presenting the peptides and co-culturing the APCs and T cells for a period of time to allow presentation and recognition of the peptide on the surface of APCs by one or more T cells in the population. In provided embodiments, the T cells include a population of the stimulated T cells.

The T cells (e.g. stimulated T cells) and APCs (e.g. B cells or monocyte-derived DCs) can be present in a culture at a ratio of T cells to APC of 1:100 to 100:1, such as 1:50 to 50:1, 1:25 to 25:1, 1:10 to 10:1, or 1:5 to 5:1. In some embodiments, the ratio of T cells (e.g. stimulated T cells) to APC is at or about 1:100, at or about 1:50, at or about 1:25, at or about 1:10, at or about 1:5, at or about 1:2.5, at or about 1:1, at or about 2:5:1, at or about 5:1, at or about 10:1, at or about 25:1, at or about 50:1 or at or about 100:1, or any value between any of the foregoing. n some embodiments, the ratio of T cells (e.g. stimulated T cells) to APC is between 20:1 and 1:1, between 15:1 and 1:1, between 10:1 and 1:1, between 5:1 and 1:1, or between 2.5:1 and 1:1. In some embodiments, the ratio of T cells (e.g. stimulated T cells) to APC is between 1:20 and 1:1, between 1:15 and 1:1, between 1:10 and 1:1, between 1:5 and 1:1, or between 1:2.5 and 1:1. In particular embodiments, co-culture will be performed by mixing the T cells, e.g. population of stimulated T cells, and APC (e.g. B cells or monocyte-derived DC) at approximately a 3:1 ratio. In some embodiments, co-culture will be performed by mixing the T cells, e.g. population of stimulated T cells, and APC (e.g. B cells or monocyte-derived DC) at approximately a 1:1 ratio.

In some embodiments, one or more recombinant cytokine for sustaining T cells is added to the co-culture. In some embodiments, the recombinant cytokine can include one or more of IL-2, IL-7, IL-15 or IL-21. In some embodiments, the co-culturing is carried out in the presence of recombinant IL-2, IL-15 and IL-7. In some embodiments, the co-culturing is carried out in the presence of a IL-2. In some embodiments, the co-culturing is carried out in the presence of IL-15 and IL-17, which, in some aspects does not additionally include IL-2.

The recombinant cytokine generally is a recombinant human protein. In particular embodiments, the recombinant cytokine is present in the cell culture medium during the co-culture at a concentration of at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

In provided embodiments, the co-culture of T cells with APCs/peptide can also be carried out with a T cell adjuvant, such as any described in Section II. In some aspects, the T cell adjuvant is a costimulatory agonist, such as a Tumor Necrosis Factor Super Family Receptor (TNFSR) agonists including but not limited to agonists of OX40 and 41BB. In some embodiments, the T cell adjuvant is an apoptosis inhibitors including but not limited to caspase inhibitors or inhibitors of the Fas/Fas ligand axis.

The co-culture of APCs and T cells can be incubated at a temperature suitable for the presentation of peptides on MHC and the activation of T cells in the culture, for example, at least about 25 degrees Celsius, generally at least about 30 degrees, and generally at or about 37 degrees Celsius. In some embodiments, the incubation is carried out for up to 96 hours. The incubation can be carried out for 24 hours to 96 hours, such as at or about 24 hours, at or about 36 hours, at or about 48 hours, at or about 60 hours, at or about 72 hours, at or about 84 hours or at or about 96 hours, or for a time between any of the foregoing. In particular embodiments, the co-culture is incubated for 24 to 48 hours.

In some embodiments, at the end of the co-culturing tumor reactive T cells are separated from APCs present in the co-culture. In some embodiments, the separation can include methods that select away or remove the APCs. In some embodiments, the separation can include methods that positively select or retain the T cells present in the co-culture. In some embodiments, total T cells in the co-culture can be selected. In particular embodiments, tumor reactive T cells or T cells that express one or more activation markers associated with tumor-reactive T cells can be selected.

### D. Selection of Tumor Reactive T cells

In embodiments of the provided methods, the methods involve enrichment or selection of tumor reactive T cells or T cells that are likely or suspected of being tumor reactive T cells by selecting or isolating T cells that are surface positive for one or more T cell activation markers associated with tumor reactive T cells. In some embodiments, T cells that are surface positive for one or more activation marker is further selected or enriched from a population of T cells that have been isolated or selected from a biological sample, such as described in Section I.B.1. In some embodiments, T cells that are surface positive for one or more activation marker is further selected or enriched from the population of stimulated T cells, such as described in Section I. B.2. In some embodiments, T cells that are surface positive for one or more activation marker is further selected or enriched from a population of T cells after their co-culture with APCs, such as described in Section I.C. In some embodiments, the methods can include a combination of any of the above selections for obtaining or enriching in tumor reactive T cells or T cells that are likely or suspected of being tumor reactive T cells. In some embodiments, the enriched population of cells is used in subsequent processing steps, such as subsequent processing steps involving incubation, stimulation or activation, and/or expansion in accord with one or more steps of any of the provided methods.

In aspects of any of the provided embodiments, tumor-reactive T cells and/or T cells expressing at least one T cell activation marker associated with tumor-reactive T cells are enriched from a biological sample in accord with any of the provided methods. In some aspects, prior to or concurrently with the enrichment, T cells can be enriched or selected from a biological sample, such as based on T cell markers CD3, CD4 or CD8. In some embodiments, enriching for a T cell that is surface positive for one or more cell surface marker includes any method for separation based on such markers. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

In some embodiments, prior to the further expansion of T cells from the co-culture, the provided methods further involve enrichment or selection of tumor reactive T cells or T cells that are likely or suspected of being tumor reactive T cells. In some embodiments, such enrichment includes selecting or isolating T cells from the co-culture that are surface positive for one or more T cell activation markers associated with tumor reactive T cells. In some embodiments, T cells selected from the co-culture results in a population of T cells enriched for CD3+ T cells or CD4+ cells and CD8+ cells, that are further positive for one of more of such T cell activation marker. In some embodiments, such cells include or are enriched for tumor-reactive T cells or T cells associated with tumor-reactive T cells. For example, such CD3+ T cells, or CD4⁺ and/or CD8⁺ populations, can be further sorted into sub-populations by positive or negative selection for markers expressed or expressed to a relatively higher degree on tumor-reactive T cells or on T cells having expression of T cell activation markers associated with tumor-reactive T cells. In particular embodiments, the enriched population of cells is cultured under conditions for expansion, such as described in Section I.E.

In some aspects, positive selection is carried out for one or more T cell activation marker. When a T cell is activated by a target or mutant peptide it begins to express upregulation markers such as, but not limited to, CD107, CD107a, CD39, CD103, CD137 (4-1BB), CD59, CD90, CD38, CD30, CD154, CD252, CD134 (OX40), CD258, CD256, PD-1, TIM-3 and/or LAG-3. In particular embodiments, the upregulation marker is CD107, CD107a, CD39, CD137, CD59, CD90, CD38, or CD103. These markers can then be used to select reactive cells. In particular, among T cell activation markers are those that are upregulated and/or whose expression is specifically detected following antigen stimulation of T cells, such that antigen specific effectors can be identified as a surrogate of an antigen that is activating or stimulating the cells. For example, following antigen-induced stimulation, human T-cells undergo dynamic functional and phenotypic changes, including upregulated surface expression of multiple activation-associated molecules, such as CD25, CD69, CD38 and others. The upregulation of surface molecules provides the opportunity to identify and isolate antigen-specific T-cells, such as tumor-reactive T cells, through antibody binding of the upregulated determinant and subsequent enrichment by flow cytometry, including by methods involving magnetic separation and fluorescence-activated cell sorting (FACS).

In some embodiments, the T cell activation marker is selected for any one or more CD107, CD107a, CD39, CD103, CD137 (4-1BB), CD59, CD90, CD38, CD30, CD154, CD252, CD134, CD258, CD256, PD-1, TIM-3 and/or LAG-3. In some embodiments, the T cell activation marker is selected from any one or more of CD107, CD107a, CD39, CD103, CD59, CD90, CD38, CD30, CD154, CD252, CD134, CD258 and/or CD256. In some embodiments, the T cell activation marker is selected from any one or more of CD107a, CD39, CD103, CD59, CD90 and/or CD38.

In some embodiments, the T cell activation marker is or includes CD107a. CD107a is a lysosomal associated protein that is normally found on the T cell Surface. Upon TCR triggering, degranulation of CD8 T cells can occur rapidly, and CD107 and other lysosomal proteins can be transported to the cell membrane to facilitate the release of perforin and granzyme. For example, in some cases CD107 expression can be detected on antigen specific CD8 T cells, such as as early as 30 minutes post-stimulation. (Betts et al. (2003) J. Immunol. Methods 281:6578).

In some embodiments, the T cell activation marker is or includes CD39. In some embodiments, the T cell activation marker is or includes CD103. In some embodiments, the T cell activation marker is or includes CD59. In some embodiments, the T cell activation marker is or includes CD90. In some embodiments, the T cell activation marker is or includes CD38.

In some embodiments, the T cell activation marker is or includes CD137 (41BB). In some embodiments, the T cell activation marker is or includes CD134 (OX40).

In some embodiments, tumor-reactive T cells or T cells associated with tumor-reactive T cells are selected, enriched or isolated based on positive surface expression of at least two or more T cell activation markers, such as at least 3, 4, 5 or 6 T cell activation markers. In some embodiments, the tumor-reactive T cells or T cells associated with tumor-reactive T cells are selected, enriched or isolated based on positive surface expression of two or more of PD-1, TIM-3, LAG-3, CD137, CD107, CD107a, CD39, CD103, CD59, CD90, CD38, CD30, CD154, CD252, CD134, CD258 and/or CD256.

In some embodiments, the at least two T cell activation markers are selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38.

In some embodiments, the tumor-reactive T cells or T cells associated with tumor-reactive T cells are selected, enriched or isolated based on positive surface expression of PD-1, TIM-2, LAG-3 and/or CD137 and at least one other T cell activation marker. In some embodiments, the tumor-reactive T cells or T cells associated with tumor-reactive T cells are selected, enriched or isolated based on positive surface expression of CD137 and at least one other T cell activation marker. In some embodiments, the at least one other T cell activation marker is selected from one or more of PD-1, TIM-3, LAG-3, CD107, CD107a, CD137, CD39, CD103, CD59, CD90, CD38, CD30, CD154, CD252, CD134, CD258 and CD256. In some embodiments, the at least one other T cell activation marker is selected from one or more of CD107, CD107a, CD39, CD103, CD59, CD90, CD38, CD30, CD154, CD252, CD134, CD258 and CD256. In some embodiments, the at least one other T cell activation marker is selected from one or more of CD107a, CD39, CD103, , CD59, CD90 and CD38. In some embodiments, the tumor-reactive T cells or T cells associated with tumor-reactive T cells are selected, enriched or isolated based on positive surface expression of CD107a and CD137, CD38 and CD137, CD103 and CD137, CD59 and CD137, CD90 and CD137 and CD38 and CD137.

In some embodiments, the T cell activation marker includes CD137 (41BB) and CD134 (OX40).

In some embodiments, tumor-reactive T cells are selected using an MHC tetramer bound to a mutation-associated or tumor-associated peptide. In some embodiments, the tetramers are prepared using MHC class I or MHC class II algorithms. In some embodiments, the tetramer is detectably labeled, such as fluorescently labeled. In some embodiments, the tetramer is HLA-matched to the subject from which the source of biological cells is obtained. In some embodiments, selection of cells using an MHC tetramer is directly from a cell source, e.g. peripheral blood, for a sample from a subject. In some embodiments, selection of cells using an MHC tetramer is after selecting or enriching T cells that are surface positive for a T cell activation marker.

Methods of isolating, selecting and/or enriching for cells can be by any of a variety of methods, such as by positive or negative selection based, such as by using any methods as described in Section I.B above. In some embodiments, methods can include immunoaffinity-based selections. In some embodiments, the T cells can be enriched or sorted a variety of ways including, but not limited to, magnetic bead separation, fluorescent cell sorting, and disposable closed cartridge based cell sorters. In particular aspects, one or more T cell activation markers can be used to select reactive cells using, but not limited to, florescent antibodies, nanoparticles or beads on cell selection equipment, but not limited to, the CliniMACS, Sony FX500 or the Tyto cell sorting systems (Miltenyi).

In some embodiments, the selections produces an enriched population of cells, such as a population of cells enriched for CD3+ T cells or CD4+ cells and CD8+ cells, that are further positive for one of more of such T cell activation marker. In some embodiments, such cells include or are enriched for tumor-reactive T cells or T cells associated with tumor-reactive T cells. In some embodiments, the enriched population of cells is used in subsequent processing steps, such as subsequent processing steps involving incubation, stimulation or activation, and/or expansion in accord with one or more steps of any of the provided methods.

In some embodiments, the enriched population of cells are enriched cells from a starting sample as describe above, in which the percentage of cells of a particular phenotype, e.g. tumor-reactive CD3+ T cells or CD3+ T cells surface positive for one or more T cell activation marker, in the enriched population of cells in increased by at least 10%, 20%, 30%, 40%, 50%, 60%, 70%, 80%, 90%, 100%, 500%, 1000%, 5000% or more greater than the percentage of such cells in the starting sample. In some embodiments, the purity of tumor-reactive CD3+ T cells or CD3+ T cells surface positive for one or more T cell activation marker in the enriched composition, i.e. the percentage of cells positive for the selected cell surface marker versus total cells in the population of enriched cells, is at least 90%, 91%, 92%, 93%, 94%, and is generally at least 95%, 96%, 97%, 98%, 99% or greater.

### E. Further Expansion and Harvesting

In some embodiments, the T cells from the co-culture, or selected T cells therefrom, are further incubated under conditions to expand the cells ex vivo following the co-culture. The incubation is carried out in the presence of one or more T cell stimulatory agent(s) under conditions for stimulating the T cells, such as to expand the T cells. The T cell stimulatory agent(s) can include any as described in Section B.2 above.

Either before the co-culture in the presence of APCs or after the selection of reactive cells, the T cells are incubated with one or more T-cell stimulating agents of lymphocytes, such as but not limited to anti-CD3 and anti-CD28 presented by APC's or as soluble antibodies, to produce a population of activated T cells. In provided embodiments, this expansion can occur over the course of 7-20 days in the presence of a costimulatory agonist, such as a Tumor Necrosis Factor Super Family Receptor (TNFSR) agonists including but not limited to agonists of OX40 and 41BB, and/or in the presence of apoptosis inhibitors including but not limited to caspase inhibitors or inhibitors of the Fas/Fas ligand axis. These soluble agonists and apoptosis inhibitors can be present in the culture for up to the maximum culture time of the expansion step or a minimum of 24 hours. In general, the culturing and incubations can occur in the presence of recombinant cytokines (e.g. IL-2, IL-7, IL-15 and/or IL-21). The expansion methods can be carried out under GMP conditions, including in a closed automated system and using serum free medium. Upon reaching a therapeutic dose after expansion the product can be concentrated and frozen in crypreservation medium. Also provided herein are populations of T cells produced by methods described herein and pharmaceutical compositions thereof.

In some of any of the provided embodiments, the T cell stimulatory agent(s) is selected from an agent that initiates TCR/CD3 intracellular signaling and/or an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include theUHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the T cell stimulatory agent(s) can include adding adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

In general, the culturing and incubations can occur in the presence of recombinant cytokines. In some embodiments, the cytokine is added or is exogenous to the culture media. In some of any of the provided embodiments, the culturing is carried out in the presence of a recombinant cytokine selected from the group consisting of IL-2, IL-15, IL-7 and IL-21. In some embodiments, the culturing and incubation is carried out in the presence of recombinant IL-2, IL-15 and IL-7. In some embodiments, the culturing is carried out in the presence of a IL-2. In some embodiments, the culturing is carried out in the presence of IL-15 and IL-17, which, in some aspects does not additionally include IL-2.

The recombinant cytokine generally is a recombinant human protein. In particular embodiments, the recombinant cytokine is present in the cell culture medium during the incubation at a concentration of at least or at least about 0.5 IU/mL, at least or at least about 1.0 IU/mL, at least or at least about 5 IU/mL, at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

In provided embodiments, this expansion can occur in the presence of a T cell adjuvant, such as any described in Section II. In some aspects, the T cell adjuvant is a costimulatory agonist, such as a Tumor Necrosis Factor Super Family Receptor (TNFSR) agonists including but not limited to agonists of OX40 and 41BB. In some embodiments, the T cell adjuvant is an apoptosis inhibitors including but not limited to caspase inhibitors or inhibitors of the Fas/Fas ligand axis. These soluble agonists and apoptosis inhibitors can be present in the culture for up to the maximum culture time of the expansion step or a minimum of 24 hours.

The sorted or selected T cells can be expanded using a cell expansion system by transfer to the cell to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 x 10⁶ cells/mL to 1.5 x 10⁶ cells/mL. In some embodiments, the density is at or about 0.5 x 10⁶ cells/mL, 0.75 x 10⁶ cells/mL, 1 x 10⁶ cells/mL, 1.25 x 10⁶ cells/mL or 1.5 x 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

In some embodiments, expansion is carried out using a Xuri cell expansion system bioreactor. The cells can be seeded at 0.5-1.5 million cells per mL. The cells can be cultured under static or rocking conditions. The medium can be added in bolus or on a perfusion schedule. In embodiments, the bioreactor maintains the temperature at or near 37°C and CO2 levels at or near 5%. The volume of the culture can be maintained at approximately 0.5 L to 1.0 L. In some embodiments, the expansion is carried out for 7-14 days such as 7-10 days. In some aspects, expansion results in a 100 million to 50 billion cells after the expansion and/or in a fold-expansion of 10 to 1000-fold expansion.

In some embodiments, expansion is carried out using a Miltenyi Prodigy bioreactor. The cells can be seeded at 0.5-1.5 million cells per mL. The cells can be cultured under static or shaking conditions. The medium can be added in bolus or on a perfusion schedule. In embodiments, the bioreactor maintains the temperature at or near 37°C and CO2 levels at or near 5%. The volume of the culture can be maintained at approximately 70 mL to 400 mL. In some embodiments, the expansion is carried out for 7-14 days such as 7-10 days. In some aspects, expansion results in a 100 million to 3 billion cells after the expansion and/or in a 10 to 1000-fold expansion.

In some embodiments, expansion is carried out using a gas-permeable bag. The cells can be seeded at 0.5-1.5 million cells per mL. The cells can be cultured under static conditions. In embodiments, the bioreactor maintains the temperature at or near 37°C and CO2 levels at or near 5%. In such aspects, when the cell concentration exceeds 2.0 million cells per mL, medium can be added to bring the cell concentration to between 0.5 and 1.0 million cells per mL. If the volume reaches the maximum volume of the bag the cells would be added to a larger bag or multiple bags for culture under the same conditions. In some embodiments, the expansion is carried out for 7-14 days such as 7-10 days.

The expansion methods can be carried out under GMP conditions, including in a closed automated system and using serum free medium. In some embodiments, any one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, the incubation with the T cell stimulatory agent(s) for expansion of tumor-reactive cells is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation with the T cell stimulatory agent(s) for expansion of tumor-reactive cells is carried out for 7-21 days, such as 7 days, 8 days, 9 days, 10 days, 11 days, 12 days, 13 days, 14, days, 15 days, 16 days, 17 days, 18 days, 19 days, 20 days or 21 days, or any value between any of the foregoing. In some embodiments, the incubation is carried out for 7-14 days. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days. In some cases, media can be exchanged daily, every other day, every third day, every 5^{th} day or once a week during the time of the culture or incubation. In some embodiments, the stimulating agents (e.g. cytokines, anti-CD3) are replenished at each media exchange.

In some embodiments, the methods of culturing for expanding cells in accord with any of the provided methods is carried out until a threshold amount of cells, such as tumor-reactive cells or cells positive for one or more T cell activation marker, is obtained. In some embodiments, the method of culturing for expanding cells in accord with any of the provided method is carried out until up to 30 days from the time of enriching the lymphocytes. For example, in particular embodiments, the method of culturing for expanding cells in accord with any of the provided methods is carried out until up to 30 days from the time of initiating the culturing. In some embodiments, the method of culturing for expanding cells in accord with any of the provided method is carried out until up to 20 days after the initiation of the first expansion. In some embodiments, the method of culturing for expanding cells in accord with any of the provided methods is carried out until up to 20 days after initiation of the co-culturing. In some of any of the provided embodiments, harvesting is carried out within 20 days after initiation of the culturing and/or the enriching of T cells comprising tumor-reactive cells. In some aspects, the cells are harvested at a time that is between at or about 7 days, at or about 8 days, at or about 9 days, at or about 10 days, at or about 11 days, at or about 12 days, at or about 13 days, at or about 14 days, at or about 15 days, at or about 16 days, at or about 17 days, at or about 18 days, at or about 19 dayas, at or about 20 days, at or about 21 days, at or about 22 days, at or about 22 days, at or about 23 days, at or about 24 days, at or about 25 days, at or about 26 days, at or about 27 days, at or about 28 days, at or about 29 days, at or about 30 days, or any value between any of the foregoing. In some of any of the provided embodiments, the cells are harvested 7 to 20 days, 7 to 14 days, 7 to 10 days, 10 to 20 days, 10 to 14 days or 14 to 20 days after the initiation of the culturing. It is understood that reference to the number of days is with reference to days in which the cells are present in a culture and do not include time in which the cells from any one or more of the steps may be stored under conditions for cryopreservation.

In some of any of the provided embodiments, the culturing is carried out until a threshold amount of cells is achieved that is between at or about 0.5 x 10⁸ and at or about 50 x 10⁹ total cells or total viable cells, between at or about 0.5 x 10⁸ and at or about 30 x 10⁹ total cells or total viable cells, between 0.5 x 10⁸ and at or about 12 x 10⁹ total cells or total viable cells, between at or about 0.5 x 10⁸ and at or about 60 x 10⁸ total cells or total viable cells, between at or about 0.5 x 10⁸ and at or about 15 x 10⁸ total cells or total viable cells, between at or about 0.5 x 10⁸ and at or about 8 x 10⁸ total cells or total viable cells, between at or about 0.5 x 10⁸ and at or about 3.5x 10⁸ total cells or total viable cells, between at or about 0.5 x 10⁸ and at or about 1 x 10⁸ total cells or total viable cells, between 1 x 10⁸ and at or about 50 x 10⁹ total cells or total viable cells, between at or about 1 x 10⁸ and at or about 30 x 10⁹ total cells or total viable cells, between 1 x 10⁸ and at or about 12 x 10⁹ total cells or total viable cells, between at or about 1 x 10⁸ and at or about 60 x 10⁸ total cells or total viable cells, between at or about 1 x 10⁸ and at or about 15 x 10⁸ total cells or total viable cells, between at or about 1 x 10⁸ and at or about 8 x 10⁸ total cells or total viable cells, between at or about 1 x 10⁸ and at or about 3.5x 10⁸ total cells or total viable cells, between at or about 3.5 x 10⁸ and at or about 50 x 10⁹ total cells or total viable cells, between at or about 3.5 x 10⁸ and at or about 30 x 10⁹ total cells or total viable cells, between at or about 3.5 x 10⁸ and at or about 12 x 10⁹ total cells or total viable cells, between at or about 3.5 x 10⁸ and at or about 60 x 10⁸ total cells or total viable cells, between at or about 3.5 x 10⁸ and at or about 15 x 10⁸ total cells or total viable cells, between at or about 3.5 x 10⁸ and at or about 8 x 10⁸ total cells or total viable cells, between at or about 8 x 10⁸ and at or about 50 x 10⁹ total cells or total viable cells, between at or about 8 x 10⁸ and at or about 30 x 10⁹ total cells or total viable cells, between at or about 8 x 10⁸ and at or about 12 x 10⁹ total cells or total viable cells, between at or about 8 x 10⁸ and at or about 60 x 10⁸ total cells or total viable cells, between at or about 8 x 10⁸ and at or about 15 x 10⁸ total cells or total viable cells, between at or about 15 x 10⁸ and at or about 50 x 10⁹ total cells or total viable cells, between at or about 15 x 10⁸ and at or about 30 x 10⁹ total cells or total viable cells, between at or about 15 x 10⁸ and at or about 12 x 10⁹ total cells or total viable cells, between at or about 15 x 10⁸ and at or about 60 x 10⁸ total cells or total viable cells, between at or about 60 x 10⁸ and at or about 50 x 10⁹ total cells or total viable cells, between at or about 60 x 10⁸ and at or about 30 x 10⁹ total cells or total viable cells, between at or about 60 x 10⁸ and at or about 12 x 10⁹ total cells or total viable cells, between at or about 12 x 10⁹ and at or about 50 x 10⁹ total cells or total viable cells, between at or about 12 x 10⁹ and at or about 30 x 10⁹ total cells or total viable cells, or between at or about 30 x 10⁹ and at or about 60 x 10⁹ total cells or total viable cells, each inclusive.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more.

Upon reaching a therapeutic dose after expansion the product can be concentrated and frozen in cryopreservation medium. Also provided herein are populations of T cells produced by methods described herein and pharmaceutical compositions thereof.

### II. T Cell Modulatory Agents or Adjuvants

In aspects of any of the provided methods, a population of T cells is incubated in the presence of one or more T cell adjuvant. In particular embodiments, the incubation is carried out under conditions in which the adjuvant promotes or increases the survival of T cells in the population, reduces or prevents apoptosis and/or sustains expansion of cells by the provided methods. In some embodiments, the T cell adjuvant is or can include a costimulatory agonist, such as a Tumor Necrosis Factor Super Family Receptor (TNFSR) agonists including but not limited to agonists of OX40 and 41BB. In some embodiments, the T cell adjuvant is or can include an apoptosis inhibitor including but not limited to caspase inhibitors or inhibitors of the Fas/Fas ligand axis. In some embodiments, the T cell adjuvant is a check point modulator, such as a checkpoint inhibitor, including but not limited to antagonists of PD-1. In some embodiments, the T cell adjuvant is or can include a heat shock protein inhibitor including but not limited to inhibitors of the Hsp90 protein.

In aspects of the provided methods, the one or more T cell adjuvant (e.g. costimulatory agonist and/or apoptosis inhibitor and/or checkpoint inhibitor) can be included during one or more or all of the steps of the provided methods. In some embodiments, the one or more T cell adjuvant is included during the first or initial expansion of T cells from the biological sample. In some embodiments, the one or more T cell adjuvant is including during the second or final expansion of T cells after enrichment of tumor-reactive T cells from the co-culture. In some embodiments, the one or more T cell adjuvant is included during both the first expansion and the second expansion. In some cases, the one or more T cell adjuvant is included during the co-culture of T cells with the APCs/peptide neoepitopes.

In embodiments of any of the provided methods, the incubation with each of the at least one T cell adjuvant, such as one or more costimulatory agonist, apoptosis inhibitor, checkpoint inhibitor, or heat shock protein inhibitor, is independently continued during the entire course of the culturing or during a portion thereof. In some embodiments, the incubation with each of the at least one T cell adjuvant is for no more than 14 days, no more than 12 days, no more than 10 days, no more than 7 days, no more than 5 days, no more than 3 days or no more than 2 days. In some embodiments, the incubation with each of the at least one T cell adjuvant is independently for 12 hours to 96 hours, such as 24 hours to 48 hours, such as generally at or about 48 hours.

### A. Agents or Adjuvants

### 1. Costimulatory Agonist

In aspects of any of the provided methods, a population of T cells is incubated in the presence of one or more costimulatory agonist. In particular embodiments, the costimulatory agonist is a molecule that specifically binds to a costimulatory molecule on the surface of T cells to stimulate one or more intracellular signal in the cell and/or to stimulate one or more functional or biological activity of the T cell. In some embodiments, the agonist promotes the survival and activity of the T cell. In some embodiments, the costimulatory molecule is a member of the tumor necrosis factor superfamily of receptors (TNFSR). Exemplary costimulatory molecules include, but are not limited to, 4-1BB, OX40, GITR and CD27. In some embodiments, the costimulatory agonist is a 4-1BB agonist, an OX40 agonist, a GITR agonist or a CD27 agonist.

In some embodiments, the costimulatory agonist is or comprises an antibody or antigen-binding fragment that specifically binds to the costimulatory receptor.

In some embodiments, the costimulatory agonist is or comprises an extracellular binding domain, or a specific binding portion thereof, of a ligand of the costimulatory receptor. In some cases, the extracellular binding domain, or a specific binding fragment thereof, is provided as fusion protein with another polypeptide, such as to increase binding avidity of the agonist. For example, in some cases the polypeptide is a multimerization domain that can promote dimerization, trimerization, tetramerization or pentamerization of the molecule. In particular embodiments, the fusion protein is a dimer. In some embodiments, the multimerization domain includes any sequence of amino acids that can interact with a complementary multimerization domain to form a stable protein-protein interaction to produce a multimer of the polypeptide molecule with another polypeptide molecule. For example, a multimerization domain can be a molecule that is able to form disulfide bonds with a complementary molecule. Exemplary multimerization domains include immunoglobulin sequences or portions thereof, leucine zippers, hydrophobic regions, hydrophilic regions, and compatible protein-protein interaction domains. The multimerization domain, for example, can be an immunoglobulin constant region or domain, such as, for example, the Fc domain or portions thereof from IgG, including IgG1, IgG2, IgG3 or IgG4 subtypes, IgA, IgE, IgD and IgM and modified forms thereof. In some embodiments, the costimulatory agonist is an Fc fusion protein.

In some embodiments, the costimulatory agonist is an OX40 (CD134) agonist. OX40, a cell surface glycoprotein and member of the tumor necrosis factor receptor family (TNFRSF), is expressed on T-lymphocytes and provides a co-stimulatory signal for the proliferation and survival of activated T-cells. OX40 generally is not constitutively expressed on resting naive T cells, unlike CD28. OX40 is a secondary co-stimulatory immune checkpoint molecule, which, in some aspects, is expressed after 24 to 72 hours following activation; its ligand, OX40L, is also not expressed on resting antigen presenting cells, but is following their activation. Expression of OX40 is dependent on full activation of the T cell; in some cases, such as without CD28 stimulation, expression of OX40 is delayed and its expression is lower. OX40 can be expressed on T cells in the body (co-culture with tumor), after activation (e.g. with an anti-CD3, such asOKT3/anti-CD28) or after an ex vivo co-culture of APC induced to present a tumor antigen target. Binding of OX40 by OX40L triggers an activation of the OX40 pathway. In some embodiments, activation of this pathway leads to upregulation of other pathways leading to increased activation, survival, memory response, and reduction of immune suppressive activity.

In some embodiments, the OX40 agonist may be an antibody or antigen-binding fragment or a fusion protein capable of binding to human or mammalian OX40. In some embodiments, an OX40 agonist binds to human OX40, such as human OX40 expressed on the surface of a T cell. In some embodiments the OX40 agonist specifically binds to OX40 and abrogates antibody-dependent cellular toxicity (ADCC), for example NK cell cytotoxicity. In some embodiments, the OX40 agonist abrogates antibody-dependent cell phagocytosis (ADCP). In some embodiments, the OX40 agonist abrogates complement-dependent cytotoxicity (CDC). In some aspects, when an OX40 agonist binds to the OX40 protein receptor, it triggers a co-stimulatory signal that is associated with increased production of T cells and inflammatory cytokines. OX40 agonists for use in the provided methods include any known to a skilled artisan.

In some embodiments, an OX40 agonist is a fusion protein. OX40 fusion proteins include those comprising an Fc domain fused to a portion of OX40L, see e.g. Sadun et al., (2009) J. Immunother., 182:1481-89. In some embodiments, a multimeric OX40 agonist, such as a trimeric or hexameric OX40 agonist (with three of six ligand binding domains) may be used. Trimeric (trivalent) or hexameric (or hexavalent) or greater fusion proteins containing three TNFRSF binding domains and as a fusion with an Fc are known and can be used, see e.g. Gieffers et al. (2013) Cancer Therapeutics, 12:2735-47.

In some embodiments, the OX40 agonist is a fusion protein in which one or more domains of OX40L is covalently linked to one or more additional protein domains. Exemplary OX40L fusion proteins that can be used as OX40 agonists are described in U.S. Patent Nos. 6,312,700; 7,622,444, International Patent Application Publication Nos. WO2011109789; and WO2010105068. In some embodiments, the OX40 agonist includes an OX40L fusion polypeptide that self- assembles into a multimeric (e.g., trimeric or hexameric) OX40L fusion protein. Such fusion proteins are described, e.g., in Morris et al. (2007) Mol Immunol. 44(12): 3112-3121, U.S. Patent No. 7,959,925. A specific fusion protein that can be used according to some embodiments provided herein is MEDI6383 (produced by AZY/Medlmmune), a human OX40 ligand fusion protein, see e.g. U.S. Patent No. 6,312,700.

In some embodiments, an OX40 agonist is an antibody or antigen-binding fragment that specifically binds OX40. Exemplary OX40 agonists for use in the provided methods include, but are not limited to, tavolixizumab (also known as MEDI0562 or MEDI-0562), 11D4 (see U.S. Patent Nos. 7,960,515; 8,236,930; 9,028,824), 18D8 (see e.g. U.S. Patent Nos. 7,960,515; 8,236,930; 9,028,824); Hu119-122 (see e.g. U.S. Patent Nos. 9,006,399 and 9,163,085, and International Patent Publication No. WO2012/027328); Hu106-222 (see e.g. U.S. Patent Nos. 9,006,399 and 9,163,085, and International Patent Publication No. WO2012/027328); MEDI6469 (also known as 9B12; see e.g. Weinberg et al. (2006) J. Immunother., 29:575-585); pogalizumab (also known as MOXR0916 and RG7888; Genentech, Inc.); GSK3174998 (GlaxoSmithKline), or PF-04518600 (PF-8600; Hamid et al. (2016) Journal of Clinical Immunology, 34:3079); BMS 986178; or an antigen-binding fragment of any of the foregoing. An OX40 agonist also includes any binding molecule, such as any antibody or antigen-binding fragment, that contains six CDRs as contained in tavolizizumab, 11D4, 18D8, Hu119-122 , Hu106-22, MED16469, pogalizumab, GSK3174998, PF-04518600 or BMS 986178.

In some embodiments, the OX40 agonist is an OX40 agonist set forth in any of International Patent Application Publication Nos. WO 95/12673, WO 95/21925, WO 2006/121810, WO 2012/027328, WO 2013/028231, WO 2013/038191, and WO 2014/148895; European Patent Application EP 0672141; U.S. Patent Application Publication Nos. US 2010/136030, US 2014/377284, US 2015/190506, and US 2015/132288 (including clones 20E5 and 12H3); and U.S. Patent Nos. 7,504,101, 7,550,140, 7,622,444, 7,696,175, 7,960,515, 7,961,515, 8,133,983, 9,006,399, and 9,163,085. An OX40 agonist also may include commercially available antibodies such as L106 BD (Pharmingen Product #340420); ACT35 (Santa Cruz Biotechnology, Catalog #20073); or anti-mCD134/mOX40 (clone OX86), commercially available from InVivoMAb, BioXcell Inc, West Lebanon, NH.

Other OX40 agonists that can be used according to any of the provided embodiments include nucleotides, expression vectors and peptides, such as disclosed for example in Linch et al. (2015) Front Oncol. 5: 34, U.S. Patent No. 6,312,700 and U.S. Application Publication No. 20140271677.

In some embodiments, the costimulatory agonist is a 4-1BB (CD137) agonist. In some embodiments, the 4-1BB agonist may be an antibody or antigen-binding fragment or a fusion protein capable of binding to human or mammalian 4-1BB. In some embodiments, a 4-1BB agonist binds to human 4-1BB, such as human 4-1BB expressed on the surface of a T cell. 4-1BB (CD137, tumor necrosis factor receptor superfamily 9) is an inducible costimulatory receptor expressed on activated T and natural killer (NK) cells. 4-1BB ligation on T cells triggers a signaling cascade that results in upregulation of antiapoptotic molecules, cytokine secretion, and enhanced effector function. In dysfunctional T cells that have a decreased cytotoxic capacity, 4-1BB ligation demonstrates a potent ability to restore effector functions. On NK cells, 4-1BB signaling can increase antibody-dependent cell-mediated cytotoxicity. Agonistic monoclonal antibodies targeting 4-1BB have been developed to harness 4-1BB signaling for cancer immunotherapy. Preclinical results in a variety of induced and spontaneous tumor models suggest that targeting 4-1BB with agonist antibodies can lead to tumor clearance and durable antitumor immunity.

In some embodiments the 4-1BB agonist binds specifically to 4-1BB in a manner sufficient to reduce toxicity. In some embodiments, the 4-1BB agonist is an agonistic 4-lBB monoclonal antibody or fusion protein that abrogates antibody-dependent cellular toxicity (ADCC), for example NK cell cytotoxicity. In some embodiments, the 4-lBB agonist is an agonistic 4-1BB monoclonal antibody or fusion protein that abrogates antibody-dependent cell phagocytosis (ADCP). In some embodiments, the 4-lBB agonist is an agonistic 4-lBB monoclonal antibody or fusion protein that abrogates complement-dependent cytotoxicity (CDC).

In some embodiments, a 4-1BB agonist is a fusion protein. 4-1BB fusion proteins include those comprising an Fc domain fused to 4-1BBL. In some embodiments, the fusion protein is a dimeric (divalent), trimeric (trivalent) or hexameric (hexavalent) or greater fusion comprising two or more, such as three, four or more, domains of 4-1BBL for binding 4-1BB fused to an Fc.

In an embodiment, the 4- 1BB agonist is a 4-1BB agonistic fusion protein described in International Patent Application Publication Nos. WO 2008/025516 A1, WO 2009/007120 A1, WO 2010/003766 A1, WO 2010/010051 A1, and WO 2010/078966 A1; U.S. Patent Application Publication Nos. US 2011/0027218 Al, US 2015/0126709 Al, US 2011/0111494 A1, US 2015/0110734 A1, and US 2015/0126710 A1; and U.S. Patent Nos. 9,359,420, 9,340,599, 8,921,519, and 8,450,460.

In some embodiments, a 4-1BB agonist is an antibody or antigen-binding fragment that specifically binds 4-1BB. In some embodiments, the 4-1BB agonist is EU-101 (Eutilex Co. Ltd.) utomilumab, or urelumab, or an antigen-binding fragment thereof. In some embodiments, the 4-1BB agonist is utomilumab (also known as PF-05082566, PF-2566, or MOR-7480). The preparation and properties of utomilumab and its variants and fragments are described in U.S. Patent Nos. 8,821,867; 8,337,850; and 9,468,678, and International Patent Application Publication No. WO 2012/032433 A1. In some embodiments, the 4-1BB agonist is urelumab (also known as BMS-663513 or 20H4,9.h4a. The preparation and properties of urelumab and its variants and fragments are described in U.S. Patent Nos. 7,288,638 and 8,962,804. An OX40 agonist also includes any binding molecule, such as any antibody or antigen-binding fragment, that contains six CDRs as contained in utomilumab or urelumab.

In an embodiment, the 4- 1BB agonist is selected from the group consisting of 1D8, 3Elor, 4B4 (BioLegend 309809), H4-1BB-Ml27 (BD Pharmingen 552532), BBK2 (Thermo Fisher MS621PABX), 145501 (Leinco Technologies B591), the antibody produced by cell line deposited as ATCC No. HB-11248 and disclosed in U.S. Patent No. 6,974,863, 5F4 (BioLegend 31 1503), C65-485 (BD Pharmingen 559446), antibodies disclosed in U.S. Patent Application Publication No. US 2005/0095244, antibodies disclosed in U.S. Patent No. 7,288,638 (such as 20H4.9-IgGl (BMS-663031)), antibodies disclosed in U.S. Patent No. 6,887,673 (such as 4E9 or BMS-554271), antibodies disclosed in U.S. Patent No. 7,214,493, antibodies disclosed in U.S. Patent No. 6,303,121, antibodies disclosed in U.S. Patent No. 6,569,997, antibodies disclosed in U.S. Patent No. 6,905,685 (such as 4E9 or BMS-554271), antibodies disclosed in U.S. Patent No. 6,362,325 (such as 1D8 or BMS-469492; 3H3 or BMS-469497; or 3El), antibodies disclosed in U.S. Patent No. 6,974,863 (such as 53A2); antibodies disclosed in U.S. Patent No. 6,210,669 (such as 1D8, 3B8, or 3El), antibodies described in U.S. Patent No. 5,928,893, antibodies disclosed in U.S. Patent No. 6,303,121, antibodies disclosed in U.S. Patent No. 6,569,997, antibodies disclosed in International Patent Application Publication Nos. WO 2012/177788, WO 2015/119923, and WO 2010/042433, and fragments, derivatives, conjugates, variants, or biosimilars thereof.

In some embodiments, the costimulatory agonist is a CD27 agonist. In some embodiments the CD27 agonist binds specifically to CD27 in a manner sufficient to reduce toxicity. In some embodiments, the CD27 agonist is an agonistic CD27 monoclonal antibody or fusion protein that abrogates antibody-dependent cellular toxicity (ADCC), for example NK cell cytotoxicity. In some embodiments, the CD27 agonist is an agonistic CD27 monoclonal antibody or fusion protein that abrogates antibody-dependent cell phagocytosis (ADCP). In some embodiments, the CD27 agonist is an agonistic CD27 monoclonal antibody or fusion protein that abrogates complement-dependent cytotoxicity (CDC).

In some embodiments, a CD27 agonist is an antibody or antigen-binding fragment that specifically binds CD27. In a particular embodiment, the CD27 agonist is the monoclonal antibody varlilumab ( also known as CDX-1127 or IFS), is an antigen-binding fragment thereof. The preparation and properties of varlilumab are described in International Patent Application Publication No. WO 2016/145085 A2 and U.S. Patent Application Publication Nos. US 2011/0274685 A1 and US 2012/0213771 A1.

In some embodiments, a CD27 agonist is a fusion protein. CD27 fusion proteins include those comprising an Fc domain fused to a ligand of CD27 (CD70). In some embodiments, the fusion protein is a dimeric (divalent), trimeric (trivalent) or hexameric (hexavalent) or greater fusion comprising two or more, such as three, four or more CD70 domains for binding CD27 fused to an Fc.

In an embodiment, the CD27 agonist is a CD27 agonist described in U.S. Patent Application Publication No. US 2014/0112942 A1, US 2011/0274685 A1, or US 2012/0213771 Al, or International Patent Application Publication No. WO 2012/004367 A1.

In some embodiments, the costimulatory agonist is a GITR agonist. In some embodiments the GITR agonist binds specifically to GITR in a manner sufficient to reduce toxicity. In some embodiments, the GITR agonist is an agonistic GITR monoclonal antibody or fusion protein that abrogates antibody-dependent cellular toxicity (ADCC), for example NK cell cytotoxicity. In some embodiments, the GITR agonist is an agonistic GITR monoclonal antibody or fusion protein that abrogates antibody-dependent cell phagocytosis (ADCP). In some embodiments, the GITR agonist is an agonistic GITR monoclonal antibody or fusion protein that abrogates complement-dependent cytotoxicity (CDC).

In some embodiments, a GITR agonist is an antibody or antigen-binding fragment that specifically binds GITR. In an embodiment, the GITR agonist is the agonistic, anti-GITR monoclonal antibody TRX518 (TolerRx, Inc.), also known as 6C8 and Ch-6C8-Agly. The preparation, properties, and uses of 6C8 and 2F8 antibodies, and their variants, are described in U.S. Patent Nos. 7,812,135; 8,388,967; and 9,028,823.

In some embodiments, the GITR agonist is the monoclonal antibody 1D7, or an antigen-binding fragment thereof. The preparation and properties of 1D7 are described in U.S. Patent Application Publication No. US 2015/0064204 A1. In an embodiment, the GITR agonist is the monoclonal antibody 33C9, or an antigen-binding fragment thereof. The preparation and properties of 33C9 are described in U.S. Patent Application Publication No. US 2015/0064204 A1. In an embodiment, the GITR agonist is the monoclonal antibody 33F6, or is an antigen-binding fragment thereof. The preparation and properties of 33F6 are described in U.S. Patent Application Publication No. US 2015/0064204 A1. In an embodiment, the GITR agonist is the monoclonal antibody 34G4, or is an antigen-binding fragment thereof. The preparation and properties of 34G4 are described in U.S. Patent Application Publication No. US 2015/0064204 A1. In an embodiment, the GITR agonist is the monoclonal antibody 35B 10, or is an antigen-binding fragment thereof. The preparation and properties of 35B1O are described in U.S. Patent Application Publication No. US 2015/0064204 A1. In an embodiment, the GITR agonist is the monoclonal antibody 41E11, or is an antigen-binding fragment thereof. The preparation and properties of 41E11 are described in U.S. Patent Application Publication No. US 2015/0064204 Al. In an embodiment, the GITR agonist is the monoclonal antibody 41GS, or is an antigen-binding fragment thereof. The preparation and properties of 41G5 are described in U.S. Patent Application Publication No. US 2015/0064204 A1. In an embodiment, the GITR agonist is the monoclonal antibody 42A11, or is an antigen-binding fragment thereof. The preparation and properties of 42A11 are described in U.S. Patent Application Publication No. US 2015/0064204 A1. In an embodiment, the GITR agonist is the monoclonal antibody 44C1, or is an antigen-binding fragment thereof. The preparation and properties of 44C1 are described in U.S. Patent Application Publication No. US 2015/0064204 A1. In an embodiment, the GITR agonist is the monoclonal antibody 45A8, or is an antigen-binding fragment thereof. The preparation and properties of 45A8 are described in U.S. Patent Application Publication No. US 2015/0064204 A1. In an embodiment, the GITR agonist is the monoclonal antibody 46E11, or is an antigen-binding fragment thereof. The preparation and properties of 46E11 are described in U.S. Patent Application Publication No. US 2015/0064204 A1. In an embodiment, the GITR agonist is the monoclonal antibody 48H12, or is an antigen-binding fragment thereof. The preparation and properties of 48H12 are described in U.S. Patent Application Publication No. US 2015/0064204 A1. In an embodiment, the GITR agonist is the monoclonal antibody 48H7, or is an antigen-binding fragment thereof. The preparation and properties of 48H7 are described in U.S. Patent Application Publication No. US 2015/0064204 A1. In an embodiment, the GITR agonist is the monoclonal antibody 49D9, or is an antigen-binding fragment thereof. The preparation and properties of 49D9 are described in U.S. Patent Application Publication No. US 2015/0064204 Al. In an embodiment, the GITR agonist is the monoclonal antibody 49E2, or is an antigen-binding fragment thereof. The preparation and properties of 49E2 are described in U.S. Patent Application Publication No. US 2015/0064204 A1. In an embodiment, the GITR agonist is the monoclonal antibody 48A9, or is an antigen-binding fragment thereof. The preparation and properties of 48A9 are described in U.S. Patent Application Publication No. US 2015/0064204 Al. In an embodiment, the GITR agonist is the monoclonal antibody 5H7, or is an antigen-binding fragment thereof. The preparation and properties of 5H7 are described in U.S. Patent Application Publication No. US 2015/0064204 A1. In an embodiment, the GITR agonist is the monoclonal antibody 7A10, or is an antigen-binding fragment thereof. The preparation and properties of 7A10 are described in U.S. Patent Application Publication No. US 2015/0064204 A1. In an embodiment, the GITR agonist is the monoclonal antibody 9H6, or is an antigen-binding fragment thereof. The preparation and properties of 9H6 are described in U.S. Patent Application Publication No. US 2015/0064204 A1.

In an embodiment, the GITR agonist is an agonistic anti-GITR monoclonal antibody with described in U.S. Patent No. 8,709,424; U.S. Patent Application Publication Nos. US 2012/0189639 A1 and US 2014/0348841 A1, and International Patent Application Publication No. WO 2011/028683 A1 (Merck Sharp & Dohme Corp.). In an embodiment, the GITR agonist is an agonistic, anti-GITR monoclonal antibody selected from the group consisting of 36E5, 3D6, 61 G6, 6H6, 61F6, 1D8, 17F10, 35D8, 49Al, 9E5, and 31H6, and antigen-binding fragments thereof. The structure, properties, and preparation of these antibodies are described in U.S. Patent No. 8,709,424; U.S. Patent Application Publication Nos. US 2012/0189639 A1 and US 2014/0348841 Al.

In an embodiment, the GITR agonist is a GITR agonist described in International Patent Application Publication Nos. WO 2013/039954 A1 and WO 2011/028683 Al; U.S. Patent Application Publication Nos. US 2013/0108641 Al, US 2012/0189639 A1, and US 2014/0348841 A1; and U.S. Patent Nos. 7,812,135; 8,388,967; and 9,028,823.

In embodiments of any of the provided methods, the ratio of T cells (e.g. tumor-reactive T cells) to costimulatory agonists (cells to moles) in the expansion method is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 500, about 1 to 1000, or about 1 to 10000.

In embodiments of any of the provided methods, the one or more co-stimulatory agonist is added to the cell culture medium during the incubation. In some embodiments, each of the one or more costimulatory agonist is indepedently added at a concentration ranging between at or about 0.1 µg/mL to at or about 100 µg/mL, at or about 0.1 µg/mL and at or about 50 µg/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 µg/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive.

In some embodiments, a costimulatory agonist is administered to a subject prior to isolation or selection of T cells for carrying out the culture methods for expansion. In such embodiments, it is contemplated that tumor reactive T cells or T cells surface positive for one or more activation marker as described is rejuvenated in vivo prior to ex vivo isolation, selection and/or enrichment for culturing cells in accord with the provided methods. In some such embodiments, the a costimulatory agonist is administered to a subject by infusing a dose selected from the group consisting of about 5 mg, about 8 mg, about 10 mg, about 20 mg, about 25 mg, about 50 mg, about 75 mg, 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, and about 2000 mg, or a value between any of the foregoing. In some embodiments, an effective dosage of a costimulatory agonist disclosed herein is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg.

In an embodiment, a costimulatory agonist is administered weekly. In an embodiment, a costimulatory agonist is administered every two weeks. In an embodiment, a costimulatory agonist is administered every three weeks. In an embodiment, a costimulatory agonist is administered monthly. In an embodiment, a costimulatory agonist is administered intravenously in a dose of 8 mg given every three weeks for 4 doses over a 12-week period. In an embodiment, a costimulatory agonist is administered at a lower initial dose, which is escalated when administered at subsequent intervals administered monthly. For example, the first infusion can deliver 300 mg of a costimulatory agonist, and subsequent weekly doses could deliver 2,000 mg of a costimulatory agonist for eight weeks, followed by monthly doses of 2,000 mg of a costimulatory agonist.

### 2. Immune Checkpoint Inhibitors

In some embodiments, the T cell modulatory agent is an immune checkpoint inhibitor that inhibits an immune checkpoint pathway. The immune system has multiple inhibitory pathways that are involved in maintaining self-tolerance and for modulating immune responses. It is known that tumors can use certain immune-checkpoint pathways as a major mechanism of immune resistance, particularly against T cells that are specific for tumor antigens (Pardoll, 2012, Nature Reviews Cancer 12:252-264*).* Because many such immune checkpoints are initiated by ligand-receptor interactions, they can be readily blocked by antibodies against the ligands and/or their receptors.

Therefore, therapy with antagonistic molecules blocking an immune checkpoint pathway, such as small molecules, nucleic acid inhibitors (e.g., RNAi) or antibody molecules, are becoming promising avenues of immunotherapy for cancer and other diseases.

As used herein, the term "immune checkpoint inhibitor" refers to molecules that totally or partially reduce, inhibit, interfere with or modulate one or more checkpoint proteins. Checkpoint proteins regulate T cell activation or function. These proteins are responsible for co-stimulatory or inhibitory interactions of T cell responses. Immune checkpoint proteins regulate and maintain self-tolerance and the duration and amplitude of physiological immune responses.

Immune checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptor ligands. Illustrative immune checkpoint molecules that may be targeted for blocking or inhibition include, but are not limited to, PD1 (CD279), PDL1 (CD274, B7-H1), PDL2 (CD273, B7-DC), CTLA-4, LAG3 (CD223), TIM3, 4-1BB (CD137), 4-1BBL (CD137L), GITR (TNFRSF18, AITR), CD40, OX40 (CD134, TNFRSF4), CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8+ (αβ) T cells), CD160 (also referred to as BY55) and CGEN-15049. In some embodiments, the immune checkpoint inhibitor is an antibody. Immune checkpoint inhibitors include antibodies, or antigen binding fragments thereof, or other binding proteins, that bind to and block or inhibit the activity of one or more of PD1, PDL1, PDL2, CTLA-4, LAG3, TIM3, 4-1BB, 4-1BBL, GITR, CD40, OX40, CXCR2, TAA, B7-H3, B7-H4, BTLA, HVEM, GAL9, B7H3, B7H4, VISTA, KIR, 2B4, CD160, and CGEN-15049. Illustrative immune checkpoint inhibitors include Ipilimumab (anti-CTLA4), Pembrolizumab (anti-PD1), Tremelimumab (CTLA-4 blocking antibody), anti-OX40L (e.g. oxelumbab),, and PD-L1 monoclonal antibody (Anti-B7-H1; MEDI4736).

In some embodiments, the checkpoint inhibitor inhibits the activity of PD1. Programmed cell death 1 (PD1) is an immune checkpoint protein that is expressed in B cells, NK cells, and T cells (Shinohara et al., 1995, Genomics 23:704-6*;* Blank et al., 2007, Cancer Immunol Immunother 56:739-45*;* Finger et al., 1997, Gene 197:177-87*;* Pardoll, 2012, Nature Reviews Cancer 12:252-264*).* The major role of PD1 is to limit the activity of T cells in peripheral tissues during inflammation in response to infection, as well as to limit autoimmunity *(*Pardoll, 2012, Nature Reviews Cancer 12:252-264)*.* PD1 expression is induced in activated T cells and binding of PD1 to one of its endogenous ligands acts to inhibit T-cell activation by inhibiting stimulatory kinases and also acting to inhibit the TCR "stop signal" (Pardoll, 2012, Nature Reviews Cancer 12:252-264)*.* PD1 is highly expressed on regulatory T cells and may increase their proliferation in the presence of ligand (Pardoll, 2012, Nature Reviews Cancer 12:252-264)*.* Anti-PD 1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer (Topalian et al., 2012, N Engl J Med 366:2443-54*;* Lipson et al., 2013, Clin Cancer Res 19:462-8*;* Berger et al., 2008, Clin Cancer Res 14:3044-51*;* Gildener-Leapman et al., 2013, Oral Oncol 49:1089-96*;* Menzies & Long, 2013, Ther Adv Med Oncol 5:278-85)*.* Exemplary anti-PD 1 antibodies include nivolumab (Opdivo by BMS), pembrolizumab (Keytruda by Merck), pidilizumab (CT-011 by Cure Tech), lambrolizumab (MK-3475 by Merck), and AMP-224 (Merck).

In some embodiments, the checkpoint inhibitor inhibits the activity of PD-L1. PD-L1 (also known as CD274 and B7-H1) and PD-L2 (also known as CD273 and B7-DC) are ligands for PD1, found on activated T cells, B cells, myeloid cells, macrophages, and some types of tumor cells. The complex of PD1 and PD-L1 inhibits proliferation of CD8+ T cells and reduces the immune response (Topalian et al., 2012, N EnglJ Med 366:2443-54*;* Brahmer et al., 2012, N Eng J Med 366:2455-65)*.* Anti-PD-L1 antibodies have been used for treatment of non-small cell lung cancer, melanoma, colorectal cancer, renal-cell cancer, pancreatic cancer, gastric cancer, ovarian cancer, breast cancer, and hematologic malignancies (Brahmer et al., N Eng J Med 366:2455-65*;* Ott et al., 2013, Clin Cancer Res 19:5300-9*;* Radvanyi et al., 2013, Clin Cancer Res 19:5541*;* Menzies & Long, 2013, Ther Adv Med Oncol 5:278-85*;* Berger et al., 2008, Clin Cancer Res 14:13044-51)*.* Exemplary anti-PD-L1 antibodies include MDX-1105 (Medarex), MEDI4736 (Medimmune) MPDL3280A (Genentech), BMS-935559 (Bristol-Myers Squibb) and MSB0010718C.

In some embodiments, the checkpoint inhibitor inhibits the activity of CTLA-4. Cytotoxic T-lymphocyte-associated antigen (CTLA-4), also known as CD152, is a co-inhibitory molecule that functions to regulate T-cell activation. CTLA-4 is a member of the immunoglobulin superfamily that is expressed exclusively on T-cells. CTLA-4 acts to inhibit T-cell activation and is reported to inhibit helper T-cell activity and enhance regulatory T-cell immunosuppressive activity (Pardoll, 2012, Nature Reviews Cancer 12:252-264)*.* Anti-CTLA-4 antibodies have been used in clinical trials for the treatment of melanoma, prostate cancer, small cell lung cancer, non-small cell lung cancer (Robert & Ghiringhelli, 2009, Oncologist 14:848-61*;* Ott et al., 2013, Clin Cancer Res 19:5300*;* Weber, 2007, Oncologist 12:864-72*;* Wada et al., 2013, J TranslMed 11:89*).* Exemplary anti-CTLA-4 antibodies include ipilimumab (Bristol-Myers Squibb) and tremelimumab (Pfizer). Ipilimumab has received FDA approval for treatment of metastatic melanoma *(*Wada et al., 2013, J Transl Med 11:89)*.*

In some embodiments, the checkpoint inhibitor inhibits the activity of LAG-3. Lymphocyte activation gene-3 (LAG-3), also known as CD223, is another immune checkpoint protein. LAG-3 has been associated with the inhibition of lymphocyte activity and in some cases the induction of lymphocyte anergyh. LAG-3 is expressed on various cells in the immune system including B cells, NK cells, and dendritic cells. LAG-3 is a natural ligand for the MHC class II receptor, which is substantially expressed on melanoma-infiltrating T cells including those endowed with potent immune-suppressive activity (Goldberg et al., Curr Top Microbiol Immunol (344) 269-278, 2011)*.* Exemplary anti-LAG-3 antibodies include BMS-986016, also known as relatlimab. IMP321 is a soluble version of the immune checkpoint molecule LAG-3, which activates dendritic cells, increasing antigen presentation.

In some embodiments, the checkpoint inhibitor inhibits the activity of TIM-3. T cell immunoglobulin domain and mucin domain-3 (TIM-3), also known as CD366, was initially identified on activated Th1 cells and has been shown to be a negative regulator of the immune response. Blockade of TIM-3 promotes T cell mediated anti-tumor immunity and has anti-tumor activity in a range of mouse tumor models. Combinations of TIM-3 blockade with other immunotherapeutic agents such as anti-PDL1 antibodies and others, can be additive or synergistic in increasing anti-tumor effects. TIM-3 expression has been associated with a number of different tumor types including melanoma, NSCLC and renal cancer, and additionally, expression of intratumoral TIM-3 has been shown to correlate with poor prognosis across a range of tumor types including NSCLC, cervical, and gastric cancers. Exemplary anti-TIM3 antibodies include TSR-022 and LY3321367

### 3. Apoptosis Inhibitor

In aspects of any of the provided methods, a population of T cells is incubated in the presence of one or more inhibitors of apoptosis or of an apoptotic signaling pathway in a cell (hereinafter "apoptosis inhibitor"). In some embodiment, the inclusion of an apoptosis inhibitor during the ex vivo manufacturing process of a T cell therapy results in an improved yield of T cells of interest during the expansion process. In particular aspects, such methods are used in connection with ex vivo manufacturing of tumor-reactive T cells, which represent a rare and infrequent endogenous population of cells. Even when such cells are enriched ex vivo by described co-culture methods they still may be susceptible to apoptosis during the process of expanding the cells. The provided methods rejuvenate such cells by increasing proliferation and supporting their activation and expansion while preventing or reducing apoptosis.

In some aspects, one or more phenotypes indicative of absence of apoptosis is decreased in cells produced by the provided methods. Apoptosis is a process of programmed cell death that includes a series of stereotyped morphological and biochemical events that lead to characteristic cell changes and death. These changes include blebbing, cell shrinkage, nuclear fragmentation, chromatin condensation, chromosomal DNA fragmentation, and global mRNA decay. Apoptosis is a well characterized process, and specific molecules associated with various stages are well known in the art. In the early stages of apoptosis, changes in the cellular and mitochondrial membrane become apparent. Biochemical changes are also apparent in the cytoplasm and nucleus of the cell. For example, the early stages of apoptosis can be indicated by activation of certain caspases, e.g., 2, 8, 9, and 10. The middle to late stages of apoptosis are characterized by further loss of membrane integrity, chromatin condensation and DNA fragmentation, include biochemical events such as activation of caspases 3, 6, and 7.

In certain embodiments, cells produced by the provided methods, or therapeutic T cell compositions provided herein, have a reduced percentage or frequency of cells positive for a marker of apoptosis. Various apoptosis markers are known to those of ordinary skill in the art and include, but are not limited to, an increase in activity of one or more caspases i.e. an activated caspase, an increase in PARP cleavage, activation and/or translocation of Bcl-2 family proteins, members of the cell death pathway, e.g., Fas and FADD, presence of nuclear shrinkage (e.g., monitored by microscope) and presence of chromosome DNA fragmentation (e.g., presence of chromosome DNA ladder) or with apoptosis assays that include TUNEL staining, and Annexin V staining. Annexin V is a protein that preferentially binds with high affinity phosphatidylserine (PS), which is a lipid that translocates from the inner to the outer leaflet of the plasma membrane during apoptosis. In some embodiments, cells produced by the provided methods, or therapeutic T cell compositions provided herein, have a reduced percentage or frequency of cells positive for expression of one or more factors associated with apoptosis, including pro-apoptotic factors known to initiate apoptosis, e.g., members of the death receptor pathway, activated members of the mitochondrial (intrinsic) pathway, such as Bcl-2 family members, e.g., Bax, Bad, and Bid, and caspases. In certain embodiments, cells produced by the provided methods, or therapeutic T cell compositions provided herein, have a reduced percentage or frequency of cells positive for staining with an indicator, e.g. Annexin V molecule, that will preferentially bind to cells undergoing apoptosis when incubated with or contacted to a cell composition. In any of such embodiments, the reduced frequency or percentage of such cells is reduced compared to a therapeutic T cell composition produced by a similar process but in which such process does not include incubation with the apoptosis inhibitor. In some embodiments, apoptosis is reduced by greater than at or about 1.5-fold, greater than at or about 2-fold, greater than at or about 3-fold, greater than at or about 5-fold, greater than at or about10-fold or more.

In particular embodiments, the apoptosis inhibitor is an inhibitor of the Fas/Fas ligand axis or is an inhibitor of caspase, both of which are involved in inducing apoptosis particularly of activated T cells. In aspects of the provide methods the apoptosis inhibitor can reduce or disrupt signaling mediated by the Fas/Fas-ligand axis and/or mediated by caspases.

In some embodiments, the apoptosis inhibitor inhibits apoptosis by disrupting the Fas/Fas-ligand axis (CD95/CD95L axis). In some aspects, the apoptosis inhibitor inhibits apoptosis induced or mediated by CD95. Fas ligand (FasL or CD95L) is a type-II transmembrane protein that belongs to the tumor necrosis factor (TNF) family. Its binding with its receptor induces apoptosis. Fas ligand/receptor interactions play an important role in the regulation of the immune system and the progression of cancer. The activation of T-cells leads to their expression of Fas ligand. T cells are initially resistant to Fas-mediated apoptosis during clonal expansion, but become progressively more sensitive the longer they are activated, ultimately resulting in activation-induced cell death (AICD). In some aspects, this process is necessary in vivo to prevent an excessive immune response and eliminate autoreactive T-cells. Humans and mice with deleterious mutations of Fas or Fas ligand develop an accumulation of aberrant T-cells, leading to lymphadenopathy, splenomegaly, and lupus erythematosus.

In aspects of the provided methods, a population of T cells, such as a population containing tumor-reactive T cells, is incubated or contacted with an apoptosis inhibitor that disrupts or blocks the interaction between Fas and Fas ligand, in which such incubation is carried out concurrently or subsequently to activation of the T cells by antigen and/or by one or more T cell stimulatory agent(s) that activates or stimulates T cells in the population. In some embodiments, activation of T cells can upregulate expression of Fas ligand where it can interact with Fas also expressed on the cell surface, thereby engaging Fas and causing apoptosis. In some embodiments, an apoptosis inhibitors that blocks this interaction can be a binding molecule that specifically binds to Fas or Fas ligand to block their interactions, thereby reducing or blocking at least partially the Fas signaling pathway and/or apoptosis in the cell. Methods for determining and/or assessing Fas signal pathway activity are known to the person skilled in the art and are, for example, described by Lavrik et. al. (2012) Cell Death Differ., 19(1):36-41.

An inhibitor according to the disclosure may act on the protein level and/or the nucleic acid level. Inhibitors acting on the protein level may be selected from antibodies, proteins and/or small molecules. Inhibitors acting on the nucleic acid level are for example antisense molecules, RNAi molecules and/or ribozymes. The inhibitor binds to Fas (CD95) and/or the Fas ligand (CD95L). In a further embodiment, the Fas/Fas ligand interaction may be inhibited.

In some embodiments, the inhibitor is an antibody or a functional fragment thereof. In some aspects, the inhibitor being an antibody may bind to Fas (CD95). In some embodiments, the inhibitor being an antibody may bind to CD95L. An example of an antibody binding CD95L is Nok-1 or an antigen-binding fragment thereof, see e.g. Catalog No. 16-9919-81, ThermoFisher Scientific, Waltham MA).

In some embodiments, the apoptosis inhibitor is a soluble protein that can specifically bind to Fas ligand. In some embodiments, the apoptosis inhibitor is a soluble CD95 receptor molecule containing an extracellular portion of CD95 but without a transmembrane domain. Soluble CD95 receptor molecules are described in EP-A-0595659 or EP-A-0965637. In some embodiments, the apoptosis inhibitor is or includes CD95 receptor peptides, such as described in WO99/65935.

In some embodiments, the apoptosis inhibitor is a fusion protein that binds to Fas ligand. In a particular embodiment, the apoptosis inhibitor contains the extracellular domain of Fas (CD95) or a specific binding protein thereof that binds to Fas ligand, in which the extracellular domain or a specific binding portion is fused to a heterologous polypeptide, such as an Fc immunoglobulin molecule. In some embodiments, the soluble Fas molecule is any as described in WO99/144330 or WO99/50413. In some embodiments, the soluble Fas molecule is the molecule known as FLINT or DCR3 or a fragment thereof.

In particular embodiments the apoptosis inhibitors binds to Fas ligand (CD95 ligand) and is a fusion protein containing the extracellular Fas (CD95) domain and an Fc domain, in particular a human Fc domain. In an embodiment, an apoptosis inhibitor includes an extracellular domain of Fas, such including all or a contiguous of the extracellular domain of mature CD95 set forth as amino acids 26-173 of the CD95 (see e.g. UniProt Accesion No. P25445; U.S. Pat. No. 5,891,434). In some embodiments, the CD95 is a human CD95 and contains an extracellular domain with the following sequence (amino acids 26-173 of human CD95):

QVTDINSKGLELRKTVTTVETQNLEGLHHDGQFCHKPCPPGERKARDCTVNGDEP
DCVPCQEGKEYTDKAHFSSKCRRCRLCDEGHGLEVEINCTRTQNTKCRCKPNFFC
NSTVCEHCDPCTKCEHGIIKECTLTSNTKCKEEGSRSN

In some embodiments, the Fas (CD95)-Fc fusion protein includes any as described in WO2014/013039 or WO2014/013037. In some embodiments, the extracellular Fas (CD95) domain of the fusion protein comprises the amino acid sequence up to amino acid 170, 171, 172 or 173 of human CD95. In particular embodiments, the fusion protein contains amino acids 26-172 of human CD95. In some embodiments, the Fc domain or functional fragment thereof comprises the CH2 and/or CH3 domain of an immunoglobulin, and optionally at least a part of the hinge region domain or a modified immunoglobulin domain derived therefrom. The immunoglobulin domain may be an IgG, IgM, IgD, or IgE immunoglobulin domain or a modified immunoglobulin domain derived, therefrom. In some embodiments, the Fc domain is an Fc of an IgG that contains at least a portion of a constant IgG immunoglobulin domain. The IgG immunoglobulin domain may be selected from IgG1, IgG2, IgG3 or IgG4 domains or from modified domains therefrom. In some embodiments, the Fc is a human Fc domain, such as a IgG Fc domain, e.g. a human IgG1 Fc domain. In particular embodiments, the extracellular domain of Fas or a specific binding fragment thereof is fused to an Fc immunoglobulin molecule including the hinge region e.g. from the human IgG1 molecule. A fusion protein comprising an extracellular CD95 domain and a human Fc domain is described in WO 95/27735 or WO2004/085478.

In some embodiments, the Fas (CD95)-Fc fusion protein is APG101 (asunercept) or is a functional fragment thereof.

In some embodiments, the Fas (CD95)-Fc fusion protein is CAN008 or is a functional fragment thereof.

In some embodiments, the apoptosis inhibitor inhibits apoptosis induced or mediated by caspase. Caspases are a family of related cysteine proteases that play an important role as modulators of cellular functions, including functions that result in apoptosis and inflammation. Caspase activation and regulation is tightly controlled through a number of mechanisms. Seven caspases, described above, are specifically involved in the process of apoptosis. Caspases are further sub-divided into iniator and effector caspases based on the lenfth of the pro-domain and activation mechanism. The iniator caspases can be recruited to caspase-activating complexes as a result of their elongated pro-domain. The effector caspases 3 and 7 are activated by proteolytic cleavage. They can cleave a wide variety of cellular proteins which can result in nuclear condensation, DNA fragmentation, plasma membrane blebbing, and eventually cell death. Inhibition of caspase mediated apoptosis can result from inhibiton of one, or any of the caspase pathway components described above.

Apoptosis via caspase activation can be initated in a number of overlapping ways, including via the mitochondrial pathway, via the death receptor pathway (I.e., Fas/FasL, TNF/TNF receptor), via the endoplasmic reticulum stress pathway, and via the apoptosis-inducing protease granzyme B.

In particular embodiments, an apoptosis inhibitor that is an inhibitor of caspase results in reduced activation of caspase in cells of the population. In certain embodiments, caspase activation can be detected by methods known to the person of ordinary skill. In some embodiments, an antibody that binds specifically to an activated caspase (i.e., binds specifically to the cleaved polypeptide) can be used to detect caspase activation. In another example, a fluorochrome inhibitor of caspase activity (FLICA) assay can be utilized to detect caspase-3 activation by detecting hydrolysis of acetyl Asp-Glu-Val-Asp 7-amido-4-methylcoumarin (Ac-DEVD-AMC) by caspase-3 (i.e., detecting release of the fluorescent 7-amino-4-methylcoumarin (AMC)). FLICA assays can be used to determine caspase activation by a detecting the product of a substrate processed by multiple caspases (e.g., FAM-VAD-FMK FLICA). Other techniques include The CASPASE-GLO^{®} caspase assays (PROMEGA) that use luminogenic caspase-8 tetrapeptide substrate (Z-LETD-aminoluciferin), the caspase-9 tetrapeptide substrate (Z-LEHD-aminoluciferin), the caspase-3/7 substrate (Z-DEVD-aminoluciferin), the caspase-6 substrate (Z-VEID-aminoluciferin), or the caspase-2 substrate (Z-VDVAD-aminoluciferin).

Examples for apoptosis inhibitors include both pan- and caspase specific inhibitors. Examples for apoptosis inhibitors include caspase inhibitors such as Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8), Wedelolactone, NS3694, NSCI and Z- fluoromethyl ketone Z-VAD-FMK and any flouromethyl ketone variant therein (I.e., Z-FA-FMK, Z-VAD(OH)-FMK, Z-DEVD-FMK, Z-VAD(OM2)-FMK, Z-VDVAD-FMK, etc.) In some embodiments, the caspase inhibitor is a caspase-specific inhibitor. In some embodiments, the apoptosis inhibitor is a pan-caspase inhibitor.

In particular embodiments, the caspase inhibitor is XIAP. In some aspects, XIAP is able to stop apoptotic cell death that is induced by overproduction of caspases, such as via its ability to bind to and inhibit caspase 3, 7 and 9. The BIR2 domain of XIAP inhibits caspase 3 and 7, while BIR3 binds to and inhibits caspase 9.

In particular embodiments, the caspase inhibitor is Z-VAD-FMK (carbobenzoxy-valyl-alanyl-aspartyl-[O-methyl]- fluoromethylketone). In some aspects, Z-VAD-FMK is able to stop apoptotic cell death that is induced by caspases, such as via its ability to bind the active site of several caspase proteases.

In embodiments of any of the provided methods, the ratio of T cells (e.g. tumor-reactive T cells) to apoptosis inhibitor (cells to moles) in the expansion method is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 500, about 1 to 1000, or about 1 to 10000.

In embodiments of any of the provided methods, the one or more apoptosis inhibitor is added to the cell culture medium during the incubation. In some embodiments, each of the one or more apoptosis inhibitor is indepedently added at a concentration ranging between at or about 0.1 µg/mL to at or about 100 ng/mL, at or about 0.1 µg/mL and at or abouu 50 ng/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 ng/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive.

In some embodiments, the apoptosis inhibitor is added at a concentration of between at about 0.5 µM and at about 50 µM, between at about 0.5 µM and at about 40 µM, between at about 0.5 µM and at about 30 µM, between at about 0.5 µM and at about 20 µM, between at about 0.5 µM and at about 10 µM, between at about 0.5 µM and at about 5 µM, between at about 0.5 µM and at about 1 µM, between at about 1 µM and at about 50 µM, between at about 1 µM and at about 40 µM, between at about 1 µM and at about 30 µM, between at about 1 µM and at about 20 µM, between at about 1 µM and at about 10 µM, or between at about 1 µM and at about 5 µM.. In some embodiments, the concentration is at or about 2 µM. In some embodiments, the concentration is at or about 10 µM. In some embodiments, the concentration is at or about 25 µM.

In particular embodiments, a T cell modulatory agent is an inhibitor of heat shock proteins. Heat shock proteins (Hsps) are a diverse group of proteins which include molecular chaperones that can be produced by cells in reponse to stress. Stressors can include but are not limited heat, oxidative stress, infection, ischemia, exposure to heavy metals, and nutrient deficiency. Some Hsps have demonstrable anti-apoptotic effects, for example Hsp70 is described as attenuating apoptosis via inhibition of the mitochondrial translocation of Bax protein. Other Hsps are involved in signaling cascades which can promote the apoptotic reponse, like Hsp 10 which is implicated in the activation of pro-caspase 3 *(*Ikwegbue et al., Pharmaceuticals 11(1): 2, 2018*).*

In certain embodiments, the Hsp inhibitor is an inhibitor of Hsp90. Hsp90 is an ATP-dependent protein chaperone that negatively inhibits Hsp70, despite Hsp90 and Hsp70 cooperating to prevent the dangerous aggregation of protein via heat-shock factor 1 in reponse to stress. Overexpresion of Hsp90 has been observed to result in protein stabilization, cell proliferation, angiogenesis, and increased survival of cancer cells. Hsp90 has also been demonstrated to stabilize several receptors involved in oncogenic signaling pathways, including EGFR *(*Chatterjee et al., Int J Mol Sci (18)9, 2017*).* For these reasons and others, Hsp90 inhibitors have been evaluated in preclinical models of cancer as well as multiple phase I and II studies as both a single agent and in combination with other agents *(*Spreafico et al., Brit J of Cancer (112) 650-659, 2015*).*

Examples for hsp inhibitors include but are not limited to MKT-077, Dihydropyrimidines (I.e., SW02, MAL2-IIB, MAL3-101, NSC630668 etc.), flavonoids (I.e., epigallocatechin, myricetin etc.), 15-DSG, Apoptozole, VER-155008, Aptamer A17, Aptamer A8, cmHSP70.1. Examples for hsp90 inhibitos include but are not limited to 17-AAg, 17-DMAG, IPI-504, NVP-AUY922, AT13387, Ganetespib, KW-2478, CNF-2024 (BIIB021), Debio 0932, PU-H71, MPC-310, SNX-5422, Ds-2248, XL-888, TAS-116, and NVP-HSP990.

### B. Exemplary Expansion Methods

In certain embodiments, methods for manufacturing T cells which express an endogenous TCR that recognize peptides on the surface of a target cell are provided.

In some embodiments methods include, but are not limited to the steps of (1) enriching a population of lymphocytes obtained from a donor subject, including but not limited to peripheral blood lymphocytes, lymph node sourced lymphocytes or tumor infiltrating lymphocytes, to produce a first population of T cells; (2) stimulating the first population of T cells with one or more first T-cell stimulating agents of lymphocytes, such as but not limited to anti-CD3 and anti-CD28 presented by APC's or as soluble antibodies (e.g. anti-CD3 and/or anti-CD28 antibodies) and/or recombinant cytokines (e.g. IL-2, IL-7, IL-15 and/or IL-21), to produce a second population of T cells containing activated T cells in which T cells have been expanded by the stimulation, wherein the stimulation is performed in a closed system using serum free medium; (3) co-culturing the second population of T cells in the presence of antigen presenting cells that present one or more MHC-associated non-native peptide, in which the peptides are peptide neoepitopes and the co-culture results in a third population of T cells containing activated T cells reactive to peptides present on the APCs in the culture ; (4) separating antigen presenting cells from the third population of T cells in a closed system to result in a fourth population of T cells that is enriched for tumor reactive T cells, in which the separating can be carried out by selecting from the third population of T cells a population of T cells containing endogenous TCR that are reactive to peptides present on the APCs based on one or more T cell activation markers on T cells following the culture in the presence of nucleated cells presenting peptides, in which the selecting is in a closed system using serum free medium. In some embodiments, this selection can be performed using florescent antibodies, nanoparticles or beads on cell selection equipment, but not limited to, the CliniMACS, Sony FX500 or the Miyltenyi Tyto cell sorting systems; and (5) expanding the fourth population of selected cells in the presence of a second T-cell stimulating agents of lymphocytes, such as but not limited to anti-CD3 and anti-CD28 as soluble antibodies (e.g. anti-CD3 and/or anti-CD28 antibodies) and/orrecombinant cytokines (e.g. IL-2, IL-7, IL-15 and/or IL-21) in a closed automated system using serum free medium. Upon reaching a therapeutic dose after expansion the cell composition can be concentrated and frozen in cryopreservation medium. In some embodiments, the stimulating of the first population of cells can occur over the course of 7-10 days in the presence of a costimulatory agonist, such as a TNFSR agonist including but not limited to agonists of OX40 and 41BB, and/or in the present of apoptosis inhibitors including but not limited to caspase inhibitors or inhibitors of Fas/Fas ligand and/or in the presence of a checkpoint inhibitor (e.g. anti-PD1 antibody) and/or in the presense of any of the T cell modulatory agents described herein. In some embodiments, the co-culture can occur over the course of 1-7 days in the presence of a costimulatory agonist, such as a TNFSR agonist including but not limited to agonists of OX40 and 41BB, and/or in the present of apoptosis inhibitors including but not limited to caspase inhibitors or inhibitors of Fas/Fas ligand and/or in the presence of a checkpoint inhibitor (e.g. anti-PD1 antibody) and/or in the presense of any of the T cell modulatory agents described herein. In some embodiments, expanding the fourth population of selected cells can occur over the course of 7-10 days in the presence of a costimulatory agonist, such as a TNFSR agonist including but not limited to agonists of OX40 and 41BB, and/or in the presence of a checkpoint inhibitor (e.g. anti-PD1 antibody) and/or in the present of apoptosis inhibitors including but not limited to caspase inhibitors or inhibitors of Fas/Fas ligand and/or in the presense of any of the T cell modulatory agents described herein. In particular embodiments, the first T cell stimulating agents includes at least one recombinant cytokine from IL-2, IL-7, IL-15 and IL-21, such as recombinant IL-2. In particular embodiments, the second T cell stimulating agents includes at least one recombinant cytokine from IL-2, IL-7, IL-15 and IL-21, such as recombinant IL-2. Also provided herein are populations of T cells produced by methods described herein and pharmaceutical compositions thereof.

In some embodiments, the methods include, but are not limited to the steps of (1) enriching a population of lymphocytes obtained from a donor subject; including but not limited to peripheral blood lymphocytes, lymph node sourced lymphocytes or tumor infiltrating lymphocytes, to produce a first population of T cells; (2) stimulating the population with one or more first T-cell stimulating agents of lymphocytes, such as but not limited to anti-CD3 and anti-CD28 presented by APC's or as soluble antibodies (e.g. anti-CD3 and/or anti-CD28 antibodies) and/or recombinant cytokines (e.g. IL-2, IL-7, IL-15 and/or IL-21), to produce a second population of T cells containing activated T cells in which T cells have been expanded by the stimulation, wherein the stimulation is performed in a closed system using serum free medium; (3) co-culturing the second population of T cells in the presence of antigen presenting cells that present one or more MHC-associated non-native peptide, in which the peptides are peptide neoepitopes and the co-culture results in a third population of T cells containing activated T cells reactive to peptides present on the APCs in the culture; (4) separating antigen presenting cells from the third population of T cells in a closed system to result in a fourth population of T cells that is enriched for tumor reactive T cells, in which the separating can be carried out by selecting from the third population of T cells a population of T cells containing endogenous TCR that are reactive to peptides present on the APCs based on one or more T cell activation marker on T cells following the culture in the presence of nucleated cells presenting peptides, and in which the selecting is in a closed system using serum free medium. In some embodiments, this selection can be performed using florescent antibodies, nanoparticles or beads on cell selection equipment, but not limited to, the CliniMACS, Sony FX500 or the Miltenyi Tyto cell sorting systems; and (5) expanding the fourth population of selected cells in the presence of T-cell stimulating agents of lymphocytes, such as but not limited to anti-CD3 and anti-CD28 as soluble antibodies (e.g. anti-CD3 and/or anti-CD28 antibodies) and recombinant cytokines (e.g. IL-2, IL-7, IL-15 and/or IL-21) in a closed automated system using serum free medium. In particular embodiments, the first T cell stimulating agents includes at least one recombinant cytokine from IL-2, IL-7, IL-15 and IL-21, such as recombinant IL-2. In particular embodiments, the second T cell stimulating agents includes at least one recombinant cytokine from IL-2, IL-7, IL-15 and IL-21, such as recombinant IL-2. Upon reaching a therapeutics dose after expansion the product will be concentrated and frozen in cryopreservation medium. Also provided herein are populations of T cells produced by methods described herein and pharmaceutical compositions thereof.

In some embodiments, the methods include, enriching a population of lymphocytes obtained from a donor subject, including but not limited to peripheral blood lymphocytes, lymph node sourced lymphocytes or tumor infiltrating lymphocytes,by selecting a population of T cells containing endogenous TCR that are reactive to peptides presented on APCs in vivo, based on one or more T cell activation markers on T cells directly from the sample, in which the selecting is in a closed system using serum free medium. In some embodiments, this selection can be performed, but not limited to, using fluorescent antibodies, nanoparticles or beads on cell selection equipment, but not limited to, the CliniMACS, Sony FX500 or the Miltenyi Tyto cell sorting systems.

In some embodiments, the method for expansion can occur over the course of 7-20 days in the presence of costimulatory agonists, such as TNFSFR agonists including but not limited to agonists of OX40 and 41BB, and/or in the present of apoptosis inhibitors including but not limited to caspase inhibitors or inhibitors of Fas/Fas ligand, and/or in the presence of a checkpoint inhibitor (e.g. anti-PD1 antibody) and/or in the presense of any of the T cell modulatory agents described herein. In any of the provided methods, the method is carried out with an apoptosis inhibitor that is a caspase inhibitor. In any of the provided methods, the method is carried out with a costimulatory agonist, such as an agonist of 4-1BB, CD27, OX40 or GITR. In any of the provided methods, the method is carried out with a checkpoint inhbititor, such as an anti-PD-1 antibody. These soluble agonists and inhibitors, such as apoptosis inhibitors or checkpoint inhibitors, can be included for up to the maximum culture time of the expansion method, or any step of the method, or a minimum of 24 hours. The costimultoary agonists and/or apoptosis inhibitors and/or other modulatory agents can be used during at least a portion of the initial stimulation, co-culture, selection, or expansion in the provided method. In some embodiments, these agonists can be used during at least a portion of the method that includes, but not limited to, prior to selection, after selection, during expansion, and between 5 and 1 days prior to harvest and cryopreservation of the cell product. Upon reaching a therapeutics dose after expansion the product will be concentrated and frozen in crypreservation medium. Also provided herein are populations of T cells produced by methods described herein and pharmaceutical compositions thereof.

### III. COMPOSITIONS AND PHARMACEUTICAL FORMULATIONS

Provided herein are compositions containing expanded T cells such as produced by any of the provided methods. In some embodiments, the compositions contain tumor reactive T cells or T cells containing an endogenous TCR specific to a tumor-associated antigen, e.g. neoantigen. In particular, among the provided compositions are compositions of cells that are enriched for tumor reactive T cells or T cells containing an endogenous TCR specific to a tumor-associated antigen, e.g. neoantigen.

In some embodiments, the composition comprises about 5-99% tumor-reactive T cells or or T cells surface positive for the one or more T cell activation marker, or any percentage of such cells between 5 and 99% inclusive. In some embodiments, the composition can include an increased or greater percentages of tumor-reactive CD3+ T cells or of CD3+ T cells surface positive for the one or more T cell activation marker relative to total CD3+ T cells or total cells in the composition compared to the percentage of such tumor-reactive CD3+ T cells or of CD3+ T cells surface positive for the one or more T cell activation marker relative to total CD3+ T cells or total cells naturally present in the subject or biological sample from which the cells were isolated. In some embodiments, the percentage is increased at least or at least about 2-fold, 3-fold, 4-fold, 5-fold, 10-fold, 20-fold, 30-fold, 40-fold, 50-fold, 60-fold, 70-fold, 80-fold, 90-fold, 100-fold, 150-fold, 200-fold or more. In such embodiments, the one or more T cell activation marker can be any as described, such as any one or more of CD107a, CD39, CD103, CD59, CD90 and/or CD38.

In some embodiments, the composition can include at least at or about 20%, at least at or about 30%, at least at or about 40%, at least at or about 50%, at least at or about 60%, at least at or about 65%, at least at or about 70%, at least at or about 75%, at least at or about 80%, at least at or about 81%, at least at or about 82%, at least at or about 83%, at least at or about 84%, at least at or about 85%, at least at or about 86%, at least at or about 87%, at least at or about 88%, at least at or about 89%, at least at or about 90%, at least at or about 91%, at least at or about 92%, at least at or about 93%, at least at or about 94%, at least at or about 95%, at least at or about 96%, at least at or about 97%, at least at or about 98%, at least at or about 99%, or substantially 100% tumor-reactive CD3+ T cells or CD3+ T cells surface positive for one or more activation marker. In some embodiments, the composition comprises more than 30% tumor reactive CD3+ T cells or CD3+ T cells surface positive for one or more activation marker. In some embodiments, the composition comprises more than 40% tumor reactive CD3+ T cells or CD3+ T cells surface positive for one or more activation marker. In some embodiments, the composition comprises more than 50% tumor reactive CD3+ T cells or CD3+ T cells surface positive for one or more activation marker. In some embodiments, the composition comprises more than 60% tumor reactive CD3+ T cells or CD3+ T cells surface positive for one or more activation marker. In some embodiments, the composition comprises more than 70% tumor reactive CD3+ T cells or CD3+ T cells surface positive for one or more activation marker. In some embodiments, the composition comprises more than 80% tumor reactive CD3+ T cells or CD3+ T cells surface positive for one or more activation marker. In some embodiments, the composition comprises more than 90% tumor reactive CD3+ T cells or CD3+ T cells surface positive for one or more activation marker. In such embodiments, the one or more T cell activation marker can be any as described, such as any one or more of CD107a, CD39, CD103, CD59, CD90 and/or CD38.

In some embodiments, the tumor reactive CD3+ T cells or CD3+ T cells surface positive for one or more activation marker can be present in the composition in a therapeutically effective amount. An effective amount of cells can vary depending on the patient, as well as the type, severity and extent of disease. Thus, a physician can determine what an effective amount is after considering the health of the subject, the extent and severity of disease, and other variables.

In certain embodiments, the number of such cells in the composition is a therapeutically effective amount. In some embodiments, the amount is an amount that reduces the severity, the duration and/or the symptoms associated with cancer, viral infection, microbial infection, or septic shock in an animal. In some embodiments, a therapeutically effective amount is a dose of cells that results in a reduction of the growth or spread of cancer by at least 2.5%, at least 5%, at least 10%, at least 15%, at least 25%, at least 35%, at least 45%, at least 50%, at least 75%, at least 85%, by at least 90%, at least 95%, or at least 99% in a patient or an animal administered a composition described herein relative to the growth or spread of cancer in a patient (or an animal) or a group of patients (or animals) not administered the composition. In some embodiments, a therapeutically effective amount is an amount to result in cytotoxic activity resulting in activity to inhibit or reduce the growth of cancer, viral and microbial cells.

In some embodiments, the composition comprises an amount of tumor reactive CD3+ T cells or CD3+ T cells surface positive for one or more activation marker that is from at or about 10⁵ and at or about 10¹² tumor reactive CD3+ T cells or CD3+ T cells surface positive for one or more activation marker, or from at or about 10⁵ to at or about 10⁸ tumor reactive CD3+ T cells or CD3+ T cells surface positive for one or more activation marker, or from at or about 10⁶ and at or about 10¹² tumor reactive CD3+ T cells or CD3+ T cells surface positive for one or more activation marker, or from at or about 10⁸ and at or about 10¹¹ tumor reactive CD3+ T cells or CD3+ T cells surface positive for one or more activation marker, or from at or about 10⁹ and at or about 10¹⁰ tumor reactive CD3+ T cells or CD3+ T cells surface positive for one or more activation marker. In some embodiments, the composition comprises greater than or greater than at or about 10⁵ tumor reactive CD3+ T cells or CD3+ T cells surface positive for one or more activation marker, at or about 10⁶ tumor reactive CD3+ T cells or CD3+ T cells surface positive for one or more activation marker, at or about 10⁷ tumor reactive CD3+ T cells or CD3+ T cells surface positive for one or more activation marker, at or about 10⁸ tumor reactive CD3+ T cells or CD3+ T cells surface positive for one or more activation marker, at or about 10⁹ tumor reactive CD3+ T cells or CD3+ T cells surface positive for one or more activation marker, at or about10¹⁰ tumor reactive CD3+ T cells or CD3+ T cells surface positive for one or more activation marker, at or about 10¹¹ tumor reactive CD3+ T cells or CD3+ T cells surface positive for one or more activation marker, or at or about 10¹² tumor reactive CD3+ T cells or CD3+ T cells surface positive for one or more activation marker. In some embodiments, such an amount can be administered to a subject having a disease or condition, such as to a cancer patient. In such embodiments, the one or more T cell activation marker can be any as described, such as any one or more of CD107a, CD39, CD103, CD59, CD90 and/or CD38.

In some embodiments, the composition comprises CD3+ T cells as a percentage of total cells in the population that is greater than or greater than about 60%, greater than or greater than about 70%, greater than or greater than about 80%, greater than or greater than about 90% or greater than or greater than about 95%. In some embodiments, the composition contains CD4+ T cells and CD8+ T cells as a percentage of total cells in the population that is greater than or greater than about 60%, greater than or greater than about 70%, greater than or greater than about 80%, greater than or greater than about 90% or greater than or greater than about 95%. In particular embodiments, the composition contains a ratio of CD8+ T cells to CD4+ T cells that is between at or about 1:100 and at or about 100:1, between at or about 1:50 and at or about 50:1, between at or about 1:25 and at or about 25:1, between at or about 1:10 and at or about 10:1, between at or about 1:5 and at or about 5:1, or between at or about 1:2.5 and at or about 2.5:1.

In some embodiments, the volume of the composition is at least or at least about 10 mL, 50 mL, 100 mL, 200 mL, 300 mL, 400 mL or 500 mL, such as is from or from about 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL, each inclusive. In some embodiments, the composition has a cell density of at least or at least about 1 x 10⁵ cells/mL, 5 x 10⁵ cells/mL, 1 × 10⁶ cells/mL, 5 x 10⁶ cells/mL, 1 × 10⁷ cells/mL, 5 x 10⁷ cells/mL or 1 × 10⁸ cells/ mL. In some embodiment, the cell density of the composition is between or between about 1 × 10⁵ cells/mL to 1 × 10⁸ cells/mL, 1 × 10⁵ cells/mL to 1 x 10⁷ cells/mL, 1 × 10⁵ cells/mL to 1 x 10⁶ cells/mL, 1 × 10⁶ cells/mL to 1 × 10⁷ cells/mL, 1 × 10⁶ cells/mL to 1 × 10⁸ cells/mL, 1 × 10⁶ cells/mL to 1 × 10⁷ cells/mL or 1 × 10⁷ cells/mL to 1 × 10⁸ cells/mL, each inclusive.

Among the compositions are pharmaceutical compositions and formulations for administration, such as for adoptive cell therapy. In some embodiments, the engineered cells are formulated with a pharmaceutically acceptable carrier.

A pharmaceutically acceptable carrier can include all solvents, dispersion media, coatings, antibacterial and antifungal agents, isotonic and absorption delaying agents, and the like, compatible with pharmaceutical administration (Gennaro, 2000, Remington: The science and practice of pharmacy, Lippincott, Williams & Wilkins, Philadelphia, PA). Examples of such carriers or diluents include, but are not limited to, water, saline, Ringer's solutions, dextrose solution, and 5% human serum albumin. Liposomes and non-aqueous vehicles such as fixed oils may also be used. Supplementary active compounds can also be incorporated into the compositions. The pharmaceutical carrier should be one that is suitable for NK cells, such as a saline solution, a dextrose solution or a solution comprising human serum albumin.

In some embodiments, the pharmaceutically acceptable carrier or vehicle for such compositions is any non-toxic aqueous solution in which the NK cells can be maintained, or remain viable, for a time sufficient to allow administration of live NK cells. For example, the pharmaceutically acceptable carrier or vehicle can be a saline solution or buffered saline solution. The pharmaceutically acceptable carrier or vehicle can also include various bio materials that may increase the efficiency of NK cells. Cell vehicles and carriers can, for example, include polysaccharides such as methylcellulose (M. C. Tate, D. A. Shear, S. W. Hoffman, D. G. Stein, M. C. LaPlaca, Biomaterials 22, 1113, 2001), chitosan (Suh J K F, Matthew H W T. Biomaterials, 21, 2589, 2000; Lahiji A, Sohrabi A, Hungerford D S, et al., J Biomed Mater Res, 51, 586, 2000), N-isopropylacrylamide copolymer P(NIPAM-co-AA) (Y. H. Bae, B. Vernon, C. K. Han, S. W. Kim, J. Control. Release 53, 249, 1998; H. Gappa, M. Baudys, J. J. Koh, S. W. Kim, Y. H. Bae, Tissue Eng. 7, 35, 2001), as well as Poly(oxyethylene)/poly(D,L-lactic acid-co-glycolic acid) (B. Jeong, K. M. Lee, A. Gutowska, Y. H. An, Biomacromolecules 3, 865, 2002), P(PF-co-EG) (Suggs L J, Mikos A G. Cell Trans, 8, 345, 1999), PEO/PEG (Mann B K, Gobin A S, Tsai A T, Schmedlen R H, West J L., Biomaterials, 22, 3045, 2001; Bryant S J, Anseth K S. Biomaterials, 22, 619, 2001), PVA (Chih-Ta Lee, Po-Han Kung and Yu-Der Lee, Carbohydrate Polymers, 61, 348, 2005), collagen (Lee C R, Grodzinsky A J, Spector M., Biomaterials 22, 3145, 2001), alginate (Bouhadir K H, Lee K Y, Alsberg E, Damm K L, Anderson K W, Mooney D J. Biotech Prog 17, 945, 2001; Smidsrd O, Skjak-Braek G., Trends Biotech, 8, 71, 1990).

In some embodiments, the composition, including pharmaceutical composition, is sterile. In some embodiments, isolation or enrichment of the cells is carried out in a closed or sterile environment, for example, to minimize error, user handling and/or contamination. In some embodiments, sterility may be readily accomplished, e.g., by filtration through sterile filtration membranes.

Also provided herein are compositions that are suitable for cryopreserving the provided T cells, including tumor-reactive T cells or T cells surface positive for one or more activation marker. In some embodiments, the composition comprises a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to - 150 °C, such as or about 80 °C ± 6.0 ° C.

In some embodiments, the cryopreserved cells are prepared for administration by thawing. In some cases, the cells can be administered to a subject immediately after thawing. In such an embodiment, the composition is ready-to-use without any further processing. In other cases, the cells are further processed after thawing, such as by resuspension with a pharmaceutically acceptable carrier, incubation with an activating or stimulating agent, or are activated washed and resuspended in a pharmaceutically acceptable buffer prior to administration to a subject.

### IV. METHODS OF TREATMENT AND THERAPEUTIC APPLICATIONS

Provided herein are compositions and methods relating to the provided therapeutic cell compositions described herein for use in treating diseases or conditions in a subject such as a cancer. Such methods and uses include therapeutic methods and uses, for example, involving administration of the therapeutic cells, or compositions containing the same, to a subject having a disease, condition, or disorder. In some cases, the disease or disorder is a tumor or cancer. In some embodiments, the cells or pharmaceutical composition thereof is administered in an effective amount to effect treatment of the disease or disorder. Uses include uses of the cells or pharmaceutical compositions thereof in such methods and treatments, and in the preparation of a medicament in order to carry out such therapeutic methods. In some embodiments, the methods thereby treat the disease or condition or disorder in the subject.

In some embodiments, the methods of treatment comprise administering an effective amount of a composition containing tumor reactive CD3+ T cells or CD3+ T cells surface positive for one or more activation marker. Such compositions can include any as described herein, including compositions produced by the provided methods.

In some embodiment, a subject (e.g. autologous) is administered from at or about 10⁵ to at or about 10¹²CD3+ T cells produced by any of the provided methods, or from at or about 10⁵ to at or about 10⁸ CD3+ T cells produced by any of the provide methods, or from at or about 10⁶ and at or about 10¹² CD3+ T cells produced by any of the provided methods, or from at or about 10⁸ and at or about 10¹¹ CD3+ T cells produced by any of the provided methods, or from at or about 10⁹ and at or about 10¹⁰ CD3+ T cells produced by any of the provided methods. In some embodiments, the therapeutically effective amount for administration comprises greater than or greater than at or about 10⁵ CD3+ T cells produced by any of the provided methods, at or about 10⁶ CD3+ T cells produced by any of the provided methods, at or about 10⁷ CD3+ T cells produced by any of the provided methods, at or about 10⁸ CD3+ T cells produced by any of the provided methods, at or about 10⁹ CD3+ T cells produced by any of the provided methods, at or about10¹⁰ CD3+ T cells produced by any of the provided methods, at or about 10¹¹ CD3+ T cells produced by any of the provided methods, or at or about 10¹² CD3+ T cells produced by any of the provided methods. In some embodiments, such an amount can be administered to a subject having a disease or condition, such as to a cancer patient. In some embodiments, the number of T cells are administered are viable T cells.

In some embodiment from at or about 10⁵ to at or about 10¹² tumor reactive CD3+ T cells or CD3+ T cells surface positive for one or more activation marker, or from at or about 10⁵ to at or about 10⁸ tumor reactive CD3+ T cells or CD3+ T cells surface positive for one or more activation marker, or from at or about 10⁶ and at or about 10¹² tumor reactive CD3+ T cells or CD3+ T cells surface positive for one or more activation marker, or from at or about 10⁸ and at or about 10¹¹ tumor reactive CD3+ T cells or CD3+ T cells surface positive for one or more activation marker, or from at or about 10⁹ and at or about 10¹⁰ tumor reactive CD3+ T cells or CD3+ T cells surface positive for one or more activation marker are administered to the individual. In some embodiments, the therapeutically effective amount for administration comprises greater than or greater than at or about 10⁵ tumor reactive CD3+ T cells or CD3+ T cells surface positive for one or more activation marker, at or about 10⁶ tumor reactive CD3+ T cells or CD3+ T cells surface positive for one or more activation marker, at or about 10⁷ tumor reactive CD3+ T cells or CD3+ T cells surface positive for one or more activation marker, at or about 10⁸ tumor reactive CD3+ T cells or CD3+ T cells surface positive for one or more activation marker, at or about 10⁹ tumor reactive CD3+ T cells or CD3+ T cells surface positive for one or more activation marker, at or about10¹⁰ tumor reactive CD3+ T cells or CD3+ T cells surface positive for one or more activation marker, at or about 10¹¹ tumor reactive CD3+ T cells or CD3+ T cells surface positive for one or more activation marker, or at or about 10¹² tumor reactive CD3+ T cells or CD3+ T cells surface positive for one or more activation marker. In some embodiments, such an amount can be administered to a subject having a disease or condition, such as to a cancer patient. In such embodiments, the one or more T cell activation marker can be any as described, such as any one or more of CD107a, CD39, CD103, CD59, CD90 and/or CD38.

In some embodiments, the amount is administered as a flat dose. In other embodiments, the amount is administered per kilogram body weight of the subject.

In some embodiments, the composition, such as produced by any of the provided methods of containing tumor-reactive T cells or T cells surface positive for a T cell activation marker, are administered to an individual soon after expansion according to the provided methods. In other embodiments, the expanded T cells, such as expanded tumor-reactive T cells or T cells surface positive for a T cell activation marker, are cryopreserved prior to administration, such as by methods described above. For example, the T cells, such as tumor-reactive T cells or T cells surface positive for a T cell activation marker, can be stored for greater than 6, 12, 18, or 24 months prior to administration to the individual. Such cryopreserved cells can be thawed prior to the administration.

In some embodiments, the provided compositions containing tumor-reactive T cells or T cells surface positive for a T cell activation marker, can be administered to a subject by any convenient route including parenteral routes such as subcutaneous, intramuscular, intravenous, and/or epidural routes of administration.

In some embodiments, the compositions containing tumor-reactive T cells or T cells surface positive for a T cell activation marker may be administered in a single dose. Such administration may be by injection, e.g., intravenous injection. In some embodiments, tumor-reactive T cells or T cells surface positive for a T cell activation marker may be administered in multiple doses. Dosing may be once, twice, three times, four times, five times, six times, or more than six times per year. Dosing may be once a month, once every two weeks, once a week, or once every other day. Administration of such compositions and cells may continue as long as necessary.

In some embodiments, the subject is administered a lymphodepleting thereapy prior to the administration of the dose of cells from a provided compositions, such as produced by any of the provided methods or containing tumor-reactive T cells or T cells surface positive for a T cell activation marker. The lymphodepleting therapy can include administration of fludarabine and/or cyclophosphamide (the active form being referred to as mafosfamide) and combinations thereof. Such methods are described in Gassner et al. (Cancer Immunol Immunother . 201 1 , 60(1):75-85, Muranski, et al, Nat Clin Pract Oncol, 2006 3(12):668-681, Dudley, et al., J Clin Oncol 2008, 26:5233-5239, and Dudley, et al., J Clin Oncol. 2005, 23(10):2346-2357. In some embodiments, the fludarabine is administered at a dosage of 10 mg/kg/day, 15 mg/kg/day, 20 mg/kg/day, 25 mg/kg/day, 30 mg/kg/day, 35 mg/kg/day, 40 mg/kg/day, or 45 mg/kg/day, or a dosage amount between a range of any of the foregoing. In some embodiments, the fludarabine is for 2-7 days, such as for 3-5 days, such as at or about 3 days, at or about 4 days or at or about 5 days. In some embodiments, the cyclophosphamide is administered at a dosage of 100 mg/m2/day, 150 mg/m2/day, 175 mg/m2/day, 200 mg/m2/day, 225 mg/m2/day, 250 mg/m2/day, 275 mg/m2/day, or 300 mg/m2/day. In some embodiments, the cyclophosphamide is administered intravenously (i.e., i.v.). In some embodiments, the cyclophosphamide treatment is for 2-7 days, such as 3-5 days, at or about 3 days, at or about 4 days or at or about 5 days. The lymphodepleting therapy is administered prior to the provided cell compositions. In some embodiments, the lymphodepleting therapy is carried out within a week of the administration of the provided cell compositions, such as 5-7 days prior to the administration of the dose of cells.

The compositions described herein can be used in a method for treating hyperproliferative disorders. In a preferred embodiment, they are for use in treating cancers. In some aspects, the cancer can be a melanoma, ovarian cancer, cervical cancer, lung cancer, bladder cancer, breast cancer, head and neck cancer, renal cell carcinoma, acute myeloid leukemia, colorectal cancer, and sarcoma. In some embodiments, the cancer is a cancer with a high mutational burden. In some embodiments, the cancer is melanoma, lung squamous, lung adenocarcinoma, bladder cancer, lung small cell cancer, esophageal cancer, colorectal cancer, cervical cancer, head and neck cancer, stomach cancer or uterine cancer.

In some embodiments, the cancer is an epithelial cancer. In some embodiments, the cancer is selected from non-small cell lung cancer (NSCLC), CRC, ovarian cancer, breast cancer, esophageal cancer, gastric cancer, pancreatic cancer, cholangiocarcinoma cancer, endometrial cancer. In some embodiments, the breast cancer is HR+/Her2- breast cancer. In some embodiments, the breast cancer is a triple negative breast cancer (TNBC). In some embodiments, the breast cancer is a HER2+ breast cancer.

In some embodiments, the subject has a cancer that is is a hematological tumor. Non-limiting examples of hematological tumors include leukemias, including acute leukemias (such as 11q23- positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some embodiments, the subject has a solid tumor cancer. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some embodiments, the cancer is a skin cancer. In particular embodiments, the cancer is a melanoma, such as a cutaneous melanoma. In some embodiments, the cancer is a merkel cell or metastatic cutaneous squamous cell carcinoma (CSCC).

In some embodiments, the tumor is a carcinoma, which is a cancer that develops from epithelial cells or is a cancer of epithelial origin. In some embodiments, the cancer arises from epithelial cells which include, but are not limited to, breast cancer, basal cell carcinoma, adenocarcinoma, gastrointestinal cancer, lip cancer, mouth cancer, esophageal cancer, small bowel cancer and stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that effect epithelial cells throughout the body.

In some embodiments, the subject has a cancer that is a gastrointestinal cancer involving a cancer of the gastrointestinal tract (GI tract), including cancers or the upper or lower digestive tract, or an accessory organ of digestion, such as esophagus, stomach, biliary system, pancreas, small intestine, large intestine, rectum or anus. In some embodiments, the cancer is an espphageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular embodiments, the cancer is a colorectal cancer.In some embodiments, the cancer is a colorectal cancer. Colorectal cancer (CRC) is a common tumor of increasing incidence, which, in many cases, does not response to checkpoint inhibition or other immunotherapy. This is the case even though such cancers have properties that are associated with response, e.g. a reasonably high mutation rate and well established association of prognosis with level of T cell infiltration.

In some embodiments, the cancer is an ovarian cancer. In some embodiments, the cancer is a triple-negative breast cancer (TNBC).

In some embodiments, the cancer is lung cancer. In some embodiments, the cancer is a breast cancer. In some embodiments, the cancer is a colorectal cancer. In some embodiments, the cancer is pancreatic cancer. In some embodiments, the cancer is a merkel cell cancer. In some embodiments, the cancer is a metastatic cutaneous squamous cell carcinoma (CSCC). In some embodiments, the cancer is a melanoma.

In some embodiments, the subject is one whose cancer is refractory to, and or who has relapsed following treatment with, a checkpoint blockade, such as an anti-PD1 or anti-PD-L1 therapy.

In some embodiments, the subject is the same subject from with the biological sample was obtained for producing the therapeutic cell composition. In some such embodiments, the provided methods of treatment is an adoptive cell therapy with a therapeutic composition containing T cells autologous to the subject.

In some embodiments, the cell compositions provided herein are autologous to the subject to be treated. In such embodiments, the starting cells for expansion are isolated directly from a biological sample from the subject as described herein, in some cases including with enrichment for T cells surface positive for one or more T cell activation marker as described, and cultured under conditions for expansion as provided herein. In some embodiments, the culturing includes incubation with one or more T cell adjuvant, such as a costimulatory agonist and/or an apoptosis inhibitor and incubation with one or more T cell stimulatory agent(s) as described. In some aspects, the biological sample from the subject is or includes a tumor or lymph node sample and such sample tumor and an amount of such tissue is obtained, such as by resection or biospsy (e.g. core needle biopsy or fine-needle aspiration). In some embodiments, the biological sample from the subject is or includes a peripheral blood sample such as an apheresis sample. In some embodiments, following the culturing under conditions for expansion the cells are formulated and optionally cryopreserved for subsequent administration to the same subject for treating the cancer.

In some embodiments, the cell compositions provided herein are allogenic to the subject to be treated. In some aspects, the subject from which the cells are derived or isolated is a healthy subject or is not known to have a disease or conditions, such as a cancer. In such embodiments, the starting cells for expansion are isolated directly from a biological sample from such a subject as described herein, in some cases including with enrichment for T cells surface positive for one or more T cell activation marker as described, and cultured under conditions for expansion as provided herein. In some embodiments, the culturing includes incubation with one or more T cell adjuvant, such as a costimulatory agonist and/or an apoptosis inhibitor and incubation with one or more T cell stimulatory agent(s) as described. In some aspects, the biological sample from the subject is or includes a tumor or lymph node sample and such sample tumor and an amount of such tissue is obtained, such as by resection or biospsy (e.g. core needle biopsy or fine-needle aspiration). In some embodiments, the biological sample from the subject is or includes a peripheral blood sample such as an apheresis sample. In some embodiments, following the culturing under conditions for expansion the cells are formulated and optionally cryopreserved for subsequent administration to the a different subject for treating a cancer in such different subject.

In some embodiments, the provided methods can be carried out with one or more other immunotherapies. In some embodiments, the immunotherapy is an immune modulating agent that is an immune checkpoint inhibitor. In some embodiments, the immune checkpoint inhibitor specifically binds a molecule selected from among CD25, PD-1, PD-L1, PD-L2, CTLA-4, LAG-3, TIM-3, 4-1BB, GITR, CD40, CD40L, OX40, OX40L, CXCR2, B7-H3, B7-H4, BTLA, HVEM, CD28, TIGIT and VISTA. In some embodiments, the immune checkpoint inhibitor is and antibody or antigen-binding fragment, a small molecule or a polypeptide. In some embodiments, the immune checkpoint inhibitor is selected from among nivolumab, pembrolizumab, pidilizumab, MK-3475, BMS-936559, MPDL3280A, ipilimumab, tremelimumab, IMP31, BMS-986016, urelumab, TRX518, dacetuzumab, lucatumumab, SEQ-CD40, CP-870, CP-893, MED16469, MEDI4736, MOXR0916, AMP-224, and MSB001078C, or is an antigen-binding fragment thereof.

In some embodiments, the provided methods include combination therapy of a cell therapy as described and PD-1 or PD-L1 inhibitors. A PD-1 or PD-L1 inhibitor can include binding antibodies, antagonists, or inhibitors (i.e., blockers).

In an embodiment, the PD-I inhibitor is nivolumab (commercially available as OPDIVO from Bristol-Myers Squibb Co.), or biosimilars, antigen-binding fragments, conjugates, or variants thereof. Nivolumab is a fully human IgG4 antibody blocking the PD-I receptor. In an embodiment, the anti-PD-I antibody is an immunoglobulin G4 kappa, anti-(human CD274) antibody. Nivolumab is assigned Chemical Abstracts Service (CAS) registry number 946414-94-4 and is also known as 5C4, BMS-936558, 1\tIDX-1106, and ONO-4538. The preparation and properties of nivolumab are described in U.S. Patent No. 8,008,449 and International Patent Publication No. WO 2006/121168.

In another embodiment, the PD-1 inhibitor comprises pembrolizumab ( commercially available as KEYTRUDA from Merck & Co., Inc., Kenilworth, NJ, USA), or antigen-binding fragments, conjugates, or variants thereof. Pembrolizumab is assigned CAS registry number 1374853-91-4 and is also known as lambrolizumab, MK-3475, and SCH-900475. The properties, uses, and preparation of pembrolizumab are described in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2.

In an embodiment, the PD-LI inhibitor is durvalumab, also known as MEDI4736 (which is commercially available from Medimmune, LLC, Gaithersburg, JVIaryland, a subsidiary f AstraZeneca plc.), or antigen-binding fragments, conjugates, or variants thereof. In an embodiment, the PD-LI inhibitor is an antibody disclosed in U.S. Patent No. 8,779,108 or U.S. Patent Application Publication No. 2013/0034559.

In an embodiment, the PD-LI inhibitor is avelumab, also known as MSB0010718C (commercially available from Merck KGaA/EMD Serono), or antigen-binding fragments, conjugates, or variants thereof. The preparation and properties of avelumab are described in U.S. Patent Application Publication No. US 2014/0341917 A1.

In an embodiment, the PD-LI inhibitor is atezolizumab, also known as MPDL3280A or RG7446 (commercially available as TECENTRIQ from Genentech, Inc., a subsidiary of Roche Holding AG, Basel, Switzerland), or antigen-binding fragments, conjugates, or variants thereof. In an embodiment, the PD-LI inhibitor is an antibody disclosed in U.S. Patent No. 8,217,149. In an embodiment, the PD-LI inhibitor is an antibody disclosed in U.S. Patent Application Publication Nos. 2010/0203056 A1, 2013/0045200 A1, 2013/0045201 A1, 2013/0045202 A1, or 2014/0065135 A1. The preparation and properties of atezolizumab are described in U.S. Patent No. 8,217,149.

### V. KITS AND ARTICLES OF MANUFACTURE

Provided herein are articles of manufacture and kits comprising the provided compositions containing or enriched for tumor-reactive T cells or T cells surface positive for a T cell activation marker. In some embodiments, the compositions are produced by any of the provided methods.

Kits can optionally include one or more components such as instructions for use, devices and additional reagents (e.g., sterilized water or saline solutions for dilution of the compositions and/or reconstitution of lyophilized protein), and components, such as tubes, containers and syringes for practice of the methods. In some embodiments, the kits can further contain reagents for collection of samples, preparation and processing of samples, and/or reagents for quantitating the amount of one or more surface markers in a sample, such as, but not limited to, detection reagents, such as antibodies, buffers, substrates for enzymatic staining, chromagens or other materials, such as slides, containers, microtiter plates, and optionally, instructions for performing the methods. Those of skill in the art will recognize many other possible containers and plates and reagents that can be used in accord with the provided methods.

In some embodiments, the kits can be provided as articles of manufacture that include packing materials for the packaging of the cells, antibodies or reagents, or compositions thereof, or one or more other components. For example,the kits can contain containers, bottles, tubes, vial and any packaging material suitable for separating or organizing the components of the kit. The one or more containers may be formed from a variety of materials such as glass or plastic. In some embodiments, the one or more containers hold a composition comprising cells or an antibody or other reagents for use in the methods. The article of manufacture or kit herein may comprise the cells, antibodies or reagents in separate containers or in the same container.

In some embodiments, the one or more containers holding the composition may be a single-use vial or a multi-use vial, which, in some cases, may allow for repeat use of the composition. In some embodiments, the article of manufacture or kit may further comprise a second container comprising a suitable diluent. The article of manufacture or kit may further include other materials desirable from a commercial, therapeutic, and user standpoint, including other buffers, diluents, filters, needles, syringes, therapeutic agents and/or package inserts with instructions for use.

In some embodiments, the kit can, optionally, include instructions. Instructions typically include a tangible expression describing the cell composition, optionally, other components included in the kit, and methods for using such. In some embodiments, the instructions indicate methods for using the cell compositions for administration to a subject for treating a disease or condition, such as in accord with any of the provided embodiments. In some embodiments, the instructions are provided as a label or a package insert, which is on or associated with the container. In some embodiments, the instructions may indicate directions for reconstitution and/or use of the composition.

### VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

The term "epitope" means a short peptide derived from a protein antigen, wherein the peptide binds to a major histo compatibility complex (MHC) molecule and is recognized in the MHC-bound context by a T cell. The epitope may bind an MHC class I molecule (e.g., HLA-A1 HLA-A2 or HLA-A3) or an MHC class II molecule.

The term "T cell adjuvant" refers to an agent or molecule that promotes T cell survival, rescues cells from apoptosis, sustains expansion and/or increases cytokine production. Exemplary T cell adjuvants include, for example, T cell costimulatory agonists or apoptosis inhibitors.

The terms "agonist" and "agonistic," such as with reference to a costimulatory agonist, refers to or describes a molecule which is capable of, directly or indirectly, substantially inducing, promoting or enhancing biological activity or activation mediated by a costimulatory receptor, such as OX40 or 4-1BB or other costimulatory receptor. An agonist can be an antibody or antigen-binding fragment or can be a ligand of a costimulatory receptor. For example, an agonist can be a biologically active ligand which binds to its complementary biologically active receptor and activates the latter either to cause a biological response in the receptor or to enhance preexisting biological activity of the receptor.

The term "allogeneic" as used herein means a cell or tissue that is removed from one organism and then infused or adoptively transferred into a genetically dissimilar organism of the same species.

The term "autologous" as used herein means a cell or tissue that is removed from the same organism to which it is later infused or adoptively transferred.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD. Among the provided antibodies are antibody fragments.

An "antibody fragment" or "antigen-binding fragment" refers to a molecule other than a conventional or intact antibody that comprises a portion of an conventional or intact antibody containing at least a variable region that binds an antigen. Examples of antibody fragments include but are not limited to Fv, single chain Fvs (sdFvs), Fab, Fab', Fab'-SH, F(ab')₂; diabodies; linear antibodies; an single-domain antibodies comprising only the V_{H} region (VHH).

As used herein, "bind," "bound" or grammatical variations thereof refers to the participation of a molecule in any attractive interaction with another molecule, resulting in a stable association in which the two molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs.

The term "biological sample" means a quantity of a substance from a living thing or formerly living thing. Such substances include, but are not limited to, blood, (for example, whole blood), plasma, serum, urine, amniotic fluid, synovial fluid, endothelial cells, leukocytes, monocytes, other cells, organs, tissues, bone marrow, lymph nodes and spleen.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

The term "concurrently" is used herein to refer to a procedure, such as an incubation, selection, enrichment or administration, involving two or more agents, where at least part of the particular procedure with one agent overlaps in time with at least a second agent.

The term "intermittently" is used herein to refer to a procedure, such as an incubation, selection, enrichment or administration, involving two or more agents, where the particular procedure involving each agent do not occur at regular intervals or are not continuous or stop and start repeatedly with periods in between.

The term "sequentially" is used herein to refer to a procedure, such as an incubation, selection, enrichment or administration, involving two or more agents, where the particular procedure involving each agent do not overlap in time.

As used herein, "isolated" or "purified with reference to a peptide, protein or polypeptide refers to a molecule which is substantially free of all other polypeptides, contaminants, starting reagents or other materials, or substantially free from chemical precursors or other chemicals when chemically synthesized. Preparations can be determined to be substantially free if they appear free of readily detectable impurities as determined by standard methods of analysis, such as high-performance liquid chromatography (HPLC), thin-layer chromatography (TLC) or capillary electrophoresis (CE), used by those of skill in the art to assess such purity, or sufficiently pure such that further purification would not detectably alter the physical and chemical properties of the substance.

As used herein, the term "recombinant" refers to a cell, microorganism, nucleic acid molecule, or vector that has been modified by introduction of an exogenous, such as heterologous, nucleic acid molecule, or refers to a cell or microorganism that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent molecule. The term "recombinant" also can refer to a protein product expressed from such a nucleic acid molecule or vector or from such cell or microorganism to which is introduced or modified with an exogenous nucleic acid.

As used herein, a composition refers to any mixture of two or more products, substances, or compounds, including cells. It may be a solution, a suspension, liquid, powder, a paste, aqueous, non-aqueous or any combination thereof.

As used herein, "optional" or "optionally" means that the subsequently described event or circumstance does or does not occur, and that the description includes instances where said event or circumstance occurs and instances where it does not. For example, an optionally substituted group means that the group is unsubstituted or is substituted.

The term "pharmaceutical composition" refers to a composition suitable for pharmaceutical use in a mammalian subject, often a human. A pharmaceutical composition typically comprises an effective amount of an active agent (e.g., cells, such as expanded in accord with the provided methods) and a carrier, excipient, or diluent. The carrier, excipient, or diluent is typically a pharmaceutically acceptable carrier, excipient or diluent, respectively.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and are compatible with other ingredients of the formulation. The pharmaceutically acceptable carrier is appropriate for the formulation employed.

Reference to "population of cells" herein is meant to refer to a number of cells that share a common trait. A population of cells generally contains a plurality of cells, such as greater than at or about 100 cells, at or about 1000 cells, and typically range from 1 × 10⁴ to 1 × 10¹⁰ in number.

The term "soluble" as used herein in reference to proteins, means that the protein is not bound, immobilized or attached to a particle, such as a cell or solid support, e.g. a bead. For example, a soluble protein includes a protein that is not bound as a transmembrane protein to the cell membrane of a cell. In some cases, solubility of a protein can be improved by linkage or attachment, directly or indirectly via a linker, to another molecule such as an Fc domain, which, in some cases, also can improve the stability and/or half-life of the protein. In some aspects, a soluble protein is an Fc fusion protein.

The term "specifically binds" as used herein means the ability of a protein, under specific binding conditions, to bind to a target protein such that its affinity or avidity is at least 10 times as great, but optionally 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule but may specifically bind to more than one target molecule. In some cases, a specifically binding protein may bind to a protein that has similarity in structural conformation with the target protein (e.g., paralogs or orthologs). Those of skill will recognize that specific binding to a molecule having the same function in a different species of animal (i.e., ortholog) or to a molecule having a substantially similar epitope as the target molecule (e.g., paralog) is possible and does not detract from the specificity of binding which is determined relative to a statistically valid collection of unique non-targets (e.g., random polypeptides). Solid-phase ELISA immunoassays, ForteBio Octet or Biacore measurements can be used to determine specific binding between two proteins. Generally, interactions between two binding proteins have dissociation constants (Kd) less than about 1x10⁻⁵ M, and often as low as about 1 × 10⁻¹² M. In certain aspects of the present disclosure, interactions between two binding proteins have dissociation constants of less than about 1x10⁻⁶ M, 1x10⁻⁷ M, 1x10⁻⁸ M, 1x10⁻⁹ M, 1x10⁻¹⁰ M, or 1x10⁻¹¹ M or less.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, a "subject" is a mammal, such as a human or other animal, and typically is human. The subject can be male or female and can be any suitable age, including infant, juvenile, adolescent, adult, and geriatric subjects.

The terms "effective amount" or "therapeutically effective amount" refer to a quantity and/or concentration of a therapeutic composition, such as containing cells, e.g. expanded in accord with the provide methods, that when administered to a patient yields any manner in which the symptoms of a condition, disorder or disease or other indication, are ameliorated or otherwise beneficially altered. An effective amount for treating a disease or disorder may be an amount that relieves, lessens, or alleviates at least one symptom or biological response or effect associated with the disease or disorder, prevents progression of the disease or disorder, or improves physical functioning of the patient. In particular aspects, there is a statistically significant inhibition of disease progression as, for example, by ameliorating or eliminating symptoms and/or the cause of the disease. In the case of cell therapy, the effective amount is an effective dose or number of cells administered to a patient. In some embodiments the patient is a human patient.

As used herein, "disease," disorder" or "condition" refers to a pathological condition in an organism resulting from cause or condition including, but not limited to, infections, acquired conditions, genetic conditions, and characterized by identifiable symptoms. In particular, it is a condition where treatment is needed and/or desired.

The terms "treating," "treatment," or "therapy" of a disease or disorder as used herein mean slowing, stopping or reversing the disease or disorders progression, as evidenced by decreasing, cessation or elimination of either clinical or diagnostic symptoms, by administration of a disclosed immunomodulatory protein or disclosed engineered cells either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. "Preventing," "prophylaxis," or "prevention" of a disease or disorder as used herein refers to the administration of a disclosed immunomodulatory protein or disclosed engineered cells expressing a disclosed immunomodulatory protein, either alone or in combination with another compound, to prevent the occurrence or onset of a disease or disorder or some or all of the symptoms of a disease or disorder or to lessen the likelihood of the onset of a disease or disorder. For example, in the context of cancer, the terms "treatment" or, "inhibit," "inhibiting" or "inhibition" of cancer refers to at least one of: a statistically significant decrease in the rate of tumor growth, a cessation of tumor growth, or a reduction in the size, mass, metabolic activity, or volume of the tumor, as measured by standard criteria such as, but not limited to, the Response Evaluation Criteria for Solid Tumors (RECIST), or a statistically significant increase in progression free survival (PFS) or overall survival (OS).

The term "antigen" refers to a molecule that can induce an immune response. Typically, an antigen is a molecule that is capable of being bound by a recognition site on an immune molecule, such as an antibody or T cell receptor if presented by major histocompatibility complex (MHC) molecules. An antigen can have one or more epitopes in which each epitope that is part of the antigen can be bound by a recognition site of an antibody or TCR/MHC complex. In some embodiments, an antigen is capable of inducing a humoral immune response or a cellular immune response leading to the activation of B lymphocytes and/or T lymphocytes.

As used herein, a "tumor-associated antigen" or "tumor-specific antigen" refers to a protein or other molecule that is found only on cancer cells and not on normal cells.

As used herein, "neoantigen" refers to an antigen to which the immune system has not previously been exposed, such as one that arises by alteration of host antigens by viral infection, neoplastic transformation, drug metabolism or other manner. In particular aspects, a neoantigen is an antigen encoded by tumor-specific mutated genes or that is a new antigen that develops in a tumor cell.

The term "in vivo" refers to an event that takes plane in a mammalian subject's body.

The term "ex vivo" refers to an event that takes place on or in a tissue or cells from a mammalian subject but outside of the mammalian subject's body. Typically the event is carried out in an external environment. In particular aspects, an ex vivo procedure includes any in which an organ, cell or tissue is taken from a subject, typically a living body, for a treatment or procedure and then returned to the subject.

The term "in vitro" refers to an event that takes place in a test system, such as in a laboratory.

As used herein, a kit is a packaged combination that optionally includes other elements, such as additional reagents and instructions for use of the combination or elements thereof.

The term "package insert" is used to refer to instructions customarily included in commercial packages of therapeutic products, that contain information about the indications, usage, dosage, administration, combination therapy, contraindications and/or warnings concerning the use of such therapeutic products.

As used herein, an "article of manufacture" is a product that is made and, in some cases, that can be sold. In some embodiments, the term can refer to compositions contained in articles of packaging, such as in a container.

It is understood that aspects and embodiments of the invention described herein include "comprising," "consisting," and "consisting essentially of" aspects and embodiments.

### VII. Examples

The following examples are included for illustrative purposes only and are not intended to limit the scope of the invention.

### Example 1 Assessment of Tumor Processing Methodologies in Obtaining a Population of Tumor-derived T-Cells

Tumors from patients with colorectal cancer (CRC) or melanoma were processed as described below and resultant infiltrating T-cell populations were analyzed for cell count viability.

### A. Colorectal Cancer

Tumors were sourced from primary tumors in patients with colorectal cancer and shipped overnight in HypoThermosol at 4 °C. Tumors were either processed as fragment or single cell suspension (SCS) cultures.

For fragment cultures, tumors were minced into fragments 1 - 8 mm in diameter, and each 1 - 8 mm fragment was placed into a well of culture vessel, either a gas-permeable 24-well culture plate or conventional 6-well plate, in the presence of Roswell Park Memorial Institute (RPMI) containing 5% human serum or serum free OpTmizer medium (ThermoFisher). Media was supplemented with either 300 or 6000 IU/mL recombinant IL-2, and also contained gentamicin at 10 µg/ml, Immune Cell Serum Replacement (ThermoFisher) at between 2 and 5% according to manufacturer's recommendations, and a final concentration of 2.0 mM of a L-alanyl-L-glutamine dipeptide form of glutamine (GlutaMAX Supplement; Thermofisher). Fragment cultures were maintained and monitored visually until a cell count was performed between approximately day 5 and 11 of culture.

For SCS cultures, tumors were also minced into fragments 1 - 8 mm in diameter. Fragments were then homogenized in a closed system using the Miltenyi GentleMACS in the presence or absence of an enzyme to digest the tumor, either an enzyme cocktail from the Miltenyi Tumor Dissociation Kit, human (part 130-095-929) used according to the manufacturer's recommendation, Collagenase I/II blend (Nordmark, Collagenase NB 4G Proved Grade, part: S1746503) or Collagenase IV (Worthington Biomedical part: LS004130) at 1 mg/ml or 5 mg/ml. Fragments designated for SCS with homogenization and enzyme digestion were incubated with the enzyme cocktail or collagenase for a total of 60 minutes. Immediately following the generation of SCS, cell counts and viability assessments were performed on the NC-200 Automated Cell Counter (ChemoMetec).

As shown in **FIG. 3A****,** SCS cultures with or without enzymatic digestion yielded more total viable cells (TVC) than was obtained following culture from CRC tumor fragments. Depicted in **FIG. 3B** the percent of viable cells was similar in cultures generated from fragments or SCS generated by homogenization in the presence of enzymes.

### B. Melanoma

Tumors were sourced from primary tumors in patients with melanoma and shipped overnight in HypoThermosol at 4 °C. Cells were cultured similarly as described above.

Briefly, for fragment cultures tumors were minced into fragments 1 - 8 mm in diameter, and each 1 - 8 mm fragment was cultured in a well of a gas-permeable 24-well culture plate or conventional 6-well plate in the presence of RPMI containing 5% human serum or serum free OpTmizer medium supplemented with recombinant IL-2 at a concentration of 300 IU/ml or 6000 IU/ml. The media also contained gentamicin at 10 µg/ml, and a final concentration of 2.0 mM of a L-alanyl-L-glutamine dipeptide form of glutamine (GlutaMAX Supplement; Thermofisher). Fragment cultures were maintained and monitored visually until a cell count was performed between day 5 and 9 of culture.

To generate SCS cultures, tumor fragments were homogenized using the Miltenyi GentleMACS in the presence or absence of an enzyme, which included Collagenase IV at a concentration of 1 mg/mL or 5 mg/mL or Collagenase I/II blend at 1 mg/mL or 5 mg/mL (Nordmark, Collagenase NB 4G Proved Grade, part: S1746503).. As above, cell count and viability assessments were performed immediately following the generation of SCS using the NC-200 Automated Cell Counter (Chemometec).

Depicted in **FIG. 4A****,** cultures generated from melanoma tumor fragments yielded more total viable cells than SCS generated by homogenization and dissociation with enzymes. As shown in **FIG. 4B****,** fragment cultures also had a higher percent viable cells than cells in SCS cultures, irrespective of enzymatic homogenization.

### Example 2 Assessment of T Cell Expansion Kinetics of Tumor-Derived Cells

Tumors were processed as described in Example 1 to generate 1 - 8 mm in diameter fragments or SCS cultures, which were then incubated under conditions to expand T cell populations present within the tumor. Cultures were grown under various tested conditions in the presence of recombinant IL-2 as described below in order to assess cellular expansion. Among the conditions that were tested were the effect of the type of culture plate, culture media, and concentration of IL-2 on cell expansion.

### A. Culture Conditions

Single cell suspensions (SCSs) obtained by homogenization and enzyme digestion of primary tumors from donor patients with CRC or melanoma, as described in Example 1, were cultured in a conventional 6-well plate or a gas-permeable 24-well culture plate. Where possible, multiple conditions from each donor were initiated and averaged (error bars represent ± standard deviation). For 6-well plates, cells were seeded between 250,000 and 1,000,000 cells/mL and for gas-permeable 24-well plates cells were seeded between 5,000 and 750,000 cells/mL. In both cases, the cells were seeded in either RPMI containing 5% human serum or serum free OpTmizer medium with recombinant IL-2 supplemented at a concentration of IL-2 of 300 IU/mL or 6000 IU/mL. The media also contained gentamicin at 10 µg/ml and a final concentration of 2.0 mM of a L-alanyl-L-glutamine dipeptide form of glutamine (GlutaMAX Supplement; Thermofisher). The cells were incubated for up to 31 days, typically 14 to 21 days, wherein 50% of cellular media was exchanged every other day beginning on the 5th day of culture.

For expansion from tumor fragments, an individual 1 - 8 mm tumor fragment, obtained as described in Example 1 from primary tumors of donor patients with CRC or melanoma, was placed in a well of a gas-permeable 24-well culture plate or a 6-well plate, and cultured in either RPMI containing 5% human serum or serum free OpTmizer medium with recombinant IL-2 supplemented at a concentration of 300 IU/mL or 6000 IU/mL. The media also contained gentamicin at 10 µg/ml, and a final concentration of 2.0 mM of a L-alanyl-L-glutamine dipeptide form of glutamine (GlutaMAX Supplement; Thermofisher). The cells were incubated for up to 31 days, such as typically 14 to 21 days, wherein 50% of cellular media was exchanged every other day beginning on the 5th day of culture.

For all conditions, cell counts were performed approximately every other day using the NC-200 Automated Cell Counter (Chemometec) and samples were collected for fluorescence-activated cell sorting (FACS). After completion of the expansion phase (e.g. day 14 - 31), cells were washed in PBS then cryopreserved in the presence of a cryoprotectant. Cryopreservation was carried out using CoolCell devices (Corning) or the VIA Freeze (GE Healthcare).

### B. Results

### 1. Growth Curves

Growth curves of expansion of SCS obtained from tumor fragments from CRC donor patients following expansion culture in different culture vessels are shown in **FIGS. 5A and 5B****.** The results shown are from cultures incubated with recombinant IL-2 at both 300 IU/mL or 6000 IU/mL concentrations, and in either media type, but are separated based on the source of starting cells. As shown, it was possible to expand tumor-derived cells from SCS from tumors of CRC donors under these conditions. In some donors greater than 2-fold and even as high as 10-fold or more expansion was observed in this initial expansion phase of cells obtained directly from CRC tumors.

Expansion achieved from SCS compared to tumor fragments from CRC tumor biopsy products were assessed. As shown in **FIGS. 5C and 5D****,** it was possible to expand cells from CRC tumors under these conditions whether extracted and cultured as fragments or as SCS. However, in general, a greater expansion was achieved in CRC cultures extracted as SCS, as evidenced by higher total cell numbers **(****FIG. 5C****)** and fold expansion **(****FIG. 5D****)** compared to culturing cells extracted via fragments.

Growth curves of expansion of cells cultured as extracted tumor fragments, or as SCSs from tumor fragments, from different melanoma donors following expansion culture in different culture vessels are shown in **FIGS. 6A and 6B****.** The results shown are from cultures incubated with recombinant IL-2 at both 300 IU/mL or 6000 IU/mL concentrations, and in either media type, but are separated based on the source of starting cells. As shown substantial expansion was observed in melanoma cultures extracted as tumor fragments in either culture vessel, whereas less expansion was observed for melanoma cells cultured as SCS.

Consistent with previous observations, tumor cells from certain donors were not amenable to expansion, irrespective of tumor type, indicative of inherent variability in expansion potential between donors and furthermore between tumor fragments of the same donor tumor. Larger scale methods in which tumor fragments from a donor patient are pooled during culture would be expected to mitigate against intra-tumor variability by combining tumor fragments from the same donor tumor.

### 2. Growth Assessment by Cellular Media

Expanded cultures generated as described above in RPMI media containing either 5% human serum or a serum replacement formulation (OpTmizer media) were compared after expansion for between 14 and 21 days. The results shown are from cultures incubated with recombinant IL-2 at both 300 IU/mL or 6000 IU/mL concentrations, and in either type of culture vessel, but are separated based on the type of media. The results for CRC tumors are from culture of SCS obtained from tumor fragments **(****FIG. 7A and 7B****),** while the results from melanoma tumors are from culture of tumor fragments **(****FIG. 8A and 8B****).**

For both tumor types, an increase in total cell number **(****FIG. 7A** **and** **FIG. 8A****)** and fold-expansion **(****FIG. 7B** **and** **FIG. 8B****)** was observed by culture in either the 5% human serum or serum replacement media for both tumor types. In the samples tested, there was a trend to improved expansion using OpTmizer media, as evidenced by higher overall cell number at the end of the initial expansion phase **(****FIG. 7A** **and** **FIG. 8A****).**

### 3. IL-2 Concentration

The effect of different IL-2 concentrations during the expansions from the different tumor types was compared. The cultures were expanded as described above in RPMI media containing either 5% human serum or a serum replacement formulation (OpTmizer media) for between 14 and 21 days, in either 300 IU/mL or 6000 IU/mL recombinant IL-2. The results shown are from cultures incubated in the presence of either media type, and in either type of culture vessel, but are separated based on the IL-2 concentration. The results for CRC tumors are from culture of SCS obtained from tumor fragments **(****FIG. 9A and 9B****),** while the results from melanoma tumors are from culture of tumor fragments **(****FIG. 10A and 10B****).**

For both tumor types, the results showed similar expansion of cells grown in either a high or low concentration of IL-2, as evidenced by similar total cell numbers after expansion **(****FIG. 9A** **and** **FIG. 10A****)** as well as fold expansion **(****FIG. 9B** **and** **FIG. 10B****).** These data support the observation that doses of IL-2 of about 300 IU/mL support expansion, and that a high dose of IL-2, such as 6000 IU/mL, is not necessary for cellular expansion for either CRC or melanoma cultures.

Together, the results show that while expansion can be donor and moreover, tumor sample dependent, CRC tumor infiltrating T-cells were grown successfully from SCS cultures and melanoma infiltrating T-cells from fragment cultures across multiple donors. It was similarly observed that for both melanoma and CRC derived T-cell cultures, the addition of high concentrations of IL-2 did not result in an appreciably distinct expansion response when compared with a lower dose.

### Example 3 Assessment of Anti-CD3 Stimulation on Expansion of Tumor-Derived Cells

Cells processed from melanoma tumor fragments as described in Examples 1 and 2, were cultured in the presence or absence of 50 ng/mL OKT3, a human anti-CD3 monoclonal antibody. Cell cultures were carried out to between 14 and 31 days in either a conventional 6-well plate or a gas permeable culture plate with RPMI or OpTmizer media. The cultures also were supplemented with 300 or 6000 IU/mL recombinant IL-2, 10 µg/mL gentamicin and a final concentration of 2.0 mM of a L-alanyl-L-glutamine dipeptide form of glutamine (GlutaMAX Supplement; Thermofisher). About 50% of cellular media was exchanged every other day beginning on the 5th day of culture as described previously. Cells were then counted using the NC-200 Automated Cell Counter (Chemometec).

The results shown are from cultures incubated with recombinant IL-2 at both 300 IU/mL or 6000 IU/mL concentrations, different medias, and in either type of culture vessel, but are separated based on the presence of absence of anti-CD3 stimulation. Cells from donor 6 were tested in both the presence or absence of anti-CD3 stimulation, and demonstrated 2-4 fold expansion in all conditions, with a 13-fold expansion observed in OpTmizer media supplemented with 300 IU/mL IL-2 with incubation in the absence of anti-CD3 stimulation (-OKT3). The results shown in **FIGS. 11A-11B** demonstrate that CD3 stimulation via OKT3 supported T cell expansion, but did not substantially impact total cell number **(****FIG. 11A****)** or fold expansion **(****FIG. 11B****).** These data are consistent with a finding that anti-CD3 stimulation (e.g. via OKT3) may not be necessary for the expansion of cells from tumor cultures.

### Example 4 Assessment of Post-Stimulation CD4+ and CD8+ Activation Markers

T-cells from three healthy donors were thawed, rested overnight in OpTmizer media supplemented with 300 IU/mL recombinant IL-2, and then activated using 50 ng/mL OKT3, a human anti-CD3 monoclonal antibody. Specific markers of activation on the CD4+ and CD8+ cell populations were measured using flow cytometry over a time course of 3 - 48 hours. Specifically, the following markers were assessed: CD38 and CD39 **(****FIG. 12A** **and** **FIG. 13A****),** CD134 and CD137 **(****FIG. 12B** **and** **FIG. 13B****),** and CD69 and CD90 **(****FIG. 12C** **and** **FIG. 13C****).**

Results for expression of activation markers on the surface of CD8 cells are shown in **FIGS. 12A-12C****,** which demonstrates the kinetics of upregulation of markers on CD8+ T cells in the 48 hours following stimulation with OKT3 compared to culture in the absence of OKT3. In some cases, some basal level of the markers can be seen on day 0 before stimulation. As shown, all assessed markers were upregulated to some extent during this time course, with the highest percentage of cells being upregulated for markers CD38 **(****FIG. 12A****),** CD134 **(****FIG. 12B****)** and CD69 **(****FIG. 12C****)** during this study.

Results for expression of activation markers on the surface of CD4 cells are shown in **FIGS. 13A-13C****,** which demonstrates the kinetics of upregulation of markers on CD4+ T cells in the first 48 hours following stimulation with OKT3 compared to the culture in the absence of OKT3 . As shown, all assessed markers were upregulated to some extent during this time course, with the highest percentage of cells being upregulated for markers CD38 **(****FIG. 13A****),** CD137 **(****FIG. 13B****)** and CD69 **(****FIG. 13C****)** during this study.

Taken together, these data support that expression of the above markers can be used as upregulation markers to select for T cells that have been activated, including under conditions of activation that would be expected to stimulate signaling via the TCR-CD3 complex as would occur following co-culture with antigen presenting cells presenting neoantigenic peptides.

### Example 5 Determination of Donor Cell Phenotype and Cellular Viability

T cells were sourced from primary tumors in patients with melanoma or CRC as described in Example 1. Cells from the tumors were extracted either as tumor fragments or as a SCS as described in Example 1, and then were assessed for T cell phenotype by flow cytometry.

For tumor fragments, each 1 - 8 mm fragment was placed into a well of culture vessel, either a gas-permeable 24-well culture plate or conventional 6-well plate, and incubated for between 5 and 11 days in the presence of RPMI containing 5% human serum or serum free OpTmizer medium (ThermoFisher). Media was supplemented with either 300 or 6000 IU/mL recombinant IL-2, and also contained gentamicin at 10 µg/ml, and a final concentration of 2.0 mM of a L-alanyl-L-glutamine dipeptide form of glutamine (GlutaMAX Supplement; Thermofisher). The incubation also was carried out with or without 50 ng/mL of anti-CD3 antibody OKT3. Fragment cultures were monitored visually until it was determined that a cell count could be performed (typically between day 5 and 9 of culture), and then cells were stained ad analyzed by flow cytometry for T cell markers.

Alternatively, for SCS cultures, tumors were minced into fragments 1 - 8 mm in diameter, then homogenized in the presence or absence of Collagenase IV (Worthington Biomedical part: LS004130) at 1 mg/ml or 5 mg/ml or Collagenase NB4G Proved Grade (Nordmark Biomedicals; Catalog No. S1746503) at 1 mg/mL. After incubation with enzyme for about 90 minutes, cells were immediately stained and analyzed by flow cytometry for T cell markers.

The gating hierarchies were designed as follows: first, the percentage of CD3+ cells from a parent population of total cell events were recorded, followed by the percentage of viable CD4+ cells from the CD3+ parent populations and next the percentage of viable CD8+ cells from the same parent CD3+ population. The memory T-cell populations (Tem) were then calculated based on their respective CD4+ and CD8+ parent populations. Thus, CD4/Tem was determined from the parent population of viable CD4+ cells, while CD8/Tem was determined from the parent populations of viable CD8+ cells. Thus, the results, as depicted in FIG. 12, are the percentage of CD3+ cells from a parent population of total cell events recorded, which were sorted in a hierarchy into subpopulations as a percentage of the respective parent population in the hierarchy. **FIG. 14** depicts the percentage of viable cells positive for select T cell markers in single cell suspensions immediately after extraction of tumor fragments by homogenization and enzyme digestion from an exemplary CRC donor (donor 1).

The percentage of CD3+ cells was compared in SCS samples that had been extracted by homogenization only (no collagenase) or by homogenization following digestion with a low concentration of collagenase (1 mg/mL) or a high concentration of collagenase (5 mg/mL). Results from a second CRC and a melanoma patient are shown in **FIG. 15A** and **FIG. 15B****,** respectively. As shown in **FIG. 15A****,** the results demonstrate an increased recovery of CD3+ T cells in SCSs from a CRC donor following homogenization and digestion with a low concentration of collagenase. Although the percent of CD3+ cells in SCSs from a melanoma door was lower, the results also demonstrate that homogenization and digestion with a low concentration of collagenase yielded the highest percentage of CD3+ T cells **(****FIG. 15B****).** Taken together, these observations demonstrate relative high purity of cells from SCS from melanoma tumors can be achieved and may support that SCS is a viable source of melanoma derived CD3+ cells.

The percentage of CD3+ cells in SCSs extracted from tumors of an additional exemplary CRC donor also was assessed. In addition, in this same donor, the percentage of CD3+ T cells in the SCSs immediately following homogenization and digestion was compared to (1) the percentage of CD3+ cells after culture of SCSs with 300 IU/mL IL-2 (low) or 6000 IU/mL IL-2 (high) for 6 days, or (2) the percentage of CD3+ cells following culture of individual tumor fragments for up to 6 days with 300 IU/mL IL-2 (low) or 6000 IU/mL (high) in the presence of absence of CD3 stimulation (OKT3). As shown in **FIG. 15C****,** the percent of CD3 cells in baseline (day 0) SCS was substantially higher than the percent of CD3+ cells in cultures obtained following culture of tumor fragments with IL-2 or OKT3 for 6 days. Similar results from culture of tumor fragments from two additional donors was observed, in which the percentage of CD3+ cells in cultures obtained following culture of CRC-derived tumor fragments with IL-2 and/or OKT3 for 11 days **(****FIG. 15D****)** or 9 days **(****FIG. 15E****)** also generally showed a low yield when extracting tumor cells from CRC tumor fragments under various assessed conditions. These results are consistent with a finding that SCSs from tumor biopsies of CRC patients may be more capable of providing an increased number of T cells for expansion than cells obtained from culture of tumor fragments.

In contrast to the results from culture of tumor fragments for CRC patients, FIG. 16 shows that a high percentage of CD3+ T cells can be obtained from culture of melanoma tumor fragments under various conditions, such as presence of low (300 IU/mL) or high (6000 IU/mL) concentrations of IL-2, presence of absence of CD3 stimulation (OKT3) or different media. The results depicted in **FIG. 16** are from a Day 0 culture. These results are consistent with a finding that culture of tumor fragments from melanoma patients may be more capable of providing an increased number of T cells for expansion than cells obtained from SCSs of tumor biopsies.

### Example 6 Quantification of Activation of Tumor Derived T Cells Following Co-Culture with Antigen Presenting Cells

T cells were sourced from primary tumors in patients with melanoma or CRC as tumor fragments, as described in Example 1. Following 5 days of culture in serum free OpTmizer medium (ThermoFisher) supplemented with 300 IU/mL recombinant IL-2, gentamicin at 10 µg/ml, Immune Cell Serum Replacement (ThermoFisher) at between 2 and 5% according to manufacturer's recommendations, and a final concentration of 2.0 mM of a L-alanyl-L-glutamine dipeptide form of glutamine (GlutaMAX Supplement; Thermofisher), tumor derived cells were washed with OpTmizer medium before being centrifuged at 300 x g for 5 minutes and suspended at 2 x 10⁶ cells/mL. Cells were then seeded into a conventional 6-well culture plate at 10,000,000 cells/well.

In a parallel culture, antigen presenting Dendritic Cells (DCs) were differentiated from PBMCs obtained from the same patient (autologous) as sourced T cells. Cryovials of frozen PBMC isolated from apheresed donors were thawed from liquid nitrogen storage. Cells were thawed in a ten-fold volume of 1X DPBS (Gibco) and counted (NucleoCounter NC200). After washing, cells were immediately used for CD14 microbead positive selection (MACS Miltenyi) according to manufacturer kit instructions. Purified CD14 (monocyte) cells were counted, the cells were resuspended in DendriMACs (MACS Miltenyi) and seeded at a density of 0.5-2 x 10⁶ cells per mL in the appropriate culture flask. GM-CSF (100 ng/mL) and IL-4 (20 ng/mL) were added to cultures to promote differentiation into immature dendritic cells. Monocytes were cultured and differentiated for a total of 5 days, with a 50% addition of medium equal to 50% of the starting amount of medium on day 2.

Coding transcripts of tumor specific peptides were identified autologously for each patient from whole exome sequencing and RNA sequencing as described in Parkhurst, Maria R., et al. "Unique neoantigens arise from somatic mutations in patients with gastrointestinal cancers." Cancer discovery 9.8 (2019): 1022-1035. Whole-exome sequencing (WES) of patient samples was performed on snap-frozen, unfixed, tumor tissue and normal peripheral blood cells (normal source). Alignments of sequences from tumor vs. normal samples were performed using novoalign MPI from novocraft (http://www.novocraft.com/) to human genome build hg19. Duplicates were marked using Picard's MarkDuplicates tool. Insertion deletion realignment and base recalibration was carried out according to the GATK best practices workflow (https://www.broadinstitute.org/gatk/). Post cleanup of data, pileup files were created using samtools mpileup (http://samtools.sourceforge.net) and Varscan2, (http://varscan.sourceforge.net), SomaticSniper (http://gmt.genome.wustl.edu/packages/somaticsniper/), Strelka (https://sites.google.com/site/strelkasomaticvariantcaller/), and Mutect (https://www.broadinstitute.org/gatk/). VCF files were merged using GATK CombineVariants tools and annotated using Annovar (http://annovar.openbioinformatics.org). Variants (mutations) present in patient tumors were then annotated using Annovar (http://annovar.openbioinformatics.org).

The following filters were used to generate an initial list of putative mutations for evaluation: (1) a tumor and normal coverage of greater than 10, (2) a variant allele frequency (VAF) of 7% or above, (3) variant read counts of 4 or above, (4) and two of the four callers identifying mutations. For insertions and deletions, the same cutoffs were used except only a single caller identifying the mutation was required to pass filters, as these were only called by varscan and strelka. Tables of amino acid sequences corresponding the mutant residue joined to the 12 amino acids encoded by regions upstream and downstream of single nucleotide variants (SNVs) (Nmers) were generated for those variants which passed the four filters. For frame-shifted transcripts, sequences were translated until a stop codon was generated in either the normal coding region or in the 3' un-translated region. The Integrative Genomics Viewer (IGV, Broad Institute), which allows mapped alignments to be visualized, was then used to carry out manual curation of the variant calls. Changes were made to the sequences of Nmers when manual curation revealed non-synonymous changes resulting from additional somatic variants or germline variants present within transcripts encoding the Nmers. Variants inferred from reads containing multiple mis-matched nucleotides, insertion/deletions mapping to different locations in different reads, and variants corresponding to frequent SNPs were flagged for removal.

Variants that were detected in more than one patients tumor but in fewer than 2.5% of total tumors were flagged but included in the list of variants that passed. Variant transcripts only annotated in the ENSEMBL database generally represent unverified coding regions and were also removed. Variants flagged as being known single nucleotide polymorphisms or were present in multiple tumors were not automatically removed but were further evaluated using IGV, as removing potential false positives, which are unlikely to encode products recognized by T cells, was less critical than removing candidates that could represent false negatives.

These sequencing data were then used to generate a peptide pool representing mutated peptide associated with the tumor and wild-type peptide associated with non-diseased peripheral blood cells.

Synthetic peptides were synthesized via Fmoc chemistry. For indels, 25 amino acid peptides were synthesized overlapping by 10 amino acids based on the translation of the frame-shifted sequence until the next stop codon. In some cases, peptides of minimal epitopes were synthesized. Peptides were dissolved in DMSO and mixed in equal volumes.

Differentiated DCs were loaded with a varying number and concentration of peptides from a peptide pool identified as described above before they were added to the tumor derived culture at several ratios of Tumor Cells: DC. DCs and tumor derived cells were then co-cultured at 37°C for 6 hours at 5% CO₂ before the culture was gently agitated and cells in suspension recovered. Recovered cells were then sorted via flow cytometry for activated T-cells using markers of T cell activation 4-1BB and OX40.

**FIG. 17A and 17B** show tumor derived T cell activation over a peptide range of 20 to 0.1 ng/mL. As shown in **FIG. 17A****,** T cell co-culture with DCs loaded with each of three peptide concentrations tested resulted in readily detectable levels of 4-1BB/OX40+ T cells, including as high as approximately 80% at 1 ng/mL peptide. Increase in T cell activation marker expression as compared to cells cultured with unloaded DCs is shown in **FIG. 17B****,** where 0.1 ng/mL resulted in the largest delta but all three concentrations of peptide resulted in a positive fold change. These data demonstrate that lower peptide concentrations less than 20 ng/mL may result in increased upregulation of T cell activation markers (upregulation markers) following co-culture.

**FIG. 18** similarly depicts tumor derived T cell activation as a function of 41BB/OX40 expression in studies in which DCs were pulsed with one or two peptides for surface presentation during co-culture. Shown in **FIG. 18A** and again in **FIG. 18B** as fold change, DCs which were loaded with only one peptide were more markedly more efficient at activating T cells in co-culture.

As shown in **FIG. 19****,** markers of T cell activation 41BB and OX40 were substantially upregulated when tumor derived T cells were co-cultured with DCs at a ratio of 1:2 (T cells: DC) as compared to 1:1.

### Example 7 Enrichment and Recovery of Activated T-Cells via Cell Sorting

T-cells from a healthy donor were isolated by immunoaffinity-based selection and then cryopreserved. The T cells were thawed and rested overnight, and then were activated with 50 ng/mL OKT3 for 24 - 48 hours prior to staining with anti-CD4 FITC (BD), anti-CD8 PerCPCy.5.5 (BD), anti-CD134 (Beckman Couleter), and anti-CD137 (MACs Miltenyi). Cells were brought to a concentration of approximately 20 x 10⁶ cells/mL and sorted using the BD FACSAriaII at a sort rate of approximately 15,000 events per second. A gate was drawn around cells expressing CD134, CD137, or both CD134 and CD137 and sorted into a single population. This was the positive sorted population. Cells lacking both CD134 and CD137 expression were sorted into a separate population. This was the negative sorted population. After sorting, cells from the positive and negative sorted populations and the unsorted population were analyzed on an alternative flow cytometer to verify purity and assess recovery rates.

As shown in **FIG. 20****,** the unsorted tumor derived T cell population (pre-sort) were compared to the positive sorted population which was collected after co-culture with mutant peptide loaded autologous dendritic cells as described previously in Example 6 and sorted into 41BB/OX40 positive populations. It was observed that this gating strategy resulted in an increased enrichment for percent reactive TCR for three donors **(****FIG. 20A****)** and average Class I reactivity **(****FIG. 20B****).**

Total cell recovery from cell sorting is shown with respect to total cell input in **FIG. 21A****.** Similarly seen in **FIG. 21B****,** percent recovery from two independent runs was approximately 80%. The results demonstrate that it is possible to obtain a high recovery of cells after selection and sorting of cells positive for upregulation markers.

**FIG. 22** depicts CD4+ population purity via flow cytometry of healthy donor T cells activated with OKT3 and stained as described above. Cells were first gated on CD4+, then the population expressing the highest intensity of CD134+ was next gated and the outputs displayed showing CD4+ vs. CD8+ and CD137+ vs. CD134+. These data support the use of these markers for gating a high purity population of tumor infiltrating T cells.

### Example 8 Post-Sort Expansion of Activated Tumor Derived T Cells

T cells sourced from a primary CRC tumor were processed as described in Example 1, and then were co-cultured with peptide presenting dendritic cells, using methods as described in Example 6. Briefly, isolated tumor infiltrating lymphocytes were cultured with autologous DCs that were loaded to express peptide associated with healthy tissue (Wild-type, WT), peptide associated with tumor tissue (Mutant), or were not loaded with peptide at all (No peptide). A control subpopulation of T cells were cultured without DCs (Unactivated). After the co-culture, the cells were sorted via the fluorescence enabled Sony FX500 based on surface expression of activation markers 4-1BB and OX40.

Cells were then seeded into a gas permeable 24 well culture plate at 250,000-1,000,000 cells/cm2 in serum free OpTmizer medium supplemented with recombinant IL-2 at a concentration of 300 IU/mL, gentamicin at 10 µg/mL, and 2.0 mM of a L-alanyl-L-glutamine dipeptide form of glutamine (GlutaMAX Supplement; Thermofisher). Cells were incubated for a total of 7 days with 50% of the media exchanged every other day beginning on culture day 5. Cell counts were performed using NC-200 Automated Cell Counter (Chemometec). on each culture day.

As shown in **FIG. 23A** and again in **FIG. 23B** as fold expansion, each tumor infiltrating lymphocyte (TIL) T cell population tested underwent measurable expansion between culture days 3 and 5 and continued to trend upwards at the 7 day conclusion of the culture period. Tumor infiltrating T cells cultured with DCs loaded with mutant tumor-associated peptide reached the highest total cell number over the course of the experiment.

Using the data above, a theoretical mathematical model shown in **FIG. 23C** was created to predict the relationship between the number of cells recovered after sorting and the expected number of cells present in culture following the expansion phase.

### Example 9 Monte Carlo Modeling Ex Vivo Expansion of Tumor Derived T Cells

In complement to the deterministic point analysis in Example 8, a first probabilistic Monte Carlo simulation was designed to forecast the number of tumor infiltrating lymphocytes resultant from a first expansion as described in Example 2. Monte Carlo simulations of likely total viable and total reactive T cell numbers post extraction and a first expansion were run by substituting a probability distribution for two factors of inherent uncertainty, recovery efficiency and fold expansion capacity. The results were iteratively calculated tens of thousands of times as a normal distribution wherein a mean value of cells recovered was defined for low and mid recovery, and a mean value for fold change in expansion was defined for low, mid, and high expansion potentials. Distributions were then calculated for total viable T cell number as well as total reactive T cells.

For the initial Monte Carlo simulations, wherein probable T cell outputs are calculated for a first expansion, test cases were run to model low recovery/low expansion, mid recovery/low expansion, mid recovery/mid expansion, and mid recovery/high expansion conditions. Values for the mean and standard deviation for both recovery and expansion variables were assigned as follows: (1) low recovery was defined as culturing a total of 20 million viable cells from the processed tumor at a standard deviation of 6 million, (2) mid recovery was defined as 50 or 60 million cells with a standard deviation of 15 million, (3) a low first fold expansion was defined as 50 fold with a standard deviation of 11, (4) mid expansion was defined as 75 fold with a standard deviation of 15, and (5) high expansion was defined as 500 fold with a standard deviation of 160.

Data for each test case from the first Monte Carlo simulations A are displayed in **Table E1** below.

| **Table E1: Monte Carlo Simulations for a first expansion** | | | | |
|---|---|---|---|---|
| | | ***Total Viable Tumor Infiltrating T Cells*** | | ***Total Reactive Tumor Infiltrating T Cells*** |
| **Test Conditions** | **Assumptions** | **Statistics** | **Forecast Values** | **Forecast Values** |
| Low Recovery/ Low Expansion | Mean = 20 | Mean | 1.99 x10⁷ | 1.59 x10⁶ |
| | Std. Dev = 6 | Median | 1.99 x10⁷ | 1.51 x10⁶ |
| | Mean = 50 | Probability Distribution (10-90^{th} Percentile) | 1.22 x10⁷ - 2.76 x10⁷ | 7.48 x10⁵ - 2.53 x10⁶ |
| | Std Dev = 11 | | | |
| Mid Recovery/Low Expansion | Mean = 50 | Mean | 4.99 x10⁷ | 3.99 x10⁶ |
| | Std. Dev = 15 | Median | 5.00 x10⁷ | 3.81 x10⁶ |
| | Mean = 50 | Probability Distribution (10-90^{th} Percentile) | 3.05 x10⁷ - 6.93 x10⁷ | 1.85 x10⁶ - 6.34 x10⁶ |
| | Std Dev = 11 | | | |
| Mid Recovery/Mid Expansion | Mean = 60 | Mean | 6.00 x10⁷ | 4.81 x10⁶ |
| | Std. Dev = 15 | Median | 5.99 x10⁷ | 4.63 x10⁶ |
| | Mean = 75 | Probability Distribution (10-90^{th} Percentile) | 4.07 x10⁷ - 7.91 x10⁷ | 4.07 x10⁷ - 7.91 x10⁷ |
| | Std Dev = 15 | | | |
| Mid Recovery/High Expansion | Mean = 60 | Mean | 4.78 x10⁷ | 5.99 x10⁷ |
| | Std. Dev = 15 | Median | 4.60 x10⁷ | 6.00 x10⁷ |
| | Mean = 500 | Probability Distribution (10-90^{th} Percentile) | 4.07 x10⁷ - 7.94 x10⁷ | 2.43 x10⁶ - 7.40 x10⁶ |
| | Std Dev = 160 | | | |

Using these data, a second set of Monte Carlo simulations were designed to predict the final number of reactive tumor infiltrating lymphocytes following co-culture with APCs, sorting via flow cytometry, and a second expansion as described in Example 8. A fixed value for percentage of reactive T cells present in the population of total T cells cultured from either tumor fragments or SCS was assigned to a mean of 8% and a standard variation of 2.50. Following tens of thousands of iterative calculations, data for each test case from the second Monte Carlo simulations are depicted in Table E2 below.

| **Table E2: Monte Carlo Simulations for a second and final expansion** | | | |
|---|---|---|---|
| | | ***Total Reactive Tumor Infiltrating T Cells*** | |
| **Test Conditions** | **Assumptions** | **Statistics** | **Forecast Values** |
| Low Recovery/ Low Expansion | Mean = 20 | Mean | 7.96 x10⁷ |
| | Std. Dev = 6 | Median | 7.34 x10⁷ |
| | Mean = 50 | Probability Distribution (10-90^{th} Percentile) | 3.41 x10⁷ - 1.34 x10⁸ |
| | Std Dev = 11 | | |
| Mid Recovery/Low Expansion | Mean = 50 | Mean | 2.00 x10⁸ |
| | Std. Dev = 15 | Median | 1.85 x10⁸ |
| | Mean = 50 | Probability Distribution (10-90^{th} Percentile) | 8.44 x10⁷ - 3.33 x10⁸ |
| | Std Dev = 11 | | |
| Mid Recovery/Mid Expansion | Mean = 60 | Mean | 3.61 x10⁸ |
| | Std. Dev = 15 | Median | 3.39 x10⁸ |
| | Mean = 75 | Probability Distribution (10-90^{th} Percentile) | 1.68 x10⁸ - 5.80 x10⁸ |
| | Std Dev = 15 | | |
| Mid Recovery/High Expansion | Mean = 60 | Mean | 2.39 x10⁹ |
| | Std. Dev = 15 | Median | 2.19 x10⁹ |
| | Mean = 500 | Probability Distribution (10-90^{th} Percentile) | 9.57 x10⁸ - 4.06 x10⁹ |
| | Std Dev = 160 | | |

Recovery and expansion potential of tumor infiltrating reactive T cells from a first expansion following tumor processing or a second expansion following downstream co-culture with APCs are factors which are inherently variable across donors and within a tumor cell population. The expected range of T cell numbers generated by the process described here is contained within the 10^{th} and 90^{th} percentiles. Cell numbers below the 10^{th} percentile are unlikely to be generated and will likely not result in a usable drug product. Therefore, the observations from the Monte Carlo simulations in **Tables E1 and E2** support that in all scenarios between the 10^{th} and 90^{th} percentiles, given a range of levels of variability for expansion potentials, the method described herein will likely provide a robust T cell output that is approximate to the number of cells which would be required for therapeutic dosing.

### Example 10 Assessment of Tumor-reactive TCR Enrichment by IFN-gamma production and TCR clonality

T cells sourced from primary tumor of patients with ovarian cancer (Sample A), CRC (Sample B) or melanoma (Sample C) were processed from tumor fragments as described in Example 1. Following the initial expansion, T cells were then co-cultured with peptide presenting, autologous dendritic cells for 6 hours using methods substantially as described in Example 6. For the co-culture, autologous DCs were loaded with either a mutant single long peptide (e.g. 25 mer) unique to the patient tumor or a wild-type single long peptide that was not-mutated compared to normal sample from the patient. After the co-culture, tumor-reactive T cells were enriched by staining the cells for expression of 4-1BB (CD137) and/or OX40 (CD134) and sorting the cells by fluorescence-activated cell sorting (FACS). Cells that were positive for either or both of 41BB and OX40 were collected as the "positive" population (also called "mutant enriched" population) and cells that were double negative for 41BB and OX40 were collected as the "negative" population (also called "wild-type unenriched" population).

The mutant and wild-type T cell populations were then cultured for 16 hours in media alone or under conditions to stimulate IFN-gamma secretion. Unsorted, unenriched T cells (bulk T cells) from the co-culture that had not been sorted based on 41BB and OX40 expression were included as a pre-selection control and were similarly stimulated. Culture supernatant was collected and IFN-gamma secretion levels were determined by ELISA.

The percentage of T cells in the sorted population expressing a TCR reactive to the peptide neo-epitope was determined by single cell TCR sequencing . TCR clonality in the T cell populations also was determined by single cell RNA- sequencing for the TCR-beta and TCR-alpha chains.

### 1. Sample A (Ovarian Cancer)

The mutant and wild-type enriched T cell populations, or control bulk T cells, produced from Sample A tumor cells were cultured for 16 hours in media alone or were stimulated by culture with anti-CD28 and anti-CD49d antibodies along with either the minimal peptide epitope (8mer) corresponding to the mutant peptide (neo-epitope) or the wild-type peptide from the respective patient tumor. As shown in **FIG. 24A****,** bulk T cells exhibited improved reactivity, as evidenced by increased IFN-gamma secretion, following culture with the neo-epitope compared to culture media alone. The ability to produce IFN-gamma was further increased in the mutant enriched T cell population that was stimulated with the neo-epitope, but no difference was observed in the wild-type enriched T cell population following stimulation in media alone versus stimulation with the neo-epitope conditions. Additionally, the wild-type unenriched T cell population still included some degree of neo-antigen reactive T cells, as evidenced by their upregulation of IFN-gamma secretion compared to media alone. This data indicates that the bulk T cells following co-culture contain a neoantigen reactive population, which is enriched by sorting based on expression of 41BB and OX40. Further, the results also demonstrate the specificity of neo-antigen enrichment.

Analysis of neoepitope-specific TCRs by RNA sequencing and flow cytometry showed an enrichment of TCR "A" neoantigen-specific TCRs in the mutant enriched T cell populations with 17% neoantigen-specific TCRs compared to 2% in the initial bulk T cell population or 0.1% in the wild-type enriched T cell population **(****FIG. 24B****).** The TCR clonality of T cells in the unselected population (wild-type enriched T cell population) compared to selected population (mutant enriched T cell population) is shown in **FIG. 24C****,** which shows that the incoming TCR diversity is high the unsorted T cell population and that enrichment of unique TCR clones is achieved in the selected population. **FIG. 24D** demonstrates that the Pre- (bulk) and post-sort cell populations contain CD4 and CD8 cells, indicating that class I and class II reactive cells are present in the enriched population,

### 2. Sample B (CRC Patient)

The mutant and wild-type enriched T cell populations, or control bulk T cells, produced from Sample B tumor cells were cultured for 16 hours in media alone or were stimulated in response to a general TCR stimulation using an anti-CD3 antibody (OKT3). As shown in **FIG. 25A****,** all T cell populations displayed functionality (i.e. IFNg production) after co-culture and sorting in response to the general TCR stimulation.

Analysis of neoepitope-specific TCRs showed an enrichment of neoantigen "B"-specific TCRs in the mutant enriched T cell populations with 71% neoantigen-specific TCRs compared to 42% in the initial bulk T cell population or 17% in the wild-type enriched T cell population **(****FIG. 25B****).** Compared to the bulk T cells after co-culture, this represents an approximate 1.7-fold enrichment in the tumor-reactive T cells in the sorted T cell population, and an approximate 2.5-fold reduction in tumor-reactive T cells in the non-sorted T cell population. The TCR clonality of T cells in the unselected population (wild-type enriched T cell population) compared to selected population (mutant enriched T cell population) is shown in **FIG. 25C****,** which shows that the incoming TCR diversity is high in the unsorted T cell population (807 unique TCR clones) and that enrichment of unique TCR clone is achieved in the selected population (64 unique TCR clones). **FIG. 25D** demonstrates that the pre- (bulk) and post-sort cell populations contain CD4 and CD8 cells, indicating that class I and class II reactive cells are present in the enriched population.

### 3. Sample C (Melanoma Patient)

T cells in the mutant and wild-type enriched T cell populations, or control bulk T cells, produced from Sample C tumor cells were assessed for neoepitope-specific TCRs by RNA sequencing and flow cytometry and TCR clonality. The results showed an enrichment of neoantigen "C"-specific TCRs in the mutant enriched T cell populations with 33% neoantigen-specific TCRs compared to 5% in the initial bulk T cell population or 4% in the wild-type enriched T cell population **(****FIG. 26A****).** Compared to the bulk T cells after co-culture, this represents an approximate 7-fold enrichment in the tumor-reactive T cells in the sorted T cell population, and no enrichment in tumor-reactive T cells in the non-sorted T cell population. The TCR clonality of T cells in the unselected population (wild-type enriched T cell population) compared to selected population (mutant enriched T cell population) is shown in **FIG. 26B****,** which shows that the incoming TCR diversity is high in the unsorted T cell population (182 unique TCR clones) and that enrichment of unique TCR clone is achieved in the selected population (15 unique TCR clones). **FIG. 26C** demonstrates that the pre- (bulk) and post-sort cell populations contain CD4 and CD8 cells, indicating that class I and class II reactive cells are present in the enriched population.

### 4. Conclusion

Together, the results show that the incoming TCR diversity is high in the unsorted T cell population (e.g. 100-900 TCRs). This unsorted population produces a low level of IFNgamma (e.g. 5-25 pg/mL). After sorting of the TCR population based on activation markers, e.g. OX40/41BB, the TCR population is enriched in a reactive population of TCR's (e.g. 15-64 TCRs) that produce higher IFNgamma (e.g. 65.3-98.6 pg/mL) than the unsorted and negative sorted population (5 pg/mL) of TCRs. The results indicate that this is specific activation consistent with enrichment of tumor-reactive T cells as it is not seen in the wild-type, unsorted co-cultures.

### Example 11 Assessment of Effect of T cell Adjuvants on T cell Viability

Cells were expanded from PBMC derived from Ficoll gradient separation from three healthy donor's apheresis material. PBMCs were seeded at 2 x 10⁶ cells/ml in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher) and activated with human anti-CD3 antibody OKT3 antibody for 48 hours. Cells were next seeded into gas permeable 100M culture vessels and expanded for 7 - 14 days to achieve a large bank of T cells, and cryopreserved. Previously expanded human T cells from three healthy donors were thawed and then seeded into 96-well culture plates to a final cell density of 5 x 10⁵ cells/mL with a test adjuvant agent at a range of concentrations in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher). Half of wells were additionally supplemented with 50 ng/mL OKT3, a human anti-CD3 monoclonal antibody. In total, 15 test adjuvant agents were tested for their impact on cell viability in the presence and absence of anti-CD3 activation (see **Table E3).** Cells were cultured for 6 days total, including a 50% medium exchange on culture day 3, and monitored for total viable CD3+ cell count.

| **Table E3: Compounds and concentrations used in high throughput screen** | |
|---|---|
| Compound | Concentration |
| ***Antibodies*** | |
| Tavolixizumab, Oxelumab, Ipilimumab, Tocilizumab, Urelumab, Pembrolizumab, Varlilumab, anti-GITR MK-1248 | 0.625 µg/mL - 10 µg/mL |
| FasL (Human anti-FasL antibody) | 0.625 µg/mL - 10 µg/mL |

| ***Small Molecule Inhibitors*** | |
|---|---|
| Z-VAD-FMK | 1.5625 µM - 25 µM |
| NVP-HSP990 | 15.625 nM-250 nM |

| ***Cytokines*** | |
|---|---|
| IL-7 | 62.5 IU/ml - 1000 IU/ml |
| IL-15 | 62.5 IU/ml - 1000 IU/ml |
| IL-21 | 62.5 IU/ml - 1000 IU/ml |
| *DMSO (Control)* | *0%* - *1% v*/*v* |

Total viable CD3+ cell count for cells grown in the absence and presence of OKT3 stimulation are shown in **FIGS. 27A-C** and **FIGS 28A-C,** respectively. The results shown are for the following concentrations of adjuvant: 10 µg/mL for tested antibodies (Tavolixizumab, Oxelumab, Ipilimumab, Tocilizumab, Urelumab, Pembrolizumab, Varlilumab, anti-GITR MK-1248, anto-human FasL); 25 µM for Z-VAD-FMK pan-caspase inhibitor; 250 nM for HSP inhibitor NVP-HSP990; and 1000 IU/mL for cytokine (IL-7, IL-15 or IL-21).

Overall, toxicity was not observed for any of the compounds tested, indicating these compounds would not be detrimental to TIL manufacturing. While inherent donor variability was observed, treatment with anti-PD1 antibody Pembrolizumab, anti-OX40L antibody Oxelumab, and pan-caspase inhibitor Z-VAD-FMK resulted in consistently higher viable cell counts than the DMSO treatment control, irrespective of activation status.

Dose response curves for IL-7 and IL-15, shown in **FIG. 29A** and **FIG. 29B****,** respectively, showed a dose dependent response, wherein cell number increased with increasing concentration. These data support that IL-7 and IL-15 at this range of tested concentrations may be beneficial for potentiating total T cell number during culture.

### Example 12 T Cell Expansion with Fas Ligand or Caspase Inhibition

Apoptotic inhibitors directed against Fas- and caspase-mediated pathways were assessed to determine effects on tumor reactive T cells during manufacturing. The studies were carried out in healthy donors, and thus, stimulation of the T cells were carried out with anti-CD3 (OKT3) or anti-CD3/anti-CD28 stimulation in order to mimic conditions present in the tumor microenvironment (TME). Constant activation signals present in the tumor microenvironment, as can be stimulated by anti-CD3 or anti-CD3/anti-CD28 activation can be detrimental to T cell growth. Cellular viability and projected cell numbers were used to compare the impact of modulation of apoptotic pathways in both a transient and a continuous activation assay, the latter of which more closely recapitulates the tumor microenvironment.

### A. Anti-CD3 Stimulation

PBMC from three healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 2.14 x 10⁵ cells/mL (7.5 x 10⁵ cells/cm²). Cells were activated for 48 hours using 50 ng/ml OKT3, a human anti-CD3 monoclonal antibody, and were additionally treated with agents as described below.

Culture wells were assigned to one of five treatment groups, as follows: (1) a no inhibitor culture control which contained only cellular media as described with no additional apoptotic modulator; (2) 2 µM of the pan-caspase inhibitor Z-VAD-FMK added to the media only on culture day 0 (transient); (3) 2 µM of the pan-caspase inhibitor Z-VAD-FMK added on culture day 0 and additionally replenished at the same concentration with each media exchange (continuous); (4). 500ng/ml of Fas ligand (FasL) blocking antibody NOK-1 (BioLegend) added to the culture media only on day 0 (transient); or (5) 500ng/ml of Fas ligand (FasL) blocking antibody NOK-1 (BioLegend) added to the culture media only on day 0 and additionally replenished each media exchange (continuous inhibition).

Cultures were maintained for at least 13 days with a 50% media exchange every other day beginning on culture day 2. Cell counts and viability were monitored every other day. When cells reached 3 x 10⁶ cells/ml, 1.5 x 10⁶ cells were sub-cultured into a new well of a 24-well gas permeable culture plate with 7 mL final volume of medium and the culture continued as described above.

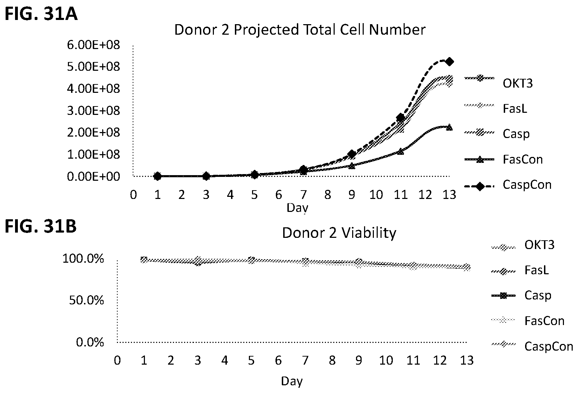

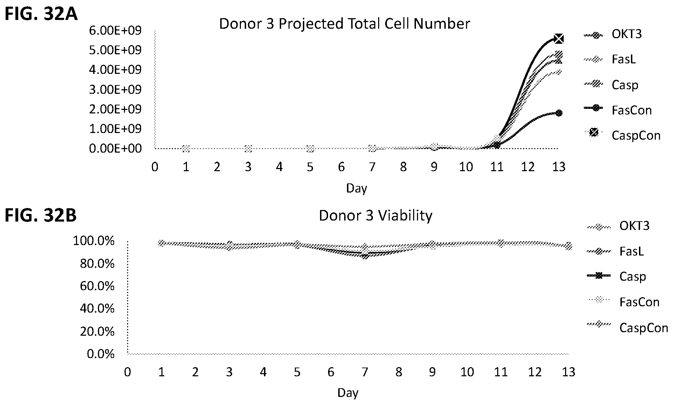

Total cell number and cell viability for each of the three donors are shown in **FIGS. 30A-30B** (donor 1), **FIGS. 31A-31B** (donor 2) and **FIGs. 32A-32B** (donor 3). Viability remained high for all of the treatment conditions throughout the culture period, however the condition with the continuous FasL blockade showed nominally lower viability than the rest. These cells also grew the slowest and their growth plateaued before any of the other conditions, across all donors. Cell cultures with the caspase inhibitor present continuously in the media showed the greatest cell growth across donors, while the control and transient treatment conditions grew similarly. Transient treatment with FasL blocking antibody NOK-1 also resulted in considerable T cell expansion.

These results show that the use of a caspase inhibitor may be useful during the expansion steps of TIL manufacturing to maximize expansion and maintain viability, particularly in high density cultures. Transient use of FasL blockade during conditions of T cell activation, co-culture, or processing in the presence of other cell types, especially tumor cells, may also be useful for blocking Fas signaling in a pro-apoptotic environment.

### B. Anti-CD3/anti-CD28 Stimulation

PBMC from two healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 1.5 x 10⁵ cells/mL (5.0 x 10⁵ cells/cm²).

Culture wells were assigned to one of two treatment groups, transient or continuous activation. For transient activation, anti-CD3/anti-CD28 paramagnetic beads (Dyanbeads^{™}) were added to the culture media at a ratio of 1 bead per cell starting on culture day 0, and then were removed during media exchange on day 2. For continuous activation, anti-CD3/anti-CD28 paramagnetic beads (Dyanbeads^{™}) were added to the culture media at a ratio of 1 bead per cell starting on culture day 0, and then added again on Day 4, and again on Day 6 at a ratio of 1 bead per cell. The anti-CD3/anti-CD28 paramagnetic beads were not removed on Day 2, 4 or 6.

For both the continuous and transient activation, culture wells were assigned to one of five treatment groups as described above for 10 conditions total per donor.

Cell counts and viability were monitored every other day. When cells reached 3 x 10⁶ cells/ml, 1.5 x 10⁶ cells were sub-cultured into a new well of a 24-well gas permeable culture plate with 7 mL final volume of medium and the culture continued as described above.

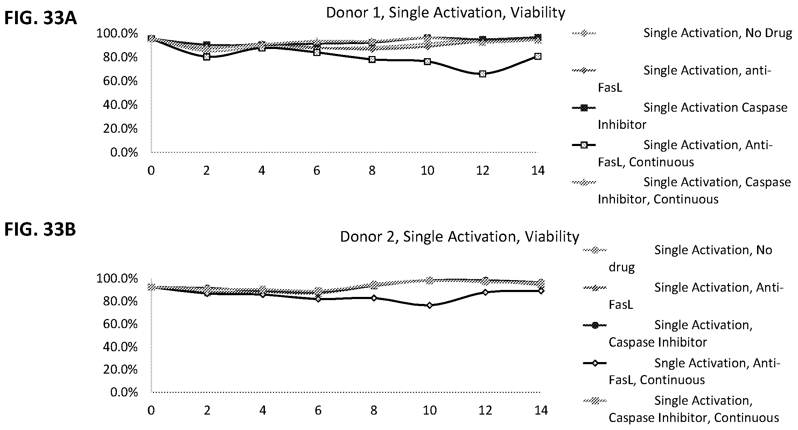

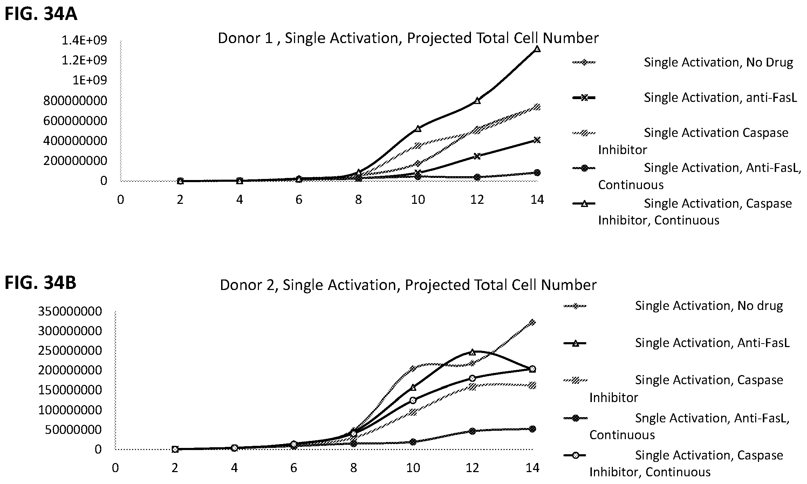

Cellular viability for the single activation with anti-CD3/anti-CD28 (transient activation) treatment groups are shown in **FIG. 33A** (donor 1) and **FIG. 33B** (donor 2), and total cell number for the same treatments are shown in **FIG. 34A** (donor 1) and **FIG. 34B** (donor 2), respectively. While inherent donor variability was observed, viability remained high for all of the treatment conditions exposed to the transient activation (single activation) stimulus. Viability remained high for all of the treatment conditions except for that of the conditions with continuous blockade of FasL, where both viability and total viable cell number declined over time (FIGS. 33A-B).

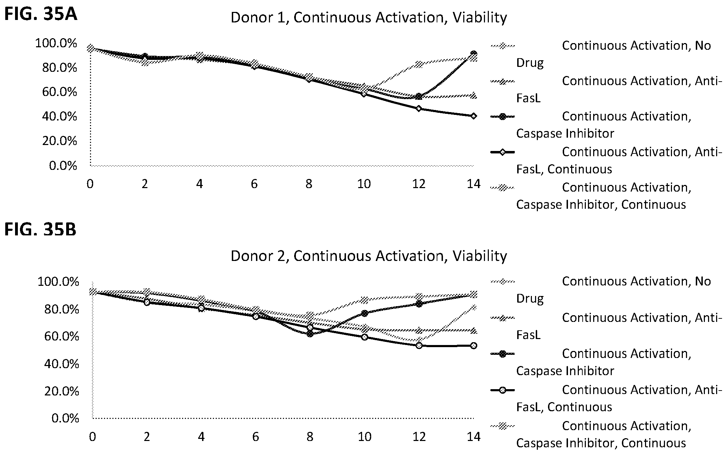

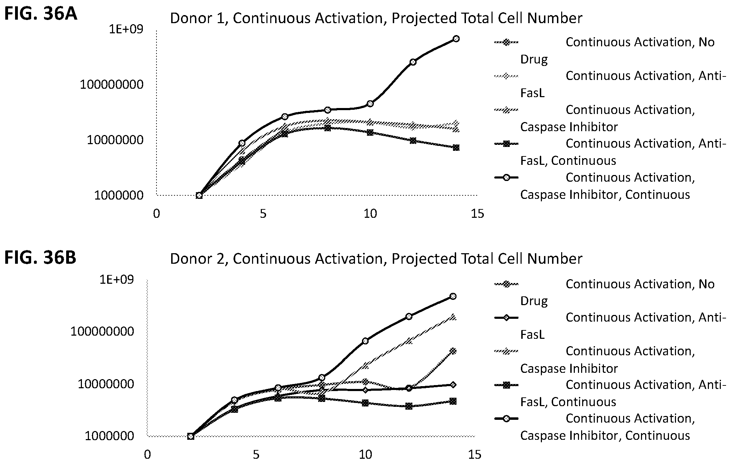

Cellular viability for the continuous activation with anti-CD3/anti-CD28 treatment groups are shown in **FIG 35A** (donor 1) and **FIG. 35B** (donor 2), and total cell number for the same treatments are shown in **FIG. 36A** (donor 1) and **FIG. 36B** (donor 2). . When exposed to a continuous activation with anti-CD3/anti-CD28, resembling the native tumor microenvironment, both total viable cell number and viability differed more between treatment conditions than conditions involving only a transient activation event. In the continuously activated populations, it appeared that the cells exposed to the caspase inhibitor, both transiently and continuously, outperformed the other conditions, with the continuous caspase inhibition outperforming the transient condition. Additionally, the cells exposed to FasL blockade showed the greatest decline in both total cell number and viability, while the cells transiently exposed to FasL blockade and no additional treatment performed similarly.

These results indicate that use of caspase inhibition during culture can improve the ability of cells to perform in environments that may be hostile to normal T cell growth, such as when cells are processed directly from the tumor which may be constitutively presenting activation signals similarly to this assay system. These results also indicate that continuous blockade of FasL signaling may be detrimental to T cell growth in conditions of both transient and continuous T cell activation, and that blockade of FasL does not affect T cell growth as strongly when it is provided transiently.

### Example 13 Assessment of Caspase Inhibition in Tumor Processing

A CRC tumor from a donor was processed as described in Example 1 using a Collagenase I/II blend (Nordmark, Collagenase NB 4G Proved Grade, part: S 1746503) to generate fragment or SCS cultures. Both tumor fragment and SCS cultures were maintained in gas permeable 24-well culture plates using OpTmizer media supplemented with 300 IU/mL recombinant IL-2, gentamicin at 10 µg/ml, Immune Cell Serum Replacement (ThermoFisher) at between 5%, and a final concentration of 2.0 mM of a L-alanyl-L-glutamine dipeptide form of glutamine (GlutaMAX Supplement; Thermofisher). Half of cultures contained media additionally supplemented with 2 µM of pan-caspase inhibitor Z-VAD-FMK which was replenished at each media exchange. Cultures were incubated at about 37° C for a minimum of 18 days with a 50% medium exchange occurring every 2-3 days following culture day 5. Cell counts were performed on day 5 and at each medium exchange using the NC-200 Automated Cell Counter (ChemoMetec).

**FIG. 37A-C** shows the fold expansion (**FIG. 37A**), total viable cells (**FIG. 37B**) and percent viability (**FIG. 37C**), of both SCS and tumor fragment derived cultures grown in the presence or absence of Z-VAD-FMK. **FIG. 37A** and **FIG. 37B** demonstrate that the outgrowth of cells was superior in the tumor fragment-derived condition containing the pan-caspase inhibitor. Additionally, as seen in **FIG. 37C****,** cell viability for this condition was also high. Cell viability was similarly high for those cells cultured as a SCS in the presence caspase inhibition, even though outgrowth of T cells was not observed for the SCS conditions. These data indicate that caspase inhibition can be a mechanism to maintain high viability and outgrowth for T cells grown from tumors.

### Example 14 Evaluation of Checkpoint Modulators and Costimulatory Agonist Antibodies on T Cell Phenotype

PBMC from two healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 5.0 x 10⁵ cells/cm². Cells were activated for 48 hours using 50 ng/ml OKT3, a human anti-CD3 monoclonal antibody, and were additionally treated with agents as described below. Following 48 hours in culture, cells were analyzed for phenotype.

Cells were divided into 6 treatment groups, as follows: Ipilimumab (anti-CTLA4), Pembrolizumab (anti-PD1), Tavolixizumab (anti-TNFRSF4), Urelumab (anti-CD137), and Varlilumab (anti-CD27), as well as a no added agent control. For all test groups, cells in each treatment group were cultured in the presence of the monoclonal antibodies at 0.5, 1, 10, or 20 µg/mL.

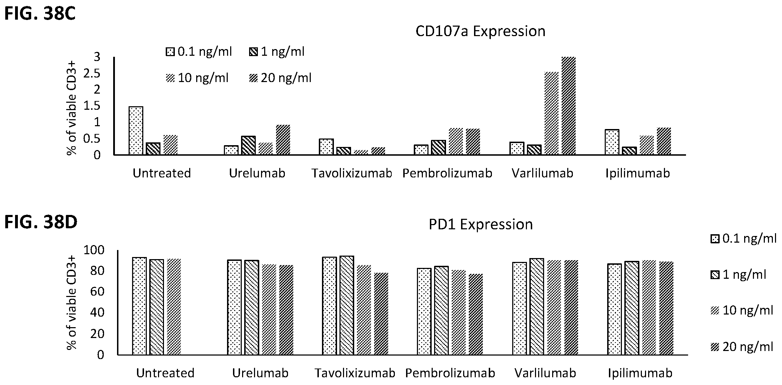

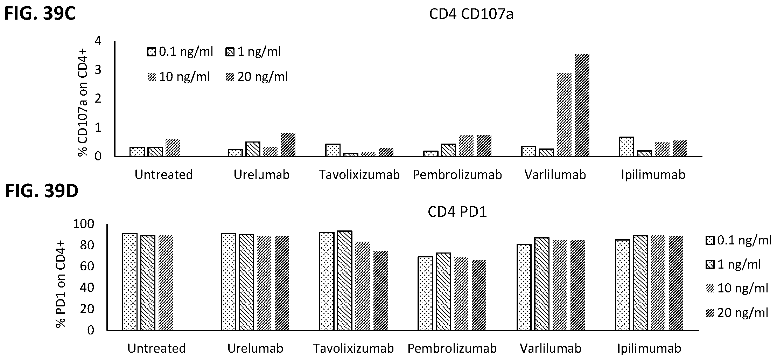

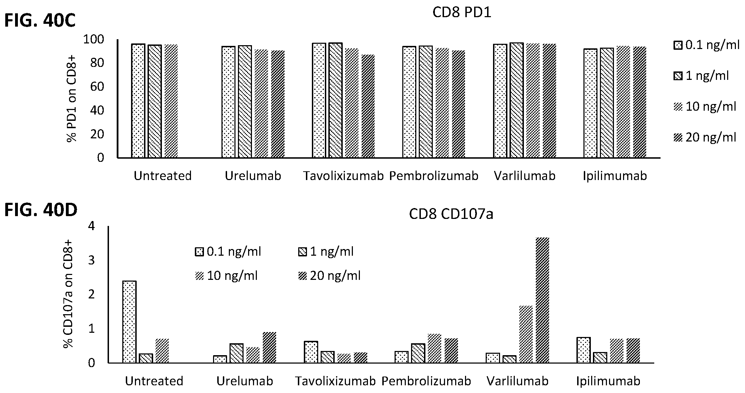

None of the tested antibodies appeared to affect the memory differentiation state of the T cells. T cell phenotype was assessed for CD4+ and CD8+ cells independently via flow cytometry for markers of activation OX40, 41BB, CD107a, and PD1. Results are shown in **FIG. 38** (CD3+), **FIG 39** (CD4+) and **FIG. 40** (CD8+). Higher concentrations of Varlilumab, an agonist anti-CD27 antibody, promoted 41BB and CD107a expression on CD3+ T cells **(****FIG. 38B** **and** **38C****),** CD4+ T cells **(****FIG. 39B** **and** **39C****)** and CD8+ T cells **(****FIG. 40B** **and** **40C****).** Urelumab, an agonist CD137 receptor antibody, promoted 41BB expression on CD4 T cells (**FIG. 39B**) and CD8+ T cells (**FIG. 40B**). Pembrolizumab, an anti-PD-1 antagonist, decreased PD1 expression on CD4 T cells (**FIG. 39D****)**

These data support that the use of monoclonal antibody modulators for use in T cell expansion as a means of moderating cellular activation status.

## Claims

1. A method for manufacturing tumor-reactive T cells, the method comprising:
(1) culturing T cells by a process that comprises:
(a) incubating a population of cells comprising T cells from a biological sample obtained from a subject that has a tumor with a first T cell stimulatory agent(s) under conditions to stimulate expansion of T cells of the population to produce a population of stimulated T cells, wherein the first T cell stimulatory agent(s) comprises the presence of one or more recombinant cytokines;
(b) co-culturing the population of stimulated T cells in the presence of antigen presenting cells (APCs) under conditions in which the APCs have been induced to present one or more peptides from a tumor-associated antigen from the subject, thereby generating a population containing T cells comprising tumor reactive T cells;
(c) enriching from the co-culture the population of tumor reactive T cells reactive to the one or more peptides, wherein said tumor-reactive T cells comprise an endogenous TCR that is reactive to a tumor-associated antigen, thereby producing a population of T cells enriched for tumor-reactive T cells; and
(d) incubating the population of T cells enriched in tumor reactive T cells with a second T cell stimulatory agent(s) under conditions to stimulate expansion of T cells in the population, wherein the second T cell stimulatory agent(s) comprises the presence of one or more recombinant cytokines; and
wherein one or more steps of the culturing is carried out in the presence of at least one T cell adjuvant that is: (i) an apoptosis inhibitor that inhibits caspase activation or activity, (ii) a costimulatory agonist which is a tumor necrosis factor receptor superfamily (TNFRSF) agonist, or (iii) a checkpoint inhibitor which inhibits the activity of an immune checkpoint selected from the group consisting of PD-1/PD-L1, CTLA-4, OX40, LAG-3, TIM-3 and B7-H3; and
(2) harvesting cells produced by the method to produce a composition of expanded T cells enriched in tumor reactive T cells.

2. The method of claim 1, wherein:
(1) the apoptosis inhibitor inhibits one or more of caspase 2, a caspase 8, a caspase 9, a caspase 10, a caspase 3, a caspase 6 or a caspase 7;
(2) the apoptosis inhibitor is selected from the group consisting of Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8), Wedelolactone, NS3694, NSCI and Z-fluoromethyl ketone Z-VAD-FMK or a flouromethyl ketone variant thereof;
(3) the apoptosis inhibitor is a pan-caspase inhibitor that inhibits activation or activity of two or more caspases;
(4) the apoptosis inhibitor is Z-VAD-FMK; and/or
(5) the apoptosis inhibitor is selected from the group consisting of Z-FA-FMK, Z-VAD(OH)-FMK, Z-DEVD-FMK, Z-VAD(OM2)-FMK, and Z-VDVAD-FMK,
optionally wherein:
(a) the apoptosis inhibitor is added at a concentration of between at about 0.5 µM and at about 50 µM, between at about 0.5 µM and at about 40 µM, between at about 0.5 µM and at about 30 µM, between at about 0.5 µM and at about 20 µM, between at about 0.5 µM and at about 10 µM, between at about 0.5 µM and at about 5 µM, between at about 0.5 µM and at about 1 µM, between at about 1 µM, at about 1 µM at about 50 µM, between at about 1 µM and at about 40 µM, between at about 1 µM and at about 30 µM, between at about 1 µM and at about 20 µM, between at about 1 µM and at about 10 µM, between at about 1 µM and at about 5 µM, each inclusive, more optionally wherein the concentration is at or about 2 µM, at or about 10 µM or at or about 25 µM; or
(b) the apoptosis inhibitor is added at a concentration of between at about 0.5 µg/mL and at or about 25 µg/mL, between at or about 0.5 µg/mL and at or about 10 µg/mL, between at or about 0.5 µg/mL and at or about 5 µg/mL, between at or about 0.5 µg/mL and at or about 1 µg/mL, between at or about 1 µg/mL and at or about 25 µg/mL, between at or about 1 µg/mL and at or about 10 µg/mL, between at or about 1 µg/mL and at or about 5 µg/mL, between at or about 5 µg/mL and at or about 25 µg/mL, between at or about 5 µg/mL and at or about 10 µg/mL, or between at or about 10 µg/mL and at or about 25 µg/mL, each inclusive.

3. The method of claim 1, wherein the costimulatory agonist is an antibody or antigen-binding fragment that specifically binds a TNFRSF member or is a fusion protein comprising an extracellular domain or binding portion thereof of a ligand of a TNFRSF member, more optionally wherein the TNFRSF member is selected from OX40, 4-1BB, GITR and CD27.

4. The method of claim 1, wherein:
(1) the immune checkpoint is selected from PD-1/PD-L1, optionally wherein the checkpoint inhibitor is an anti-PD-1 antibody or the checkpoint inhibitor is an anti-PDL1 antibody;
(2) the immune checkpoint is OX40, optionally wherein the checkpoint inhibitor is an anti-OX40L antibody; or
(3) the immune checkpoint is CTLA-4, optionally wherein the checkpoint inhibitor is an anti-CTLA-4 antibody.

5. The method of any of claims 1-4, wherein:
(1) the first T cell stimulatory agent(s) comprise a recombinant cytokine selected from the group consisting of IL-10, IL-1a, IL-5, IL-7, IL-12, IL-23p40, IL-16, IL-17A, IL-15, IL-22, IL-2, IL-4, IL-6, IL-8, IL-10, IL12p70, IL13, and IL-1b; or
(2) the second T cell stimulatory agent(s) comprise a recombinant cytokine selected from the group consisting of IL-10, IL-1a, IL-5, IL-7, IL-12, IL-23p40, IL-16, IL-17A, IL-15, IL-22, IL-2, IL-4, IL-6, IL-8, IL-10, IL12p70, IL13, and IL-1b,
optionally wherein the concentration of each of the one or more recombinant cytokine individually is 100 IU/mL to 6000 IU/mL.

6. The method of any of claims 1-5, wherein the second T cell stimulatory agent(s) further comprises an anti-CD3 antibody, optionally OKT3, optionally wherein the concentration of the anti-CD3 antibody is at or about 50 ng/mL.

7. The method of any of claims 1-6, wherein the second T cell stimulating agent comprises feeder cells that are non-dividing peripheral blood mononuclear cells (PBMC), optionally wherein the feeder cells are gamma irradiated.

8. The method of any of claims 1-7, wherein the antigen presenting cells are nucleated cells such as dendritic cells, mononuclear phagocytes, B lymphocytes, endothelial cells or thymic epithelium, optionally wherein the antigen presenting cells are dendritic cells.

9. The method of any of claims 1-8, wherein:
the one or more peptides comprises at least one neoepitope from tumor-associated antigens from the subject; and/or
the one or more peptide comprises an individual peptide or a pool of peptides.

10. The method of any of claims 1-9, wherein the co-culture ratio of antigen presenting cells to T cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1, optionally wherein the co-culture ratio of antigen presenting cells to T cells is or is about 1:1.

11. The method of any of claims 1-10, wherein the co-culturing is for 2 hours to 24 hours, optionally wherein the co-culturing is for at or about 6 hours.

12. The method of any of claims 1-11, wherein the enriching for the tumor reactive T cells comprises selection of T cells surface positive for the one or more T cell activation markers, optionally wherein the one or more T cell activation marker is selected from the group consisting of CD107, CD107a, CD39, CD103, CD137 (4-1BB), CD59, CD69, CD90, CD38, CD30, CD154, CD252, CD134, CD258, CD256, PD-1, TIM-3 and LAG-3, more optionally wherein the one or more T cell activation marker is CD134 and/or CD137.

13. The method of any of claims 1-12, wherein:
(1) one or more of the steps of the method is carried out in a closed system;
(2) the incubating with the first T cell stimulatory agent(s) is for 7 to 21 days, optionally 7 to 14 days;
(3) the incubating with the second T cell stimulatory agent(s) is for 7 to 21 days, optionally 7 to 14 days; and/or
(4) the method comprises formulating the harvested cells with a cryoprotectant.

14. The method of any of claims 1-13, wherein:
(1) the biological sample is a peripheral blood sample, a lymph node sample, or a tumor sample;
(2) the biological sample is a resected tumor and the population of cells comprising T cells are one or more tumor fragments from the resected tumor; or
(3) the biological sample is a resected tumor and the population of cells comprising T cells are processed as a single cell suspension by homogenization and/or enzymatic digestion of one or more tumor fragments from the resected tumor.

15. A composition comprising expanded T cells enriched in tumor reactive T cells for use in a method of treating a subject having a cancer, wherein the method comprises manufacturing the composition using the method of any one of claims 1-14 and administering the composition to a subject having a tumor a therapeutic dose of the composition, wherein the cells of the administered composition are autologous to the subject.

## Patentansprüche

1. Verfahren zur Herstellung von Tumor-reaktiven T-Zellen, wobei das Verfahren Folgendes umfasst:
(1) Züchten von T-Zellen durch ein Verfahren, das umfasst:
(a) Inkubieren einer Zellpopulation, die T-Zellen aus einer biologischen Probe umfasst, die von einem Subjekt erhalten wurde, das einen Tumor hat, mit einem ersten T-Zell-stimulierenden Agens bzw. ersten T-Zell-stimulierenden Agenzien unter Bedingungen, um die Expansion von T-Zellen der Population zu stimulieren, um eine Population von stimulierten T-Zellen zu erzeugen, wobei das eine oder die mehreren ersten T-Zell-stimulierenden Agenzien die Anwesenheit von einem oder mehreren rekombinanten Zytokinen umfassen;
(b) Co-Kultivierung der Population stimulierter T-Zellen in Gegenwart von Antigen-präsentierenden Zellen (APCs) unter Bedingungen, unter denen die APCs dazu veranlasst wurden, ein oder mehrere Peptide von einem Tumor-assoziierten Antigen aus dem Subjekt zu präsentieren, wodurch eine Population erzeugt wird, die T-Zellen enthält, die tumorreaktive T-Zellen umfassen;
(c) Anreichern, aus der Co-Kultur, der Population tumorreaktiver T-Zellen, die auf das eine oder die mehreren Peptide reagieren, wobei die tumorreaktiven T-Zellen einen endogenen TCR umfassen, der mit einem tumorassoziierten Antigen reaktiv ist, wodurch eine Population von T-Zellen erzeugt wird, die mit tumorreaktiven T-Zellen angereichert ist; und
(d) Inkubieren der Population von T-Zellen, die an tumorreaktiven T-Zellen angereichert sind, mit einem oder mehreren zweiten T-Zell-stimulierenden Agenzien unter Bedingungen, die die Expansion von T-Zellen in der Population stimulieren, wobei das eine oder die mehreren zweiten T-Zell-stimulierenden Agenzien die Anwesenheit von einem oder mehreren rekombinanten Zytokinen umfassen; und
wobei ein oder mehrere Schritte der Kultivierung in Gegenwart von mindestens einem T-Zell-Adjuvans durchgeführt werden, das Folgendes ist: (i) ein Apoptose-Inhibitor, der die Caspase-Aktivierung oder -Aktivität hemmt, (ii) ein kostimulatorischer Agonist, der ein Agonist der Tumornekrosefaktor-Rezeptor-Superfamilie (TNFRSF) ist, oder (iii) ein Checkpoint-Inhibitor, der die Aktivität eines Immun-Checkpoints, ausgewählt aus der Gruppe bestehend aus PD-1/PD-Ll, CTLA-4, OX40, LAG-3, TIM-3 und B7-H3, hemmt; und
(2) Ernten der durch das Verfahren hergestellten Zellen zur Herstellung einer Zusammensetzung von expandierten T-Zellen, die mit tumorreaktiven T-Zellen angereichert sind.

2. Verfahren nach Anspruch 1, wobei:
(1) der Apoptoseinhibitor eine oder mehrere von Caspase 2, einer Caspase 8, einer Caspase 9, einer Caspase 10, einer Caspase 3, einer Caspase 6 oder einer Caspase 7 inhibiert;
(2) der Apoptose-Inhibitor ausgewählt ist aus der Gruppe bestehend aus Emricasan (IDN- 6556, PF-03491390), NAIP (Neuronalapoptose-Inhibitorprotein; BIRC1), cIAP1 und cIAP2 (zellulärer Inhibitor der Apoptose, Cellular Inhibitor of Apoptosis 1 and 2; BIRC2 bzw. BIRC3), XIAP (X chromosom-bindendes IAP; BIRC4), Survivin (BIRC5), BRUCE (Apollon; BIRC6), Livin (BIRC7) und Ts-IAP (Testisspezifisches IAP; BIRC8), Wedelolacton, NS3694, NSCI und Z-Fluormethylketon Z-VAD-FMK oder eine Flouromethylketon-Variante davon;
(3) der Apoptose-Inhibitor ein Pan-Caspase-Inhibitor ist, der die Aktivierung oder Aktivität von zwei oder mehr Caspasen hemmt;
(4) der Apoptoseinhibitor Z-VAD-FMK ist; und/oder
(5) der Apoptoseinhibitor ausgewählt ist aus der Gruppe bestehend aus Z-FA-FMK, Z-VAD(OH)-FMK, Z-DEVD-FMK, Z-VAD(OM2)-FMK und Z-VDVAD-FMK,
wobei optional:
(a) der Apoptose-Inhibitor in einer Konzentration von zwischen bei etwa 0,5 µM und bei etwa 50 µM, zwischen bei etwa 0,5 µM und bei etwa 40 µM, zwischen bei etwa 0,5 µM und bei etwa 30 µM, zwischen bei etwa 0,5 µM und bei etwa 20 µM, zwischen bei etwa 0,5 µM und bei etwa 10 µM, zwischen bei etwa 0,5 µM und bei etwa 5 µM, zwischen bei etwa 0,5 µM und bei etwa 1 µM, zwischen bei etwa 1 µM, bei etwa 1 µM und bei etwa 50 µM, zwischen bei etwa 1 µM und bei etwa 40 µM, zwischen bei etwa 1 µM und bei etwa 30 µM, zwischen bei etwa 1 µM und bei etwa 20 µM, zwischen bei etwa 1 µM und bei etwa 10 µM, zwischen bei etwa 1 µM und bei etwa 5 µM, jeweils einschließlich hinzugegeben wird, wobei optionaler die Konzentration bei oder etwa 2 µM, bei oder etwa 10 µM oder bei oder etwa 25 µM liegt; oder
(b) der Apoptose-Inhibitor in einer Konzentration von zwischen bei etwa 0,5 µg/ml und bei oder etwa 25 µg/ml, zwischen bei oder etwa 0,5 µg/ml und bei oder etwa 10 µg/ml, zwischen bei oder etwa 0,5 µg/ml und bei oder etwa 5 µg/ml, zwischen bei oder etwa 0,5 µg/ml und bei oder etwa 1 µg/ml, zwischen bei oder etwa 1 µg/ml und bei oder etwa 25 µg/ml, zwischen bei oder etwa 1 µg/ml und bei oder etwa 10 µg/ml, zwischen bei oder etwa 1 µg/ml und bei oder etwa 5 µg/ml, zwischen bei oder etwa 5 µg/ml und bei oder etwa 25 µg/ml, zwischen bei oder etwa 5 µg/ml und bei oder etwa 10 µg/ml, oder zwischen bei oder etwa 10 µg/ml und bei oder etwa 25 µg/ml, jeweils einschließlich hinzugegeben wird.

3. Verfahren nach Anspruch 1, wobei der kostimulatorische Agonist ein Antikörper oder ein Antigen-bindendes Fragment ist, das spezifisch ein TNFRSF-Mitglied bindet, oder ein Fusionsprotein ist, das eine extrazelluläre Domäne oder einen bindenden Teil davon eines Liganden eines TNFRSF-Mitglieds umfasst, wobei optionaler das TNFRSF-Mitglied ausgewählt ist aus OX40, 4-IBB, GITR und CD27.

4. Verfahren nach Anspruch 1, wobei:
(1) der Immun-Checkpoint ausgewählt ist aus PD-1/PD-Ll, wobei optional der Checkpoint-Inhibitor ein Anti-PD-I-Antikörper ist oder der Checkpoint-Inhibitor ein Anti-PDLI-Antikörper ist;
(2) der Immun-Checkpoint OX40 ist, wobei optional der Checkpoint-Inhibitor ein Anti-OX40L-Antikörper ist; oder
(3) der Immun-Checkpoint CTLA-4 ist, wobei optional der Checkpoint-Inhibitor ein Anti-CTLA-4-Antikörper ist.

5. Verfahren nach einem der Ansprüche 1-4, wobei:
(1) das eine oder die mehreren ersten T-Zell-stimulierenden Mittel ein rekombinantes Zytokin umfassen, das aus der Gruppe ausgewählt ist, die aus IL-10, IL-1a, IL-5, IL-7, IL-12, IL-23p40, IL-16, IL-1 7A, IL-15, IL-22, IL-2, IL-4, IL-6, IL-8, IL-10, IL12p70, IL13 und IL-1b besteht; oder
(2) das eine oder die mehreren zweiten T-Zell-stimulierenden Mittel ein rekombinantes Zytokin umfassen, das aus der Gruppe ausgewählt ist, die aus IL-10, IL-1a, IL-5, IL-7, IL-12, IL-23p40, IL-16, IL-1 7A, IL-15, IL-22, IL-2, IL-4, IL-6, IL-8, IL-10, IL12p70, IL13 und IL-1b besteht,
wobei optional die Konzentration jedes der einen oder mehreren rekombinanten Zytokine 100 IU/ml bis 6000 IU/ml betragen kann.

6. Verfahren nach einem der Ansprüche 1-5, wobei das eine oder die mehreren zweiten T-Zell-stimulierenden Agenzien ferner einen Anti-CD3-Antikörper, optional OKT3, umfassen, wobei optional die Konzentration des Anti-CD3-Antikörpers bei oder etwa 50 ng/ml liegt.

7. Verfahren nach einem der Ansprüche 1-6, wobei das zweite T-Zell-stimulierende Mittel Feederzellen umfasst, die nicht-teilende periphere mononukleare Blutzellen (PBMC) sind, wobei optional die Feederzellen gammabestrahlt werden.

8. Verfahren nach einem der Ansprüche 1-7, wobei die Antigen-präsentierenden Zellen kernhaltige Zellen wie dendritische Zellen, mononukleäre Phagozyten, B-Lymphozyten, Endothelzellen oder Thymusepithel sind, wobei optional die Antigen-präsentierenden Zellen dendritische Zellen sind.

9. Verfahren nach einem der Ansprüche 1-8, wobei:
das eine oder die mehreren Peptide mindestens ein Neoepitop aus tumorassoziierten Antigenen des Subjekts umfassen; und/oder
das eine oder die mehreren Peptide ein einzelnes Peptid oder einen Pool von Peptiden umfasst.

10. Verfahren nach einem der Ansprüche 1-9, wobei das Co-Kultur-Verhältnis von Antigen-präsentierenden Zellen zu T-Zellen zwischen 20:1 und 1:1, zwischen 15:1 und 1:1, zwischen 10:1 und 1:1, zwischen 5:1 und 1:1, zwischen 2,5:1 und 1:1, zwischen 1:20 und 1:1, zwischen 1:15 und 1:1, zwischen 1:10 und 1:1, zwischen 1:5 und 1:1, oder zwischen 1:2,5 und 1:1 beträgt, wobei optional das Co-Kultur-Verhältnis von Antigen-präsentierenden Zellen zu T-Zellen 1:1 beträgt oder etwa beträgt.

11. Verfahren nach einem der Ansprüche 1-10, wobei die Co-Kultivierung 2 Stunden bis 24 Stunden dauert, optional wobei die Co-Kultivierung etwa 6 Stunden dauert.

12. Verfahren nach einem der Ansprüche 1-11, wobei die Anreicherung für die tumorreaktiven T-Zellen die Selektion von T-Zellen umfasst, die oberflächenpositiv für den einen oder die mehreren T-Zellaktivierungsmarker sind, wobei optional der eine oder die mehreren T-Zellaktivierungsmarker ausgewählt sind aus der Gruppe bestehend aus CD107, CD107a, CD39, CD103, CD137 (4-1BB), CD59, CD69, CD90, CD38, CD30, CD154, CD252, CD134, CD258, CD256, PD-1, TIM-3 und LAG-3, wobei optionaler der eine oder mehrere T-Zell-Aktivierungsmarker CD134 und/oder CD137 ist.

13. Verfahren nach einem der Ansprüche 1-12, wobei:
(1) einer oder mehrere der Schritte des Verfahrens in einem geschlossenen System durchgeführt werden;
(2) die Inkubation mit dem einen oder den mehreren ersten T-Zell-stimulierenden Mitteln für 7 bis 21 Tage, optional 7 bis 14 Tage, erfolgt;
(3) die Inkubation mit dem einen oder den mehreren zweiten T-Zell-stimulierenden Mitteln für 7 bis 21 Tage, optional 7 bis 14 Tage, erfolgt; und/oder
(4) das Verfahren das Formulieren der geernteten Zellen mit einem Kälteschutzmittel umfasst.

14. Verfahren nach einem der Ansprüche 1-13, wobei:
(1) die biologische Probe eine periphere Blutprobe, eine Lymphknotenprobe oder eine Tumorprobe ist;
(2) die biologische Probe ein resezierter Tumor ist und die T-Zellen umfassende Zellpopulation ein oder mehrere Tumorfragmente aus dem resezierten Tumor sind; oder
(3) die biologische Probe ein resezierter Tumor ist und die T-Zellen umfassende Zellpopulation als Einzelzellsuspension durch Homogenisierung und/oder enzymatische Verdauung eines oder mehrerer Tumorfragmente aus dem resezierten Tumor verarbeitet wird.

15. Zusammensetzung, die expandierte T-Zellen umfasst, die mit tumorreaktiven T-Zellen angereichert sind, zur Verwendung in einem Verfahren zur Behandlung eines Subjekts mit Krebs, wobei das Verfahren die Herstellung der Zusammensetzung unter Verwendung des Verfahrens nach einem der Ansprüche 1-14 und die Verabreichung einer therapeutischen Dosis der Zusammensetzung an ein Subjekt mit einem Tumor umfasst, wobei die Zellen der verabreichten Zusammensetzung autolog für das Subjekt sind.

## Revendications

1. Procédé de création de cellules T réactives à une tumeur, le procédé comprenant :
(1) la mise en culture de cellules T par un processus qui comprend :
(a) l'incubation d'une population de cellules comprenant des cellules T provenant d'un échantillon biologique obtenu auprès d'un sujet, qui a une tumeur, avec un ou des premier(s) agent(s) stimulateur(s) de cellules T, dans des conditions pour stimuler le développement de cellules T de la population pour produire une population de cellules T stimulées, dans lequel le(s) premier(s) agent(s) stimulateur(s) de cellules T comprend (comprennent) la présence d'une ou de plusieurs cytokines recombinantes ;
(b) la mise en coculture de la population de cellules T stimulées en présence de cellules présentant un antigène (« Antigen Presenting Cells », APCs), dans des conditions dans lesquelles les APCs ont été induites à présenter un ou plusieurs peptides provenant d'un antigène, associé à une tumeur, provenant du sujet, générant ainsi une population contenant des cellules T comprenant des cellules T réactives à une tumeur ;
(c) l'enrichissement, à partir de la coculture, de la population de cellules T réactives à une tumeur réactives à l'un ou aux plusieurs peptides, dans lequel lesdites cellules T réactives à une tumeur comprennent un TCR endogène qui est réactif à un antigène associé à une tumeur, produisant ainsi une population de cellules T enrichies en cellules T réactives à une tumeur ; et
(d) l'incubation de la population de cellules T enrichies en cellules T réactives à une tumeur avec un ou des second(s) agent(s) stimulateur(s) de cellules T, dans des conditions pour stimuler le développement de cellules T dans la population, dans lequel le(s) second(s) agent(s) stimulateur(s) de cellules T comprend (comprennent) la présence d'une ou de plusieurs cytokines recombinantes ; et
dans lequel une ou plusieurs étapes de la mise en culture est ou sont effectuée(s) en présence d'au moins un adjuvant de cellule T qui est : (i) un inhibiteur d'apoptose qui inhibe l'activation ou l'activité caspase, (ii) un agoniste costimulateur qui est un agoniste de superfamille de récepteur de facteur de nécrose de tumeur (« Tumor Necrosis Factor Receptor SuperFamily », TNFRSF), ou (iii) un inhibiteur de point de contrôle qui inhibe l'activité d'un point de contrôle immunitaire sélectionné parmi le groupe constitué de PD-1/PD-L1, CTLA-4, OX40, LAG-3, TIM-3 et B7-H3 ; et
(2) la récolte de cellules produites par le procédé, pour produire une composition de cellules T développées enrichies en cellules T réactives à une tumeur.

2. Procédé de la revendication 1, dans lequel :
(1) l'inhibiteur d'apoptose inhibe une ou plusieurs caspases parmi une caspase 2, une caspase 8, une caspase 9, une caspase 10, une caspase 3, une caspase 6 ou une caspase 7 ;
(2) l'inhibiteur d'apoptose est sélectionné parmi le groupe constitué de : Emricasan (IDN-6556, PF-03491390), NAIP (« Neuronal Apoptosis Inhibitory Protein » ; BIRC1), cIAP1 et cIAP2 (inhibiteur cellulaire d'apoptose 1 et 2 ; BIRC2 et BIRC3, respectivement), XIAP (« X-chromosome binding IAP » ; BIRC4), survivine (BIRC5), BRUCE (Apollon ; BIRC6), livine (BIRC7) et Ts-IAP (« Testis-specific IAP » ; BIRC8), Wédélolactone, NS3694, NSCI et Z-fluorométhyl-cétone Z-VAD-FMK ou une variante de flourométhyl-cétone de celle-ci ;
(3) l'inhibiteur d'apoptose est un inhibiteur de pan-caspase qui inhibe l'activation ou l'activité de deux ou de plus de deux caspases ;
(4) l'inhibiteur d'apoptose est Z-VAD-FMK ; et/ ou
(5) l'inhibiteur d'apoptose est sélectionné parmi le groupe constitué de : Z-FA-FMK, Z-VAD(OH)-FMK, Z-DEVD-FMK, Z-VAD(OM2)-FMK, et Z-VDVAD-FMK, facultativement dans lequel :
(a) l'inhibiteur d'apoptose est ajouté en une concentration d'entre environ à 0,5 µM et environ à 50 µM, entre environ à 0,5 M et environ à 40 µM, entre environ à 0,5 M et environ à 30 µM, entre environ à 0,5 M et environ à 20 µM, entre environ à 0,5 M et environ à 10 µM, entre environ à 0,5 M et environ à 5 µM, entre environ à 0,5 M et environ à 1 µM, entre environ à 1 µM, environ à 1 µM environ à 50 µM, entre environ à 1 µM et environ à 40 µM, entre environ à 1 µM et environ à 30 µM, entre environ à 1 µM et environ à 20 µM, entre environ à 1 µM et environ à 10 µM, entre environ à 1 µM et environ à 5 µM, chaque inclusive, plus facultativement dans lequel la concentration est à ou environ à 2 µM, à ou environ à 10 µM ou à ou environ à 25 µM ; ou
(b) l'inhibiteur d'apoptose est ajouté en une concentration d'entre environ à 0,5 µg/mL et à ou environ à 25 µg/mL, entre à ou environ à 0,5 µg/mL et à ou environ à 10 µg/mL, entre à ou environ à 0,5 µg/mL et à ou environ à 5 µg/mL, entre à ou environ à 0,5 µg/mL et à ou environ à 1 µg/mL, entre à ou environ à 1 µg/mL et à ou environ à 25 µg/mL, entre à ou environ à 1 µg/mL et à ou environ à 10 µg/mL, entre à ou environ à 1 µg/mL et à ou environ à 5 µg/mL, entre à ou environ à 5 µg/mL et à ou environ à 25 µg/mL, entre à ou environ à 5 µg/mL et à ou environ à 10 µg/mL, ou entre à ou environ à 10 µg/mL et à ou environ à 25 µg/mL, chacune incluse.

3. Procédé de la revendication 1, dans lequel l'agoniste costimulateur est un anticorps ou fragment de liaison à l'antigène qui lie spécifiquement un membre TNFRSF ou est une protéine de fusion comprenant un domaine extracellulaire ou une portion de liaison de celui-ci d'un ligand d'un membre TNFRSF, plus facultativement dans lequel le membre TNFRSF est sélectionné parmi OX40, 4-IBB, GITR et CD27.

4. Procédé de la revendication 1, dans lequel :
(1) le point de contrôle immunitaire est sélectionné parmi PD-1/PD-L1, facultativement dans lequel l'inhibiteur de point de contrôle est un anticorps anti-PD-1 ou l'inhibiteur de point de contrôle est un anticorps anti-PDL1 ;
(2) le point de contrôle immunitaire est OX40, facultativement dans lequel l'inhibiteur de point de contrôle est un anticorps anti-OX40L ; ou
(3) le point de contrôle immunitaire est CTLA-4, facultativement dans lequel l'inhibiteur de point de contrôle est un anticorps anti-CTLA-4.

5. Procédé de quelconques des revendications 1 à 4, dans lequel :
(1) le(s) premier(s) agent(s) stimulateur(s) de cellules T comprend (comprennent) une cytokine recombinante sélectionnée parmi le groupe constitué de : IL-10, IL-1a, IL-5, IL-7, IL-12, IL-23p40, IL-16, IL-17A, IL-15, IL-22, IL-2, IL-4, IL-6, IL-8, IL-10, IL12p70, IL13, et IL-1b ; ou
(2) le(s) second(s) agent(s) stimulateur(s) de cellules T comprend (comprennent) une cytokine recombinante sélectionnée parmi le groupe constitué de : IL-10, IL-1a, IL-5, IL-7, IL-12, IL-23p40, IL-16, IL-17A, IL-15, IL-22, IL-2, IL-4, IL-6, IL-8, IL-10, IL12p70, IL13, et IL-1b,
facultativement dans lequel la concentration de chacun de l'un ou des plusieurs recombinant cytokine est individuellement 100 IU/mL à 6 000 IU/mL.

6. Procédé de quelconques des revendications 1 à 5, dans lequel le(s) second(s) agent(s) stimulateur(s) de cellules T comprend (comprennent) en outre un anticorps anti-CD-3, facultativement OKT3, facultativement dans lequel la concentration de l'anticorps anti-CD-3 est à ou environ à 50 ng/mL.

7. Procédé de quelconques des revendications 1 à 6, dans lequel le second agent stimulateur de cellules T comprend des cellules nourricières qui sont des cellules mononucléaires de sang périphérique (« Peripheral Blood Mononuclear Cells », PBMC) ne se divisant pas, facultativement dans lequel les cellules nourricières sont gamma-irradiées.

8. Procédé de quelconques des revendications 1 à 7, dans lequel les cellules présentant un antigène sont des cellules nuclées telles que des cellules dendritiques, des phagocytes mononucléaires, des lymphocytes B, des cellules endothéliales ou épithéliales thymiques, facultativement dans lequel les cellules présentant un antigène sont des cellules dendritiques.

9. Procédé de quelconques des revendications 1 à 8, dans lequel :
l'un ou les plusieurs peptides comprend (comprennent) au moins un néoépitope provenant d'antigènes associés à une tumeur provenant du sujet ; et/ou
l'un ou les plusieurs peptides comprend (comprennent) un peptide individuel ou une réserve de peptides.

10. Procédé de quelconques des revendications 1 à 9, dans lequel le rapport de coculture de cellules présentant un antigène par rapport à des cellules T est entre 20 : 1 et 1 : 1, entre 15 : 1 et 1 : 1, entre 10 : 1 et 1 : 1, entre 5 : 1 et 1 : 1, entre 2,5 : 1 et 1 : 1, entre 1 : 20 et 1 : 1, entre 1 : 15 et 1 : 1, entre 1 : 10 et 1 : 1, entre 1 : 5 et 1 : 1, ou entre 1 : 2,5 et 1 : 1, facultativement dans lequel le rapport de coculture de cellules présentant un antigène par rapport à des cellules T est ou est environ 1 : 1.

11. Procédé de quelconques des revendications 1 à 10, dans lequel la mise en coculture est pendant 2 heures à 24 heures, facultativement dans lequel la mise en coculture est pendant ou environ 6 heures.

12. Procédé de quelconques des revendications 1 à 11, dans lequel l'enrichissement en cellules T réactives à une tumeur comprend la sélection de cellules T positives en surface pour l'un ou les plusieurs marqueurs d'activation de cellule T, facultativement dans lequel l'un ou les plusieurs marqueurs d'activation de cellule T est ou sont sélectionné(s) parmi le groupe constitué de : CD107, CD107a, CD39, CD103, CD137 (4-1BB), CD59, CD69, CD90, CD38, CD30, CD154, CD252, CD134, CD258, CD256, PD-1, TIM-3 et LAG-3, plus facultativement dans lequel l'un ou les plusieurs marqueur d'activation de cellule T est ou sont CD134 et/ou CD137.

13. Procédé de quelconques des revendications 1 à 12, dans lequel :
(1) une ou plusieurs des étapes du procédé est ou sont effectuée(s) dans un système fermé ;
(2) l'incubation avec le(s) premier(s) agent(s) stimulateur(s) de cellules T se fait pendant 7 à 21 jours, facultativement 7 à 14 jours ;
(3) l'incubation avec le(s) second(s) agent(s) stimulateur(s) de cellules T se fait pendant 7 à 21 jours, facultativement 7 à 14 jours ; et/ou
(4) le procédé comprend la préparation des cellules récoltées avec un cryoprotecteur.

14. Procédé de quelconques des revendications 1 à 13, dans lequel :
(1) l'échantillon biologique est un échantillon de sang périphérique, un échantillon de nœud lymphatique, ou un échantillon de tumeur ;
(2) l'échantillon biologique est une tumeur réséquée et la population de cellules comprenant des cellules T est composée d'un ou de plusieurs fragments de tumeur provenant de la tumeur réséquée ; ou
(3) l'échantillon biologique est une tumeur réséquée et la population de cellules comprenant des cellules T est traitée sous forme de suspension monocellulaire par homogénéisation et/ou digestion enzymatique d'un ou de plusieurs fragments de tumeur provenant de la tumeur réséquée.

15. Composition, comprenant des cellules T développées enrichies en cellules T réactives à une tumeur pour utilisation dans un procédé de traitement d'un sujet atteint d'un cancer, dans lequel le procédé comprend la création de la composition en utilisant le procédé de l'une quelconque des revendications 1 à 14 et l'administration de la composition à un sujet, ayant une tumeur, en une dose thérapeutique de la composition, dans lequel les cellules de la composition administrée sont autologues au sujet.
